# EUROPEAN PATENT APPLICATION

(11) **EP 4 524 128 A1**
(43) Date of publication of application: **19.03.2025**
(21) Application number: 23197821.4
(22) Date of filing: 17.09.2023
(51) Int. Cl.: C07C 235/84, A61L 27/00, C08J 3/24

(54) **BENZOPHENONE CROSSLINKERS**

(71) Applicant: Hemoteq AG, 52146 Würselen (DE)
(72) Inventor: HOFFMANN, Erika, 52159 Roetgen (DE); HOFFMANN, Michael, 52159 Roetgen (DE); WIEMER, Katharina, 52223 Stolberg (DE); HORBACH, Helmut, 52066 Aachen (DE); CASARETTO, Monika, 41069 Mönchengladbach (DE)
(74) Representative: Arth, Hans-Lothar

(57) **Abstract**

The present invention relates to crosslinkers having at least two benzophenone substituents that are covalently coupled to a benzophenone linker. The present invention further relates to compositions comprising at least one polymer and at least one crosslinker according to the present invention. The present invention also relates to medical products and medical supplies having a coating of crosslinked polymers and at least one crosslinker of the present invention, but also relates to cosmetical and hygienical products including at least one crosslinker according to the present invention.

## Description

The present invention relates to crosslinkers having at least two benzophenone substituents covalently coupled to a benzophenone linker. The present invention further relates to compositions comprising at least one polymer and at least one crosslinker according to the present invention. The present invention also relates to medical products and medical aids having a coating of crosslinked polymers and at least one crosslinker of the present invention, but also to cosmetic and hygienic products including at least one crosslinker according to the present invention.

### Background of the invention

In polymer chemistry, the term "crosslinking" generally refers to the process of forming covalent bonds to connect two or more polymer chains together. By crosslinking of the polymer chains, specific changes in the mechanical properties of polymers can be achieved. Crosslinking of polymer chains can be achieved by chemical reactions initiated by heat, pressure, pH changes or irradiation. Chemical covalent crosslinking results in improved mechanical properties, but also stabilizing properties.

Crosslinkers are generally characterized by at least two reactive groups. In the prior art, bifunctional photo- and/or thermos-reactive low-molecular compounds are known which, after activation, can form covalent bonds to other compounds.

The use of benzophenone as a photoinitiator for UV curing is known in the art. As an ingredient in cosmetics, benzophenone protects fragrances and colors in products such as creams, perfumes, soaps or even plastic packaging from destruction by UV radiation. Oxybenzone, a derivative of benzophenone, is approved under the German Cosmetics Regulation as UV protectant in sun creams. Crosslinked polymers or materials can be produced by adding benzophenone or derivatives thereof to a polymer composition, wherein the coating or material is subsequently irradiated with UV light to initiate crosslinking.

The object of the present invention is to provide improved crosslinkers which can be used to form particularly stable yet flexible and deformable, highly hydrophilic and very low friction crosslinked polymers, and medical products and medical supplies having a coating of such crosslinked polymers. A further objective is to provide cosmetic and hygiene products comprising such crosslinkers.

The objective of the present invention is solved by the teaching of the independent claims. Further advantageous features, aspects and details of the invention are evident from the dependent claims, the description, the figures, and the examples of the present application.

### Brief description of the invention

The present invention relates to crosslinkers having at least two benzophenone substituents **BP¹** and **BP²,** each covalently coupled to a benzophenone linker **Z** via at least an amide group, an ester group, a carbamate group, a urea group, or combination thereof. In particular, the at least two benzophenone substituents **BP¹** and **BP²** are both covalently coupled to the benzophenone linker **Z** via a linker **L¹** and **L²** including or consisting of a functional group selected from -OCO-, -NHCO-, -COO-, -CONH-, -NHCOO-, -OCONH-, -OCOO-, and -NHCONH-.

Surprisingly, it has been shown that crosslinkers according to the present invention can be reacted with at least one molecular colloid (e.g. a polymer) after activation via irradiation, wherein a molecule colloid network is formed. The crosslinkers of the present invention are therefore particularly advantageous for the production of molecule colloid networks of at least one polymer or polymerizate (i.e. molecular colloids) and an crosslinker as described herein.

In general, crosslinking reactions involve linking one or more individual macromolecules to form a three-dimensional network. Macromolecules can be long-chain high-molecular-weight molecules, but also branched high-molecular-weight molecules. The macromolecules used to produce the molecular colloid networks are polymers, preferably hydrophilic polymers. The polymers suitable for the preparation of the molecular colloid networks include both homopolymers, mixed polymers or copolymers. The crosslinkers according to the invention and the at least one polymer are preferably crosslinked and, after crosslinking has taken place, together form the molecular colloid network.

In general, the properties of a polymer are changed by the process of crosslinking. An increase in hardness, toughness, melting point and a decrease in solubility are usually observed. The change in properties increases with the degree of crosslinking, that is, with the proportion of crosslinked sites relative to the total amount of polymer.

Surprisingly, it has been shown that the crosslinkers according to the invention advantageously influence the properties of the resulting molecular colloid through the process of crosslinking and that the properties of the molecular colloid can be specifically controlled with these crosslinkers.

The molecular colloids and molecular colloid networks of the present invention exhibit excellent hydrophilicity, stability, biocompatibility and hemocompatibility, and are particularly suitable for applications related to medical devices and medical aids, but are not limited to the aforementioned devices and aids. The molecular colloids of the present invention can be used to provide low-friction, inflammation-free medical devices and medical aids that can be used over the long term.

The molecular colloid network formed by at least one polymer and a crosslinker according to the present invention after activation via irradiation or heat is suitable for use as or in any medical device or medical aid that can be used in or on the body on a short-term or long-term basis. Consequently the molecular colloids of the present invention can be used, among other things, as coatings for tubes, catheters, or other materials that require insertion into the body. Examples of these include, but are not limited to, balloon catheters, bladder catheters, stomach tubes, endoscopes, colonoscopes, needles, cannulas, endotracheal tubes, tracheal cannulas, implants such as stents, ocular lenses, prosthetic joints, screws, fixations, artificial exits, nasal implants, vocal cords, larynx, artificial and native valves such as cardiac or venous valves, esophageal sphincter, sphincter ani, bone graft substitutes, and ventilator tubes. However, the molecule colloids of the present invention can also be used as cartilage substitutes, nerve sheaths, occlusion of venous valves, seals ex corpora and in corpora, subcutaneous or intramuscular implants, as moisturizing and/or active ingredient gels/ointments, micro- and macrocapsules, wound protectors, breast implants, generally as filling materials, plasters, pads, etc., or as components of the products mentioned herein. Further applications of the molecular colloids of the present invention include, but are not limited to cosmetics, personal care, hair and skin care, color cosmetics, pharmaceuticals, biomaterials for delivery systems, excipients and coatings, and industrial applications, paper coatings, textile coatings, ink additive, suspending aid and adhesives. Additional applications include, but are not limited to membrane applications, lubricious coatings, compatibilizers, adhesives, polymeric surfactants and antimicrobial and antifouling coatings. Further examples include skin lotions, moisturizers, eye creams, soaps, syndets, shower and bath preparations, sunscreen preparations, insect repellents, acne preparations, shaving products, etc. Thus, an enormously diverse product range of medical devices, medical aids and cosmetical and hygienical products, e.g. personal care composition or cosmetical compositions and applications thereof is made possible.

### Description of the invention

Surprisingly it has been found that crosslinkers having at least two benzophenone substituents **BP¹** and **BP²,** each covalently coupled to a benzophenone linker Z via at least an amide group, an ester group, a carbamate group, a urea group, or combination thereof solve the above objective. In particular, the at least two benzophenone substituents **BP¹** and **BP²** are both covalently coupled to the benzophenone linker **Z** via linkers **L¹** and **L²** including or consisting of a functional group selected from -OCO-, -NHCO-, -COO-, -CONH-, -NHCOO-, -OCONH-, -OCOO-, and -NHCONH-.

### Definitions

### Linker

The term "**linker**", as used herein, refers to an organic molecule of low molecular weight that covalently links two or more organic compounds with each other. A linker generally represents a bifunctional chemical entity that binds a first compound to a second compound. A linker serves as a spacer for two or more organic compounds and can therefore advantageously increase flexibility. Linkers can have different lengths and polarities. Examples of linkers include, but are not limited to alkyl linkers, aryl linkers, aryl alkyl linkers, alkoxy linkers, aryloxy linkers, alkylhydroxy linkers, arylhydroxy linkers, alkylaryloxy linkers, alkylamino linkers, arylamino linkers, alkylarylamino linkers, each of which may be optionally substituted or unsubstituted. Polymers still having functional groups in the polymer chain can be functionalized with various organic compounds. These organic compounds can also be connected to the polymer chain via a linker, so that these organic compounds have a distance to the main chain of the polymer toincrease the flexibility of these organic compounds.

### Crosslinking

The term "**crosslinking**", as used herein, refers to reactions in which one or more individual macromolecules are linked to form a three-dimensional network. The linkage can be achieved either directly during the assembly of the macromolecules or through reactions on preexisting polymers. The process of crosslinking changes the properties of polymers. In general, an increase in hardness, toughness, melting point and a decrease in solubility are observed. The changes increase with the degree of crosslinking, the proportion of linked sites relative to the total amount of polymer. Crosslinking of already existing polymers can be accomplished either by functionalities already present in the polymer through clever choice of reaction conditions (self-crosslinking) or by the addition of multifunctional, low-molecular-weight substances. Crosslinking or UV curing are well known in the field of polymer chemistry. Examples of photo-reactive functional groups that can be used for crosslinking include, but are not limited to arylazides, azido-methyl coumarins, benzophenones, anthraquinones, certain diazo compounds, diazirines, and psoralen derivatives. Examples of direct crosslinking reactions include free radical polymerizations of monomers with two vinyl groups or polycondensation or polyaddition using monomers with two or more functional groups.

### Crosslinker

A "**crosslinker**", as used herein, refers to a bi- or multifunctional, low molecular weight compound characterized by at least two reactive groups and being capable of crosslinking individual macromolecules or polymers to form a three-dimensional network. The reactive groups of the crosslinkers according to the invention are benzophenones each of which may be optionally substituted or unsubstituted.

### Monomer

The term "**monomer**", as used herein, refers to an organic compound, generally an organic compound of low molecular weight, herein particularly an unsaturated (low molecular weight) organic compound, which has at least one chemically reactive group such as a double bond or an epoxide group, or has at least two chemically reactive groups such as two carboxy groups, two hydroxy groups or two amino groups, which can be reacted in a chemical reaction to form a long-chain unbranched or branched macromolecule consisting of monomers covalently coupled to each other.

Examples of hydrophilic monomers include, but are not limited to, hydroxyl-substituted lower alkyl acrylates and hydroxyl-substituted lower alkyl methacrylates such as hydroxyethyl acrylate, hydroxyethyl methacrylate, hydroxypropyl acrylate and hydroxypropyl methacrylate, acrylamide, methacrylamide, (lower alkyl) acrylamides and (lower alkyl) methacrylamides, N,N-dialkylacrylamides, ethoxylated acrylates, ethoxylated methacrylates such as polyethylene glycol monoacrylates, polyethylene glycol monomethacrylates, polyethylene glycol monomethyl ether acrylates, polyethylene glycol monomethyl ether acrylates, hydroxyl substituted (lower alkyl) acrylamides, hydroxyl substituted (lower alkyl) methacrylamides, hydroxyl substituted lower alkyl vinyl ethers, sodium vinyl sulfonate, sodium styrene sulfonate, 2-acrylamido-2-methyl-propanesulfonic acid, N-vinylpyrrole, N-vinyl-2-pyrrolidone, 2-vinyloxazoline, 2-vinyl-4,4'-dialkyloxazolin-5-one, 2-vinylpyridine, 4-vinylpyridine, allyl alcohol, 3-trimethylammonium-2-hydroxypropylmethacrylate chloride, vinyl alcohol, acrylonitrile, acryloyl chloride, ethylene glycol acrylate, methylolacrylamide, diacetone acrylamide, styrenesulfonic acid salt, dimethylaminoethyl methacrylate, dimethylaminoethyl methaarylamide,N-(1,1-dimethyl-3-oxobutyl)acrylamide, ethylene glycol vinyl ether, di(ethylene-glycol)vinyl ether, N-vinylpyrrolidone, 1-methyl-3-methylene-2-pyrrolidone, 1-methyl-5-methylene-2-pyrrolidone, 5-methyl-3-methylene-2-pyrrolidone, 1-ethyl-5-methylene-2-pyrrolidone, N-methyl-3-methylene-2-pyrrolidone, 5-ethyl-3-methylene-2-pyrrolidone, 1-n-propyl-3-methylene-2-pyrrolidone, 1-n-propyl-5-methylene-2-pyrrolidon, 1-isopropyl-3-methylene-2-pyrrolidone, 1-isopropyl-5-methylene-2-pyrrolidone, N-vinyl-N-methylacetamide, N-vinyl-N-ethylacetamide, N-vinyl-N-ethylformamide, N-vinylformamide, N-vinylacetamide, N-vinylisopropylamide, N-vinylcaprolactam, N-2-hydroxyethylvinylcarbamate, N-carboxy-β-alanine-N-vinyl ester, N-carboxyvinyl-β-alanine and N-carboxyvinyl-α-alanine.

Herein preferred monomers and/or oligomers are monomers as well as oligomers of known hydrophilic polymers. For example, preferred monomers are organic compounds that are at least monoolefinic unsaturated and can be polymerized. Examples of monoolefinic unsaturated monomers include, but are not limited to, acrylic acids, methacrylic acids, N-vinyl carbazoles, acrylic acid esters, acrylic acid amides, vinyls, vinylamines, vinylamides, N-vinylpyrrolidones, vinyl ethers, vinyl acetal, etc..

Examples of hydrophobic monomers include, but are not limited to, C₁-C₁₈ alkyl acrylates or C₁-C₁₈ methacrylates such as ethyl acrylate, butyl acrylate, isobutyl acrylate, hexyl acrylate, heptyl acrylate, methyl methacrylate, ethyl methacrylate, butyl methacrylate, isobutyl methacrylate, hexyl methacrylate, heptyl methacrylate, 1,3 butadiene, C₃-C₁₈ cycloalkyl acrylates or C₃-C₁₈ methacrylates such as. Cycloalkyl acrylate, isobornyl acrylate, isobornyl methacrylate, cycloalkyl methacrylate, C₃-C₁₈ alkyl acrylamides, C₃-C₁₈ methacrylamides, acrylonitrile, methacrylonitrile, vinyl C₁-C₁₈ alkanoates such as vinyl acetate, vinyl propionate, vinyl butyrate, vinyl valerate, C₂-C₁₈ alkenes, C₂-C₁₈ haloalkenes, styrene, (lower alkyl)styrene, d-methylstyrene, C₂-C₁₂ alkyl vinyl ethers, vinyl ethyl ethers, C₂-C₁₀ perfluoroalkyl acrylates, C₂-C₁₀ perfluoroalkyl methacrylates, partially fluorinated acrylates or partially fluorinated methacrylates, such as trifluoroethyl methacrylate, hexafluoroisopropyl methacrylate, hexafluorobutyl methacrylate, C₂-C₁₂ perfluoroalkylethylthiocarbonylaminoethyl acrylates and methacrylates, perfluorohexylethylthiocarbonylaminoethyl methacrylate, acryloxy and methacryloxyalkyl siloxanes, such as tristrimethylsilyloxysilylpropyl methacrylate (TRIS), 3-methacryloxy-propylpentamethyldisiloxane, N-vinylcarbazole, bis C₁-C₁₂ alkyl esters of maleic acid, fumaric acid, itaconic acid, mesaconic acid, such as e.g. dimethyl fumarate, dimethyl maleate, dibutyl maleate and dibutyl fumarate, chloroprene, vinyl chloride and vinylidene chloride.

### Polymer

The term "**polymer**", as used herein, refers to a macromolecule composed of long unbranched or branched chains of many covalently linked monomers. As used herein, macromolecules refer to molecules having an average molecular mass of at least 1,000 g/mol, preferably at least 5,000 g/mol, more preferably at least 10,000 g/mol.

Examples of polymers include, but are not limited to, polyacids, polyalcohols, polyethers, polyesters, polyesteramides, polyetherimines, polyetheramines, polyvinylcarbazoles, polyurethanes, polyacidesters, polyacidamines, polyacidamides, polyvinyls, polyvinylides, polyvinylalcohols, polyvinylamines, polyvinylamides, polyvinylpyrrolidones, polyvinylethers, polyhydroxycarboxylic acids, polylactams, polyethylene glycols, polyethylene glycol ethers, polyethylene oxides, polyoxazolines, polyacetals and polysiloxanes. Examples of known synthetic organic polymers include, but are not limited to, polyacrylamide, polyacrylic acid, polybutadiene, polymethacrylic acid, polyethyleneimine, polystyrene, polysulfonic acid, polytetrafluoroethylene, polyvinyl alcohol, polyvinyl chloride, polyethylene glycol, and polyvinyl pyrrolidone.

Particular examples of polymers include, but are not limited to, polylactonic acids, polyvalerolactones, poly-ε-decalactones, polyglycolic acids, polylactides, polyglycolides, copolymers of polylactides and polyglycolides, poly-ε-caprolactone, polypivotolactones, polycaprolactone glycolides, polygluconates, polylactic acid-polyethylene oxide copolymers, poly(L-lactide) (PLLA), poly(D,L-lactide) (PLA), polyglycolide (PGA), poly(L-lactide-co-D,L-lactide) (PLLA/PLA), poly(L-lactide-co-glycolide) (PLLA/PGA), poly(D,L-lactide-co-glycolide) (PLA/PGA), poly(glycolide-co-trimethylene carbonate) (PGA/PTMC), polycaprolactone (PCL), polycaprolactone-co-butyl acrylate, poly(phosphazene), poly(D,L-lactide-co-caprolactone) (PLA/PCL), Poly(glycolide-co-caprolactone) (PGA/PCL), polymaleic anhydrides, maleic anhydride copolymers, polymaleic acids, maleic acid copolymers, polylactams, poly(N-vinyl)-pyrrolidone (PVP), poly(N-vinyl)-pyrrolidone copolymers, N-dodecyl-pyrrolidone, N-decyl-pyrrolidone, N-octyl-pyrrolidone, N-alkyllactams, N-dodecylcaprolactam, N-decyl-caprolactam, N-octyl-caprolactam, N-dodecyl-valerolactam, N-decyl-valerolactam, N-octyl-valerolactam, polyvinyl alcohols, derivatives of polyvinyl alcohols, polyethers, polyethylene glycol (PEG), polyethylene oxide (PEO), polyethylene oxide propylene oxide, copolymers with polyethylene glycol and/or polypropylene glycol, polyoxyethylene sorbitan monolaurate, polyoxyethylene sorbitan monopalmitate, polyoxyethylene sorbitan monostearate, sorbitan monolaurate, sorbitan monopalmitate, sorbitan monostearate, polyoxyethylene oleyl ether, polyoxyethylene oleyl ether, bis-[alpha-methyl-(4-methylbenzyl)]-phenyl polyglycol ether, bis-[alpha-methyl-(4-n-dodecyl)]-phenyl-polyglycol ether, bis-(4-methyl-benzyl)-phenyl-polyglycol ether, Bis-(4-n-dodecyl-benzyl) phenyl-polyglycol ether, tris-[alpha-methyl-(4-methyl-benzyl)]-phenyl-polyglycol ether, Nonylphenol-polyglycol ether, nonylphenol diglycol ether, polyether ester multiblock polymers such as polyethylene glycol (PEG) and polybutylene terephthalate, polyhydroxybutyric acid, polyhydroxybutyrate, polyhydroxybutyrate-co-valerate, polydioxanone (PDS), poly(1,4-dioxane-2,3-dione), poly(1,3-dioxane-2-one), polyhydroxymethacrylates, polycyanoacrylates, poly-caprolactone dimethylacrylates, polycaprolactone butylacrylates, poly-g-ethylglutamate, multiblock polymers e. g. oligocaprolactone diol and oligodioxanone diol, polyglycolic acid trimethyl carbonates, poly(DTH-iminocarbonate), poly(DTE-co-DT-carbonate), poly(bisphenol A-iminocarbonate), polyesters, glycolated polyesters, polyesteramides, polyorthoesters, polytrimethyl carbonates, polyetherimines, poly[p-carboxyphenoxy)propane], polyether esters, polyalkenoxalates, copolymers of polyorthoesters, polytetrafluoroethylene (PTFE), polyvinyls, polyvinylides, polyvinylamines, polyvinylamides, polyvinyl ethers, polyvinyl chloride (PVC), polydimethylsiloxanes (PDMS), nylons, polyphosphoesters, polyhydroxypentanoic acid, polyacids, polyhydroxycarboxylic acids, polyacid esters, polyacid amines, polyacid amides, polyacrylic acids, polyacrylates, polymethyl methacrylates (PMMA), polybutyl methacrylates, polyacrylamides, polyacrylonitriles, polyamides, polyether amides, polyethylene amines, polyimides, polycarbonates, polycarbourethanes, polyvinyl ketones, polyvinyl halides, polyvinylidene halides, polyisobutylenes, polyvinylaromatics, polyvinyl esters, polyoxymethylene, polytetramethylene oxide, polyethylene, polypropylene, polyurethanes, soft polyurethanes, polyurethanes with amino acid residues in the backbone, polyetherurethanes, silicone polyetherurethanes, silicone polyurethanes, silicone polycarbonaturethanes, polyolefin elastomers, polyisobutylenes, polyvalerates, polyethylene terephthalates, polyetheretherketones, polyaryletheretherketones, lipids, fibrin, carrageenans, fibrinogen, starch, derivatives of starch, modified starch, collagen, protein-based polymers, polyamino acids, synthetic polyamino acids, zein, modified zein, polyhydroxyalkanoates, pectinic acid, actinic acid, hyaluronic acid, modified and unmodified fibrin and casein, carboxymethyl sulfates, albumin, heparan sulfate, heparin, chondroitin sulfate, dextrans, dextrins, α-cyclodextrin, β-cyclodextrin, γ-cyclodextrin, dimethyl-β-cyclodextrin, gum arabic, guar, collagen, collagen-N-hydroxysuccinimide, phospholipids, carboxymethyl chitosans, lanolin, gelatin, derivatives of gelatin, cellulose, derivatives of cellulose, modified cellulose, hydroxypropyl cellulose, ethyl cellulose, carboxymethyl cellulose, rayon, rayon triacetates, cellulose nitrates, cellulose acetates, cellulose butyrates, cellulose acetate butyrates, cellulose ethers, cellulose triacetates, ethyl vinyl acetate copolymers, polysulfones, epoxy resins, ABS resins, EPDM rubbers, agar, alginic acid, alginate, chicle, dammar, marshmallow extract, gellan (E 418), guar gum (E 412), gum arabic (E 414), gum from plantain seed husks, gum from spruce sap, locust bean gum (E 410), karaya (E 416), konjac flour (E 425), mastic, pectin, tara gum (E 417), tragacanth (E 413), xanthan gum (E 415), sago, shellac, silicones such as polysiloxanes, polydimethylsiloxanes, fluorosilicones, polyvinyl halogens, poly-para-xylylene (Parylene) such as Parylene N, Parylene C and/or Parylene D, as well as co-, block- or segment polymers and/or mixtures of the aforementioned polymers and derivatives thereof.

Examples of biocompatible polymers include, but are not limited to poly(esteramides), polyhydroxyalkanoates (PHA), poly(3-hydroxypropanoate), poly(3-hydroxybutyrate), poly(3-hydroxyvalerate), poly(3-hydroxyhexanoate), poly(3-hydroxyheptanoate), poly(3-hydroxyoctanoate), poly(4-hydroxybutyrate), poly(4-hydroxyvalerate), Poly(4-hydroxyhexanote), poly(4-hydroxyheptanoate), poly(4-hydroxyoctanoate), poly(dioxanone), poly(orthoester), poly(anhydride), poly(tyrosine carbonate), poly(tyrosine ester), poly(iminocarbonate), poly(glycolic acid-co-trimethylene carbonate), polyphosphoesters, polyphosphoesterurethanes, poly(amino acids), polycyanoacrylates, polyepoxyacrylates, polyurethane acrylates, Polyester acrylates, polyether acrylates, amine-modified polyether acrylates, polyacrylate acrylates, polycarbonate acrylates, polyepoxymethacrylates, polyurethane methacrylates, polyester methacrylates, polyether methacrylates, amine-modified polyether methacrylates, polyacrylate methacrylates, polycarbonate methacrylates, polyepoxyacrylamides, polyurethane acrylamides, polyester acrylamides, polyether acrylamides, amine-modified polyether acrylamides, polyacrylate acrylamides, polycarbonate acrylamides, polyepoxy methacrylamides, polyurethane methacrylamides, polyester methacrylamides, polyether methacrylamides, amine-modified polyether methacrylamides, polyacryl methacrylamides, polycarbonate methacrylamides, poly(trimethylene carbonate), poly(imino carbonate), polyurethanes, polyphosphazenes, silicones, polyesters, polyolefins, polyisobutylene, ethylene-alpha-olefin copolymers, acrylic polymers, acrylic copolymers, vinyl halide polymers, vinyl halide copolymers, polyvinyl chloride, polyvinyl ether, polyvinyl methyl ether, polyvinylidene halides, polyvinylidene chloride, polyacrylonitrile, polyvinyl ketones, polyvinyl aromatics, polystyrene, polyvinyl esters, polyvinyl acetate, ethylene-methyl methacrylate copolymers, acrylonitrile-styrene copolymers, ethylene-vinyl acetate copolymers, polyamides, polycaprolactam, polycarbonates, polyoxymethylenes, polyimides, polyethers, polypropylene fumarate), poly(n-butyl methacrylate), poly(sec-butyl methacrylate), poly(isobutyl methacrylate), poly(tert-butyl methacrylate), poly(n-propyl methacrylate), poly(isopropyl methacrylate), poly(ethyl methacrylate), poly(methyl methacrylate), polyurethanes, cellulose acetate, cellulose butyrate, cellulose acetate butyrate, cellophane, cellulose nitrate, cellulose propionate, cellulose ether, carboxymethyl cellulose, polyether, polyethylene glycol) (PEG), copoly(ether ester), polyalkylene oxides, poly(ethylene oxide), poloxamers, poloxamines, polypropylene oxide), poly(ether ester), polyalkylene oxalates, polyphosphazenes, silane-modified polymers poly(aspirin), poly(styrene-isoprene-styrene)-PEG (SIS-PEG), polystyrene-PEG, polyisobutylene-PEG, Polycaprolactone-PEG (PCL-PEG), PLA-PEG, Poly(methyl methacrylate)-PEG (PMMA-PEG), polydimethylsiloxane-co-PEG (PDMS-PEG), Poly(vinylidene fluoride)-PEG (PVDF-PEG), (polypropylene oxide-co-polyethylene glycol), poly(tetra-,methylene glycol), hydroxy¬functionalized poly(vinylpyrrolidone), polypropylene oxide-co-polyethylene glycol, PEGylated heparin, glycosaminoglycan (GAG), GAG derivatives, polysaccharides, polymers and copolymers of hydroxyl-containing monomers such as HEMA, Hydroxypropyl methacrylate (HPMA), hydroxypropyl methacrylamide, PEG acrylate (PEGA), PEG methacrylate, 2-methacryloyloxyethylphosphorylcholine (MPC), N-vinylpyrrolidone (VP), polymers and copolymers of carboxylic acid-containing monomers such as methacrylic acid (MA), acrylic acid (AA), alkoxy methacrylate, alkoxy acrylate, 3-trimethylsilylpropyl methacrylate (TMSPMA), as well as co-, block- or segment polymers and/or mixtures of the aforementioned polymers and derivatives thereof.

Examples of biodegradable, or bioabsorbable polymers include, but are not limited to polyvalerolactones, poly-ε-decalactones, polylactonic acid, polyglycolic acid polylactides, polyglycolides, copolymers of polylactides and polyglycolides, poly ε-caprolactone, polyhydroxybutyric acid, polyhydroxybutyrates, polyhydroxyvalerates, Polyhydroxybutyrate-co-valerate, poly(1,4-dioxane-2,3-dione), poly(1,3-dioxane-2-one), poly-p-dioxanones, polyanhydrides such as polymaleic anhydrides, polyhydroxymethacrylates, fibrin, polycyanoacrylates, poly-b-maleic acid, polycaprolactone dimethylacrylates, polycaprolactone butyl acrylates, polypivotolactones, multiblock polymers such as those e.g., of oligocaprolactone diols and oligodioxanone diols, polyether ester multiblock polymers such as PEG and polybutylene terephthalate. Polyglycolic acid trimethyl carbonates, polycaprolactone glycolides, poly-g-ethylglutamate, poly(DTH-iminocarbonate), poly(DTE-co-DT-carbonate), poly(bisphenol A-iminocarbonate), polyorthoesters, polytrimethyl carbonates, polyglycolic acid trimethyl carbonates, polyiminocarbonates, poly(N-vinyl)-pyrolidone, polyvinyl alcohols, polyorthoesters and their copolymers, lipids, carrageenans, fibrinogen, starch, collagen, protein-based polymers, polyamino acids, synthetic polyamino acids, zein, modified zein, polyhydroxyalkanoates, pectinic acid, actinic acid, modified and unmodified fibrin and casein, carboxymethyl sulfate, albumin, furthermore hyaluronic acid, heparan sulfate, heparin, chondroitin sulfate, dextran, β-cyclodextrins, and copolymers with PEG and polypropylene glycol, gum arabic, guar, gelatin, collagen, collagen-N-hydroxysuccinimide, lipids, phospholipids, modifications and copolymers and/or mixtures of the aforementioned.

Examples of biostable polymers or extremely slowly degradable polymers include, but are not limited to polyacrylic acid, polyacrylates such as polymethylmethacrylate, polybutylmethacrylate, polyacrylamide, polyacrylonitrile, polyamides, polyetheramides, polyethyleneamine, polyimides, polycarbonates, polycarbourethanes, polyvinylketones, polyvinylhalides, polyvinylidenehalides, polyvinylethers, polyisobutylenes, polyvinyl aromatics, polyvinyl esters, polyvinyl pyrollidones, polyoxymethylenes, polytetramethylene oxide, polyethylene, polypropylene, polytetrafluoroethylene, polyurethanes, polyetherurethanes silicone-polyetherurethanes, silicone-polyurethanes, silicone-polycarbonate urethanes, polyolefin elastomers, polyisobutylenes, EPDM rubbers, fluorosilicones, carboxymethyl chitosans, polyarylether ether ketones, polyether ether ketones, polyethylene terephthalate, polyvalerates, carboxymethyl cellulose, cellulose, Rayon, Rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, EPDM rubbers, silicones such as polysiloxanes, polydimethylsiloxanes, polyvinyl halogens and copolymers, cellulose ethers, cellulose triacetates,chitosans and copolymers and/or mixtures of these substances.

Examples of hydrophilic polymers include, but are not limited to, polyvinyl alcohol (PVA), polyethylene oxide (polyethylene glycol (PEG)), poly-N-vinyl-pyrrolidone, poly-N-vinyl-2-piperidone, poly-N-vinyl-2-caprolactam, poly-N-vinyl-3-methyl-2-caprolactam, poly-N-vinyl-3-methyl-2-piperidone, poly-N-vinyl-4-methyl-2-piperidone, poly-N-vinyl-4-methyl-2-caprolactam, poly-N-vinyl-3-ethyl-2-pyrrolidone, poly-N-vinyl-4,5-dimethyl-2-pyrrolidone, polyvinylimidazole, poly-N-dimethyl acrylamide, polyacrylic acid, poly-2-ethyloxazoline, heparin polysaccharides, polysaccharides, polyoxyethylene derivatives as well as co-, block- or segment polymers thereof and derivatives thereof.

Examples of polymers obtainable through non-radical polymerization include, but are not limited to, polyvinyl alcohols, polyethers, polyorthoesters, polyetherimines, polyetheramines, polyurethanes, polyhydroxycarboxylic acids, polylactams, polyethylene glycols, polyethylene oxides, polyethylene imines, polyitaconic acids, polymesaconic acids, polyglutaconic acids, polycitraconic acids, polyaconitic acids, polyisocitronic acids, polyoxaloacetic acids, polymalic acids, polylactides, glucosaminoglycans, polyglycolides, cellulose, polyoxazolines, peptides, polysaccharides, polyelectrolytes, polyacetals, polysiloxanes, polymethylene malonic acids, as well as co-, block- or segment polymers thereof and derivatives thereof. Non-radical polymerization also yields polyethylene glycols, polyethylene oxides, polypropylene glycols or polyurethanes. Polyurethanes, for example, can be synthesized from diols and diisocyanates via polyaddition reaction. Polyethylene glycols / polyethylene oxides can be synthesized from ethylene oxide by means of alkaline catalysis.

A polymer may further comprise photosensitive and/or thermosensitive groups and may represent a photosensitive and/or thermosensitive polymer that can be activated via irradiation and/or heat and can bind to surfaces, for example, with the formation of covalent bonds.

### Hydrophilic Polymer

The term "**hydrophilic polymer**", as used herein, refers to a polymer having a partition coefficient between n-octanol and water of Tow < 6.30 (log Tow < 0.80), preferably Tow < 1.80 (log Tow < 0.26), more preferably Tow < 0.63 (log Tow < -0.20), and even more preferably Tow < 0.40 (log Tow < -0.40). A "**hydrophilic monomer**" is herein preferably a monomer with a solubility in water of more than 80 g/l, in particular of more than 100 g/l at 25° C and 1 bar.

### Hydrophobic Polymer

The term "**hydrophobic polymer**" or "lipophilic polymer", as used herein, refers to a polymer having a partition coefficient between n-butanol and water of ≥0.5, preferably ≥0.7, more preferably ≥0.9, and particularly preferably ≥1.1. A "**hydrophobic monomer**" as used herein is preferably a monomer having a solubility in water of not more than 80 g/l, particularly not more than 50 g/l at 25° C and 1 bar.

### Polymer Chain

The term "**polymer chain**", as used herein, refers to an unbranched or branched chain of a plurality of covalently linked monomers. In the production of polymers, many monomers are covalently linked to form a polymer chain consisting of repeating structural units (monomer moieties). Thus, polymers represent macromolecules composed of repeating, identical or different structural units (monomer moieties). An unbranched or linear polymer is a polymer that consists of one polymer chain, the main chain. Linear polymers are formed when the monomers can only be linked to two adjacent monomers. An example of a linear polymer is polyvinylpyrrolidone (PVP). A branched polymer has additional branching points in the polymer chain, via which a side chain can be formed in the polymer chain. For example, to prepare a branched polymer, a monomer can be used that has more than one chemically reactive group, such as a tri-, quaternary or polyfunctional monomer.

### Degree of Polymerization

The term "**degree of polymerization**", as used herein, refers to the number of covalently linked monomers in the polymer chain. Macromolecules having a degree of polymerization of at least 10 or macromolecules having a polymer chain of at least 10 covalently linked monomers are herein referred to as a polymer.

### Monomer Moiety

The term "**monomer moiety**", as used herein, refers to a covalently bound monomer incorporated in a polymer chain. Thus, the term "monomer moiety", refers to a structural unit of a polymer chain. Thus, a polymer chain or a polymer consists of many monomer moieties linked together through covalent bonds. If the monomer is an unsaturated organic compound of the general formula R-CH=CH₂, wherein R is any arbitrary residue, the monomer moiety has the following structure:

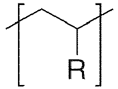

### Polymerization

The term "**polymerization**" or "polymerization reaction," as used herein, refers to a chemical reaction in which similar or different monomers are reacted to form polymers. Thus, in a polymerization reaction, linear, branched or crosslinked polymers are formed by successive reactions of monomers or oligomers. Herein, unsaturated organic compounds containing at least one double bond are preferably used as monomers:

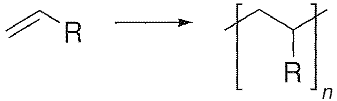

### Homopolymer and Homopolymerization

The term "**homopolymerization**", as used herein, refers to a polymerization, wherein only one type of monomer is used to produce a polymer. Thus, homopolymerization yields a polymer consisting of only one kind of monomer moiety. A polymer composed of the same monomer moieties is also referred to herein as a "**homopolymer**".

### Copolymer, Main Monomer, Comonomer and Copolymerization

The term "**copolymerization**", as used herein, refers to a polymerization. wherein two or more different monomers are used to produce a polymer. Thus, copolymerization yields a polymer, wherein the polymer chain is composed of different monomer moieties. A polymer that is built up from two or more different monomer moieties is therefore herein referred to as a "**copolymer**". The two or more different monomers may be present in the copolymer in equimolar ratio or in some other ratio. Generally, the monomer that primarily determines the physical and mechanical properties of the resulting copolymer is referred to as the "**main monomer**". The term "**comonomer**", as used herein, refers to a (second) monomer used in a copolymerization in addition to the main monomer. Copolymerization is commonly used in the prior art to tailor the physical and mechanical properties of a homopolymer to specific requirements, e.g., to control wetting properties or improve solubility. In this case, only small amounts or even substantial amounts of a comonomer can also be used. One advantage in using comonomers is that comonomers are also incorporated into the polymer chain and are covalently bound in the polymer and thus cannot be leached out. Comonomers can also be used to achieve crosslinking in order to obtain a crosslinked polymer. Since a copolymer consists of at least two types of monomers, copolymers can be classified according to how these monomers are arranged along the polymer chain. Linear copolymers consist of a single main chain and comprise alternating copolymers, random copolymers and block copolymers. Branched copolymers consist of a main chain with one or more polymeric side chains and may be grafted.

### Random Copolymer

The term "random copolymer", as used herein, refers to a copolymer in which the distribution of the main monomer and comonomer in the chain follows a statistical distribution. If the ratio of monomers in a section corresponds to the molar ratio, the distribution is said to be random.

### Block Copolymer

The term "**block copolymer**", as used herein, refers to a copolymer consisting of longer segments or blocks of each of two or more different monomers.

### Graft Copolymer

The term "**graft copolymer**", as used herein, refers to a copolymer in which polymer chains of a monomer are grafted onto the backbone of a polymer or copolymer.

### Alternating Copolymer

The term "**alternating copolymer**", as used herein, refers to a copolymer in which the two monomers are arranged alternately.

### Blocks

The term "**blocks**", as used herein, refers to segments in a polymer chain of a copolymer that are composed of only one type of a monomer and therefore have the structural characteristics of the homopolymer of said monomer. Herein, blocks preferably consist of at least two, more preferably at least three, further preferably at least four, even more preferably at least five, further preferably at least six, even more preferably at least seven, and further preferably at least eight identical monomers. A copolymer composed of blocks of monomer moieties of a first monomer divided by blocks of a second monomer moiety differs from an ordinary block polymer in that the polymer chain contains only blocks of the first monomer and no blocks of the second monomer are present. Thus, in a copolymer composed of blocks of a first monomer divided by a second monomer, the second monomer has only covalent linkages to the monomer moieties of the first monomer and no directly covalently linked moieties of the second monomer are present in the polymer chain. The first monomer is referred to herein as the main monomer and the second monomer is referred to as the comonomer. It should be understood that the size of the blocks, and thus the number of the main monomer in a block, also depends on the amount of comonomers in the polymer chain, i.e. the smaller the amount of comonomers the larger the number of the main monomer in the blocks. Thus, the size of the blocks decreases as more comonomer is used in the copolymerization. If the main monomer and the comonomer are ultimately present in a ratio of 1:1, an alternating copolymer is obtained in which the main monomer and the comonomer are arranged alternately in the polymer chain. In such an alternating copolymer, the blocks therefore consist of only one monomer moiety of the main monomer, but there are still no directly covalently linked comonomers in the polymer chain. It should be further understood that the size of the blocks, and thus the number of blocks of the main monomer of a block, also depends on the method of preparation of the copolymer. If the copolymer is prepared by copolymerization starting from the at least two monomers, the incorporation of the comonomer into the polymer chain can be statistically or random. Thereby, different sizes of the blocks are formed, depending on the number of blocks of the main monomer already incorporated after which a covalent linkage with a comonomer occurs. Preferred herein is that the blocks have substantially an equal number of blocks of the main monomer, that is, substantially equal sized blocks. Another possibility is to provide polymer chains of blocks of the main monomer already during copolymerization instead of the main monomer itself, i.e. oligomers of the main monomer. Chain extension can then be achieved by covalent linkage with a comonomer or with a further oligomer of the main monomer. This approach is advantageous in that a minimum number of blocks of the main monomer can be ensured in the blocks of the copolymer.

### Molecular Colloid

The term "**molecular colloid**", as used herein, generally refers to substances that are thermodynamically stable, hydrophilic systems whose particle size is determined by the linkage of 10³ to 10⁹ covalently bound atoms, preferably 10⁴ to 10⁷ covalently bound atoms. The colloid particles in the solutions of macromolecular compounds (e.g. polymers) are called molecular colloids. The molecular colloids are identical with the macromolecule (e.g. the polymer), whose structural units are connected by covalent bonds, wherein macromolecular compounds are compounds whose characteristic properties are essentially determined by the extraordinary size or length of their chain-like molecules, the molecular weights of most natural and synthetic compounds being between 10³ to 10⁹ , preferably 10⁴ and 10⁷, wherein these macromolecular compounds give colloidal solutions in which the colloidal particles are generally identical with the individual macromolecules.

### Molecular Colloid Network

The term "**molecular colloid network**", as used herein, can be formed from a molecular colloid and a crosslinker through covalent bonds, i.e. via crosslinking. The bonds of the molecular colloid network in the solid phase are covalent bonds.Thus, "molecular colloid networks" refer to molecular colloids whose macromolecules or colloid particles are covalently bound to one another inter- and intramolecularly via bi- or multifunctional compounds (crosslinkers). Polymers suitable for preparing the molecular colloid network of the present invention include homopolymers as well as mixed polymers or copolymers. Crosslinking can be achieved either directly during the assembly of the polymers or by reactions on preexisting polymers. A crosslinker and at least one molecular colloid (i.e. polymer) are preferably crosslinked and together form the molecular colloid network.

### Coefficient of Friction

The term "**coefficient of friction**", as used herein, is also called the frictional coefficient and is also abbreviated herein as CoF. The coefficient of friction indicates the ratio of the frictional force to the contact force between two bodies. In sliding friction, the friction surfaces move relative to each other. Sliding friction occurs at the contact surfaces between bodies that move relative to each other. The "coefficient of friction", as used herein, therefore herein refers to the coefficient of sliding friction *µ*_{G}.

### Alkyl

The term "**alkyl**", as used herein, refers to saturated hydrocarbon groups having from 1 to 10 carbon atoms, either straight or branched, preferably having from 1 to 8 carbon atoms, and more preferably having from 1 to 6 carbon atoms. An alkyl having a certain number of carbon atoms is referred to as e.g. "C₁-C₈-alkyl" with -CH₃, -C₂H₅, -C₃H₇, -C₄H₉, -C₅H₁₁, -C₆H₁₃, -C₇H₁₅, and -C₈H₁₇, or " linear C₁-C₆-alkyl" with -CH₃, -CH₂CH₃, -CH₂CH₂CH₃, -CH₂CH₂CH₂CH₃, -CH₂CH₂CH₂CH₂CH₃, and -CH₂CH₂CH₂CH₂CH₂CH₃; or a "branched C₁-C₆ alkyl" with -CH(CH₃)₂, -CH₂CH(CH₃)₂, -CH(CH₃)C₂H₅, -C(CH₃)₃, -CH(CH₃)C₃H₇, -CH₂CH(CH₃)C₂H₅, -CH(CH₃)CH(CH₃)₂, -C(CH₃)₂C₂H₅, -CH₂C(CH₃)₃, -CH(C₂H₅)₂, -C₂H₄CH(CH₃)₂, -C₃H₆-CH(CH₃)₂, -C₂H₄CH(CH₃)C₂H₅, -CH(CH₃)C₄H₉, -CH₂CH(CH₃)-C₃H₇, -CH(CH₃)CH₂CH(CH₃)₂, -C(CH₃)₂C₃H₇, -CH(CH₃)CH(CH₃)C₂H₅, -CH₂-CH(CH₃)-CH(CH₃)₂, -CH₂-C(CH₃)₂C₂H₅, -CH(CH₃)C(CH₃)₃, -C(CH₃)₂CH(CH₃)₂, and -C₂H₄C(CH₃)₃.

### Alkylene

The term "**alkylene**", as used herein, refers to a straight or branched divalent hydrocarbon residue having 1 to 10 carbon atoms, preferably 1 to 8 carbon atoms, and more preferably 1 to 6 carbon atoms.

### Alkenyl

The term "**alkenyl**", as used herein, refers to groups having from 2 to 10 carbon atoms, preferably from 2 to 6 carbon atoms (C₂-C₆ alkenyl), which are either straight or branched and contain at least one double bond, but optionally more than one double bond. Examples of "straight or branched C₂-C₈ -alkenyl" include, but are not limited to: -CH=CH₂, -CH₂CH=CH₂, -C(CH₃)=CH₂, -CH=CHCH₃, -C₂H₄CH=CH₂, -CH₂CH=CH-CH₃, -CH=CHC₂H₅, -CH=C(CH₃)₂, -CH₂C(CH₃)=CH₂, -CH(CH₃)CH=CH, -C(CH₃)=CHCH₃, -CH=CHCH=CH₂, -C₃H₆CH=CH₂, -C₂H₄CH=CHCH₃, -CH₂CH=CHC₂H₅, -CH=CHC₃H₇, -CH₂CH=CHCH=CH₂, -CH=CHCH=CHCH₃, -C₂H₄CH=CHCH₃, -CH₂CH=CHC₂H₅, -CH₂CH=CHCH=CH₂, -CH=CHCH=CHCH₃, -CH=CHCH₂CH=CH₂, -C(CH₃)=CHCH=CH₂, -CH=C(CH₃)CH=CH₂, -CH=CHC(CH₃)=CH₂, -CH₂CH=C(CH₃)₂, -C(CH₃)=C(CH₃)₂, -C₂H₄CH=CH₂, -CH=CHC₂H₅, -CH=C(CH₃)₂, -CH₂CH=CH-CH₃, -CH=CHCH=CH₂, C₃H₆-CH=CH₂, -CH=CHC₃H₇, -C₄H₈CH=CH₂, -CH=CHC₄H₉, -C₃H₆CH=CHCH₃, -CH₂CH=CHC₃H₇, -C₂H₄CH=CHC₂H₅, -CH₂C(CH₃)=C(CH₃)₂, -C₂H₄CH=C(CH₃)₂ and -CH=CHPh.

### Alkenylene

The term "**alkenylene**", as used herein, refers to a straight or branched divalent hydrocarbon residue having from 2 to 12 carbon atoms and one or more carbon-carbon double bonds, preferably from 2 to 8 carbon atoms and more preferably from 2 to 6 carbon atoms.

### Alkynyl

The term "**alkynyl**", as used herein, refers to groups having from 2 to 10 carbon atoms, preferably from 2 to 8 carbon atoms, which are either straight or branched and contain at least one triple bond, but optionally more than one triple bond, and additionally optionally have one or more double bonds. Examples of "straight or branched C₂-C₈ alkynyl" include, but are not limited to: -CH₂C≡CH, -C≡CH, -C≡CCH₃, -C₂H₄C≡CH, -C≡C-C₂H₅, -CH₂-C≡CCH₃, -C≡CCH=CH₂, -CH=CH-C≡CH, -C≡CC≡CH, -C₃H₆C≡CH, -C≡CC₃H₇, -C₂H₄C≡CCH₃, -CH₂C≡CC₂H₅, -CH₂C≡CCH=CH₂, -CH₂CH=CHC≡CH, -CH₂C≡CC≡CH, -C≡CCH=CHCH₃, -CH=CHC≡CCH₃, -C≡CC≡CCH₃, -C≡CCH₂CH=CH₂, -CH=CHCH₂C≡CH, -C≡CCH₂C≡CH, -C(CH₃)=CHCH=CH₂, -CH=C(CH₃)CH=CH₂, -CH=CHC(CH₃)=CH₂, -C(CH₃)=CHC≡CH, -CH=C(CH₃)C≡CH, -C≡CC(CH₃)=CH₂, -C₄H₈C≡CH, -C≡CC₄H₉, -C₃H₆C≡CCH₃, -CH₂C≡CC₃H₇, and -C=CPh.

### Cycloalkyl

The term "**cycloalkyl**", as used herein, refers to cyclic alkyl groups having 3 to 12 carbon atoms, preferably 3 to 8 carbon atoms, having a single cyclic ring or multiple fused rings that may optionally be substituted with 1 to 3 alkyl groups. Examples of cycloalkyl groups include, but are not limited to, single ring structures such as cyclopropyl, cyclobutyl, cyclopentyl, cyclooctyl, 1-methylcyclopropyl, 2-methylcyclopentyl, 2-methylcyclooctyl, and the like, or multiple ring structures, including bridged ring systems, such as adamantyl. Examples of "C₃-C₈ cycloalkyl" include, but are not limited to, cyclo-C₃H₅, cyclo-C₄H₇, cyclo-C₅H₉, cyclo-C₆H₁₁, cyclo-C₇H₁₃, and cyclo-C₈H₁₅.

### Aryl. Diaryl, Arylalkyl

The term "**aryl**", as used herein, refers to an unsaturated aromatic carbocyclic group having 6 to 12 carbon atoms and having a single ring (e.g. phenyl) or multiple fused rings (e.g. naphthyl or anthryl). Examples of aryls include phenyl, pyridyl, naphthyl, and the like. The term "**diaryl**", as used herein, refers to an unsaturated aromatic carbocyclic group having at least 6 to 12 carbon atoms and having more than one ring, that are not fused e.g. diaryl compounds such as benzophenone or derivatives thereof. The term "**arylalkyl**", as used herein, refers to an alkyl substituted with an aryl, i.e. arylalkyl groups preferably having 1 to 6 carbon atoms in the alkyl moiety and 6 to 12 carbon atoms in the aryl moiety. Such arylalkyl groups include benzyl, phenylethyl and the like. Examples of "C₆-C₈ arylalkyl" include, but are not limited to -CH₂Ph and -C₂H₄Ph. The term "**diarylalkyl**", as used herein, refers to an alkyl substituted with a diaryl, i.e. diarylalkyl groups preferably having 1 to 6 carbon atoms in the alkyl moiety and at least 6 to 12 carbon atoms in the diaryl moiety. Such diarylalkyl groups include benzoylbenzyl and the like.

### Oxy, Alkoxy, Aryloxy

The term "**oxy**", as used herein, refers to a divalent group -O-, which may have various substituents to form various oxy groups, including ethers and esters. The term "**alkoxy**" or "alkyloxy" as used herein are used interchangeably and refer to the group -OR°, wherein R° is an alkyl group, including optionally substituted alkyl groups. "**Aryloxy**" as used herein refers to the group -OR°, wherein R° is an aryl group, including optionally substituted aryl groups.

### Carbonyl

The term "**carbonyl**", as used herein, refers to the chemical group -C(O)- or C=O. Substituted carbonyl refers to the group R°-C(O)-R°, wherein each R° is independently of each other selected from optionally substituted alkyl, cycloalkyl, cycloheteroalkyl, alkoxy, carboxy, aryl, aryloxy, heteroaryl, heteroarylalkyl, acyl, alkoxycarbonyl, sulfanyl, sulfinyl, sulfonyl, and the like. Typical substituted carbonyl groups include acids, ketones, aldehydes, amides, esters, acyl halides, thioesters and the like.

Amino, Aminoalkyl, Aminoaryl, Aminoarylalkyl, Aminocarbonyl, Aminocarbonylalkyl The term "**amino**", as used herein, refers to the group -NH₂. Substituted amino refers to the group -NHR^{α}, NR^{α}R^{α}, and NR^{α}R^{α}R^{α}, wherein each R° is independently of each other selected from optionally substituted alkyl, cycloalkyl, cycloheteroalkyl, alkoxy, carboxy, aryl, aryloxy, heteroaryl, heteroarylalkyl, acyl, alkoxycarbonyl, sulfanyl, sulfinyl, sulfonyl, and the like. Typical amino groups include dimethylamino, diethylamino, trimethylammonium, triethylammonium, methylysulfonylamino, furanyl-oxy-sulfamino and others. The term "**aminoalkyl**", as used herein, refers to an alkyl group in which one or more hydrogen atoms are replaced by an amino group, including a substituted amino group. The term "**aminoaryl**", as used herein, refers to an aryl group in which one or more hydrogen atoms are replaced by an amino group, including a substituted amino group. The term "**aminoarylalkyl**", as used herein, refers to an alkylaryl group in which one or more hydrogen atoms are replaced by an amino group, including a substituted amino group. The term "**aminocarbonyl**", as used herein, refers to a carbonyl group substituted with an amino group, including a substituted amino group as defined herein, and includes amides. The term "**aminocarbonylalkyl**" refers to an alkyl substituted with an aminocarbonyl group.

### Halogen, Haloalkyl

The term "**halogen**", as used herein, refers to fluorine, chlorine, bromine and iodine. The term "**haloalkyl**", as used herein, refers to an alkyl group in which one or more hydrogen atoms are replaced by a halogen. Thus, the term haloalkyl includes monohaloalkyl, dihaloalkyl, trihaloalkyl, etc., up to perhaloalkyl. The term "C₁-C₂ haloalkyl" comprises 1-fluoromethyl, difluoromethyl, trifluoromethyl, 1-fluoroethyl, 1,1-difluoroethyl, 1,2-difluoroethyl, 1,1,1-trifluoroethyl, perfluoroethyl, etc..

### Hydroxy, Hydroxyalkyl, Hydroxyaryl, Alkyaryloxy

The term "**hydroxy**", as used herein, refers to -OH. The term "**hydroxyalkyl**", as used herein, refers to an alkyl substituted with one or more hydroxy groups. The term "**hydroxyaryl**", as used herein, refers to an aryl substituted with one or more hydroxy groups. The term "**alkylaryloxy**", as used herein, refers to an arylalkyl substituted with one or more hydroxy groups.

### Heteroalkyl, Heteroalkenyl, Heteroalkynyl, Heteroaryl, Heteroarylalkyl, Heterocycloalkyl

The terms "**heteroalkyl**", "**heteroalkenyl**", and "**heteroalkynyl**", as used herein, refer to alkyl, alkenyl, and alkynyl residues, as defined herein, in which one or more carbon atoms are each independently replaced by the same or different heteroatoms or heteroatomic groups. Heteroatoms and/or heteroatomic groups that may replace the carbon atoms include, but are not limited to, -O-, -S-, -S-O-, -NR°-, -PH-, -S(O)-, -S(O)₂-, -S(O)NR°-, -S(O)₂NR^{α}-, and the like, including combinations thereof, wherein each R° is independently selected from hydrogen, alkyl, heteroalkyl, cycloalkyl, heterocycloalkyl, aryl, and heteroaryl. The term "**heteroaryl**" refers to an aromatic heterocyclic group having 1 to 10 carbon atoms and 1 to 4 heteroatoms selected from oxygen, nitrogen and sulfur within the ring. Such heteroaryl groups may have a single ring (e.g., pyridyl or furyl) or multiple fused rings (e.g., indolizinyl or benzothienyl). The term "C₁-C₁₀ heteroaryl" refers to aromatic residues having one or more heteroatoms such as O, S, N. The term "**heteroarylalkyl**" refers to an alkyl substituted with a heteroaryl, i.e. heteroarylalkyl groups preferably having 1 to 6 carbon atoms in the alkyl moiety and 5 to 12 ring atoms in the heteroaryl moiety. Such heteroarylalkyl groups include pyridylmethyl and the like. The term "**heterocycloalkyl**" refers to a saturated or unsaturated group having a single ring or multiple fused rings, 2 to 9 carbon ring atoms and 1 to 4 hetero ring atoms selected from nitrogen, sulfur or oxygen within the ring. Such heterocyclic groups may have a single ring (e.g., piperidinyl or tetrahydrofuryl) or multiple fused rings (e.g., indolinyl, dihydrobenzofuran, or cuinuclidinyl). Examples of heterocycles include, but are not limited to, furan, thiophene, thiazole, oxazole, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, pyrrolidine, indoline and the like.

### Carboxy Group, Carboxylic Acid, Carboxylic Acid Ester

The term "**carboxy group**", as used herein, refers to the functional group -COOH. Organic compounds having one or more carboxy groups (-COOH) are referred to as "**carboxylic acids**". Carboxylic acids have the general formula R^{α}-COOH, wherein the residue R° may represent a hydrogen atom or an organic residue. A carboxylic acid with only one carboxy group is referred to herein as a "monocarboxylic acid" and a carboxylic acid with two carboxy groups is referred to herein as a "dicarboxylic acid". Examples of carboxylic acids having one or more carboxy groups include, but are not limited to, acetic acid, citric acid, acrylic acid, benzoic acid, fumaric acid, maleic acid, oleic acid and salicylic acid. "**Carboxylic acid esters**" are esters of carboxylic acids with the functional group -COOR^{β}, wherein R^{β} represents an organic residue derived from an alcohol or a phenol. Therefore, carboxylic acid esters have the general formula R^{α}-COO-R^{β}. Examples of carboxylic acid esters include, but are not limited to, alkyl acetates such as ethyl ethanoate (ethyl acetate), triglycerides, lactones such as γ-butyrolactone and also polyesters such as polyethylene terephthalate (PET). The formula **(-COO-)**, as used herein, is used to represent a carboxy group, which may be either a carboxyl group (-COOH) or an ester group (-COOR^{β}), wherein R^{β} represents an organic residue. Thus, the formula **(-COO-)** may be also used herein when a carboxy group may be optionally functionalized.

As used herein, -OCO- represents 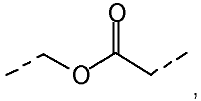 and -COO- represents 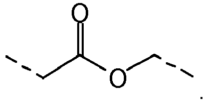 As used herein, a dashed line indicates connectivity and a solid line represents a bond.

### Carbamoyl Group, Carboxylic Acid Amides

The term "**carbamoyl group**", as used herein, refers to the functional group -CONH₂. Organic compounds having one or more carbamoyl groups (-CONH₂) are referred to as "**carboxylic acid amides**" in organic chemistry. Carboxylic acid amides are therefore derived from carboxylic acids. Carboxylic acid amides generally have the general formula R^{α}-CONH₂, wherein the residue R° may represent a hydrogen atom or an organic residue. Carboxylic acid amides with the functional group -CONH₂ are also referred to as primary carboxylic acid amides. If one or both hydrogen atoms on the nitrogen are replaced by an organic residue, secondary carboxylic acid amides of the general formula R^{α}-CONHR^{β} or tertiary carboxylic acid amides of the general formula R^{α}-CONR^{β}R^{β'} are obtained, wherein the residues R^{β} and R^{β'} represent organic residues. Carboxylic acid amides can be prepared by reacting reactive carboxylic acid derivatives, such as carboxylic acid chlorides or carboxylic acid anhydrides with ammonia or primary or secondary amines. In this context, a carboxylic acid amide with only one carbamoyl group is referred to as a "monocarboxylic acid amide" and a carboxylic acid amide with two carbamoyl groups is referred to as a "dicarboxylic acid amide". Examples of carboxylic acid amides having one or more carbamoyl groups include, but are not limited to, formamide, *N,N*-dimethylformamide (DMF), acetamide, ε-caprolactam, N-vinylpyrrolidone, urea, polyamides such as peptides, proteins, or synthetic polyamides such as polyhexamethylene adipic acid amide (nylon). The formula **(-CONH-)**, as used herein, is used to represent a carbamoyl group, which may be either a primary carbamoyl group (-CONH₂) or a secondary carbamoyl group (-CONHR^{β}), wherein R^{β} represents an organic residue. Thus, the formula **(-CONH-)** may be also used herein when a carbamoyl group may be optionally functionalized with an organic residue.

Herein, the functional group -NHCO- represents 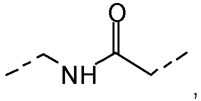 and -CONH-represents 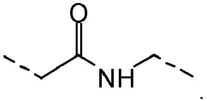 As used herein, a dashed line indicates connectivity and a solid line represents a bond.

### Carboxylic Acid Derivate

The term "**carboxylic acid derivative**", as used herein, refers to an organic compound having one or more functional groups formally derived from the carboxy group -COOH. In particular, the term carboxylic acid derivative, as used herein, refers to carboxylic acid esters of the general formula R^{α}-COO-R^{β}, primary carboxylic acid amides of the general formula R^{α}-CONH₂ and secondary carboxylic acid amides of the general R^{α}-CONH-R^{β}, wherein the residues R^{α} and R^{β} represent organic residues. Particularly preferably, the term carboxylic acid derivative herein refers to carboxylic acid esters of the general formula R^{α}-COO-R^{β} and secondary carboxylic acid amides of the general formula R^{α}-CONH-R^{β}, wherein the residues R^{α} and R^{β} represent organic residues. In an organic compound with more than one carboxy group, one or more of these carboxy groups may be present as ester or amide. If a dicarboxylic acid is present, one or both carboxy groups may be functionalized with an organic residue or may have one or two carbamoyl groups instead of the carboxy groups.

### Carbamate Group

The term "**carbamate group**", as used herein, refers to the functional group -NHCOO-, or -OCONH-. Carbamates which are formally derived from carbamic acid (NH2COOH) generally have the general formula R^{β}R^{β'}NCOOR^{α}, wherein the residues R°, R^{β} and R^{β'}represent organic residues.

Herein, the functional group -NHCOO- represents 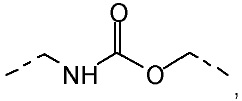 and - OCONH- represents 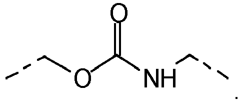 As used herein, a dashed line indicates connectivity and a solid line represents a bond.

### Urea Group

The term "**urea group**", as used herein, refers to the functional group -NHCONH-Urea is an organic compound with chemical formula CO(NH₂)₂. This amide has two amino groups (-NH₂) joined by a carbonyl functional group (-C(=O)-). Ureas refers to a class of chemical compounds that share the same functional group, a carbonyl group attached to two organic amine residues: R^{α}R^{α'}N-C(=O)-NR^{β}R^{β'}, wherein the organic residues R^{α}, R^{α'}, R^{β} and R^{β'} are hydrogen, or represent organic residues.

Herein, the functional group -NHCONH- represents 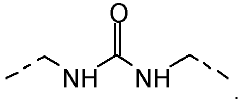 As used herein, a dashed line indicates connectivity and a solid line represents a bond.

### Carbonate Ester Group

The term "**carbonate ester group**", as used herein, refers to the functional group -OCOO-. Carbonate esters are esters of carbonic acid. This functional group consists of a carbonyl group flanked by two alkoxy groups. The general structure of these carbonates is R^{α}-O-C(=O)-O-R^{β}, wherein the residues R^{α} and R^{β} represent organic residues.

Herein, the functional group -OCOO- represents 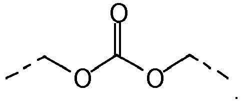 As used herein, a dashed line indicates connectivity and a solid line represents a bond.

### Organometallic Group

The term "**organometallic group**", as used herein, refers to chemical groups containing at least one carbon atom and at least one metal or electropositive element atom bound via a more or less polar covalent bond. In this context, the organic group can either be bound to the metal or element via a single, double or even triple bond, or can be linked multiple times with the metal or element atom.

### Silyl Group

The term "**silyl group**", as used herein, refers to chemical groups containing at least one carbon atom and at least one silicium atom bound via a more or less polar covalent bond. In this context, the silicium atom can either be bound to an oxygen atom. The term "silyl group" refers to the chemical group -SiR₃, wherein R represents hydrogen or an organic residue, e.g. -CH₃. For example, silyl ethers are a group of chemical compounds which contain a silicon atom covalently bonded to an alkoxy group. The general structure is R₁R₂R₃Si-O-R₄ wherein R₄ is an alkyl group or an aryl group.

### Optionally Substituted

The term "**optionally substituted**", as used herein, with respect to the aforementioned chemical groups means that the positions of the chemical group occupied by hydrogen may be substituted by another atom, such as carbon, oxygen, nitrogen or sulfur, or a chemical group, e.g. by hydroxy, oxo, nitro, methoxy, ethoxy, alkoxy, substituted alkoxy, trifluoromethoxy, haloalkoxy, fluorine, chlorine, bromine, iodine, halogen, methyl, ethyl, propyl, butyl, alkyl, alkenyl, alkynyl, substituted alkyl, trifluoromethyl, haloalkyl, hydroxyalkyl, alkoxyalkyl, thio, alkylthio, acyl, carboxy, alkoxycarbonyl, carboxamido, substituted carboxamido, alkylsulfonyl, alkylsulfinyl, alkylsulfonylamino, sulfonamido, substituted sulfonamido, cyano, amino, substituted amino, alkylamino, dialkylamino, aminoalkyl, acylamino, amidino, amidoximo, hydroxamoyl, phenyl, aryl, substituted aryl, aryloxy, arylalkyl, arylalkenyl, arylalkynyl, pyridyl, imidazolyl, heteroaryl, substituted heteroaryl, heteroaryloxy, heteroarylalkyl, heteroarylalkenyl, heteroarylalkynyl, cyclopropyl, cyclobutyl, cyclopentyl, cyclohexyl, cycloalkyl, cycloalkenyl, cycloalkylalkyl, substituted cycloalkyl, cycloalkyloxy, pyrrolidinyl, piperidinyl, morpholino, heterocyclic, (heterocyclyl)oxy, and (heterocyclyl)alkyl; wherein preferred heteroatoms are oxygen, nitrogen, and sulfur. However, it is clear to the person skilled in the art that the term "may be substituted" refers to the replacement of a hydrogen atom with any of the above chemical groups. The person skilled in the art understands that the term "optionally substituted" refers only to sterically useful and/or synthetically feasible chemical groups.

### Benzophenone

The term "**benzophenone**", as used herein, refers to an organic compound having the following structural formula:

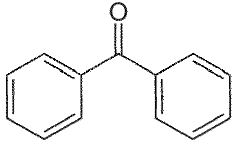

Benzophenone is often used as a photo-initiator for UV curing. Crosslinked polymer coatings or crosslinked materials can be prepared by adding benzophenone to a polymer composition and then irradiating the coating or material with UV light to effect crosslinking. Examples of benzophenones known in the prior art include, but are not limited to benzophenone, 4-phenylbenzophenone, 4-methoxybenzophenone, 4,4'-dimethoxybenzophenone, 4,4'-dimethylbenzophenone, 4,4'-dichlorobenzophenone, 4,4'-bis(di-methylamino)benzophenone, 4,4'-bis(diethylamino)benzophenone, 4,4'-bis(-methyl-ethylamino)benzophenone, 4,4'-bis(p-isopropylphenoxy)benzophenone, 4-methyl-benzophenone, 2,4,6-trimethylbenzophenone, 4-(4-methylthiophenyl)benzo-phenone, 3,3'-dimethyl-4-methoxybenzophenone, methyl-2-benzoylbenzoate, 4-(2-hydroxyethylthio)benzophenone, 4-(4-tolylthio)-benzophenone, 1-[4-(4-benzoyl-phenylsulfanyl)-phenyl]-2-methyl-2-(toluene-4-sulfonyl)-propan-1-one, 4-benzoyl-*N,N,N*-trimethylbenzene methanaminium chloride, 2-hydroxy-3-(4-benzoylphenoxy)-*N*,*N*,*N*-trimethyl-1-propanaminium chloride monohydrate, 4-(13-acryloyl1,4,7,10,13-pentaoxatridecyl)-benzophenone, 4-benzoyl-N,N-dimethyl-N-[2-(1-oxo-2-propenyl)oxy] ethylbenzene methanaminium chloride, benzophenone-3,3'-4,4'-tetracarboxylic acid dianhydride, 4-benzoylbiphenyl, 4,4'-bis(diethyl-amino)benzophenone, 4,4'-bis[2-(1-propenyl)phenoxy]benzophenone, 4-(diethyl-amino)benzophenone, 4,4'-dihydroxy benzophenone, 4-(dimethylamino)-benzophenone, 3,4-dimethylbenzophenone, 4-aminobenzophenone, 4,4'-di-aminobenzophenone, 3-hydroxybenzophenone, 4-hydroxybenzophenone, 4,4'-di-hydroxybenzophenone, 3,4-diaminobenzophenone, 2-methylbenzophenone, 3-methylbenzophenone, 4-methylbenzophenone and Michler ketone.

### Alkyl Linker

The term "**alkyl linker**", as used herein, refers to an alkylene capable of covalently linking two or more organic compounds. An "alkyl linker" represents a bifunctional chemical entity that binds a first compound to a second compound.

### C₁-C₅ Alkyl Linker

The term "**C₁-C₅ alkyl linker**", as used herein, preferably refers to a straight alkyl linker having 1 to 5 carbon atoms, i.e., a "C₁₋C₅ alkenylene". Particularly preferred C₁-C₅ alkyl linkers, as used herein, include, but are not limited to, methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

### Aromatic Linker

The term "**aromatic linker**", as used herein, refers to an aryl, diaryl or heteroaryl compound capable of covalently linking two or more organic compounds. An "aromatic linker" represents a bifunctional chemical entity that binds a first compound, herein a first benzophenone substituent, to a second compound, herein a second benzophenone substituent. An aromatic linker may have one or more substituents including, but not limited to -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, and -SOCH₃. Preferably aromatic linkes may have one or more substituents including, but not limited to -OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃.

### Benzophenone Substituent

The term "**benzophenone substituent**", as used herein, refers to a substituent present at positions of a chemical group occupied instead of hydrogen. Examples of benzophenone substituents include, but are not limited to benzoylphenyl 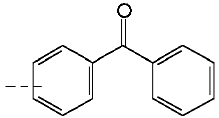 and benzoylbenzyl 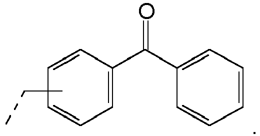 A benzophenone substituent may have one or more substituents including, but not limited to -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃. Preferably benzophenone substituent may have one or more substituents including, but not limited to -OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃.

### Benzophenone Linker

The term "**benzophenone linker**", as used herein, refers to a benzophenone molecule capable of covalently linking two or more organic compounds. A "benzophenone linker" represents a bifunctional chemical entity that binds a first compound, herein a first benzophenone substituent, to a second compound, herein a second benzophenone substituent. A benzophenone linker may have one or more substituents e.g. including, but not limited to -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃. Preferanbly a benzophenone linker may have one or more substituents including, but not limited to -OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃.

According to the present invention, benzophenone substituents, linkers such as aromatic linkers or aryl linkers, alkyl linkers, in particular benzophenone linkers may have one or more substituents selected from the group comprising or consisting of -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃. Preferably, benzophenone substituents and benzophenone linkers may have one or more substituents selected from the group comprising or consisting of -OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃. However, the benzophenone substituents or the benzophenone linker may also have substituents other than the substituents mentioned above. The skilled person is able to select further suitable substituents for benzophenone substituents or benzophenone linkers. Examples of substituents for benzophenone substituents or benzophenone linkers include, but are not limited to -F, -Cl, -Br, -I, -CN, -N₃, -OCN, -NCO, -SCN, -NCS, -COOH, -COCN, -OH, -CHO, -NO₂, -NH₂, -COCH₃, -CONH₂, -SO₃H, -SO₂NH₂, -NHCONH₂, -NHC(=NH)NH₂, -OCONH₂ -CH₃ , alkyl substituents such as -CH₃, -C₂H₅, -C₃H₇, -CH(CH₃)₂, -cyclo-C₃H₅, -C₄H₉, -CH₂CH(CH₃)₂, -CH(CH₃)C₂H₅, -C(CH₃)₃, -cyclo-C₄H₇, -CH₂-cyclo-C₃H₅, -C₅H₁₁, -CH(CH₃)C₃H₇, -CH₂CH(CH₃)C₂H₅, -CH(CH₃)CH(CH₃)₂, -C(CH₃)₂-C₂H₅, -CH₂C(CH₃)₃, -CH(C₂H₅)₂, -C₂H₄CH(CH₃)₂, -cyclo-C₅H₉, -CH₂-cyclo-C₄H₇, -C₆H₁₃, -C₃H₆CH(CH₃)₂, -C₂H₄CH(CH₃)-C₂H₅, -CH(CH₃)C₄H₉, -CH₂CH(CH₃)-C₃H₇, -CH(CH₃)CH₂CH(CH₃)₂, -CH(CH₃)CH(CH₃)C₂H₅, -CH₂CH(CH₃)CH(CH₃)₂, -CH₂C(CH₃)₂C₂H₅, -C(CH₃)₂C₃H₇, -C(CH₃)₂CH(CH₃)₂, -C₂H₄C(CH₃)₃, -CH(CH₃)C(CH₃)₃, -cyclo-C₆H₁₁, -CH₂-cyclo-C₅H₉, -C₇H₁₅, -cyclo-C₇H₁₃, -CH₂-cyclo-C₆H₁₁, -C₈H₁₇, -cyclo-C₈H₁₅, haloalkyl substituents such as -CH₂F, -CHF₂, -CF₃, -CH₂Cl, -CH₂CH₂F, -CH₂CHF₂, -CH₂CF₃, -CH₂Br, -CH₂CH₂Cl, -CH₂CH₂Br, arylalkyl substituents such as -CH₂Ph, -Ph, -C₂H₄Ph, -PhPh, alkoxy substituents such as -OCH₃, -OC₂H₅, -OC₃H₇, -OCH(CH₃)₂, -OC₄H₉, -OCH₂CH(CH₃)₂, -CH(CH₃)C₂H₅, -OC(CH₃)₃, -OCH₂-cyclo-C₃H₅, -OC₅H₁₁, -OCH(CH₃)-C₃H₇, -OCH₂CH(CH₃)-C₂H₅, -OCH(CH₃)CH(CH₃)₂, -OC(CH₃)₂-C₂H₅, -OCH₂-C(CH₃)₃, -OCH(C₂H₅)₂, -OC₂H₄CH(CH₃)₂, -OCH₂-cyclo-C₄H₇, -OC₆H₁₃, -OC₃H₆CH(CH₃)₂, -OC₂H₄CH(CH₃)C₂H₅, -OCH(CH₃)C₄H₉, -OCH₂CH(CH₃)C₃H₇, -OCH(CH₃)CH₂CH(CH₃)₂, -OCH(CH₃)CH(CH₃)C₂H₅, -OCH₂CH(CH₃)CH(CH₃)₂, -OCH₂C(CH₃)₂C₂H₅, -OC(CH₃)₂C₃H₇, -OC(CH₃)₂CH(CH₃)₂, -OC₂H₄C(CH₃)₃, -OCH(CH₃)C(CH₃)₃, -OCH₂-cyclo-C₅H₉, -OC₇H₁₅, -OCH₂-cyclo-C₆H₁₁, -OC₈H₁₇, -OCF₃, -OC₂F₅, -OCH₂Ph, -OPh; -OC₂H₄Ph, -OC₂H₅OH, -OC₃H₇OH, -OC₄H₉OH, -OC₅H₁₁OH, -OC₆H₁₃OH, -OC₂H₅COOH, -OC₃H₇COOH, -OC₄H₉COOH, -OC₅H₁₁COOH, -OC₆H₁₃COOH, -OC₂H₅NH₂, -OC₃H₇ NH₂, -OC₄H₉NH₂, -OC₅H₁₁NH₂, -OC₆H₁₃NH₂, -OC₂H₅OC₂H₅OH, -OC₂H₅OC₂H₅OC₂H₅OH, -OC₂H₅OC₂H₅OC₂H₅OC₂H₅OH, alkylamine substituents such as -NHCH₃, -NHC₂H₅, -NHC₃H₇, -NHC₄H₉, -NH-cyclo-C₃H₅, -NH[CH(CH₃)₂], -NH[C(CH₃)₃], -N(CH₃)₂, -N(C₂H₅)₂, -N(C₃H₇)₂, -N(C₄H₉)₂, -N(cyclo-C₃H₅)₂, -N[CH(CH₃)₂]₂, -N[C(CH₃)₃]₂, ester substituents such as -COOC(CH₃)₃, -COOCH₃, -COOCH₂CH₃, -COOCH₂CH₂CH₃, -COOCH(CH₃)₂, -COOCH₂Ph, -OCOCH₃, -OCOC₂H₅, -OCOC₃H₇, -OCO-cyclo-C₃H₅, -OCOC₄H₉, -OCOCH(CH₃)₂, -OCOCH₂Ph, -OCOC(CH₃)₃, amide substituents such as -NHCOCH₃, -NHCOC₂H₅, -NHCOC₃H₇, -NHCOCH₃, -NHCOC₂H₅, -NHCOC₃H₇, -NHCOC₄H₉, -NHCO-cyclo-C₃H₅, -NHCO[CH(CH₃)₂], -NHCO[C(CH₃)₃], sulfonic substituents such as -SO₂CH₃, -SO₂C₂H₅, -SO₂C₃H₇, -SO₂Ph, -SO₂-cyclo-C₃H₅, -SO₂CH(CH₃)₂, -SO₂C₄H₉, -SO₂C(CH₃)₃, sulfonamide substituents such as -NHSO₂CH₃, -NHSO₂C₂H₅, -NHSO₂C₃H₇, -CH₂SO₂NH₂, -C₂H₄SO₂NH₂, -C₃H₆SO₂NH₂, sulfonoxide substituents such as -SOCH₃, -SOC₂H₅, -SOC₃H₇, -SO-cyclo-C₃H₅, -SOCH(CH₃)₂, -SOC(CH₃)₃, carbonic ester substituents such as -OCOOCH₃, -OCOOC₂H₅, -OCOOC₃H₇, -OCOO-cyclo-C₃H₅, -OCOOC₄H₉, -OCOOCH(CH₃)₂, -OCOOCH₂Ph, -OCOOC(CH₃)₃, alkyl urea substituents such as -NHCONHCH₃, -NHCONHC₂H₅, -NHCONHC₃H₇, -NHCONHC₄H₉, -NHCONH-cyclo-C₃H₅, -NHCONH[CH(CH₃)₂], -NHCONH[C(CH₃)₃], -NHCON(CH₃)₂, -NHCON(C₂H₅)₂, -NHCON(C₃H₇)₂, -NHCON(C₄H₉)₂, -NHCON(cyclo-C₃H₅)₂, -NHCON[CH(CH₃)₂]₂, -NHCON[C(CH₃)₃]₂, alkylguanidine substituents such as -NHC(=NH)NHCH₃, -NHC(=NH)NHC₂H₅, -NHC(=NH)NHC₃H₇, -NHC(=NH)NHC₄H₉, -NHC(=NH)NH-cyclo-C₃H₅, -NHC(=NH)NH[CH(CH₃)₂], -NHC(=NH)NH[C(CH₃)₃], -NHC(=NH)N(CH₃)₂, -NHC(=NH)N(C₂H₅)₂, -NHC(=NH)N(C₃H₇)₂, -NHC(=NH)-N(cyclo-C₃H₅)₂, -NHC(=NH)N(C₄H₉)₂, -NHC(=NH)N[CH(CH₃)₂]₂, -NHC(=NH)N[C(CH₃)₃]₂, alkyl carbamate substituents such as -OCONHCH₃, -OCONHC₂H₅, -OCONHC₃H₇, -OCONHC₄H₉, -OCONH-cyclo-C₃H₅, -OCONH[CH(CH₃)₂], -OCONH[C(CH₃)₃], -OCON(CH₃)₂, -OCON(C₂H₅)₂, -OCON(C₃H₇)₂, -OCON(C₄H₉)₂, -OCON(cyclo-C₃H₅)₂, -OCON[CH(CH₃)₂]₂, -OCON[C(CH₃)₃]₂, unsaturated alkoxy substituents such as -OC₂H₄CH=CH₂, -OCH₂-CH=CHCH₃, -OC₂H₄C≡CH, -OCH₂C≡CH, -OCH₂C≡CCH₃, -OC₃H₆CH=CH₂, -OC₂H₄CH=CH-CH₃, -OCH₂CH=CHC₂H₅, -OCH₂CH=CHCH=CH₂, -OC(CH₃)=CH-CH=CH₂, -OCH₂CH=C(CH₃)₂, -OC(CH₃)=C(CH₃)₂, -OC₃H₆C≡CH, -OC₂H₄C≡CCH₃, -OCH₂C≡CC₂H₅, -OCH₂C≡CCH=CH₂, -OCH₂CH=CHC≡CH, -OCH₂C≡CC≡CH, -OC₄H₈CH=CH₂, -CH=CHC₄H₉, -OC₃H₆CH=CHCH₃, -OCH₂CH=CHC₃H₇, -OC₂H₄-CH=CHC₂H₅, -OCH₂C(CH₃)=C(CH₃)₂, -OC₂H₄CH=C(CH₃)₂, -OC₄H₈C≡CH, -OC≡CC₄H₉, -OC₃H₆C≡CCH₃, -OCH₂C≡CC₃H₇, -OC₂H₄C≡CC₂H₅, alkenyl substituents and alkynyl substituents such as -C₂H₄CH=CH₂, -CH=CHC₂H₅, -CH=C(CH₃)₂, -CH₂CH=CHCH₃, -CH=CHCH=CH₂, -C₂H₄C≡CH, -C≡CC₂H₅, -CH₂C≡CCH₃, -C≡CCH=CH₂, -CH=CHC≡CH, -C≡CC≡CH, -C₃H₆CH=CH₂, -CH=CHC₃H₇, -C₂H₄CH=CHCH₃, -CH₂CH=CHC₂H₅, -CH₂CH=CHCH=CH₂, -CH=CHCH=CHCH₃, -CH=CHCH₂CH=CH₂, -C(CH₃)=CHCH=CH₂, -CH=C(CH₃)-CH=CH₂, -CH=CHC(CH₃)=CH₂, -CH₂CH=C(CH₃)₂, -C(CH₃)=C(CH₃)₂, -C₃H₆C=CH, -C≡CC₃H₇, -C₂H₄C≡CCH₃, -CH₂C≡CC₂H₅, -CH₂C≡CCH=CH₂, -CH₂CH=CHC≡CH, -CH₂C≡CC≡CH, -C≡CCH=CHCH₃, -CH=CHC≡CCH₃, -C≡CC≡CCH₃, -C≡CCH₂CH=CH₂, -CH=CHCH₂C≡CH, -C≡CCH₂C≡CH, -C(CH₃)=CHCH=CH₂, -CH=C(CH₃)CH=CH₂, -CH=CHC(CH₃)=CH₂, -C(CH₃)=CHC≡CH, -CH=C(CH₃)C≡CH, -C≡CC(CH₃)=CH₂, -C₄H₈CH=CH₂, -CH=CHC₄H₉, -C₃H₆CH=CH-CH₃, -CH₂CH=CH-C₃H₇, -C₂H₄CH=CH-C₂H₅, -CH₂C(CH₃)=C(CH₃)₂, -C₂H₄CH=C(CH₃)₂, -C₄H₈C≡CH, -C≡CC₄H₉, -C₃H₆C≡CCH₃, -CH₂C≡CC₃H₇, -C₂H₄C≡CC₂H₅, -CH=CHPh and -C=CPh. As used herein, an optionally substituted benzophenone substituent or an optionally substituted linker, in particular an optionally substituted benzophenone linker means a benzophenone substituent or benzophenone linker, respectively, which may optionally have any of the above substituents. It is to be understood that, with respect to further use, the skilled person would consider organic compounds having one or more benzophenone substituents that do not undergo side reactions in further use.

### Lactam, N-Vinyllactam

The term "**lactam**", as used herein, refers to cyclic organic chemical compounds that have an amide bond in the ring. Example compounds include 2-pyrrolidone, also known as γ-butyrolactam, β-lactam δ-lactam or ε-caprolactam. The term "***N*-vinyllactam**" as used herein, also referred to as "vinyllactam", refers to the aforementioned lactams, but having a vinyl group on the nitrogen atom. Examples of *N*-vinyllactams include, but are not limited to, *N*-vinyl-2-pyrrolidone (NVP) or *N*-vinylcaprolactam.

### Vinyl Group. Vinyl Compound

The term "**vinyl group**", as used herein, refers to the functional group -CH=CH₂. The term "**vinyl compound**", as used herein, refers to an organic compound having at least one vinyl group as a functional group. In the prior art vinyl compounds are commonly used as monomers in polymerization reactions.

### N-Vinylpyrrolidone (NVP)

The term "***N*-vinylpyrrolidone**", also *N*-vinyl-2-pyrrolidone or NVP, as used herein, refers to a heterocyclic organic compound having the following structural formula: 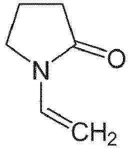 *N*-vinylpyrrolidone is widely used in the prior art in the production of polymers. *N*-vinylpyrrolidone is very reactive and can also polymerize spontaneously. Polymerization can also be initiated by UV irradiation. Due to its UV sensitivity, N-vinylpyrrolidone is used for the production of UV-curable inks, paints and coatings, and as a photoinitiator in other polymers. A variety of polymers can be prepared from N-vinylpyrrolidone, such as linear polyvinylpyrrolidones (PVP), crosslinked polyvinylpolypyrrolidone (PVPP) and numerous copolymers.

### N-Vinylpyrrolidone Moiety

The term "***N*-vinylpyrrolidone moiety**", as used herein, refers to a covalently bound *N*-vinylpyrrolidone monomer incorporated into a polymer chain. In the case of polyvinylpyrrolidone (PVP), the monomer is *N*-vinylpyrrolidone and the monomer moiety is an N-vinylpyrrolidone moiety having the following structural formula:

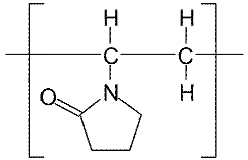

### Polyvinylpyrrolidon (PVP)

The term "**polyvinylpyrrolidone**" (PVP), as used herein, refers to a linear polymer that can be prepared by polymerization of N-vinylpyrrolidone (NVP). Thus, the polymer chain consists of many *N*-vinylpyrrolidone moieties linked together through covalent bonds. The N-vinylpyrrolidone moieties have the molecular formula C₆H₉NO and thus polyvinylpyrrolidone has the following general structural formula:

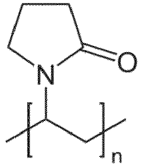

Alternative designations for polyvinylpyrrolidone include, but are not limited to, 1-ethenyl-2-pyrrolidone homopolymer, poly[1-(2-oxo-1-pyrrolidinyl)ethylene], povidone, polyvidone, 1-vinyl-2-pyrrolidinone polymer and additive E 1201. Polyvinylpyrrolidone is a hygroscopic, amorphous powder with a white to light yellow color and is readily soluble in water. Commercial designations are mainly povidone, PVP or Periston. A distinction is made here, for example, between Povidone K-30 and Povidone K-90. Povidone is also available under the trade name Kollidon^{®}. The K value is a classification commonly used in the plastics industry and is directly related to the average molar mass of the polymer. Thus, the K value can be used to indirectly infer the degree of polymerization and thus the chain length. Preferred povidones herein are povidone 90 and povidone 130. Also included herein as biodegradable polymers are the commercially available povidones Kollidon^{®} 90F with a molecular weight of 1000kDa - 1500kDa and Kollidon^{®} 30 with a molecular weight of 44kDa - 54 kDa.

Polyvinylpyrrolidone (PVP) is hydrophilic, biocompatible and non-toxic, dissolves in solvents of different polarity, exhibits excellent binding properties and shows a stabilizing effect in suspensions and emulsions. Polyvinylpyrrolidone (PVP) is used in the prior art for a variety of industrial applications, such as in personal care or cosmetic products e.g. as a thickening agent in shampoo or toothpaste, in pharmaceutical products as an excipient, binder or to increase viscosity e.g. in eye drops, as a coating component for medical products, in the food industry as a binding and thickening agent, stabilizer and coating agent and further e.g. as an additive in adhesives, in the production of batteries, ceramics, glass fibers, ink, for the production of membranes e.g. as dialysis or water treatment filters, as a binding and complexing agent in the agricultural industry or also for contact lenses and in contact lens solutions as a lubricant or wetting agent to reduce friction.

The polyvinylpyrrolidone homopolymer is a linear polymer and can be prepared by homopolymerization of *N*-vinylpyrrolidone (NVP). It is known in the art that the physical properties of the polyvinylpyrrolidone homopolymer can be varied by selective incorporation of one or more comonomers. Comonomers known from the prior art include, but are not limited to, *N*-vinylimidazole, vinyl acetate and vinylcaprolactam. For special applications, such as reducing hydrophilicity, hydrophobic comonomers, such as styrene, can also be used to provide a more hydrophobic and thus less water-soluble PVP copolymer.

### Vinylpyrrolidone Copolymer

The term "**vinylpyrrolidone copolymer**", as used herein, refers to a copolymer in which *N*-vinylpyrrolidone (NVP) is used as the main monomer. The properties of vinylpyrrolidone homopolymers (PVP) can be selectively varied by incorporating comonomers into the polymer chain. Examples of prior art comonomers include *N*-vinylimidazole, vinyl acetate and vinylcaprolactam. Vinylpyrrolidone/vinyl acetate copolymers can be used, for example, as thickeners and hydrophilic hot melt adhesives.

### Blocks of N-Vinylpyrrolidone moieties

The term "**blocks of N-vinylpyrrolidone moieties**", refers to segments in the polymer chain of a vinylpyrrolidone copolymer which are composed only of *N*-vinylpyrrolidone moieties and therefore locally exhibit the physical and mechanical properties of the polyvinylpyrrolidone homopolymer. Blocks of *N*-vinylpyrrolidone moieties herein preferably comprise at least two, more preferably at least three, further preferably at least four, even more preferably at least five, further preferably at least six, even more preferably at least seven, and further preferably at least eight identical monomer moieties. A copolymer of blocks of *N*-vinylpyrrolidone moieties divided by maleic acid moieties is characterized in that the polymer chain contains only blocks of *N*-vinylpyrrolidone moieties and no blocks of maleic acid moieties are present. Therefore, in a copolymer of blocks of *N*-vinylpyrrolidone moieties divided by maleic acid moieties, the maleic acid moieties have only covalent linkages to the *N*-vinylpyrrolidone moieties and no maleic acid moieties covalently linked to each other are present in the polymer chain of the vinylpyrrolidone copolymer. Preferably, the *N*-vinylpyrrolidone monomer is the main monomer and the maleic acid monomer is the comonomer.

### α,β-unsaturated carboxylic acid monomer

The term "**α,β-unsaturated carboxylic acid monomer**", as used herein, generally refers to α,β-unsaturated monocarboxylic acids, α,β-unsaturated dicarboxylic acids, α,β-unsaturated monocarboxylic acid amides, α,β-unsaturated monocarboxylic acid esters, α,β-unsaturated dicarboxylic acid amides, α,β-unsaturated dicarboxylic acid esters and α,β-unsaturated dicarboxylic acid anhydrides as well as derivatives of these compounds. Examples of α,β-unsaturated carboxylic acid monomers include, but are not limited to acrylic acid, methacrylic acid, ethacrylic acid, α-chloroacrylic acid, crotonic acid, maleic acid, itaconic acid, citraconic acid, mesaconic acid, cis/trans-glutaconic acid, aconitic acid, fumaric acid and mixtures thereof. The monomers also include the salts of the above acids, in particular the sodium, potassium and ammonium salts and the salts with amines. The monomers can be used as such or as mixtures of one another.

An α,β-unsaturated monocarboxylic acid monomer has the general formula: 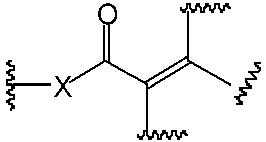 wherein X is -O-, -NH-, or -N-.

Suitable α,β-monoolefinically unsaturated monocarboxylic acids include, but are not limited to, acrylic acid, methacrylic acid, ethacrylic acid, α-chloroacrylic acid, and/or crotonic acid. In some embodiments, the α,β-unsaturated carboxylic acid monomer is preferably selected from an α,β-unsaturated monocarboxylic acid selected from the group consisting of or including acrylic acid, methacrylic acid, ethacrylic acid, α-chloroacrylic acid, crotonic acid, and derivatives thereof. Particularly preferred are α,β-unsaturated monocarboxylic acid selected from the group comprising or consisting of acrylic acid and/or methacrylic acid and derivatives thereof.

Suitable α,β-monoolefinically unsaturated monocarboxylic acid amides include, but are not limited to, methyl acrylamides, methyl methacrylamides, N-methylol acrylamides, N-methylol methacrylamides, N-butoxymethyl (meth)acrylamides, N-isobutoxymethyl(meth)acrylamides, tetrahydrofurfuryl-2-acrylamides, tetrahydrofurfuryl-2-methacrylamides, and derivatives thereof.

Suitable α,β-monoolefinically unsaturated monocarboxylic acid esters include, but are not limited to, alkoxy group-containing monomers such as 3-methoxybutyl methacrylate, 3-methoxybutyl acrylate, 2-methoxyethyl methacrylates, 2-methoxyethyl acrylate, 2-butoxyethyl acrylate, 2-butoxyethyl methacrylates, 2-ethoxyethyl acrylates, 2-ethoxyethyl methacrylate, tetrahydrofurfuryl-2-acrylate, tetrahydrofurfuryl-2-acrylate, tetrahydrofurfuryl-2-methacrylate, methyl acrylate, methyl methacrylate, methyl ethacrylate, ethyl methacrylate, ethyl acrylate, ethyl ethacrylate, n-propyl methacrylate, n-propyl acrylate, isopropyl methacrylate, isopropyl acrylate, n-butyl acrylate, n-butyl methacrylate, tert-butyl methacrylate, tert-butyl acrylate, n-pentyl methacrylate, n-pentyl acrylate, n-hexyl methacrylate, n-hexyl acrylate, n-heptyl methacrylate, n-heptyl acrylate, n-octyl methacrylate, n-octyl acrylate, 1,1,3,3-tetramethylbutyl acrylate, 1,1,3,3-tetramethylbutyl methacrylate, ethylhexyl acrylate, ethylhexyl methacrylate, n-nonyl methacrylate, n-nonyl acrylate, n-decyl methacrylate, n-decyl acrylate, n-undecyl acrylate, n-undecyl methacrylate, tridecyl methacrylate, tridecyl acrylate, myristyl methacrylate, myristyl acrylate, pentadecyl methacrylate, pentadecyl acrylate, palmityl methacrylate, palmityl acrylate, heptadecyl acrylate, heptadecyl methacrylate, nonadecyl methacrylate, nonadecyl acrylate, arrachinyl acrylate, arrachinyl methacrylate, behenyl methacrylate, behenyl acrylate, lignocerenyl methacrylate, lignocerenyl acrylate, cerotinyl acrylate, cerotinyl methacrylate, melissinyl (meth)acrylate, palmitoleinyl methacrylate, palmitoleinyl acrylate, oleyl methacrylate, oleyl acrylate, linolylacrylate, linolyl methacrylate, linolenyl methacrylate, linolenyl acrylate, stearyl methacrylate, stearyl acrylate, lauryl methacrylate and lauryl acrylate.

Suitable α,β-monoolefinically unsaturated secondary and tertiary monocarboxylic acid amides include, but are not limited to, N,N-dimethylacrylamides, N,N-dimethyl methacrylates, N,N-dimethylacrylates, N,N-diethylamino-ethyl-acrylamides, N,N-diethylamino-ethyl-methacrylamides, N,N-diisopropyl-acrylamides, N,N-diisopropyl-methacrylamides, N,N-dibutyl acrylates, N,N-dibutyl-acrylamides, N,N-dibutyl-methacrylamides, N,N-dibutyl methacrylates, diacetone acrylamides, N-tert-butyl methacrylamides, N-tert-butyl acrylamides, diacetone methacrylamides, N-(1-methylundecyl) methacrylamides, N-(1-methylundecyl)-acrylamides, N-isobornylacrylam ides, N-isobornylmethacrylam ides, N-benzyl-acrylam ides, N-adamantyl-methacrylamides, acrylamidoacetic acids, N-adamantyl-acrylamides, N-benzyl-methacrylamides, N-4-methylphenylacrylamides, methylmethacrylamides, methylacrylamides, acrylamidoacetic esters, N-diphenylmethylacrylamides, phthalimidomethylmethacrylamides, phthalimidomethylacrylamides, acrylamidohydroxyacetic acids, methacrylamidohydroxyacetic acids, methacrylamidoacetic acids, methacrylamidoacetic esters, methyl methacrylamidoacetate, methyl acrylamidoacetate, 2-methacrylamido-2-methylbutyric acid, 2-acrylamido-2-methylbutyric acid, N-(2,2,2-trichloro-1-hydroxy)ethylacrylamide, N-(2,2,2-trichloro-1-hydroxy)-ethylmethacrylamide, N,N-bis(2-cyanoethyl)methacrylamide, N-(3-hydroxy-2,2-di-methylpropyl)-acrylamide, N-(3-hydroxy-2,2-di-methylpropyl)-methacrylamide, dialkylmethacrylamide, dialkylacrylamide, p-hydroxyacrylic anilide and p hydroxy-methacrylic anilide.

Other suitable α,β-monoolefinically unsaturated monocarboxylic acid monomers include, but are not limited to, 2-hydroxy-3-[N,N-di(2-hydroxyethyl)]-propyl methacrylate, 2-hydroxy-3-[N,N-di(2-hydroxyethyl)]-propyl acrylate, 2-methoxy-3-[N,N-di(2-hydroxyethyl)]-propyl acrylate, cyclohexyl methacrylate, 2-methoxy-3-[N,N-di(2-hydroxyethyl)]-propyl methacrylate, cyclohexyl acrylate 2-hydroxy-3-[N-hydroxyethyl-N-alkyl]-propyl methacrylate, furfuryl acrylate, 2-hydroxy-3-[N-hydroxyethyl-N-alkyl]-propyl acrylate, methacrylate, 2-hydroxy-3-[N-hydroxyethyl-N-methyl]-propyl methacrylate, furfuryl methacrylate, 2-hydroxy-3-[N-hydroxyethyl-N-methyl]-propyl acrylate, isobornyl acrylate, 2-hydroxy-3-[N-ethyl-N-methyl]-propyl methacrylate, 3-cyclohexylpropyl-1-acrylate, 3-cyclohexyl propyl-1-methacrylate, 4-tert-butyl-cyclohexyl methacrylate, 2-N-morpholinoethyl acrylate, 2-N-morpholinoethyl methacrylate, 4-tert-butylcyclohexyl acrylate, 2-N-morpholino-N-hexyl (meth)acrylate, N-cyclohexyl methacrylate, N-cyclohexyl acrylate, N-isobornyl acrylate, and N-isobornyl methacrylate.

Other suitable α,β-monoolefinically unsaturated monocarboxylic acid derivatives include, but are not limited to, butyl acrylate, methyl acrylate, ethyl acrylate, propyl acrylate, octyl acrylate, heptyl acrylate, nonyl acrylate, Hexyl acrylate, n-hexyl acrylate, isopropyl acrylate, isobutyl acrylate, decyl acrylate, isodecyl acrylate, isodecyl acrylate, lauryl acrylate, stearyl acrylate, behenyl acrylate, mellissyl acrylate, methoxyethyl acrylate, hydroxyethyl acrylate, hydroxyethyl acrylate, glycidyl acrylate, 2-ethylhexyl acrylate, 2-ethoxyethyl acrylate, ethylene glycol diacrylate, propylene glycol diacrylate, diethylene glycol diacrylate, polyethylene glycol diacrylate, 1,5-pentanediol diacrylate, neopentyl glycol diacrylate, 1,6-hexanediol acrylate, 1,6-hexanediol diacrylate, polyethylene glycol diacrylate, polypropylene glycol diacrylate, pentaerythritol triacrylate, trimethylolpropane triacrylate, trimethylolpropane dipentaerythritol pentaacrylate, n-butyl acrylate, benzoin acrylate, triethylene glycol diacrylate, pentaerythritol tetraacrylate, glyceryl propoxy triacrylate, 1,3-propylene glycol diacrylate, tripropylene glycol diacrylate, 1,3-butylene glycol diacrylate, 1,4-butanediol diacrylate, diethylene glycol acrylate, butyl methacrylate, triethylene glycol diacrylate, tetraethylene glycol diacrylate, methyl methacrylate, ethyl methacrylate, propyl methacrylate, octyl methacrylate, heptyl methacrylate, nonyl methacrylate, hexyl methacrylate, n-hexyl methacrylate, isopropyl methacrylate, isobutyl methacrylate, decyl methacrylate, isodecyl methacrylate, isodecyl methacrylate, stearyl methacrylate, glycidyl methacrylate, mellissyl methacrylate, lauryl methacrylate, hydroxyethyl methacrylate, methoxyethyl methacrylate, behenyl methacrylate, 2-ethylhexyl methacrylate, 2-ethoxyethyl methacrylate, ethylene glycol dimethacrylate, propylene glycol dimethacrylate, n-butyl methacrylate, diethylene glycol dimethacrylate, polyethylene glycol dimethacrylate, 1,5-pentanediol dimethacrylate, benzoin methacrylate, neopentyl glycol dimethacrylate, 1,6-hexanediol methacrylate, methyl ethacrylate, 1,6-hexanediol dimethacrylate, polyethylene glycol dimethacrylate, polypropylene glycol dimethacrylate, pentaerythritol trimethacrylate, trimethylolpropane trimethacrylate, trimethylolpropane dipentaerythritol pentamethacrylate, pentaerythritol tetramethacrylate, triethylene glycol dimethacrylate, glyceryl propoxy tri(meth)acrylate, 1,3-propylene glycol dimethacrylate, tripropylene glycol dimethacrylate, 1,3-butylene glycol dimethacrylate, 1,4-butanediol dimethacrylate, diethylene glycol dimethacrylate, triethylene glycol dimethacrylate, tetraethylene glycol dimethacrylate, ethoxylated bisphenol A-dimethacrylate, 1,12-dodecanediol diacrylate, 1,12-dodecanediol dimethacrylate, 1,3-butylene glycol diacrylate, 1,3-butylene glycol dimethacrylate, 1,4-butanediol diacrylate, 1,4-butanediol dimethacrylate, 1,6-hexanediol diacrylate, 1,6-hexanediol dimethacrylate, alkoxylated cyclohexanedimethanol diacrylates, alkoxylated cyclohexanedimethanol dimethacrylates, alkoxylated hexanediol diacrylates, alkoxylated hexanediol dimethacrylates, alkoxylated neopentyl glycol diacrylates, alkoxylated neopentyl glycol dimethacrylates, cyclohexane dimethanol diacrylate, cyclohexane dimethanol dimethacrylate, diethylene glycol diacrylate, diethylene glycol dimethacrylate, dipropylene glycol diacrylate, dipropylene glycol dimethacrylate, ethoxylated bisphenol A diacrylates, ethoxylated bisphenol A dimethacrylates, ethylene glycol diacrylate, ethylene glycol dimethacrylate, neopentyl glycol diacrylate, neopentyl glycol dimethacrylate, polyethylene glycol diacrylates, polyethylene glycol dimethacrylates, propylene glycol diacrylates, propylene glycol dimethacrylates, propoxylated neopentyl glycol diacrylates, propoxylated neopentyl glycol dimethacrylates, tetraethylene glycol diacrylate, tetraethylene glycol dimethacrylate, triethylene glycol diacrylate, triethylene glycol dimethacrylate, tripropylene glycol diacrylate, tripropylene glycol dimethacrylate, ethoxylated trimethylolpropane triacrylates, ethoxylated trimethylolpropane trimethacrylates, propoxylated glyceryl triacrylates, propoxylated glyceryl trimethacrylates, trimethylolpropane triacrylate, trimethylolpropane trimethacrylate, Pentaerythritol triacrylate, pentaerythritol trimethacrylate, propoxylated trimethylolpropane triacrylates, propoxylated trimethylolpropane trimethacrylates, tris(2-hydroxyethyl)iso-cyanurate triacrylate, tris(2-hydroxyethyl)isocyanurate trimethacrylate, caprolactone acrylates, caprolactone methacrylates, caprolactamacrylamides, caprolactamacrylamide, caprolactammethacrylamide, valerolactone acrylates, valerolactone methacrylates, valerolactamacrylamides, valerolactammethacrylamides, butyrolactone acrylates, butyrolactone methacrylates, butyrolactamacrylamides, butyrolactammethacrylamides, propiolactone acrylates, propiolactone methacrylates, propiolactamacrylamides, propio-lactamacrylamides, propio¬lactammethacrylamides, acrylic acid, methacrylic acid, 2-(2-ethoxyethoxy)ethyl acrylate, 2-(2-ethoxyethoxy)ethyl methacrylate, 2-phenoxyethyl acrylate, 2-phenoxyethyl methacrylate, 3,3,5-trimethylcyclohexyl acrylate, 3,3,5-tri-methylcyclohexyl methacrylate, alkoxylated lauryl acrylates, alkoxylated lauryl methacrylates, alkoxylated phenol acrylates, alkoxylated phenol methacrylates, alkoxylated tetrahydrofurfuryl acrylates, alkoxylated tetrahydrofurfuryl methacrylates, lauryl acrylate, lauryl methacrylate, cyclic trimethylolpropane formal acrylate, cyclic trimethylolpropane formal methacrylate, dicyclopentadienyl acrylate, dicyclopentadienyl methacrylate, diethylene glycol methyl ether acrylate, diethylene glycol methyl ether methacrylate, ethoxylated hydroxyethyl acrylates, ethoxylated hydroxyethyl methacrylates, ethoxylated nonylphenol acrylates, ethoxylated nonylphenol methacrylates, isobornyl acrylate, isobornyl methacrylate, isodecyl acrylate, isodecyl methacrylate, isoyl acrylate, isoocyl acrylate, isoocyl methacrylate, metal acrylates, metal methacrylates, methoxypolyethylene glycol acrylates, methoxypolyethylene glycol methacrylates, octyldecyl acrylate, octyldecyl methacrylate, polypropylene glycol acrylates, polypropylene glycol methacrylates, propoxylated allyl acrylates, propoxylated allyl methacrylates, stearyl acrylate, stearyl methacrylate, tetrahydrofurfuryl acrylate, tetrahydrofurfuryl methacrylate, tridecyl acrylate, tridecyl methacrylate, triethylene glycol ethyl ether acrylate, triethylene glycol ethyl thermethacrylate, 1,12-dodecanediol diacrylate, 1,12-dodecanediol dimethacrylate, 1,3-butylene glycol diacrylate, 1,3-butylene glycol dimethacrylate, 1,4-butanediol diacrylate, 1,4-butanediol dimethacrylate, 1,6-hexane diol diacrylate, 1,6-hexane diol dimethacrylate, alkoxylated cyclohexane dimethanol diacrylates, alkoxylated hexane diol diacrylates, alkoxylated hexane diol dimethacrylates, alkoxylated neopentyl glycol diacrylates, alkoxylated neopentyl glycol dimethacrylates, cyclohexane dimethanol diacrylate, cyclohexane dimethanol dimethacrylate, diethylene glycol diacrylate, diethylene glycol dimethacrylate, dipropylene glycol diacrylate, dipropylene glycol dimethacrylate, ethoxylated bisphenol A-diacrylates, ethoxylated bisphenol A-dimethacrylates, Ethylene glycol diacrylate, ethylene glycol dimethacrylate, neopentyl glycol diacrylate, neopentyl glycol dimethacrylate, polyethylene glycol diacrylates, polyethylene glycol dimethacrylates, propylene glycol diacrylates, propylene glycol di-methacrylates, propoxylated neopentyl glycol diacrylates, propoxylated neopentyl glycol dimethacrylates, tetraethylene glycol diacrylate, tetraethylene glycol dimethacrylate, triethylene glycol diacrylate, triethylene glycol dimethacrylate, tripropylene glycol diacrylate, tripropylene glycol dimethacrylate, ethoxylated trimethylolpropane triacrylates, ethoxylated trimethylolpropane trimethacrylates, propoxylated glyceryl triacrylates, propoxylated glyceryl trimethacrylates, trimethylolpropane triacrylate, trimethylolpropane trimethacrylate, pentaerythritol triacrylate, pentaerythritol trimethacrylate, propoxylated trimethylolpropane triacrylate, propoxylated trimethylolpropane trimethacrylate, tris(2-hydroxyethyl)isocyanurate triacrylate, tris(2-hydroxy-ethyl)isocyanurate trimethacrylate, di-trimethylolpropane tetraacrylate, dipentaerythritol pentaacrylate, ethoxylated pentaerythritol tetraacrylates, dipentaerythritol pentaacrylate and pentaerythritol tetraacrylate.

Examples of polymerizable monofunctional photo- and/or thermosensitive α,β-unsaturated carboxylic acid monomers, which can also be used as photoinitiators, are generally acrylic acids (AA), methacrylic acids (MA), methacrylates and acrylamides such as 2-hydroxymethyl methacrylate (HEMA), N,N-dimethylacrylamide (DMA), diisobutylacrylamide (DBA), aminopropyl methacrylamide (APMA), N,N-diiso¬butylacrylamide (DBA), 9-anthracenylmethyl methacrylate, N-[3-methacrylamido)-propyl]-2-(carboxymethyl)-hexadecanamide, N-[3-methacryl¬amido)-propyl]-3-carboxyheptadecanamide. Other examples of such polymerizable monofunctional photo- and/or thermosensitive α,β-unsaturated carboxylic acid monomers, which may also be used as photoinitiators, include, but are not limited to dipentaerythritol pentaacrylate (PPA), thoxylated trimethylolpropane triacrylates (TTA) N,N'-diethyl-1,3-propylene bisacrylamide (EPA), and 2,2,4-trimethyl-6-[2-(2-methylacryl-oyloxy)-ethoxycarbonylamino]-hexyl-carbamoyl-oxy-ethyldimethacrylate (UDMA).

An α,β-unsaturated dicarboxylic acid monomer has the general formula: 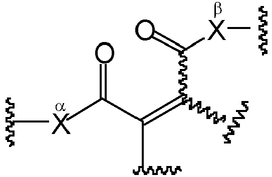 wherein X^{α} and X^{β} independently of each other represent -O-, -NH- or -N-.

Examples of α,β-unsaturated dicarboxylic acid monomers include, but are not limited to, maleic acid, itaconic acid, citraconic acid, mesaconic acid, glutaconic acid, aconitic acid, and/or fumaric acid. Monomers also include the salts of the above acids, in particular the sodium, potassium and ammonium salts and the salts with amines. The monomers can be used as such or as mixtures with one another.

Preferred α,β-monoolefinically unsaturated dicarboxylic acids include, but are not limited to, maleic acid, itaconic acid, citraconic acid, mesaconic acid, glutaconic acid, aconitic acid and/or fumaric acid. Particularly preferred herein are maleic acid and derivatives thereof. An α,β-unsaturated carboxylic acid monomer may be preferably selected from an α,β-unsaturated dicarboxylic acid or a derivative thereof, preferably selected from maleic acid, itaconic acid, citraconic acid, mesaconic acid, glutaconic acid, aconitic acid and/or fumaric acid and/or derivatives thereof.

Examples of α,β-monoolefinically unsaturated dicarboxylic acid esters include, but are not limited to, n-butyl maleic acid monoester, fumaric acid mono-ethyl ester, maleic acid monomethyl ester, maleic acid monoethyl ester, maleic acid monopropyl ester, maleic acid monobutyl ester, maleic acid monobenzyl ester, maleic acid mono(2-ethylhexyl) ester, maleic acid monohydroxyethyl ester, maleic acid monohydroxypropyl, fumaric acid monomethyl ester, fumaric acid mono-ethyl ester, fumaric acid monopropyl ester, fumaric acid monobutyl ester, fumaric acid monobenzyl ester, fumaric acid mono(2-ethylhexyl) ester, fumaric acid monohydroxyethyl ester, fumaric acid monohydroxypropyl, maleic acid dimethyl ester, maleic acid diethyl ester, maleic acid dipropyl ester, maleic acid dibutyl ester, maleic acid monobenzyl ester, maleic acid di(2-ethylhexyl) ester, maleic acid dihydroxyethyl ester, maleic acid dihydroxypropyl, fumaric acid dimethyl ester, fumaric acid monoethyl ester, fumaric acid dipropyl ester, fumaric acid monobutyl ester, fumaric acid dibenzyl ester, fumaric acid di(2-ethylhexyl) ester, fumaric acid dihydroxyethyl ester, fumaric acid monohydroxypropyl ester, itaconic acid monomethyl ester, itaconic acid monoethyl ester, itaconic acid monopropyl ester, itaconic acid monobutyl ester, itaconic acid monobenzyl ester, itaconic acid mono(2-ethylhexyl) ester, Itaconic acid monohydroxyethyl ester, itaconic acid monohydroxypropyl, itaconic acid dimethyl ester, itaconic acid diethyl ester, itaconic acid dipropyl ester, itaconic acid dibutyl ester, itaconic acid dibenzyl ester, itaconic acid di(2-ethylhexyl) ester, itaconic acid dihydroxyethyl ester, itaconic acid dihydroxypropyl ester, citraconic acid monomethyl ester, citraconic acid monoethyl ester, citraconic acid monopropyl ester, citraconic acid monobutyl ester, citraconic acid monobenzyl ester, citraconic acid mono(2-ethylhexyl) ester, citraconic acid monohydroxyethyl ester, citraconic acid monohydroxypropyl, citraconic acid dimethyl ester, citraconic acid diethyl ester, citraconic acid dipropyl ester, citraconic acid dibutyl ester, citraconic acid dibenzyl ester, citraconic acid di(2-ethylhexyl) ester, citraconic acid dihydroxyethyl ester, citraconic acid dihydroxypropyl ester, mesaconic acid monomethyl ester, mesaconic acid monoethyl ester, mesaconic acid monopropyl ester, mesaconic acid monobutyl ester, mesaconic acid monobenzyl ester, mesaconic acid mono(2-ethylhexyl) ester, mesaconic acid monohydroxyethyl ester, mesaconic acid monohydroxypropyl, mesaconic acid dimethyl ester, mesaconic acid diethyl ester, citraconic acid dipropyl ester, mesaconic acid dibutyl ester, mesaconic acid dibenzyl ester, mesaconic acid di(2-ethylhexyl) ester, mesaconic acid dihydroxyethyl ester, mesaconic acid dihydroxypropyl ester, glutaconic acid monomethyl ester, glutaconic acid monoethyl ester, glutaconic acid monopropyl ester, glutaconic acid monobenzyl ester, glutaconic acid mono(2-ethylhexyl) ester, glutaconic acid monohydroxyethyl ester, glutaconic acid monohydroxypropyl, glutaconic acid dimethyl ester, glutaconic acid diethyl ester, glutaconic acid dipropyl ester, glutaconic acid dibutyl ester, glutaconic acid dibenzyl ester, glutaconic acid di(2-ethylhexyl) ester, glutaconic acid dihydroxyethyl ester, glutaconic acid dihydroxypropyl, aconitic acid monomethyl ester, aconitic acid monoethyl ester, aconitic acid monopropyl ester, aconitic acid monobutyl ester, aconitic acid mono(2-ethylhexyl) ester, aconitic acid monohydroxyethyl ester, aconitic acid monohydroxypropyl, aconitic acid dimethyl ester, aconitic acid diethyl ester, aconitic acid dipropyl ester, aconitic acid dibutyl ester, aconitic acid dibenzyl ester, aconitic acid di(2-ethylhexyl) ester, aconitic acid dihydroxyethyl ester, aconitic acid dihydroxypropyl ester, furthermore 3-methoxybutyl, 2-methoxyethyl, 2-butoxyethyl, 2-ethoxyethyl, tetrahydrofurfuryl-2, n-propyl, isopropyl, n-butyl, tert-butyl, n-pentyl, n-hexyl, n-heptyl, n-octyl, 1,1,3,3-tetramethylbutyl, ethylhexyl, n-nonyl, n-decyl, n-undecyl, tridecyl, myristyl, pentadecyl, palmityl, heptadecyl, nonadecyl, arrachinyl, behenyl, lignocerenyl, cerotinyl, melissinyl, palmitoleinyl, oleyl, linolyl, linolenyl, stearyl, lauryl mono and diesters of maleic acid, itaconic acid, citraconic acid, mesaconic acid, glutaconic acid, aconitic acid and fumaric acid.

Examples of α,β-monoolefinically unsaturated dicarboxylic acid anhydrides include, but are not limited to, maleic anhydride and maleic anhydride derivatives.

Preferred α,β-monoolefinically unsaturated dicarboxylic acid amides include, but are not limited to, maleic acid mono- and diamides, itaconic acid mono- and diamides, fumaric acid mono- and diamides, N,N-dimethylamides, N,N-diethylamino-ethyl-amides, N,N- diisopropyl-amides, N,N-diisopropyl-amides, N,N-dibutyl-amides, N,N-dibutyl-amides, N-tert-butyl-amides, diacetone-amides, N-(1-methylundecyl)-amides, N-(1-methylundecyl)-amides, N-isobornylamides, N-adamantyl-amides, N-benzyl-amides, N-4-methylphenylamides, methylamides, N-diphenylmethylamides, phthalimidomethylamides, phthalimidoamides, amidohydroxyacetic acidsAmidoacetic acids, amidoacetic acid esters, amidoacetic acid methyl esters, N-(2,2,2-trichloro-1-hydroxy)-ethylamides, N-(2,2,2-trichloro-1-hydroxy)¬ethylamides, N,N-bis(2-cyanoethyl)amides, N-(3-hydroxy-2,2-di-methylpropyl)amides, dialkylamides of maleic acid, itaconic acid, citraconic acid, mesaconic acid, glutaconic acid, aconitic acid and fumaric acid.

### Maleic Acid

The term "**maleic acid**", also referred to as cis-butenedioic acid or cis-ethylenedicarboxylic acid, as used herein, refers to a hydrophilic dicarboxylic acid that is readily soluble in water and has the chemical formula HO₂CCH=CHCO₂H: 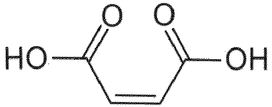 Maleic acid can undergo reactions at the carboxy groups on the one hand and reactions at the double bond on the other hand. Typical maleic acid derivatives are maleic acid esters and maleic acid amides. Maleic acid or maleic acid derivatives are used in the prior art to produce polymers, synthetic resins e.g. for refining and dyeing cotton.

The double bond of maleic acid can participate in polymerization reactions. For example, vinylpyrrolidone copolymers with maleic acid moieties can be obtained using N-vinylpyrrolidone (NVP) as the main monomer. For the copolymerization, in addition to maleic anhydride, an α,β-unsaturated maleic acid derivative can also be used as comonomer, e.g. a monoester, a diester, a monoamide or even a diamide of maleic acid, while retaining the double bond function to remain available for copolymerization with N-vinylpyrrolidone (NVP). Thus, maleic acid can first be converted to a suitable maleic acid derivative, which can be used as a comonomer in the copolymerization with N-vinylpyrrolidone (NVP). However, maleic anhydride can also be used first as a comonomer in the copolymerization with N-vinylpyrrolidone (NVP), in which case the carboxy groups can be further functionalized following the preparation of the vinylpyrrolidone copolymer.

Since maleic acid has functional groups with different reactivity - the carboxy groups and the double bond maleic acid can also serve as a linker to connect two or more compounds via covalent bonds.

Maleic acid homopolymers as well as maleic acid copolymers are known in the art, e.g. copolymers of maleic acid and styrene, styrene sulfonic acid, acrylic acid or methyl vinyl ether are commercially available. On average, these copolymers have a maleic acid content of 25-50 mol%. Since maleic acid has two carboxyl groups, such copolymers represent polyelectrolyte polymers that can be used, for example, to produce membranes. Copolymers functionalized at the carboxy group are also known in the prior art, such as poly-(styrene-co-maleic acid) with isobutyl ester or isobutyl/methyl ester functionalizations, e.g. poly-(styrene-co-maleic acid) with isobutyl ester functionalization is commercially available and has a molar ratio of 1.5:1 styrene/maleic acid with about half of the carboxyl groups present as isobutyl esters. The distribution of the isobutyl ester groups is not homogeneous, so that hydrophilic and hydrophobic regions are present in the polymer chain, which can lead to problems in forming a uniform film.

As comonomers, maleic acid and maleic acid derivatives offer the advantage that, due to steric hindrance, the ability to autopolymerize is limited and thus, advantageously, no local "maleic acid accumulations" occur in e.g. vinylpyrrolidone copolymers and, ultimately, a uniform distribution of the maleic acid moieties in the polymer chain is achieved. This provides, among other things, uniform and improved solubility in polar solvents. In addition, maleic acid offers the advantage that the carboxy groups can be functionalized with photoreactive benzophenone substituents, with which crosslinking of the polymer chain of the vinylpyrrolidone copolymer can be achieved after copolymerization, but also crosslinking to surfaces and thus binding to surfaces can be increased, resulting in improved and stable adhesion to various surfaces and materials.

### Maleic Acid Monomer

The term "**maleic acid monomer**", as used herein, refers to an α,β-unsaturated maleic acid derivative that can be used as a comonomer for copolymerization with N-vinylpyrrolidone as the main monomer to prepare vinylpyrrolidone copolymers, in particular vinylpyrrolidone copolymers composed of blocks of N-vinylpyrrolidone (NVP) moieties divided by maleic acid moieties. Examples maleic acid comonomers include, but are not limited to, maleic anhydride, maleic acid monoesters, maleic acid diesters, maleic acid monoamides, maleic acid monoamides and maleic acid esteramides: 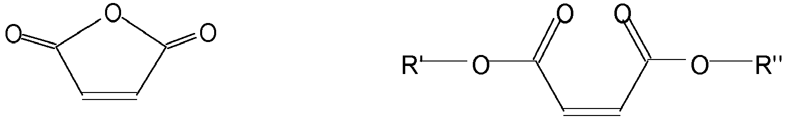 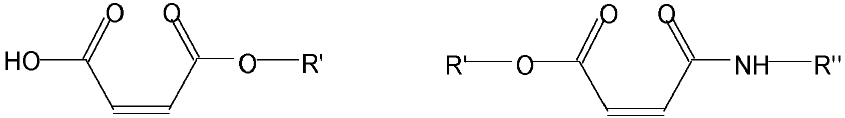 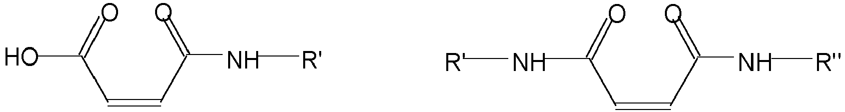 wherein R' and R" are as defined herein.

Furthermore, maleic acid derivatives substituted on the double bond, such as dimethylmaleic acid or dimethylmaleic anhydrides can also be used:

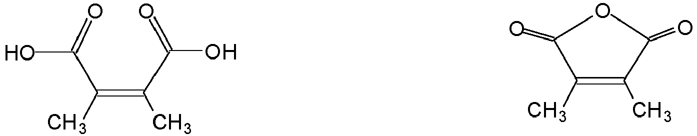

Thus, examples maleic acid derivatives that have subtituents at the double bond include, but are not limited to: 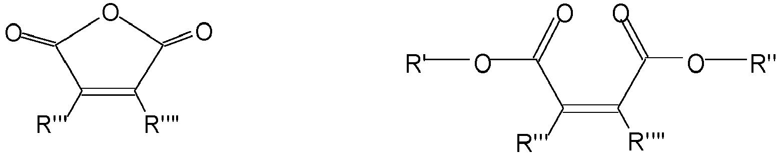 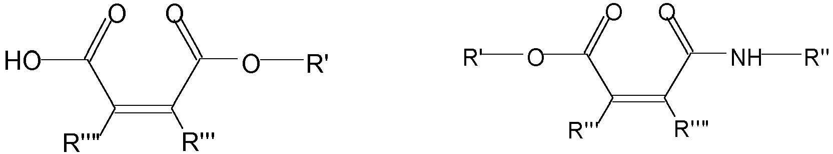 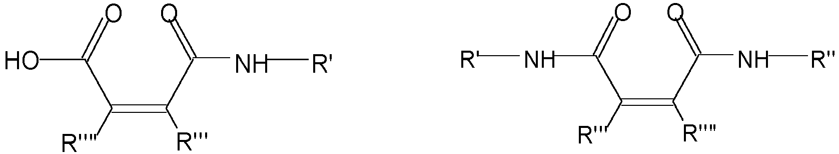 wherein R' and R" are as defined herein, and wherein R‴ and R‴′ may independently of each other represent -H, -CH₃, -CH₂CH₃, or -F.

### Maleic Acid Moiety

The term "**maleic acid moiety**", as used herein, refers to a maleic acid monomer covalently bound in a polymer chain. A maleic acid moiety having a carboxy group (-COO-) and a carbamoyl group (-CONH-) as functional groups may be represented as follows:

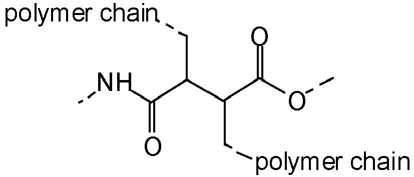

A maleic acid moiety having two carboxy groups (-COO-) or two carbamoyl groups (-CONH-) as functional groups can be represented as follows:

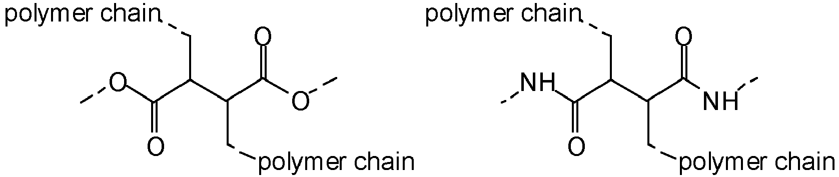

Maleic acid moieties may have as residues R' and R", a benzophenone substituent (BP), but non-benzophenone residues may be also present, which may be selected from polyethylene glycol, polactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl and an organometallic group:

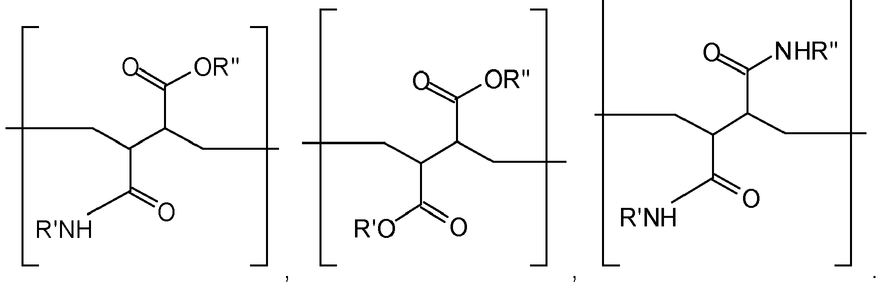

### Medical Device and Medical Aid

The term "**medical device**", as used herein, refers to "objects or substances that serve to detect, prevent, monitor, treat or alleviate diseases, but achieve this purpose primarily ("intended main effect") by physical means and not by pharmacological/immunological means or by metabolic action (Schorn G. MPG Medizinproduktegesetz, 4th edition, Wissenschaftliche Verlagsgesellschaft, Stuttgart, 2009). However, the physical effect of medical devices can certainly be supported by pharmacological, immunological or metabolic effects".

The term "**medical aid**", as used herein, refers to objects that are necessary to ensure the success of a medical treatment, to prevent an impending disability or to compensate for a disability, insofar as they are not to be regarded as general objects of daily use (Definition of the SGB V). Medical aids may mainly be used by the person concerned on his or her own responsibility.

Examples of medical devices and medical aids that can be used in or on the body for short or long periods of time, include, but are not limited to, balloon catheters, bladder catheters, stomach tubes, endoscopes, colonoscopes, needles, cannulas, endotracheal tubes, tracheal tubes, and the like, implants such as stents, eye lenses, joint prostheses, screws, fixations, artificial exits, nasal implants, vocal cords, larynx, artificial and native valves, bone substitutes, respiratory tubes, etc., but also cartilage substitutes, nerve sheaths, occlusion of venous valves, seals ex corpora and in corpora, subcutaneous or intramuscular implants, as moisturizing and/or active ingredient gel, micro- and macrocapsules, wound protection, breast implants, generally as filling material, plasters, pads, etc..

Examples of materials of medical device surfaces or medical aid surfaces include, but are not limited to polyamides (such as PA 12, PA 6, PA 66), stainless steel, nitinol, Pebax, polyethylenes, polypropylenes, polyvinyls, polystyrenes, polyimides, polyamides, PTFE, silicones, polyurethanes, polyesters such as polymethacrylates, polyacrylates, polyacrylamides, polycarbonates, polylactides, polyglycolic acids, polyglycolic acids, and many others.e.g. polymethacrylates, polyacrylates, polyacrylamides, polycarbonates, polylactides, polyglycolides, polylactic acids, polyacetates, polycarbonates etc. and their copolymers and derivatives.

### Crosslinkers according to the inventions

The present invention relates to a crosslinker of general formula (**I**):

**BP¹-L¹-Z-L²-BP²** (**I**)

wherein
L¹ represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 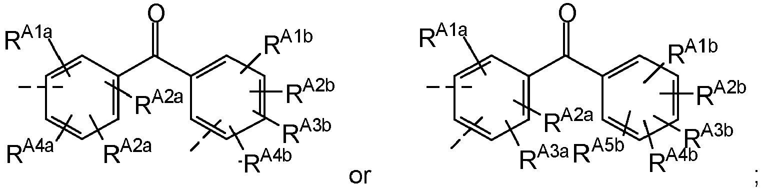
**BP¹** and **BP²** independently of each other represent 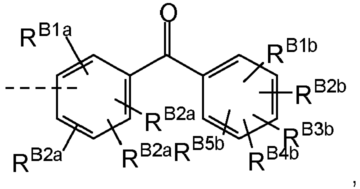
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, -C₆H₁₂R⁵CH₂-, 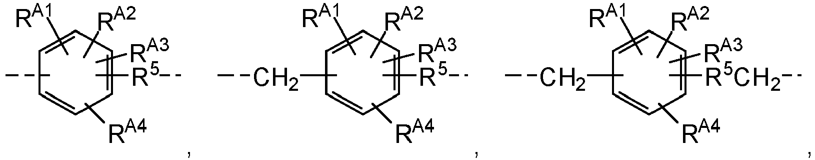 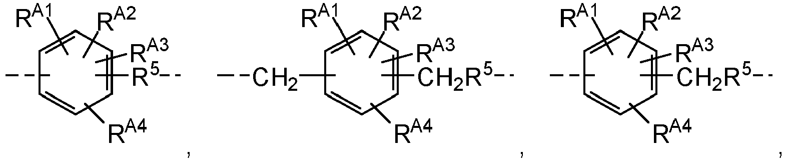 or 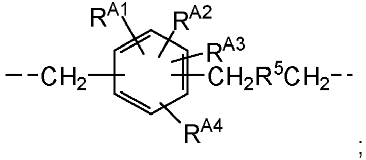
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, -CH₂R⁶C₆H₁₂-, 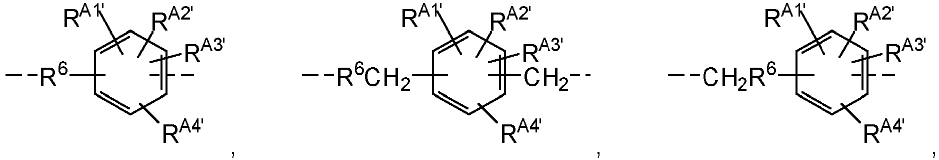 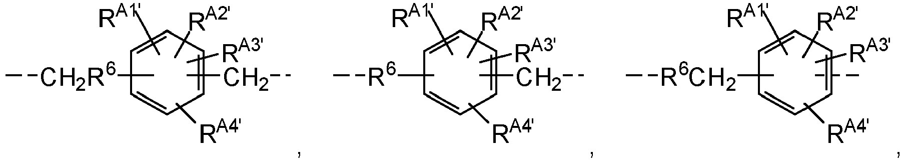 or 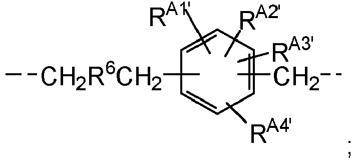
**R⁵** and **R⁶** independently of each other represent -NHCO-, -CONH-, -OCO-, -COO-, -NHCOO-, -OCONH-, -OCOO-, or -NHCONH-; and
**R^{A1}** - **R^{A4}, R^{A1'}** - **R^{A4'}**, **R^{A1a}** - **R^{A4a}**, **R^{A1b}** - **R^{A5b}**, **R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

### Benzophenone Linker Z

The crosslinkers of the present invention comprise a benzophenone linker **Z** that is functionalized with at least two benzophenone substituents **BP¹** and **BP²** via linkers **L¹** and **L²** including or consisting of a functional group selected from -OCO-, -NHCO-, -COO-, -CONH-, -NHCOO-, -O-CO-NH-, -O-CO-O-, and -NHCONH-.

The at least two benzophenone substituents **BP¹** and **BP²** may be both covalently coupled to the same aromatic ring of the benzophenone linker Z or may be covalently coupled to different aromatic rings of the benzophenone linker Z. Thus, the benzophenone linker **Z** represents 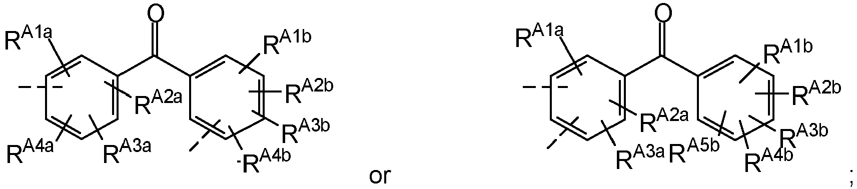 wherein **R^{A1a}** - **R^{A4a}**, and **R^{A1b}** - **R^{A5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

A benzophenone linker **Z** represented by 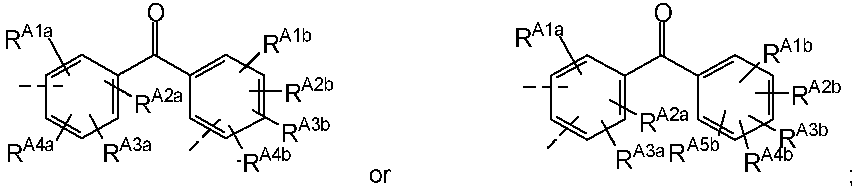 includes the following variations: 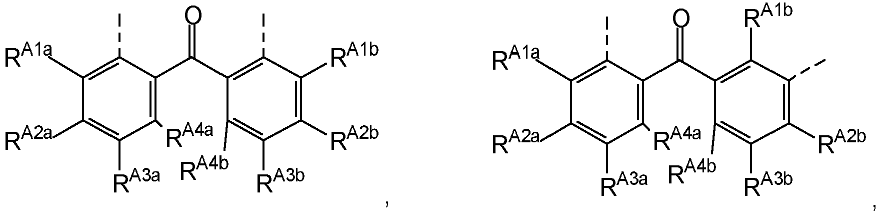 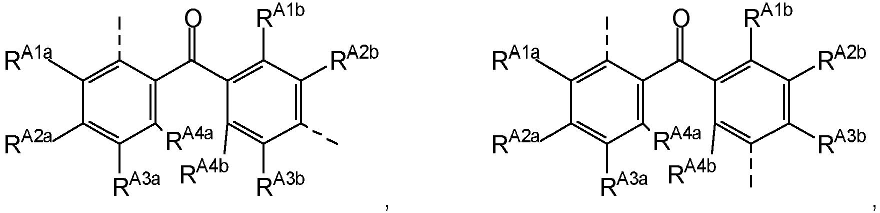 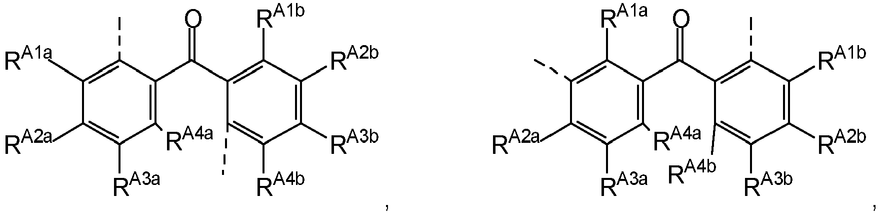 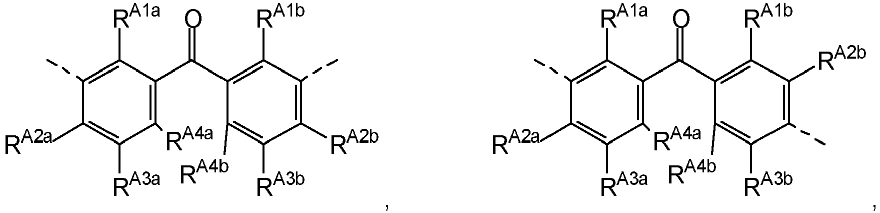 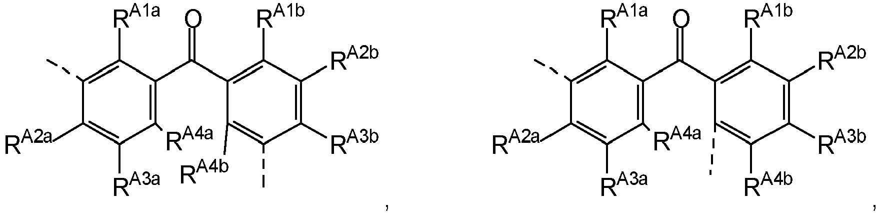 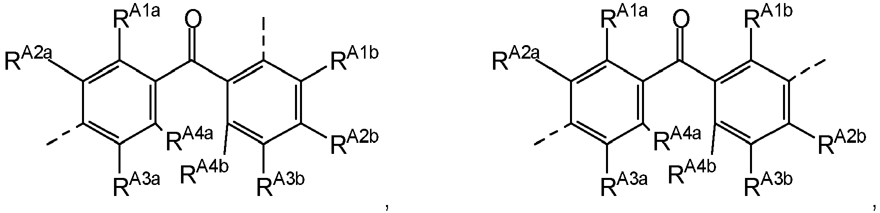 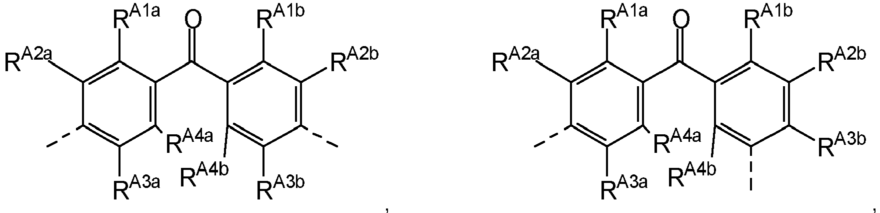 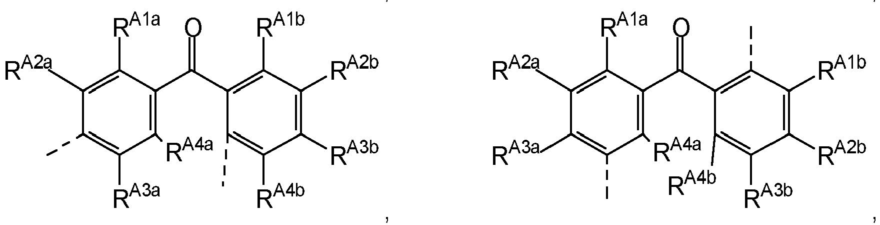 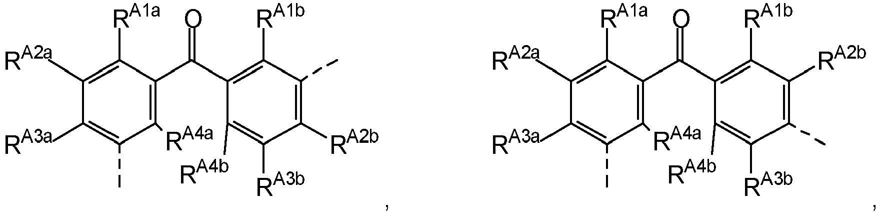 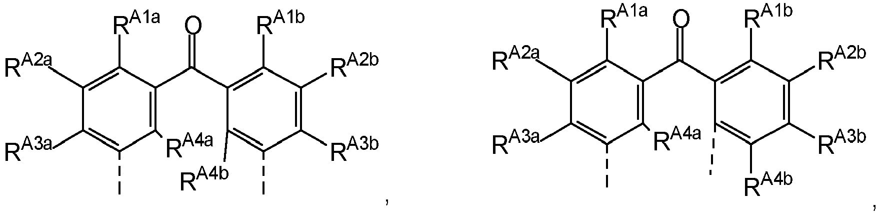 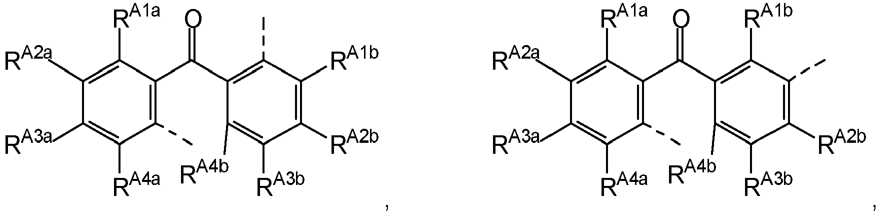 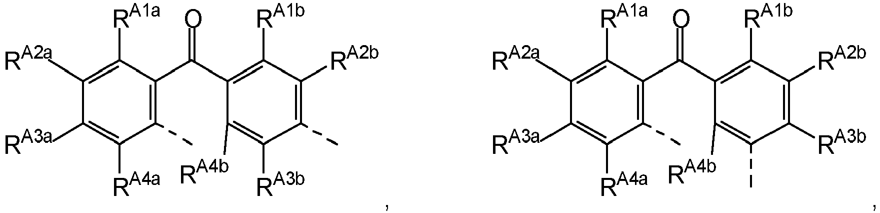 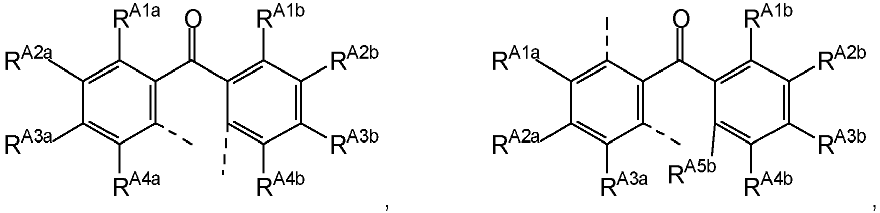 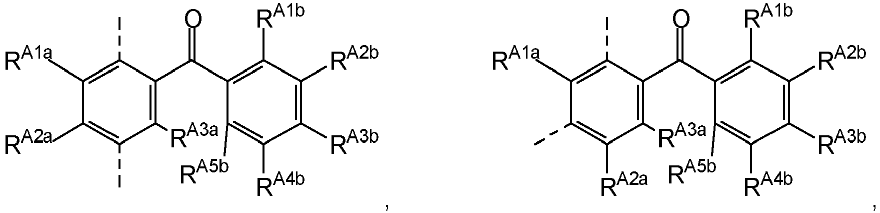 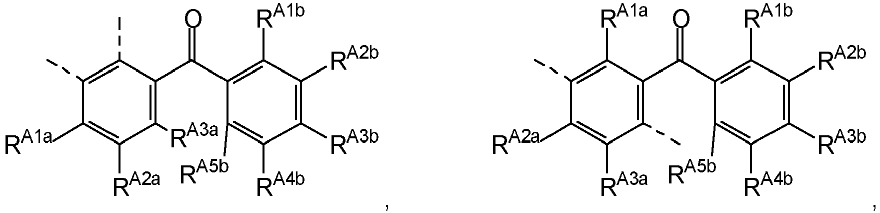 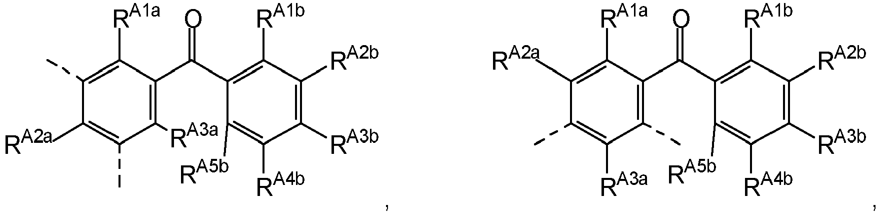 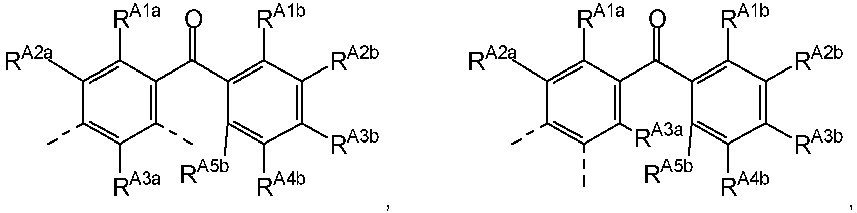 and 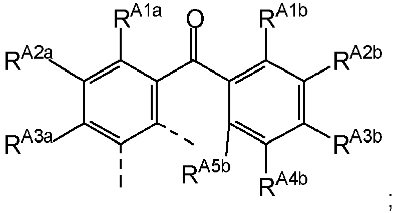 wherein **R^{A1a}** - **R^{A4a}**, and **R^{A1b}** - **R^{A5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Preferably, the at least two benzophenone substituents **BP¹** and **BP²** are covalently coupled to different aromatic rings of the benzophenone linker Z. Thus, in preferred embodiments the benzophenone linker Z represents 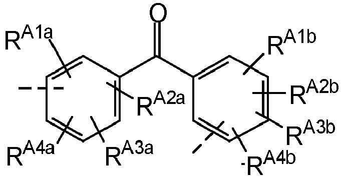 wherein **R^{A1a}** - **R^{A4a}**, and **R^{A1b}** - **R^{A4b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In preferred embodiments, at least one of **R^{A1a} - R^{A4a},** and at least one of **R^{A1b}** - **R^{A5b}** is -H. Thus, in preferred embodiments the benzophenone linker **Z** represents 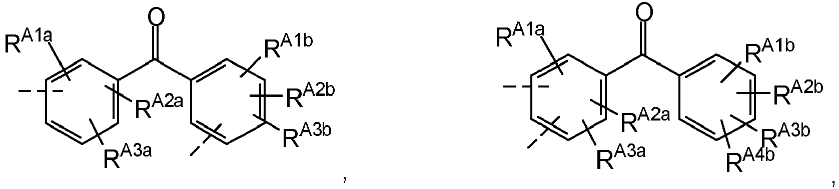 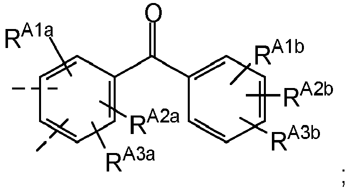 wherein **R^{A1a}** - **R^{A3a}**, and **R^{A1b}** - **R^{A4b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

More preferably, at least two of **R^{A1a}** - **R^{A4a},** and at least two of **R^{A1b}** - **R^{A5b}** are -H. Thus, in preferred embodiments the benzophenone linker **Z** represents 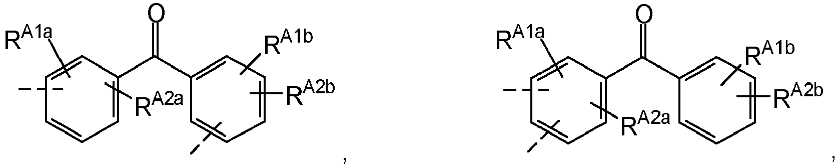 wherein **R^{A1a}** - **R^{A2a},** and **R^{A1b}** - **R^{A2b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Even more preferably, at least three of **R^{A1a}** - **R^{A4a},** and at least three of **R^{A1b}** - **R^{A5b}** are -H. Thus, in preferred embodiments the benzophenone linker **Z** represents 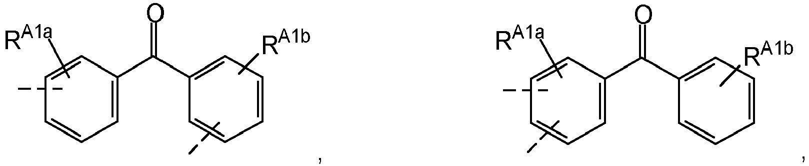 wherein **R^{A1a}** and **R^{A1b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In particular preferred embodiments the benzophenone linker **Z** represents 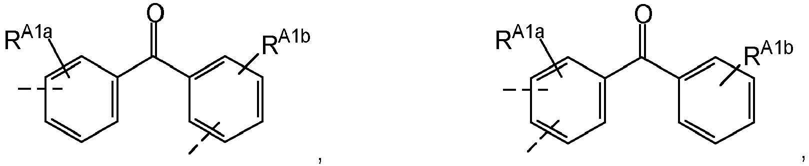 wherein **R^{A1a}** and **R^{A1b}** independently of each other represent -H, or -COOH.

In preferred embodiments, the benzophenone linker **Z** represents 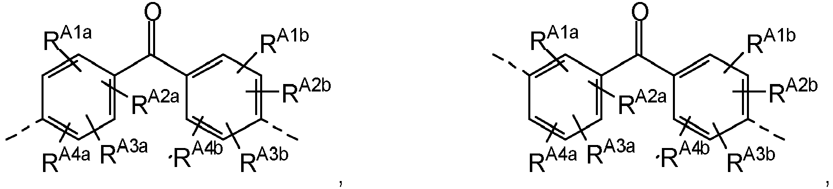 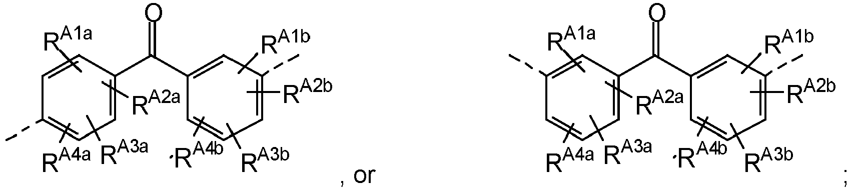 wherein **R^{A1a}** - **R^{A4a},** and **R^{A1b}** - **R^{A5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, **R^{A1a}** - **R^{A3a},** and **R^{A1b}** - **R^{A3b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃; and **R^{A4a},** and **R^{A4b}** - **R^{A5b}** represent -H.

In further preferred embodiments, **R^{A1a}** - **R^{A2a},** and **R^{A1b}** - **R^{A2b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃; and **R^{A3a}** - **R^{A4a}** and **R^{A3b}** - **R^{A5b}** represent -H.

In further preferred embodiments, **R^{A1a}** and **R^{A1b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂,-OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃; and
**R^{A2a}** - **R^{A4a},** and **R^{A2b} - R^{A5b}** represent -H.

In particular preferred embodiments, **R^{A1a}** and **R^{A1b}** independently of each other represent -H, or -COOH; and **R^{A2a}** - **R^{A4a},** and **R^{A2b}** - **R^{A5b}** represent -H.

In preferred embodiments, the benzophenone linker **Z** represents 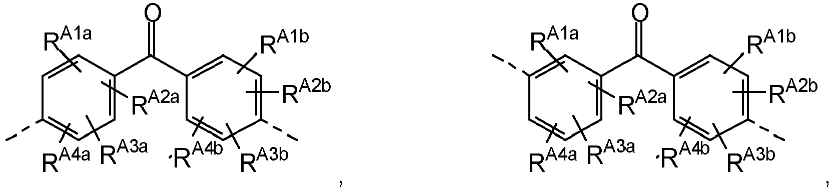 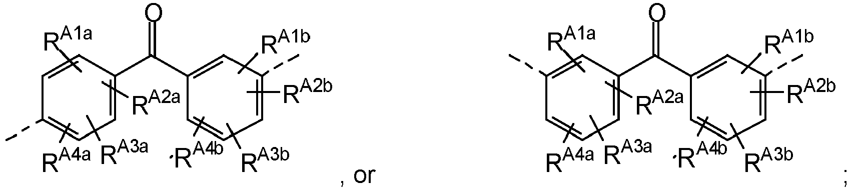 wherein **R^{A1a}** - **R^{A4a},** and **R^{A1b}** - **R^{A5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, **R^{A1a}** - **R^{A3a},** and **R^{A1b}** - **R^{A3b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃; and **R^{A4a},** and **R^{A4b}** - **R^{A5b}** represent -H.

In further preferred embodiments, **R^{A1a}** - **R^{A2a},** and **R^{A1b}** - **R^{A2b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃; and **R^{A3a}** - **R^{A4a},** and **R^{A3b}** - **R^{A5b}** represent -H.

In further preferred embodiments, **R^{A1a}** and **R^{A1b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂,-OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃; and
**R^{A2a}** - **R^{A4a},** and **R^{A2b} - R^{A5b}** represent -H.

In particular preferred embodiments, **R^{A1a}** and **R^{A1b}** independently of each other represent -H, or -COOH; and **R^{A2a}** - **R^{A4a},** and **R^{A2b}** - **R^{A5b}** represent -H.

In particular preferred embodiments, the benzophenone linker **Z** represents 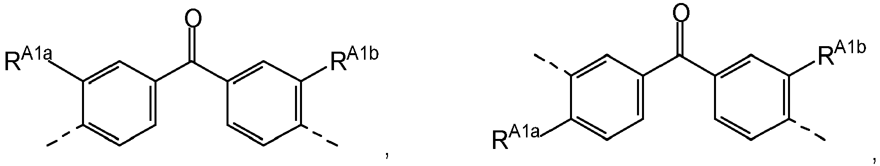 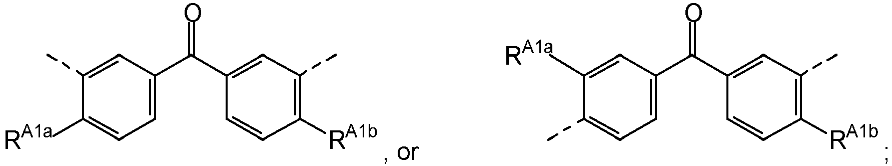 wherein **R^{A1a}** and **R^{A1b}** independently of each other represent -H, or -COOH.

### Benzophenone Substituent BP¹ and BP²

The crosslinkers of the present invention have at least two benzophenone substituents **BP¹** and **BP²** covalently coupled to a benzophenone linker Z via linkers L¹ and L² including or consisting of a functional group selected from -OCO-, -NHCO-, -COO-, -CONH-, -NHCOO-, -O-CO-NH-, -O-CO-O-, and -NHCONH-.

Thus, **BP¹** and **BP²** may independently of each other represent 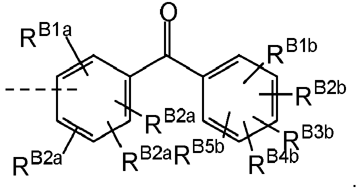 wherein **R^{B1a}** - **R^{B4a}** and **R^{B1b}** - **R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

A benzophenone substituent **BP¹** or **BP²** represented by 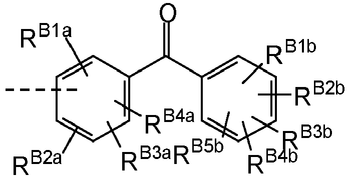 includes the following variations 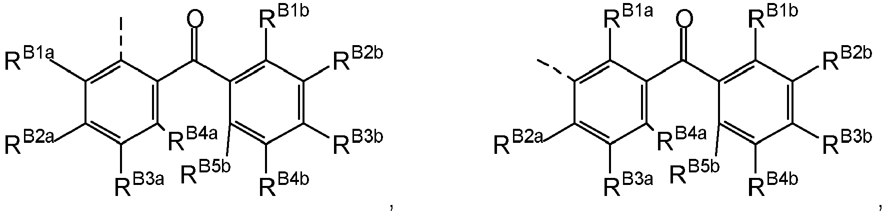 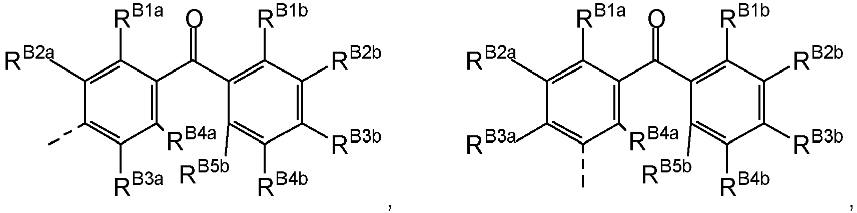 and 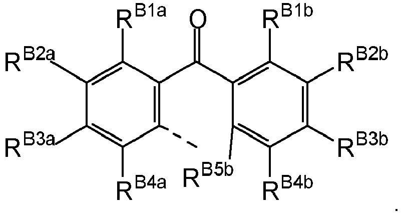 wherein **R^{B1a}** - **R^{B4a}** and **R^{B1b}** - **R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In preferred embodiments, at least one of **R^{B1a}** - **R^{B4a},** and at least one of **R^{B1b}** - **R^{B5b}** is -H. Thus, in preferred embodiments, the benzophenone substituents **BP¹** and/or **BP²** represent 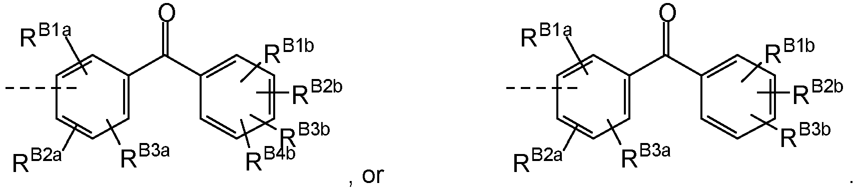 wherein **R^{B1a}** - **R^{B3a}** and **R^{B1b}** - **R^{B4b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, the benzophenone substituents **BP¹** and/or **BP²** represent 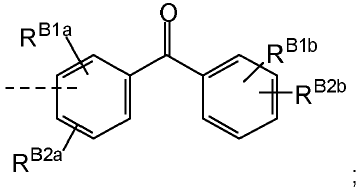 wherein **R^{B1a}** - **R^{B2a}** and **R^{B1b}** - **R^{B2b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, the benzophenone substituents **BP¹** and/or **BP²** represent 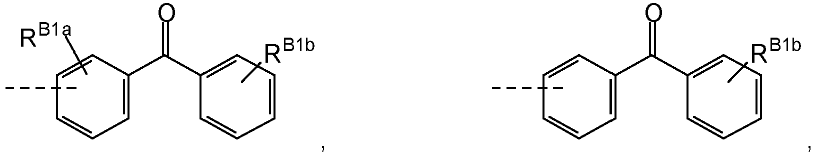 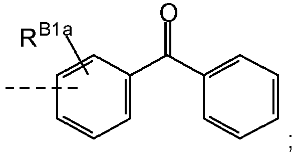 wherein **R^{B1a}** and **R^{B1b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In particular preferred embodiments, the benzophenone substituents **BP¹** and/or **BP²** represent 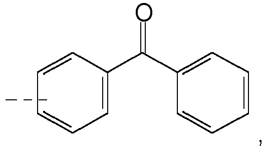 preferably

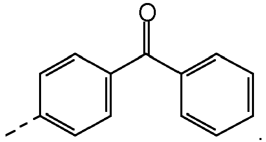

In some preferred embodiments, the benzophenone substituents **BP¹** and **BP²** may comprise different substituents.

Thus, in some embodiments **BP¹** may represent 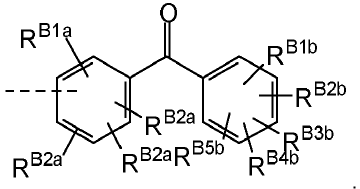 wherein **R^{B1a} - R^{B4a}** and **R^{B1b}** - **R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃; and
**BP²** may represent 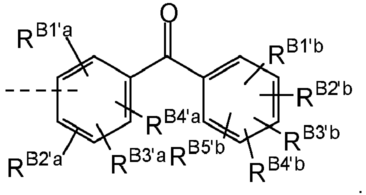 wherein **R^{B1'a}** - **R^{B4'a}** and **R^{B1'b}** - **R^{B5'b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Thus, in preferred embodiments, the benzophenone substituents **BP¹** represents 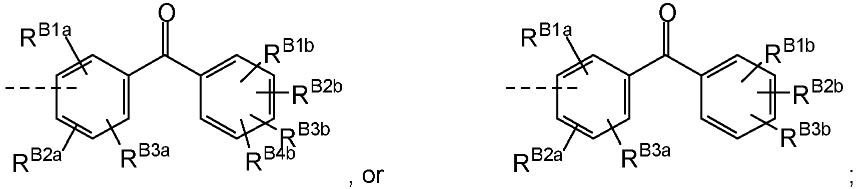 and/or
the benzophenone substituents **BP²** represents 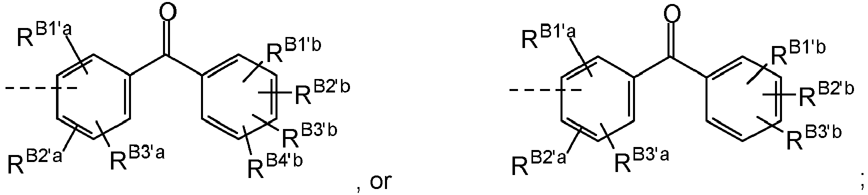 wherein **R^{B1a} - R^{B3a}**, **R^{B1b} - R^{B4b}**, **R^{B1'a} - R^{B3'a}**, **R^{B1'b} - R^{B4'b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, the benzophenone substituent **BP¹** represents 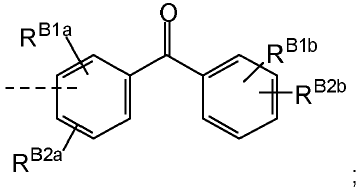 and/or
the benzophenone substituents **BP²** represents 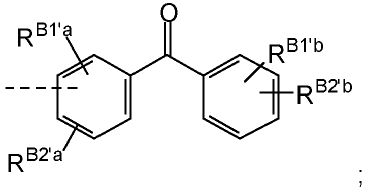 wherein **R^{B1a} - R^{B2a}**, **R^{B1b} - R^{B2b}**, **R^{B1'a} - R^{B2'a}**, **R^{B1'b} - R^{B2'b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, the benzophenone substituent **BP¹** represents 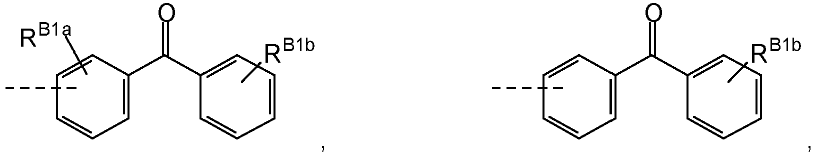 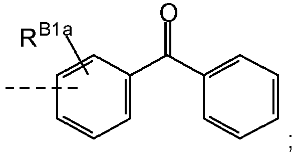 and/or
the benzophenone substituent **BP²** represents 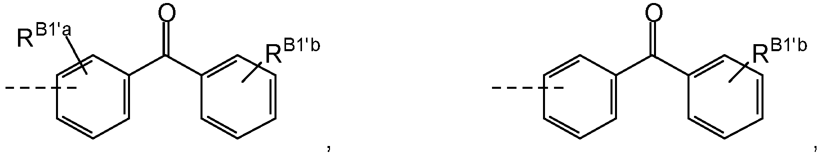 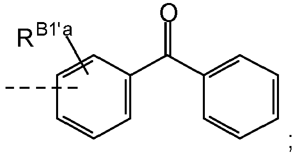 wherein **R^{B1a}, R^{B1b}, R^{B1'a},** and **R^{B1'b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

### Linker L¹ and L²

According to the invention, the at least two benzophenone substituents **BP¹** and **BP²** are both covalently coupled to the benzophenone linker **Z** via linkers **L¹** and L² including or consisting of a functional group selected from -OCO-, -NHCO-, -COO-, -CONH-, -NHCOO-, -OCONH-, -OCOO-, and -NHCONH-. As used herein,

Thus, the present invention relates to crosslinkers of general formula (I):

**BP¹-L¹-Z-L²-BP²** (I)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂- -C₅H₁₀R⁵CH₂-, -C₆H₁₂R⁵CH₂-, 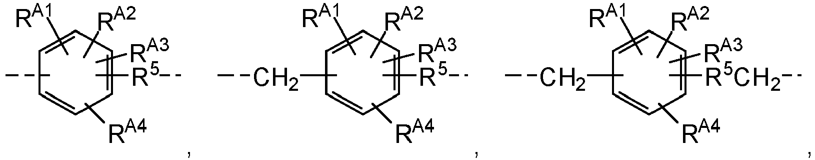 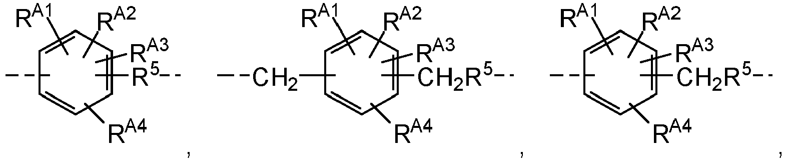 or 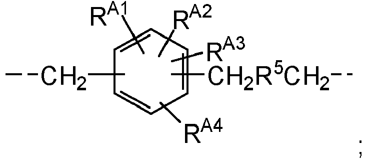
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, -CH₂R⁶C₆H₁₂-, 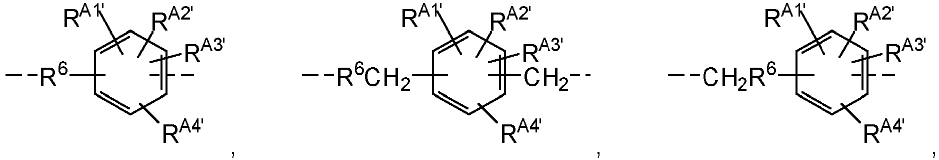 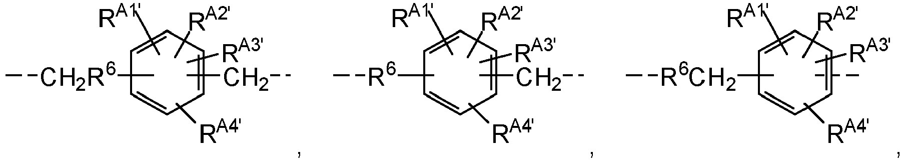 or 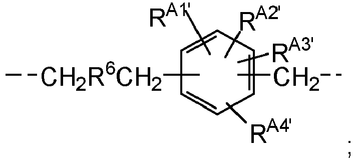
**R⁵** and **R⁶** independently of each other represent -NHCO-, -CONH-, -OCO-, -COO-, -NHCOO-, -OCONH-, -OCOO-, or -NHCONH-;
**R^{A1}** - **R^{A4},** and **R^{A1'}** - **R^{A4'}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃; and
**BP¹, BP²** and **Z** are as defined herein.

As used herein, **L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-, wherein
-R¹-NHCO-R²- represents 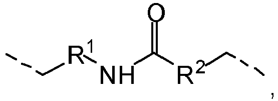 -R¹-CONH-R²- represents 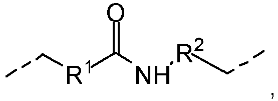 -R¹-OCO-R²- represents 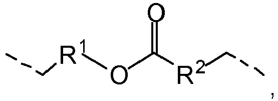 -R¹-COO-R²- represents 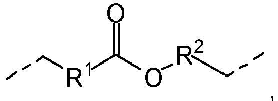 -R¹-NHCOO-R²- represents 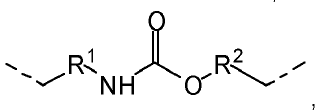 -R¹-OCONH-R²- represents 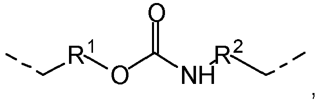 -R¹-OCOO-R²- represents 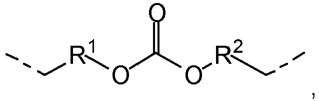 and
-R¹-NHCONH-R²- represents

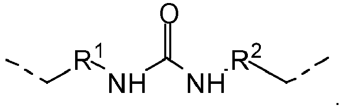

The same follows analogously for **L²** which represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-. As used herein, a dashed line indicates connectivity and a solid line represents a bond.

Preferably, the present invention relates to crosslinkers of general formula (**I**):

**BP¹-L¹-Z-L²-BP²** (**I**)

wherein **-L¹-Z-L²-** represents
-R¹-OCO-R²-Z-R³-COO-R⁴-, -R¹-COO-R²-Z-R³-OCO-R⁴-, -R¹-OCO-R²-Z-R³-OCO-R⁴-, -R¹-COO-R²-Z-R³-COO-R⁴-, -R¹-NHCO-R²-Z-R³-CONH-R⁴-, -R¹-CONH-R²-Z-R³-NHCO-R⁴-, -R¹-NHCO-R²-Z-R³-NHCO-R⁴-, -R¹-CONH-R²-Z-R³-CONH-R⁴-, -R¹-NHCOO-R²-Z-R³-OCONH-R⁴-, -R¹-OCONH-R²-Z-R³-NHCOO-R⁴-, -R¹-NHCOO-R²-Z-R³-NHCOO-R⁴-, -R¹-OCONH-R²-Z-R³-OCONH-R⁴-, -R¹-OCO-R²-Z-R³-NHCO-R⁴-, -R¹-OCO-R²-Z-R³-CONH-R⁴-, -R¹-OCO-R²-Z-R³-NHCOO-R⁴-, -R¹-OCO-R²-Z-R³-OCONH-R⁴-, -R¹-COO-R²-Z-R³-NHCO-R⁴-, -R¹-COO-R²-Z-R³-CONH-R⁴-, -R¹-COO-R²-Z-R³-NHCOO-R⁴-, -R¹-COO-R²-Z-R³-OCO-NH-R⁴-, -R¹-NHCO-R²-Z-R³-OCO-R⁴-, -R¹-NHCO-R²-Z-R³-COO-R⁴-, -R¹-NHCO-R²-Z-R³-NHCOO-R⁴-, -R¹-NHCO-R²-Z-R³-OCONH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴-, -R¹-CONH-R²-Z-R³-OCONH-R⁴-, -R¹-NHCOO-R²-Z-R³-OCO-R⁴-, -R¹-NHCOO-R²-Z-R³-COO-R⁴-, -R¹-NHCOO-R²-Z-R³-NHCO-R⁴-, -R¹-NHCOO-R²-Z-R³-CONH-R⁴-, -R¹-OCONH-R²-Z-R³-OCO-R⁴-, -R¹-OCONH-R²-Z-R³-COO-R⁴-, -R¹-OCONH-R²-Z-R³-NHCO-R⁴-, -R¹-OCONH-R²-Z-R³-CONH-R⁴-, -R¹-NHCONH-R²-Z-R³-NHCONH-R⁴-, -R¹-OCO-R²-Z-R³-NHCONH-R⁴-, -R¹-NHCO-R²-Z-R³-NHCONH-R⁴-, -R¹-COO-R²-Z-R³-NHCONH-R⁴-, -R¹-CONH-R²-Z-R³-NHCONH-R⁴-, -R¹-NHCOO-R²-Z-R³-NHCONH-R⁴-, -R¹-OCONH-R²-Z-R³-NHCONH-R⁴-, -R¹-NHCONH-R²-Z-R³-OCO-R⁴-, -R¹-NHCONH-R²-Z-R³-COO-R⁴-, -R¹-NHCONH-R²-Z-R³-NHCO-R⁴-, -R¹-NHCONH-R²-Z-R³-CONH-R⁴-, -R¹-NHCONH-R²-Z-R³-NHCOO-R⁴-, -R¹-NHCONH-R²-Z-R³-OCONH-R⁴-, -R¹-OCOO-R²-Z-R³-OCOO-R⁴-, -R¹-OCO-R²-Z-R³-OCONH-R⁴-, -R¹-NHCO-R²-Z-R³-OCOO-R⁴-, -R¹-COO-R²-Z-R³-OCOO-R⁴-, -R¹-CONH-R²-Z-R³-OCOO-R⁴-, -R¹-NHCOO-R²-Z-R³-OCOO-R⁴-, -R¹-OCONH-R²-Z-R³-OCOO-R⁴-, -R¹-OCOO-R²-Z-R³-OCO-R⁴-, -R¹-OCOO-R²-Z-R³-COO-R⁴-, -R¹-OCOO-R²-Z-R³-NHCO-R⁴-, -R¹-OCOO-R²-Z-R³-CONH-R⁴-, -R¹-OCOO-R²-Z-R³-NHCOO-R⁴- , or -R¹-OCOO-R²-Z-R³-OCONH-R²-; and **R¹, R², R³, R⁴, BP¹, BP²,** and **Z** are as defined herein.

Thus, the present invention preferably relates to crosslinkers of general formula (**I**):

**BP¹-L¹-Z-L²-BP²** (**I**)

wherein **BP¹-L¹-Z-L²-BP²** represents
BP¹-R¹-OCO-R²-Z-R³-COO-R⁴-BP², BP¹-R¹-COO-R²-Z-R³-OCO-R⁴-BP², BP¹-R¹-OCO-R²-Z-R³-OCO-R⁴-BP², BP¹-R¹-COO-R²-Z-R³-COO-R⁴-BP², BP¹-R¹-NHCO-R²-Z-R³-CONH-R⁴-BP², BP¹-R¹-CONH-R²-Z-R³-NHCO-R⁴-BP², BP¹-R¹-NHCO-R²-Z-R³-NHCO-R⁴-BP², BP¹-R¹-CONH-R²-Z-R³-CONH-R⁴-BP², BP¹-R¹-NHCOO-R²-Z-R³-OCONH-R⁴-BP², BP¹-R¹-OCONH-R²-Z-R³-NHCOO-R⁴-BP², BP¹-R¹-NHCOO-R²-Z-R³-NHCOO-R⁴-BP², BP¹-R¹-OCONH-R²-Z-R³-OCONH-R⁴-BP², BP¹-R¹-OCO-R²-Z-R³-NHCO-R⁴-BP², BP¹-R¹-OCO-R²-Z-R³-CONH-R⁴-BP², BP¹-R¹-OCO-R²-Z-R³-NHCOO-R⁴-BP², BP¹-R¹-OCO-R²-Z-R³-OCONH-R⁴-BP², BP¹-R¹-COO-R²-Z-R³-NHCO-R⁴-BP², BP¹-R¹-COO-R²-Z-R³-CONH-R⁴-BP², BP¹-R¹-COO-R²-Z-R³-NHCOO-R⁴-BP², BP¹-R¹-COO-R²-Z-R³-OCO-NH-R⁴-BP², BP¹-R¹-NHCO-R²-Z-R³-OCO-R⁴-BP², BP¹-R¹-NHCO-R²-Z-R³-COO-R⁴-BP², BP¹-R¹-NHCO-R²-Z-R³-NHCOO-R⁴-BP², BP¹-R¹-NHCO-R²-Z-R³-OCONH-R⁴-BP², BP¹-R¹-CONH-R²-Z-R³-OCO-R⁴-BP², BP¹-R¹-CONH-R²-Z-R³-COO-R⁴-BP², BP¹-R¹-CONH-R²-Z-R³-NHCOO-R⁴-BP², BP¹-R¹-CONH-R²-Z-R³-OCONH-R⁴-BP², BP¹-R¹-NHCOO-R²-Z-R³-OCO-R⁴-BP², BP¹-R¹-NHCOO-R²-Z-R³-COO-R⁴-BP², BP¹-R¹-NHCOO-R²-Z-R³-NHCO-R⁴-BP², BP¹-R¹-NHCOO-R²-Z-R³-CONH-R⁴-BP², BP¹-R¹-OCONH-R²-Z-R³-OCO-R⁴-BP², -R¹-OCONH-R²-Z-R³-COO-R⁴-BP², BP¹-R¹-OCONH-R²-Z-R³-NHCO-R⁴-BP², BP¹-R¹-OCONH-R²-Z-R³-CONH-R⁴-BP², BP¹-R¹-NHCONH-R²-Z-R³-NHCONH-R⁴-BP², BP¹-R¹-OCO-R²-Z-R³-NHCONH-R⁴-BP², BP¹-R¹-NHCO-R²-Z-R³-NHCONH-R⁴-BP², BP¹-R¹-COO-R²-Z-R³-NHCONH-R⁴-BP², BP¹-R¹-CONH-R²-Z-R³-NHCONH-R⁴-BP², BP¹-R¹-NHCOO-R²-Z-R³-NHCONH-R⁴-BP², BP¹-R¹-OCONH-R²-Z-R³-NHCONH-R⁴-BP², BP¹-R¹-NHCONH-R²-Z-R³-OCO-R⁴-BP², BP¹-R¹-NHCONH-R²-Z-R³-COO-R⁴-BP², BP¹-R¹-NHCONH-R²-Z-R³-NHCO-R⁴-BP², BP¹-R¹-NHCONH-R²-Z-R³-CONH-R⁴-BP², BP¹-R¹-NHCONH-R²-Z-R³-NHCOO-R⁴-BP², BP¹-R¹-NHCONH-R²-Z-R³-OCONH-R⁴-BP², BP¹-R¹-OCOO-R²-Z-R³-OCOO-R⁴-BP², BP¹-R¹-OCO-R²-Z-R³-OCONH-R⁴-BP², BP¹-R¹-NHCO-R²-Z-R³-OCOO-R⁴-BP², BP¹-R¹-COO-R²-Z-R³-OCOO-R⁴-BP², BP¹-R¹-CONH-R²-Z-R³-OCOO-R⁴-BP², BP¹-R¹-NHCOO-R²-Z-R³-OCOO-R⁴-BP², BP¹-R¹-OCONH-R²-Z-R³-OCOO-R⁴-BP², BP¹-R¹-OCOO-R²-Z-R³-OCO-R⁴-BP², BP¹-R¹-OCOO-R²-Z-R³-COO-R⁴-BP², BP¹-R¹-OCOO-R²-Z-R³-NHCO-R⁴-BP², BP¹-R¹-OCOO-R²-Z-R³-CONH-R⁴-BP², BP¹-R¹-OCOO-R²-Z-R³-NHCOO-R⁴-BP² , or BP¹-R¹-OCOO-R²-Z-R³-OCONH-R²-BP²; and **R¹, R², R³, R⁴, BP¹, BP², and Z** are as defined herein.

Preferably, the present invention relates to crosslinkers of general formula (**I**):

**BP¹-L¹-Z-L²-BP²** (**I**)

wherein **-L¹-Z-L²-** represents
-R¹-OCO-R²-Z-R³-COO-R⁴-, -R¹-COO-R²-Z-R³-OCO-R⁴-, -R¹-OCO-R²-Z-R³-OCO-R⁴-, -R¹-COO-R²-Z-R³-COO-R⁴-, -R¹-NHCO-R²-Z-R³-CONH-R⁴-, -R¹-CONH-R²-Z-R³-NHCO-R⁴-, -R¹-NHCO-R²-Z-R³-NHCO-R⁴-, -R¹-CONH-R²-Z-R³-CONH-R⁴-, -R¹-NHCOO-R²-Z-R³-OCONH-R⁴-, -R¹-OCONH-R²-Z-R³-NHCOO-R⁴-, -R¹-NHCOO-R²-Z-R³-NHCOO-R⁴-, -R¹-OCONH-R²-Z-R³-OCONH-R⁴-, -R¹-OCO-R²-Z-R³-NHCO-R⁴-, -R¹-OCO-R²-Z-R³-CONH-R⁴-, -R¹-OCO-R²-Z-R³-NHCOO-R⁴-, -R¹-OCO-R²-Z-R³-OCONH-R⁴-, -R¹-COO-R²-Z-R³-NHCO-R⁴-, -R¹-COO-R²-Z-R³-CONH-R⁴-, -R¹-COO-R²-Z-R³-NHCOO-R⁴-, -R¹-COO-R²-Z-R³-OCONH-R⁴-, -R¹-NHCO-R²-Z-R³-OCO-R⁴-, -R¹-NHCO-R²-Z -R³-COO-R⁴-, -R¹-NHCO-R²-Z-R³-NHCOO-R⁴-, -R¹-NHCO-R²-Z-R³-OCONH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴-, -R¹-CONH-R²-Z-R³-OCONH-R⁴-, -R¹-NHCOO-R²-Z-R³-OCO-R⁴-, -R¹-NHCO-O-R²-Z-R³-COO-R⁴-, -R¹-NHCOO-R²-Z-R³-NHCO-R⁴-, -R¹-NHCO-O-R²-Z-R³-CONH-R⁴-, -R¹-OCONH-R²-Z-R³-OCO-R⁴-, -R¹-OCO-NH-R²-Z-R³-COO-R⁴-, -R¹-OCONH-R²-Z-R³-NHCO-R⁴-, or -R¹-OCONH-R²-Z-R³-CONH-R⁴-, and **R¹, R², R³, R⁴, BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents -R¹-OCO-R²-Z-R³-COO-R⁴-, -R¹-COO-R²-Z-R³-OCO-R⁴-, -R¹-OCO-R²-Z-R³-OCO-R⁴-, -R¹-COO-R²-Z-R³-COO-R⁴-, -R¹-NHCO-R²-Z-R³-CONH-R⁴-, -R¹-CONH-R²-Z-R³-NHCO-R⁴-, -R¹-NHCO-R²-Z-R³-NHCO-R⁴-, -R¹-CONH-R²-Z-R³-CONH-R⁴-, -R¹-NHCOO-R²-Z-R³-OCONH-R⁴-, -R¹-OCONH-R²-Z-R³-NHCOO-R⁴-, -R¹-NHCOO-R²-Z-R³-NHCOO-R⁴-, or -R¹-OCONH-R²-Z-R³-OCONH-R⁴-; and **R¹**, **R², R³, R⁴, BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents -R¹-OCO-R²-Z-R³-COO-R⁴-, -R¹-COO-R²-Z-R³-OCO-R⁴-, -R¹-NHCO-R²-Z-R³-CONH-R⁴-, -R¹-CONH-R²-Z-R³-NHCO-R⁴-, -R¹-NHCOO-R²-Z-R³-OCONH-R⁴-, or -R¹-OCONH-R²-Z-R³-NHCOO-R⁴-; and **R¹**, **R², R³, R⁴, BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-OCO-R²-Z-R³-COO-R⁴-, -R¹-COO-R²-Z-R³-OCO-R⁴-, -R¹-OCO-R²-Z-R³-OCO-R⁴-, or -R¹-COO-R²-Z-R³-COO-R²; and **R¹, R², R³, R⁴, BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-NHCO-R²-Z-R³-CONH-R⁴-, -R¹-CONH-R²-Z-R³-NHCO-R⁴-, -R¹-NHCO-R²-Z-R³-NHCO-R⁴-, or -R¹-CONH-R²-Z-R³-CONH-R⁴-; and **R¹, R², R³, R⁴, BP¹, BP²,** and **Z** are as defined herein.

Preferably, the present invention relates to crosslinkers of general formula (I):

**BP¹-L¹-Z-L²-BP²** (**I**)

wherein **-L¹-Z-L²-** represents
-R¹-NHCOO-R²-Z-R³-OCONH-R⁴-, -R¹-OCONH-R²-Z-R³-NHCOO-R⁴-, -R¹-NHCOO-R²-Z-R³-NHCOO-R⁴-, or -R¹-OCONH-R²-Z-R³-OCONH-R⁴-; and **R¹**, **R², R³, R⁴, BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-OCO-R²-Z-R³-COO-R⁴-, or -R¹-COO-R²-Z-R³-OCO-R⁴-; and R¹, **R², R³, R⁴, BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-NHCO-R²-Z-R³-CO-NH-R⁴-, or -R¹-CO-NH-R²-Z-R³-NH-CO-R⁴-; and **R**¹, **R², R³, R⁴, BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-NHCOO-R²-Z-R³-OCONH-R⁴-, or -R¹-OCONH-R²-Z-R³-NHCOO-R⁴-; and **R¹, R², R³, R⁴, BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-OCO-R²-Z-R³-NHCO-R⁴-, -R¹-OCO-R²-Z-R³-CONH-R⁴-, -R¹-OCO-R²-Z-R³-NHCOO-R⁴-, -R¹-OCO-R²-Z-R³-OCONH-R⁴-, -R¹-COO-R²-Z-R³-NHCO-R⁴-, -R¹-COO-R²-Z-R³-CONH-R⁴-, -R¹-COO-R²-Z-R³-NHCOO-R⁴-, -R¹-COO-R²-Z-R³-OCONH-R⁴-, -R¹-NHCO-R²-Z-R³-OCO-R⁴-, -R¹-NHCO-R²-Z -R³-COO-R⁴-, -R¹-NHCO-R²-Z-R³-NHCOO-R⁴-, -R¹-NHCO-R²-Z-R³-OCONH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴-, -R¹-CONH-R²-Z-R³-OCONH-R⁴-, -R¹-NHCOO-R²-Z-R³-OCO-R⁴-, -R¹-NHCOO-R²-Z-R³COO-R⁴-, -R¹-NHCOO-R²-Z-R³-NHCO-R⁴-, -R¹-NHCOO-R²-Z-R³-CONH-R⁴-, -R¹-OCONH-R²-Z-R³-OCO-R⁴-, -R¹-OCONH-R²-Z-R³-COO-R⁴-, -R¹-OCONH-R²-Z-R³-NHCO-R⁴-, or -R¹-OCONH-R²-Z-R³-CONH-R⁴-, and **R¹, R², R³, R⁴, BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-OCOR²-Z-R³-NHCO-R⁴-, -R¹-OCO-R²-Z-R³-CONH-R⁴-, -R¹-OCO-R²-Z-R³-NHCOO-R⁴-, -R¹-OCO-R²-Z-R³-O-CONH-R⁴-, -R¹-COO-R²-Z-R³-NHCO-R⁴-, -R¹-COO-R²-Z-R³-CONH-R⁴-, -R¹-COO-R²-Z-R³-NHCOO-R⁴-, or -R¹-COO-R²-Z-R³-OCONH-R⁴-;
and **R¹, R², R³,** a **R⁴, BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-NHCO-R²-Z-R³-OCO-R⁴-, -R¹-NHCO-R²-Z -R³-COO-R⁴-, -R¹-NHCO-R²-Z-R³-NHCOO-R⁴-, -R¹-NHCO-R²-Z-R³-OCONH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴-, or -R¹-CONH-R²-Z-R³-OCONH-R⁴-;
and **R¹, R², R³, R⁴, BP¹, BP², and Z** are as defined herein.

Preferably, the present invention relates to crosslinkers of general formula (**I**):

**BP¹-L¹-Z-L²-BP²** (**I**)

wherein **-L¹-Z-L²-** represents
-R¹-NHCOO-R²-Z-R³-OCO-R⁴-, -R¹-NHCOO-R²-Z-R³-COO-R⁴-, -R¹-NHCOO-R²-Z-R³-NHCO-R⁴-, -R¹-NHCOO-R²-Z-R³-CONH-R⁴-, -R¹-OCONH-R²-Z-R³-OCO-R⁴-, -R¹-OCONH-R²-Z-R³-COO-R⁴-, -R¹-OCONH-R²-Z-R³-NHCO-R⁴-, and -R¹-OCONH-R²-Z-R³-CONH-R⁴-,
and **R¹, R², R³, R⁴, BP¹, BP²,** or **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-OCO-R²-Z-R³-COO-R⁴-, -R¹-COO-R²-Z-R³-OCO-R⁴-, -R¹-OCO-R²-Z-R³-OCO-R⁴-, -R¹-COO-R²-Z-R³-COO-R⁴-, -R¹-NHCO-R²-Z-R³-CONH-R⁴-, -R¹-CONH-R²-Z-R³-NHCO-R⁴-, -R¹-NHCO-R²-Z-R³-NHCO-R⁴-, -R¹-CONH-R²-Z-R³-CONH-R⁴-, -R¹-NHCOO-R²-Z-R³-OCONH-R⁴-, -R¹-OCONH-R²-Z-R³-NHCOO-R⁴-, -R¹-NHCOO-R²-Z-R³-NHCOO-R⁴-, -R¹-OCONH-R²-Z-R³-OCONH-R⁴-, -R¹-OCO-R²-Z-R³-NHCO-R⁴-, -R¹-OCO-R²-Z-R³-CONH-R⁴-, -R¹-OCO-R²-Z-R³-NHCOO-R⁴-, -R¹-OCO-R²-Z-R³-OCONH-R⁴-, -R¹-COO-R²-Z-R³-NHCO-R⁴-, -R¹-COO-R²-Z-R³-CONH-R⁴-, -R¹-COO-R²-Z-R³-NHCOO-R⁴-, -R¹-COO-R²-Z-R³-OCO-NH-R⁴-, -R¹-NHCO-R²-Z-R³-OCO-R⁴-, -R¹-NHCO-R²-Z -R³-COO-R⁴-, -R¹-NHCO-R²-Z-R³-NHCOO-R⁴-, -R¹-NHCO-R²-Z-R³-OCONH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴-, -R¹-CONH-R²-Z-R³-OCONH-R⁴-, -R¹-NHCOO-R²-Z-R³-OCO-R⁴-, -R¹-NHCOO-R²-Z-R³-COO-R⁴-, -R¹-NHCOO-R²-Z-R³-NHCO-R⁴-, -R¹-NHCOO-R²-Z-R³-CONH-R⁴-, -R¹-OCONH-R²-Z-R³-OCO-R⁴-, -R¹-OCONH-R²-Z-R³-COO-R⁴-, -R¹-OCONH-R²-Z-R³-NHCO-R⁴-, -R¹-OCONH-R²-Z-R³-CONH-R⁴-, -R¹-NHCONH-R²-Z-R³-NHCONH-R⁴-, -R¹-OCOR²-Z-R³-NHCONH-R⁴-, -R¹-NH-CO-R²-Z-R³-NHCONH-R⁴-, -R¹-COO-R²-Z-R³-NHCONH-R⁴-, -R¹-CONH-R²-Z-R³-NHCONH-R⁴-, -R¹-NHCOO-R²-Z-R³-NHCONH-R⁴-, -R¹-OCONH-R²-Z-R³-NHCONH-R⁴-, -R¹-NHCONH-R²-Z-R³-OCO-R⁴-, -R¹-NHCONH-R²-Z-R³-COO-R⁴-, -R¹-NHCONH-R²-Z-R³-NHCO-R⁴-, -R¹-NHCONH-R²-Z-R³-CONH-R⁴-, -R¹-NHCONH-R²-Z-R³-NHCOO-R⁴-, -R¹-NHCONH-R²-Z-R³-OCONH-R²-, -R¹-OCOO-R²-Z-R³-OCOO-R⁴-, -R¹-OCO-R²-Z-R³-OCO-NH-R⁴-, -R¹-NHCO-R²-Z-R³-OCOO-R⁴-, -R¹-COO-R²-Z-R³-OCOO-R⁴-, -R¹-CONH-R²-Z-R³-OCOO-R⁴-, -R¹-NHCOO-R²-Z-R³-OCOO-R⁴-, -R¹-OCONH-R²-Z-R³-OCOO-R⁴-, -R¹-OCOO-R²-Z-R³-OCO-R⁴-, -R¹-OCOO-R²-Z-R³-COO-R⁴-, -R¹-OCOO-R²-Z-R³-NHCO-R⁴-, -R¹-OCOO-R²-Z-R³-CONH-R⁴-, -R¹-OCOO-R²-Z-R³-NHCOO-R⁴-, or -R¹-OCOO-R²-Z-R³-OCONH-R²-; **R¹, R², R³,** and **R⁴** independently of each other represent a bond or -CH₂-;
and **BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-OCO-R²-Z-R³-COO-R⁴-, -R¹-COO-R²-Z-R³-OCO-R⁴-, -R¹-OCO-R²-Z-R³-OCO-R⁴-, -R¹-COO-R²-Z-R³-COO-R⁴-, -R¹-NHCO-R²-Z-R³-CONH-R⁴-, -R¹-CONH-R²-Z-R³-NHCO-R⁴-, -R¹-NHCO-R²-Z-R³-NHCO-R⁴-, -R¹-CONH-R²-Z-R³-CONH-R⁴-, -R¹-NHCOO-R²-Z-R³-OCONH-R⁴-, -R¹-OCONH-R²-Z-R³-NHCOO-R⁴-, -R¹-NHCOO-R²-Z-R³-NHCOO-R⁴-, -R¹-OCONH-R²-Z-R³-OCONH-R⁴-, -R¹-OCO-R²-Z-R³-NHCO-R⁴-, -R¹-OCO-R²-Z-R³-CONH-R⁴-, -R¹-OCO-R²-Z-R³-NHCOO-R⁴-, -R¹-OCO-R²-Z-R³-OCONH-R⁴-, -R¹-COO-R²-Z-R³-NHCO-R⁴-, -R¹-COO-R²-Z-R³-CONH-R⁴-, -R¹-COO-R²-Z-R³-NHCOO-R⁴-, -R¹-COO-R²-Z-R³-OCONH-R⁴-, -R¹-NHCO-R²-Z-R³-OCO-R⁴-, -R¹-NHCO-R²-Z -R³-COO-R⁴-, -R¹-NHCO-R²-Z-R³-NHCOO-R⁴-, -R¹-NHCO-R²-Z-R³-OCONH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴-, -R¹-CONH-R²-Z-R³-OCONH-R⁴-, -R¹-NHCOO-R²-Z-R³-OCO-R⁴-, -R¹-NHCOO-R²-Z-R³-COO-R⁴-, -R¹-NHCOO-R²-Z-R³-NHCO-R⁴-, -R¹-NHCOO-R²-Z-R³-CONH-R⁴-, -R¹-OCONH-R²-Z-R³-OCO-R⁴-, -R¹-OCONH-R²-Z-R³-COO-R⁴-, -R¹-OCONH-R²-Z-R³-NHCO-R⁴-, -R¹-OCONH-R²-Z-R³-CONH-R⁴-, -R¹-NHCONH-R²-Z-R³-NHCONH-R⁴-, -R¹-OCO-R²-Z-R³-NHCONH-R⁴-, -R¹-NHCO-R²-Z-R³-NHCONH-R⁴-, -R¹-COO-R²-Z-R³-NHCONH-R⁴-, -R¹-CONH-R²-Z-R³-NHCONH-R⁴-, -R¹-NHCOO-R²-Z-R³-NHCONH-R⁴-, or -R¹-OCONH-R²-Z-R³-NHCONH-R⁴-;
**R¹, R², R³,** and **R⁴** independently of each other represent a bond or -CH₂-;
and **BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-OCO-R²-Z-R³-COO-R⁴-, -R¹-COO-R²-Z-R³-OCO-R⁴-, -R¹-OCO-R²-Z-R³-OCO-R⁴-, -R¹-COO-R²-Z-R³-COO-R⁴-, -R¹-NHCO-R²-Z-R³-CONH-R⁴-, -R¹-CONH-R²-Z-R³-NHCO-R⁴-, -R¹-NHCO-R²-Z-R³-NHCO-R⁴-, -R¹-CONH-R²-Z-R³-CONH-R⁴-, -R¹-NHCOO-R²-Z-R³-OCONH-R⁴-, -R¹-OCONH-R²-Z-R³-NHCOO-R⁴-, -R¹-NHCOO-R²-Z-R³-NHCOO-R⁴-, -R¹-OCONH-R²-Z-R³-OCONH-R⁴-, -R¹-OCO-R²-Z-R³-NHCO-R⁴-, -R¹-OCO-R²-Z-R³-CONH-R⁴-, -R¹-OCO-R²-Z-R³-NHCOO-R⁴-, -R¹-OCO-R²-Z-R³-OCO-NH-R⁴-, -R¹-COO-R²-Z-R³-NHCO-R⁴-, -R¹-COO-R²-Z-R³-CONH-R⁴-, -R¹-COO-R²-Z-R³-NHCOO-R⁴-, -R¹-COO-R²-Z-R³-OCONH-R⁴-, -R¹-NHCO-R²-Z-R³-OCO-R⁴-, -R¹-NHCO-R²-Z-R³-COO-R⁴-, -R¹-NHCO-R²-Z-R³-NHCOO-R⁴-, -R¹-NHCO-R²-Z-R³-OCONH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴-, -R¹-CONH-R²-Z-R³-OCONH-R⁴-, -R¹-NHCOO-R²-Z-R³-OCO-R⁴-, -R¹-NHCOO-R²-Z-R³-COO-R⁴-, -R¹-NHCOO-R²-Z-R³-NHCO-R⁴-, -R¹-NHCOO-R²-Z-R³-CONH-R⁴-, -R¹-OCONH-R²-Z-R³-OCO-R⁴-, -R¹-OCONH-R²-Z-R³-COO-R⁴-, -R¹-OCONH-R²-Z-R³-NHCO-R⁴-, -R¹-OCONH-R²-Z-R³-CONH-R⁴-, -R¹-NHCONH-R²-Z-R³-NHCONH-R⁴-, -R¹-OCO-R²-Z-R³-NHCONH-R⁴-, -R¹-NHCO-R²-Z-R³-NHCONH-R⁴-, -R¹-COO-R²-Z-R³-NHCONH-R⁴-, -R¹-CONH-R²-Z-R³-NHCONH-R⁴-, -R¹-NHCOO-R²-Z-R³-NHCONH-R⁴-, or -R¹-OCONH-R²-Z-R³-NHCONH-R⁴-;
**R²** and **R³** represent a bond; **R¹** and **R⁴** represent -CH₂-;
and **BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-OCO-R²-Z-R³-COO-R⁴-, -R¹-COO-R²-Z-R³-OCO-R⁴-, -R¹-OCO-R²-Z-R³-OCO-R⁴-, -R¹-COO-R²-Z-R³-COO-R⁴-, -R¹-NHCO-R²-Z-R³-CONH-R⁴-, -R¹-CONH-R²-Z-R³-NHCO-R⁴-, -R¹-NHCO-R²-Z-R³-NHCO-R⁴-, -R¹-CONH-R²-Z-R³-CONH-R⁴-, -R¹-NHCOO-R²-Z-R³-OCONH-R⁴-, -R¹-OCONH-R²-Z-R³-NHCOO-R⁴-, -R¹-NHCOO-R²-Z-R³-NHCOO-R⁴-, -R¹-OCONH-R²-Z-R³-OCONH-R⁴-, -R¹-OCO-R²-Z-R³-NHCO-R⁴-, -R¹-OCO-R²-Z-R³-CONH-R⁴-, -R¹-OCO-R²-Z-R³-NHCOO-R⁴-, -R¹-OCO-R²-Z-R³-OCONH-R⁴-, -R¹-COO-R²-Z-R³-NHCO-R⁴-, -R¹-COO-R²-Z-R³-CONH-R⁴-, -R¹-COO-R²-Z-R³-NHCOO-R⁴-, -R¹-COO-R²-Z-R³-OCONH-R⁴-, -R¹-NHCO-R²-Z-R³-OCO-R⁴-, -R¹-NHCO-R²-Z -R³-COO-R⁴-, -R¹-NHCO-R²-Z-R³-NHCOO-R⁴-, -R¹-NHCO-R²-Z-R³-OCO-NH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴-, -R¹-CONH-R²-Z-R³-OCONH-R⁴-, -R¹-NHCOO-R²-Z-R³-OCO-R⁴-, -R¹-NHCOO-R²-Z-R³-COO-R⁴-, -R¹-NHCOO-R²-Z-R³-NHCO-R⁴-, -R¹-NHCOO-R²-Z-R³-CONH-R⁴-, -R¹-OCONH-R²-Z-R³-OCO-R⁴-, -R¹-OCONH-R²-Z-R³-COO-R⁴-, -R¹-OCO-NH-R²-Z-R³-NHCO-R⁴-, -R¹-OCO-NH-R²-Z-R³-CONH-R⁴-, -R¹-NHCONH-R²-Z-R³-NHCONH-R⁴-, -R¹-OCO-R²-Z-R³-NHCONH-R⁴-,-R¹-NHCO-R²-Z-R³-NHCONH-R⁴-, -R¹-COO-R²-Z-R³-NHCONH-R⁴-, -R¹-CONH-R²-Z-R³-NHCONH-R⁴-, -R¹-NHCOO-R²-Z-R³-NHCONH-R⁴-, or -R¹-OCONH-R²-Z-R³-NHCONH-R⁴-;
**R² and R³** represent -CH₂- ; **R¹** and **R⁴** represent a bond;
and **BP¹, BP², and Z** are as defined herein.

Preferably, the present invention relates to crosslinkers of general formula **(I):**

**BP¹-L¹-Z-L²-BP²** **(I)**

wherein **-L¹-Z-L²-** represents
-OCO-R²-Z-R³-COO-, -COO-R²-Z-R³-OCO-, -OCO-R²-Z-R³-OCO-, -COO-R²-Z-R³-COO-, -NHCO-R²-Z-R³-CONH-, -CONH-R²-Z-R³-NHCO-, -NHCO-R²-Z-R³-NHCO-, -CONH-R²-Z-R³-CONH-, -NHCOO-R²-Z-R³-OCONH-, -OCONH-R²-Z-R³-NHCOO-, -NHCOO-R²-Z-R³-NHCOO-, or -OCONH-R²-Z-R³-OCONH-;
and **BP¹, BP², Z, R²** and **R³** are as defined herein.

Preferably, the present invention relates to crosslinkers of general formula (**I**):

**BP¹-L¹-Z-L²-BP²** (**I**)

wherein **-L¹-Z-L²-** represents
-OCOCH₂-Z-CH₂COO-, -COOCH₂₋Z-CH₂OCO-, -OCOCH₂-Z-CH₂OCO-, -COOCH₂-Z-CH₂COO-, -NHCOCH₂-Z-CH₂CONH-, -CONHCH₂-Z-CH₂NHCO-, -NHCOCH₂-Z-CH₂NHCO-, -CONHCH₂-Z-CH₂CONH-, -NH-COOCH₂-Z-CH₂-OCONH-, -OCONH-CH₂-Z-CH₂-NHCOO-, -NHCOOCH₂-Z-CH₂NHCOO-, or -OCONHCH₂-Z-CH₂OCONH-;
and **BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-R¹-OCOCH₂Z-CH₂COO-R⁴-, -R¹-COOCH₂Z-CH₂OCO-R⁴-, -R¹-OCOCH₂Z-CH₂OCO-R⁴-, -R¹-COOCH₂-Z-CH₂COO-R⁴-, -R¹-NHCOCH₂Z-CH₂CONH-R⁴-, -R¹-CONHCH₂Z-CH₂NHCO-R⁴-, -R¹-NHCOCH₂-Z-CH₂NHCO-R⁴-, -R¹-CONHCH₂-Z-CH₂CONH-R⁴-, -R¹-NHCOO-CH₂-Z-CH₂OCONH-R⁴-, -R¹-OCONH-CH₂-Z-CH₂NHCOO-R⁴-, -R¹-NHCOOCH₂-Z-CH₂NHCOO-R⁴-, or -R¹-OCONHCH₂-Z-CH₂OCONH-R⁴-; and **BP¹, BP², Z, R¹** and **R⁴**are as defined herein.

Preferably, the present invention relates to crosslinkers of general formula (**I**):

**BP¹-L¹-Z-L²-BP²** (**I**)

wherein **-L¹-Z-L²-** represents
-CH₂OCO-R²-Z-R³-COOCH₂-, -CH₂COO-R²-Z-R³-OCOCH₂-, -CH₂OCO-R²-Z-R³-OCOCH₂-, -CH₂COO-R²-Z-R³-COOCH₂-, -CH₂NHCO-R²-Z-R³-CONHCH₂, -CH₂CONH-R²-Z-R³-NHCOCH₂-, -CH₂NHCO-R²-Z-R³-NHCOCH₂-, -CH₂CONH-R²-Z-R³-CONHCH₂-, -CH₂NHCOO-R²-Z-R³-OCONHCH₂-, -CH₂OCONH-R²-Z-R³-NHCOOCH₂, -CH₂-NHCOO-R²-Z-R³-NHCOOCH₂-, or -CH₂OCONH-R²-Z-R³-OCONHCH₂-; and **BP¹, BP², Z**, **R²** and **R³** are as defined herein.

Preferably, the present invention relates to crosslinkers of general formula (**I**):

**BP¹-L¹-Z-L²-BP²** (**I**)

wherein **-L¹-Z-L²-** represents
-R¹-OCO-Z-COO-R⁴-, -R¹-COO-Z-OCO-R⁴-, -R¹-OCO-Z-OCO-R⁴-, -R¹-COO-Z-COO-R⁴- -R¹-NHCO-Z-CONH-R⁴-, -R¹-CONH-Z-NHCO-R⁴-, -R¹-NHCO-Z-NHCO-R⁴-, -R¹-CONH-Z-CONH-R⁴-, -R¹-NHCOO-Z-OCONH-R⁴-, -R¹-OCONH-Z-NHCOO-R⁴-, -R¹-NHCOO-Z-NHCOO-R⁴-, or -R¹-OCONH-Z-OCONH-R⁴-;
and **BP¹, BP², Z, R¹,** and **R⁴**are as defined herein.

Preferably, the present invention relates to crosslinkers of general formula (**I**):

**BP¹-L¹-Z-L²-BP²** (**I**)

wherein **-L¹-Z-L²-** represents
-CH₂OCO-Z-COOCH₂-, -CH₂COO-Z-OCOCH₂-, -CH₂OCO-Z-OCOCH₂, -CH₂COO-Z-COOCH₂-, -CH₂NHCO-Z-CONHCH₂-, -CH₂CONH-Z-NHCOCH₂-, -CH₂NHCO-Z-NHCOCH₂-, -CH₂CONH-Z-CONHCH₂-, -CH₂NHCOO-Z-OCONHCH₂-, -CH₂OCONH-Z-NHCOOCH₂, -CH₂NHCOO-Z-NHCOOCH₂-, or -CH₂OCO-NH-Z-OCONHCH₂-; and **BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-OCO-Z-COO-, -COO-Z-OCO-, -OCO-Z-OCO-, -COO-Z-COO-, -NHCO-Z-CONH-, -CONH-Z-NHCO-, -NHCO-Z-NHCO-, -CONH-Z-CONH-, -NHCOO-Z-OCONH-, -OCONH-Z-NHCOO-, -NHCOO-Z-NHCOO-, and -OCONH-Z-OCONH-; and **BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents -CH₂OCO-Z-COOCH₂, -CH₂COO-Z-OCOCH₂-, -CH₂OCO-Z-OCOCH₂, -CH₂COO-Z-COOCH₂-, -CH₂NHCO-Z-CONHCH₂-, -CH₂CONH-Z-NHCOCH₂-, -CH₂NHCO-Z-NHCOCH₂-, or -CH₂CONH-Z-CONHCH₂-; and **BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents -OCO-Z-COO-, -COO-Z-OCO-, -OCO-Z-OCO-, -COO-Z-COO-, -NHCO-Z-CONH-, -CONH-Z-NHCO-, -NHCO-Z-NHCO-, or -CONH-Z-CONH-; and **BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents -CH₂NHCO-Z-CONHCH₂-, -CH₂CONH-Z-NHCOCH₂-, -CH₂NHCO-Z-NHCOCH₂-, or -CH₂CONH-Z-CONHCH₂-, and **BP¹, BP²,** and **Z** are as defined herein.

Preferably, the present invention relates to crosslinkers of general formula (**I**):

**BP¹-L¹-Z-L²-BP²** (**I**)

wherein **-L¹-Z-L²-** represents
-NHCO-Z-CONH-, -CONH-Z-NHCO-, -NHCO-Z-NHCO-, or -CONH-Z-CONH-;
and **BP¹, BP²,** and **Z** are as defined herein.

### Alkyl-Linker L¹ and L²

In some preferred embodiments, the linkers **L¹** and **L²** may be alkyl linkers including a functional group selected from -OCO-, -NHCO-, -COO-, -CONH-, -NHCOO-, -O-CO-NH-, -O-CO-O-, and -NHCONH-. Thus, the present invention also relates to crosslinkers of general formula (I):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, or -C₆H₁₂R⁵CH₂-;
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, or -CH₂R⁶C₆H₁₂-;
**R⁵** and **R⁶** independently of each other represent -NHCO-, -CONH-, -OCO-, -COO-, -NHCOO-, -OCONH-, -OCOO-, or -NHCONH-; and
**BP¹, BP²** and **Z** are as defined above.

In preferred embodiments, the present invention relates to crosslinkers of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, -CH₂R⁵-, -CH₂CH₂R⁵-, -CH₂CH₂CH₂R⁵-, -CH(CH₃)R⁵-, -C[(CH₃)_{2]}R⁵-, -CH₂CH(CH₃)R⁵-, -CH(CH₃)CH₂R⁵-, -CH(CH₂CH₃)R⁵-, -CH₂CH₂CH₂CH₂R⁵-, -CH₂C[(CH₃)_{2]}R⁵-, -C[(CH₃)_{2]}CH₂R⁵-, -CH(CH₃)CH₂CH₂R⁵-, -CH₂CH₂CH(CH₃)R⁵-, -CH₂CH(CH₂CH₃)R⁵-, -CH(CH₂CH₃)CH₂R⁵-, -CH₂CH(CH₃)CH₂R⁵-, -CH₂CH₂CH₂CH₂CH₂R⁵-, -CH₂CH(CH₃)CH(CH₃)R⁵-, -CH₂C[(CH₃)₂]CH₂R⁵-, -CH(CH₃)CH(CH₃)CH₂R⁵-, -C[(CH₃)_{2]}C(CH₃)R⁵-,-C(CH₃)C[(CH₃)₂]R⁵-, -CH(CH₃)CH(CH₃)R⁵-, -CH(CH₃)CH₂CH₂CH₂R⁵-, -C(CH₃)₂ CH₂CH₂R⁵-, -C(CH₂CH₃)₂R⁵-, -CH₂CH(CH₃)C₂H₄R⁵-, -CH(CH₃)C(CH₃)₂R⁵-, -CH₂CH₂C(CH₃)₂R⁵-, -CH(CH₃)₂C(CH₃)₂R⁵-, -CH₂CH₂CH₂CH₂CH₂CH₂R⁵-, -CH(CH₃)CH(CH₃)CH(CH₃)R⁵-, -CH₂CH₂CH₂CH(CH₃)₂R⁵-, -CH₂CH₂CH(CH₃)CH₂CH₂R⁵-, -CH₂CH(CH₃)CH₂CH₂CH₂R⁵-, -CH(CH₃)CH₂CH₂CH₂CH₂R⁵-, -CH(CH₃)CH₂C(CH₃)₂R⁵-, -C(CH₃)₂CH₂CH₂CH₂R⁵-, -CH(CH₃)CH(CH₃)CH₂CH₂R⁵-, -CH₂CH(CH₃)CH(CH₃)₂R⁵-, -CH₂C(CH₃)₂CH₂CH₂R⁵-, -C(CH₃)₂C(CH₃)₂R⁵-, -CH₂C(CH₂CH₃)₂R⁵, or -CH₂CH₂CH₂CH₂CH₂CH₂CH₃R⁵-; and/or
R³ represents a bond, -CH₂-, -R⁶CH₂-, -R⁶CH₂CH₂-, -R⁶CH₂CH₂CH₂-, -R⁶CH(CH₃)-, -R⁶C[(CH₃)₂]-, -R⁶CH₂CH(CH₃)-, -R⁶CH(CH₃)CH₂-, -R⁶CH(CH₂CH₃)-, -R⁶CH₂CH₂CH₂CH₂-, -R⁶CH₂C[(CH₃)₂]-, -R⁶C[(CH₃)₂]CH₂-, -R⁶CH(CH₃)CH₂CH₂-, -R⁶CH₂CH₂CH(CH₃)-, -R⁶CH₂CH(CH₂CH₃) -, -R⁶CH(CH₂CH₃)CH₂-, -R⁶CH₂CH(CH₃)CH₂-, -R⁶CH₂CH₂CH₂CH₂CH₂-, -R⁶CH₂CH(CH₃)CH(CH₃)-, -R⁶CH₂C[(CH₃)₂]CH₂-, -R⁶CH(CH₃)CH(CH₃)CH₂-, -R⁶C[(CH₃)₂]C(CH₃)-, - R⁶C(CH₃)C[(CH₃)₂] -, -R⁶CH(CH₃)CH(CH₃) -, -R⁶CH(CH₃)CH₂CH₂CH₂-, -R⁶C(CH₃)₂ CH₂CH₂-, -R⁶C(CH₂CH₃)₂-, -R⁶CH₂CH(CH₃)C₂H₄-, -R⁶CH(CH₃)C(CH₃)₂-, -R⁶CH₂CH₂C(CH₃)₂-, -R⁶CH(CH₃)₂C(CH₃)₂-, -R⁶CH₂CH₂CH₂CH₂CH₂CH₂-, -R⁶CH(CH₃)CH(CH₃)CH(CH₃)-, -R⁶CH₂CH₂CH₂CH(CH₃)₂-, -R⁶CH₂CH₂CH(CH₃)CH₂CH₂-, -R⁶CH₂CH(CH₃)CH₂CH₂CH₂-, -R⁶CH(CH₃)CH₂CH₂CH₂CH₂-, -R⁶CH(CH₃)CH₂C(CH₃)₂-, -R⁶C(CH₃)₂CH₂CH₂CH₂-, -R⁶CH(CH₃)CH(CH₃)CH₂CH₂-, -R⁶CH₂CH(CH₃)CH(CH₃)₂-, -R⁶CH₂C(CH₃)₂CH₂CH₂-, -R⁶C(CH₃)₂C(CH₃)₂-, -R⁶CH₂C(CH₂CH₃)₂, or -R⁶CH₂CH₂CH₂CH₂CH₂CH₂CH₃-;
**R⁵** and **R⁶** independently of each other represent -NHCO-, -CONH-, -OCO-, -COO-, -NHCOO-, -OCONH-, -OCOO-, or -NHCONH-; and
**BP¹, BP²** and **Z** are as defined above.

In preferred embodiments, the present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, -CH₂R⁵-, -CH₂CH₂R⁵-, -CH₂CH₂CH₂R⁵-, -CH(CH₃)R⁵-, -C[(CH₃)₂]R⁵-, -CH₂CH(CH₃)R⁵-, -CH(CH₃)CH₂R⁵-, -CH(CH₂CH₃)R⁵-, -CH₂CH₂CH₂CH₂R⁵-, -CH₂C[(CH₃)₂]R⁵-, -C[(CH₃)₂]CH₂R⁵-, -CH(CH₃)CH₂CH₂R⁵-, -CH₂CH₂CH(CH₃)R⁵-, -CH₂CH(CH₂CH₃)R⁵-, -CH(CH₂CH₃)CH₂R⁵-, -CH₂CH(CH₃)CH₂R⁵-, -CH₂CH₂CH₂CH₂CH₂R⁵-, -CH₂CH(CH₃)CH(CH₃)R⁵-, -CH₂C[(CH₃)₂]CH₂R⁵-, -CH(CH₃)CH(CH₃)CH₂R⁵-, -C[(CH₃)₂]C(CH₃)R⁵-,-C(CH₃)C[(CH₃)₂]R⁵-, -CH(CH₃)CH(CH₃)R⁵-, -CH(CH₃)CH₂CH₂CH₂R⁵-, -C(CH₃)₂ CH₂CH₂R⁵-, -C(CH₂CH₃)₂R⁵-, -CH₂CH(CH₃)C₂H₄R⁵-, -CH(CH₃)C(CH₃)₂R⁵-, -CH₂CH₂C(CH₃)₂R⁵-, -CH(CH₃)₂C(CH₃)₂R⁵-, -CH₂CH₂CH₂CH₂CH₂CH₂R⁵-, -CH(CH₃)CH(CH₃)CH(CH₃)R⁵-, or -CH₂CH₂CH₂CH(CH₃)₂R⁵-;
   preferably
**R²** represents a bond, -CH₂-, -CH₂R⁵-, -CH₂CH₂R⁵-, -CH₂CH₂CH₂R⁵-, -CH₂CH₂CH₂CH₂R⁵-, -CH₂CH₂CH₂CH₂CH₂R⁵-, or -CH₂CH₂CH₂CH₂CH₂CH₂R⁵-;
   more preferably
**R²** represents a bond, -CH₂-, -CH₂R⁵-, -CH₂CH₂R⁵-, or -CH₂CH₂CH₂R⁵-;
   more preferably
**R²** represents a bond, -CH₂-, or -CH₂CH₂R⁵-;
   most preferably
**R²** represents a bond, or -CH₂; and/or
**R³** represents a bond, -CH₂-, -R⁶CH₂-, -R⁶CH₂CH₂-, -R⁶CH₂CH₂CH₂-, -R⁶CH(CH₃)-, -R⁶C[(CH₃)₂]-, -R⁶CH₂CH(CH₃)-, -R⁶CH(CH₃)CH₂-, -R⁶CH(CH₂CH₃)-, -R⁶CH₂CH₂CH₂CH₂-, -R⁶CH₂C[(CH₃)₂]-, -R⁶C[(CH₃)₂]CH₂-, -R⁶CH(CH₃)CH₂CH₂-, -R⁶CH₂CH₂CH(CH₃)-, -R⁶CH₂CH(CH₂CH₃) -, -R⁶CH(CH₂CH₃)CH₂-, -R⁶CH₂CH(CH₃)CH₂-, -R⁶CH₂CH₂CH₂CH₂CH₂-, -R⁶CH₂CH(CH₃)CH(CH₃)-, -R⁶CH₂C[(CH₃)₂]CH₂-, -R⁶CH(CH₃)CH(CH₃)CH₂-, -R⁶C[(CH₃)₂]C(CH₃)-, - R⁶C(CH₃)C[(CH₃)₂] -, -R⁶CH(CH₃)CH(CH₃) -, -R⁶CH(CH₃)CH₂CH₂CH₂-, -R⁶C(CH₃)₂ CH₂CH₂-, -R⁶C(CH₂CH₃)₂-, -R⁶CH₂CH(CH₃)C₂H₄-, -R⁶CH(CH₃)C(CH₃)₂-, -R⁶CH₂CH₂C(CH₃)₂-, -R⁶CH(CH₃)₂C(CH₃)₂-, or -R⁶CH₂CH₂CH₂CH₂CH₂CH₂-;
   preferably
**R³** represents a bond, -CH₂-, -R⁶CH₂-, -R⁶CH₂CH₂-, -R⁶CH₂CH₂CH₂-, -R⁶CH₂CH₂CH₂CH₂-, -R⁶CH₂CH₂CH₂CH₂CH₂-, or -R⁶CH₂CH₂CH₂CH₂CH₂CH₂-;
   more preferably
**R³** represents a bond, -CH₂-, -R⁶CH₂-, -R⁶CH₂CH₂-, or -R⁶CH₂CH₂CH₂-;
   more preferably
**R³** represents a bond, -CH₂-, or -R⁶CH₂CH₂;
   most preferably
**R³** represents a bond, or -CH₂;
**R⁵** and **R⁶** independently of each other represent -NHCO-, -CONH-, -OCO-, -COO-, -NHCOO-, -OCONH-, -OCOO-, or -NHCONH-;
**BP¹, BP²** and **Z** are as defined above.

In preferred embodiments, **R⁵** and **R⁶** independently of each other may represent -NHCO-, -CONH-, -OCO-, -COO-, -NHCOO-, or -OCONH.

In further preferred embodiments, **R⁵** and **R⁶** independently of each other may represent -NHCO-, -CONH-, -OCO-, or -COO-.

In most preferred embodiments, **R⁵** and **R⁶** independently of each other may represent -NHCO-, or -CONH-.

Thus, the present invention also relates to crosslinkers of general formula (**I**):

**BP¹-L1-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, or -C₆H₁₂R^{s}CH₂-;
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, or -CH₂R⁶C₆H₁₂-;
**R⁵** and **R⁶** independently of each other represent -NHCO-, -CONH-, -OCO-, -COO-, -NHCOO-, -OCONH-, -OCOO-, or -NHCONH-,
   preferably
**R⁵** and **R⁶** independently of each other may represent -NHCO-, -CONH-, -OCO-, -COO-, -NHCOO-, or -OCONH,
   more preferably
**R⁵** and **R⁶** independently of each other may represent -NHCO-, -CONH-, -OCO-, or -COO-,
   most preferably
**R⁵** and **R⁶** independently of each other may represent -NHCO-, or -CONH-; and
**BP¹, BP²** and **Z** are as defined above.

Thus, in preferred embodiments, the present invention relates to crosslinkers of general formula (**I**):

**BP¹-L1-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, or -R¹-OCONH-R²-, ;
**L²** represents -R³-NHCO-R⁴-, -R³-CONH-R⁴-, -R³-OCO-R⁴-,-R³-COO-R⁴-, -R³-NHCOO-R⁴-, or -R³-OCONH-R⁴-;
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, or -C₆H₁₂R^{s}CH₂-;
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, or -CH₂R⁶C₆H₁₂-;
**R⁵** and **R⁶** independently of each other may represent -NHCO-, -CONH-, -OCO-, -COO-, -NHCOO-, or -OCONH,
**BP¹, BP²** and **Z** are as defined above.

Thus, in preferred embodiments, the present invention relates to crosslinkers of general formula (**I**):

**BP¹-L1-Z-L²-BP²** (**I**)

wherein
**L¹** represents -R¹-NHCO-R²-, -R¹-CONH-R²-, -R¹-OCO-R²-, or -R¹-COO-R²-;
**L²** represents -R³-NHCO-R⁴-, -R³-CONH-R⁴-, -R³-OCO-R⁴-, or -R³-COO-R⁴-;
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, or -C₆H₁₂R⁵CH₂-;
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, or -CH₂R⁶C₆H₁₂-;
**R⁵** and **R⁶** independently of each other may represent -NHCO-, -CONH-, -OCO-, or -COO-;
**BP¹**, **BP²** and **Z** are as defined above.

Thus, in preferred embodiments, the present invention relates to crosslinkers of general formula (**I**):

**BP¹-L1-Z-L²-BP²** (**I**)

wherein
**L¹** represents -R¹-OCO-R²-, or -R¹-COO-R²-;
**L²** represents -R³-OCO-R⁴-, or-R³-COO-R⁴-;
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, or -C₆H₁₂R^{s}CH₂-;
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈- -CH₂R⁶C₅H₁₀- or -CH₂R⁶C₆H₁₂-;
**R⁵** and **R⁶** independently of each other may represent -NHCO-, -CONH-, -OCO-, or -COO-;
**BP¹, BP²** and **Z** are as defined above.

Thus, in preferred embodiments, the present invention relates to crosslinkers of general formula (**I**):

**BP¹-L1-Z-L²-BP²** (**I**)

wherein
**L¹** represents -R¹-NHCO-R²-, or-R¹-CONH-R²-;
**L²** represents -R³-NHCO-R⁴-, or -R³-CONH-R⁴-;
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, or -C₆H₁₂R⁵CH₂-;
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, or -CH₂R⁶C₆H₁₂-;
**R⁵** and **R⁶** independently of each other may represent -NHCO-, -CONH-, -OCO-, or -COO-;
**BP¹**, **BP²** and **Z** are as defined above.

Thus, in further preferred embodiments, the present invention relates to crosslinkers of general formula (**I**):

**BP¹-L1-Z-L²-BP²** (**I**)

wherein
**L¹** represents -R¹-NHCO-R²-, or-R¹-CONH-R²-;
**L²** represents -R³-NHCO-R⁴-, or -R³-CONH-R⁴-;
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, or -C₆H₁₂R⁵CH₂-;
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, or -CH₂R⁶C₆H₁₂-;
**R⁵** and **R⁶** independently of each other may represent -NHCO-, or -CONH-;
**BP¹, BP²** and **Z** are as defined above.

In particularly preferred embodiments, the present invention relates to a crosslinker of general formula (**I**):

**BP¹-L1-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** - **R⁴** independently of each other represent a bond or -CH₂-; and **BP¹, BP²** and **Z** are as defined above.

Thus, in particularly preferred embodiments, the present invention relates to a crosslinker of general formula (**I**):

**BP¹-L1-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** - **R⁴** represent a bond; and **BP¹, BP²** and **Z** are as defined above.

Thus, in particularly preferred embodiments, the present invention relates to a crosslinker of general formula (**I**):

**BP¹-L1-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** - **R⁴** represent -CH₂-; and **BP¹, BP²** and **Z** are as defined above.

Thus, in particularly preferred embodiments, the present invention relates to a crosslinker of general formula (**I**):

**BP¹-L1-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** and **R⁴** represent a bond; **R²** and **R³** represent -CH₂-; and **BP¹, BP²** and **Z** are as defined above.

Thus, in particularly preferred embodiments, the present invention relates to a crosslinker of general formula (**I**):

**BP¹-L1-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** and **R⁴** represent -CH₂-;**R²** and **R³** represent a bond; and **BP¹, BP²** and **Z** are as defined above.

Thus, in preferred embodiments, the present invention relates to crosslinkers of general formula (**I**):

**BP¹-L¹-Z-L²-BP²** **(I)**

wherein
**L¹** and **L²** independently of each other represent -OCO-, -NHCO-, -COO-, -CONH-, -NHCOO-, -OCONH-, -OCOO-, -NHCONH-, -CH₂OCO-, -CH₂NHCO-, -CH₂COO-, -CH₂CONH-, -CH₂NHCOO-, -CH₂OCONH-, -CH₂OCOO-, -CH₂NHCONH-, -OCOCH₂-, -NHCOCH₂-, -COOCH₂-, -CONHCH₂-, -NHCOOCH₂-, -OCONHCH₂-, -OCOOCH₂-, -NHCONHCH₂-, -CH₂OCOO-, -CH₂NHCONH-, -CH₂OCOCH₂, -CH₂NHCOCH₂-, -CH₂COOCH₂-, -CH₂CONHCH₂-, -CH₂NHCOOCH₂-, -CH₂OCONHCH₂-, -CH₂OCOOCH₂-, or -CH₂NHCONHCH₂-, and **BP¹, BP²** and **Z** are as defined above.

Thus, in further preferred embodiments, the present invention relates to crosslinkers of general formula (**I**):

**BP¹-L1-Z-L²-BP²** (**I**)

wherein
**L¹** and **L²** independently of each other represent -OCO-, -NHCO-, -COO-, -CONH-, -CH₂OCO-, -CH₂NHCO-, -CH₂COO-, -CH₂CONH-, -OCOCH₂-, -NHCOCH₂-, -COOCH₂-, -CONHCH₂-, -CH₂OCOCH₂-, -CH₂NHCOCH₂-, -CH₂COOCH₂-, -CH₂CONHCH₂-, and **BP¹, BP²** and **Z** are as defined above.

Thus, in even further preferred embodiments, the present invention relates to a crosslinker of general formula (**I**):

**BP¹-L1-Z-L²-BP²** (**I**)

wherein
**L¹** and **L²** independently of each other represent -NHCO-, -CONH-, -CH₂NHCO-, -CH₂CONH-, -NHCOCH₂-, -CONHCH₂-, -CH₂NHCOCH₂-, -CH₂CONHCH₂-, and **BP¹, BP²** and **Z** are as defined above.

### Aryl-Linker L¹ and L²

In preferred embodiments, the present invention relates to crosslinkers of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, 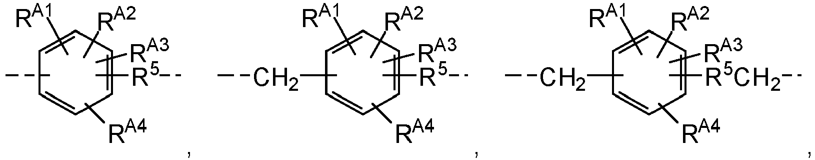 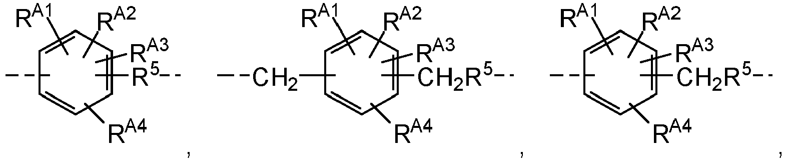 or 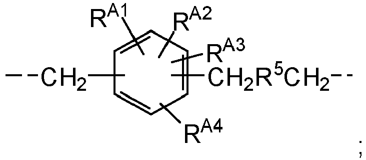
**R³** represents a bond, -CH₂-, 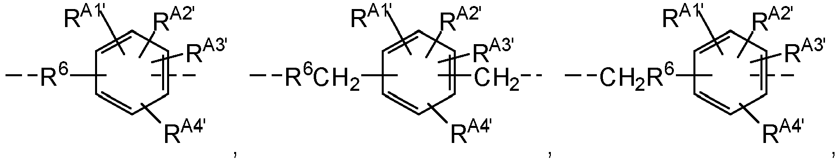 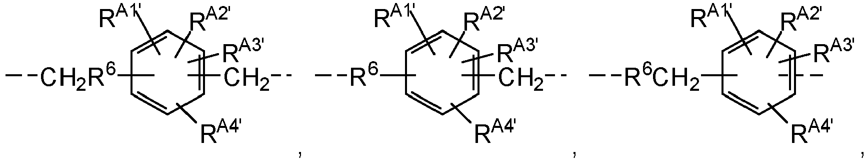 or 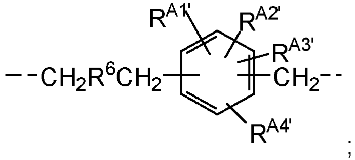
**R⁵** and **R⁶** independently of each other represent -NHCO-, -CONH-, -OCO-, -COO-, -NHCOO-, -OCONH-, -OCOO-, or -NHCONH-; and
**R^{A1}** - **R^{A4}**, and **R^{A1'}** - **R^{A4'}**, independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

An aryl linker represented by 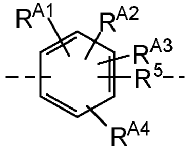 includes the following variations:

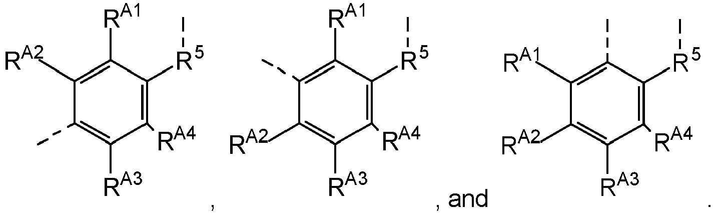

As used herein, a dashed line indicates connectivity and a solid line represents a bond. The same follows analogously for any one of 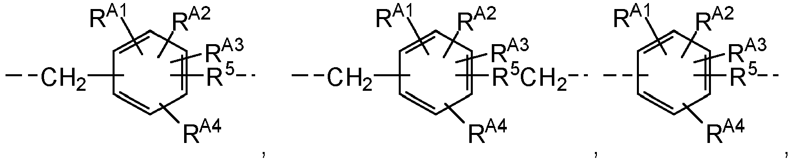 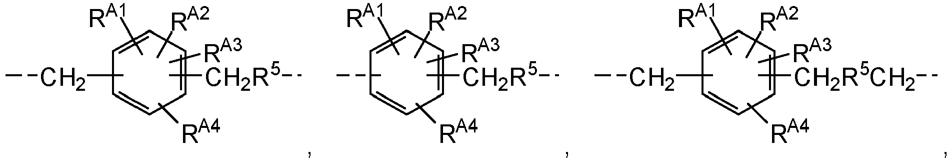 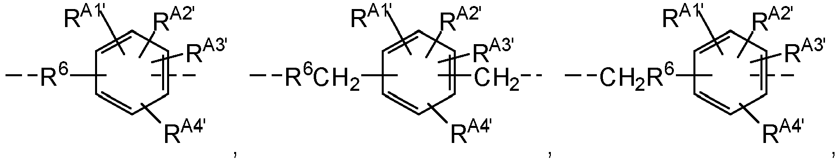 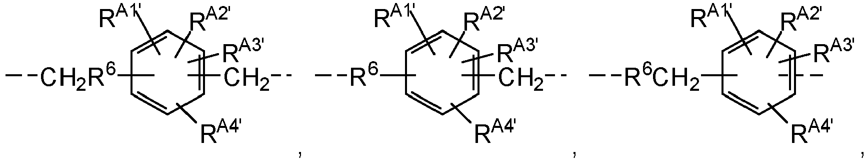 and

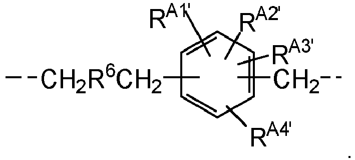

In preferred embodiments, at least one of **R^{A1}** - **R^{A4}** is -H. In preferred embodiments, at least two of **R^{A1}** - **R^{A4}** are -H. In preferred embodiments, at least three of **R^{A1}** - **R^{A4}** are -H. In some preferred embodiments, **R^{A1}** - **R^{A4}** are -H.

Thus, in preferred embodiments, **R²** may represent a bond, -CH₂-, 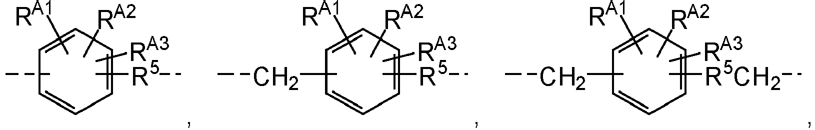 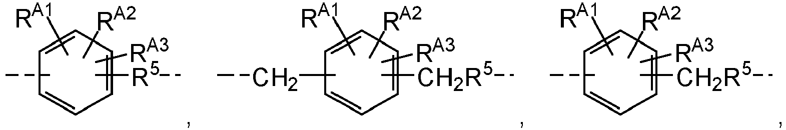 or 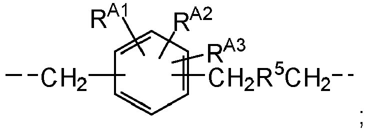 and **R^{A1}** - **R^{A3}** are as defined herein.

In some preferred embodiments, **R**² may represent a bond, -CH₂-, 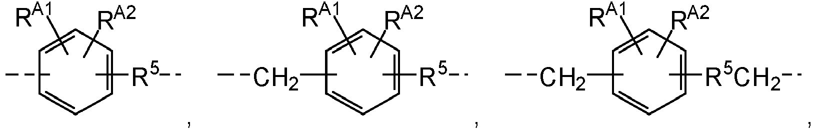 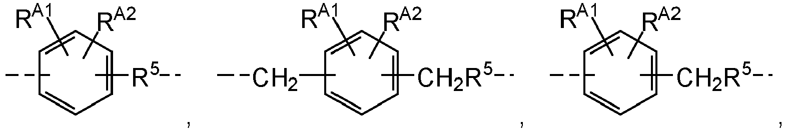 or 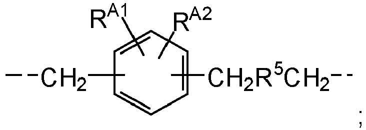 and **R^{A1}** and **R^{A2}** are as defined herein.

In some preferred embodiments, **R²** may represent a bond, -CH₂-, 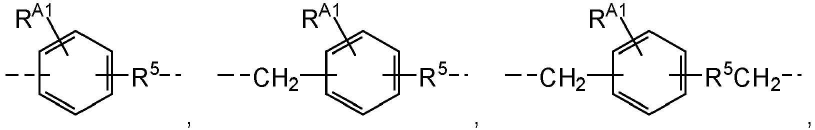 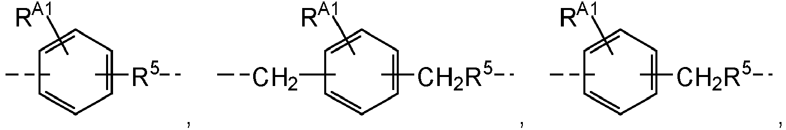 or 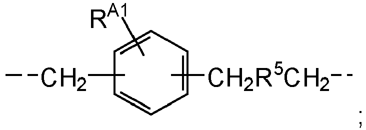 and **R^{A1}** is as defined herein.

Thus, **R²** may represent a bond, -CH₂-, 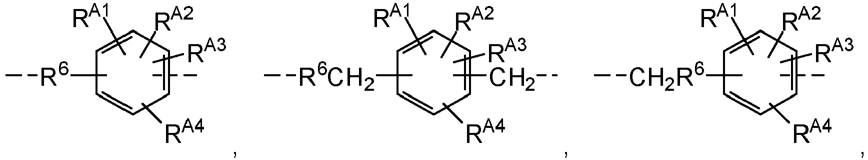 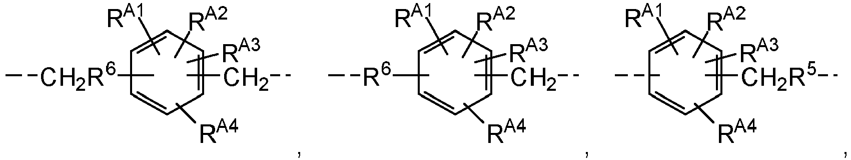 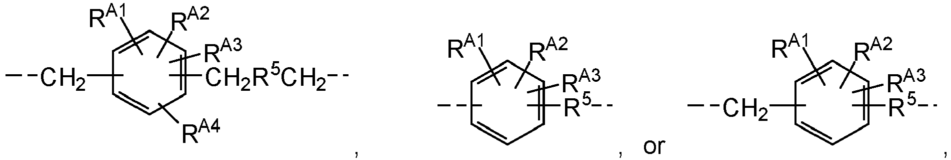 preferably 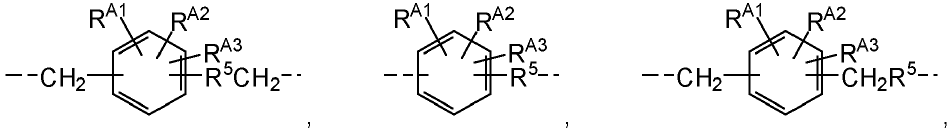 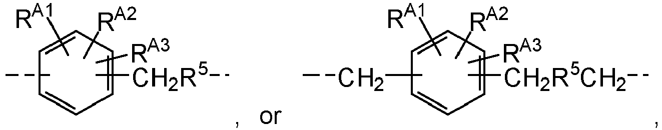 even more preferably 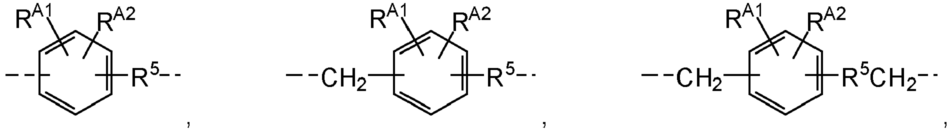 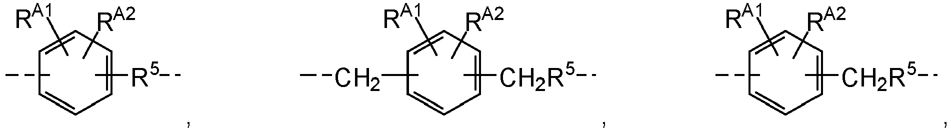 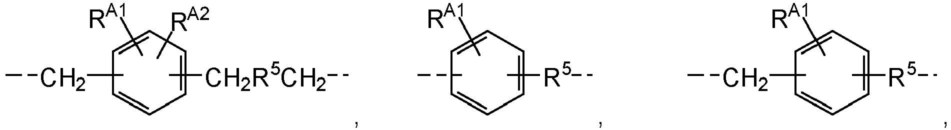 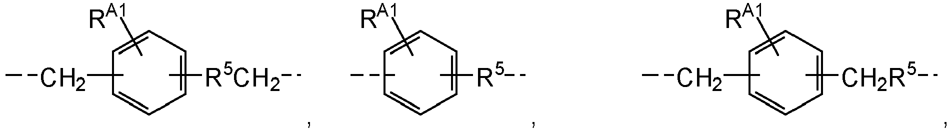 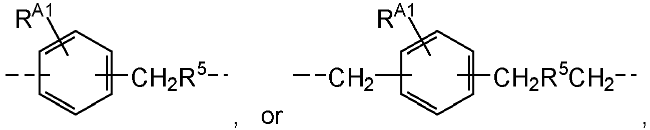 and
**R^{A1}** - **R^{A4}**, independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Thus, in preferred embodiments, **R³** may represent a bond, -CH₂-, 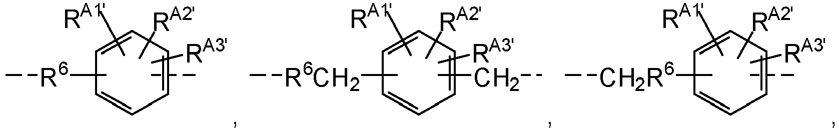 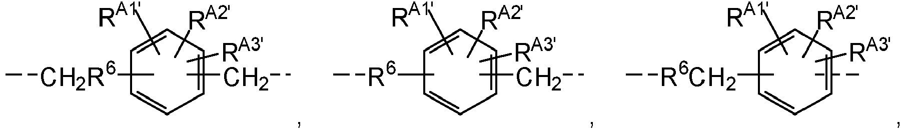 or 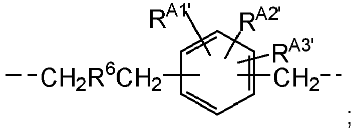 and **R^{A1'}** - **R^{A3'}** are as defined herein.

Thus, in preferred embodiments, **R³** may represent a bond, -CH₂-, 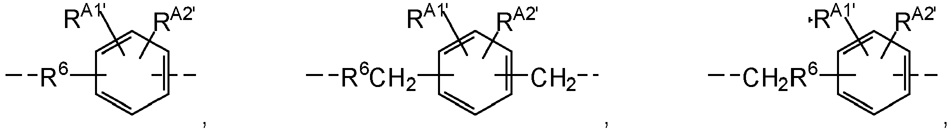 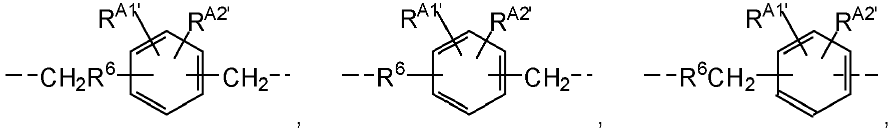 or 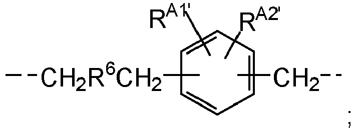 and **R^{A1'}** and **R^{A2'}** are as defined herein.

Thus, in preferred embodiments, **R³** may represent a bond, -CH₂-, 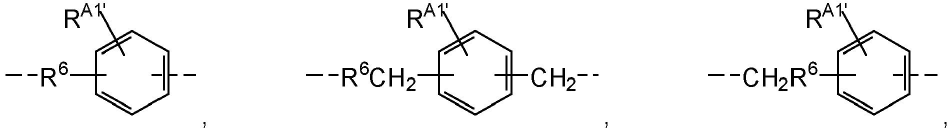 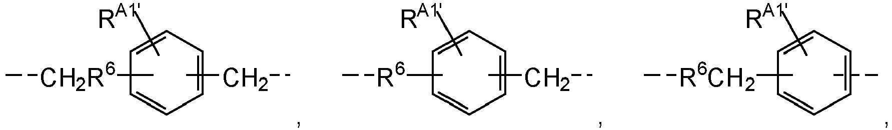 or 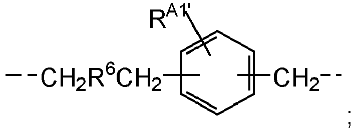 and **R^{A1'}** is as defined herein.

Thus, **R²** may represent a bond, -CH₂-, 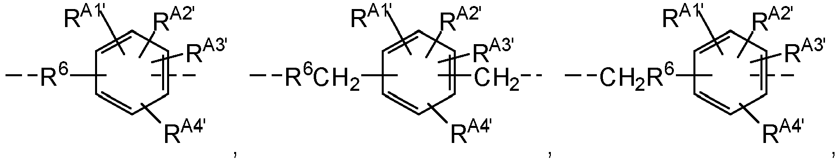 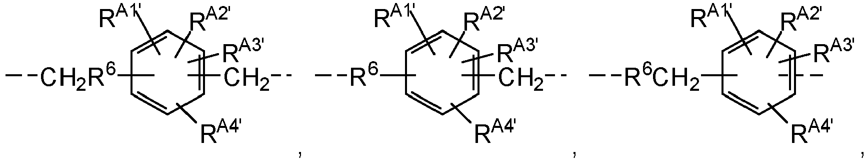 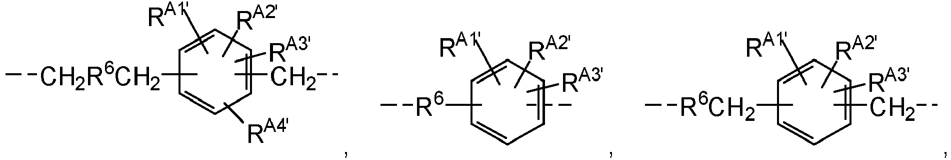 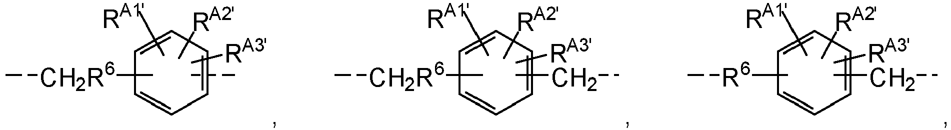 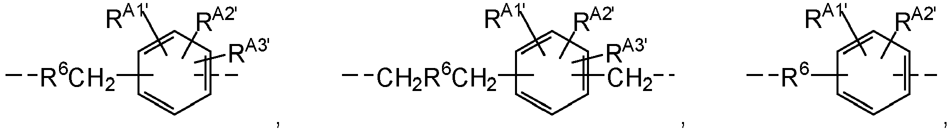 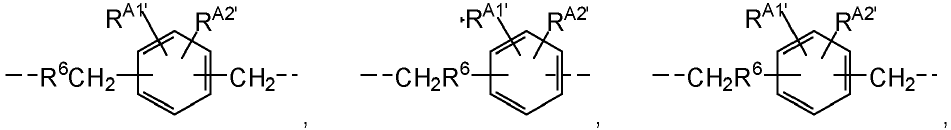 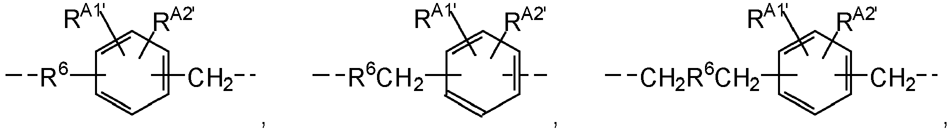 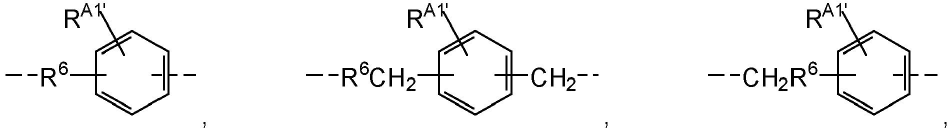 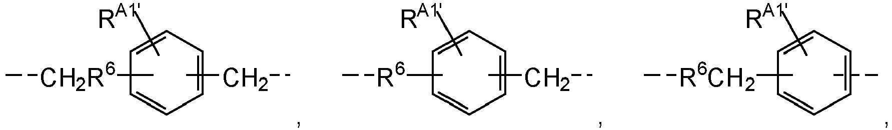 or 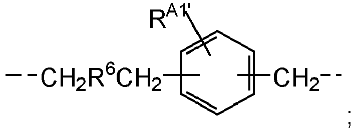 and
**R^{A1'}** - **R^{A4'}**, independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In preferred embodiments, the present invention relates to crosslinkers of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-,-R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, 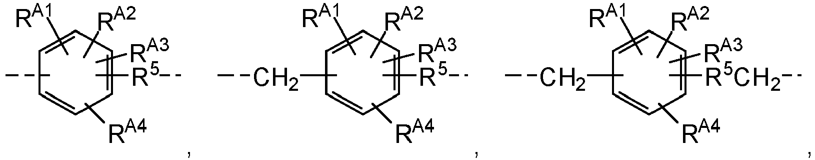 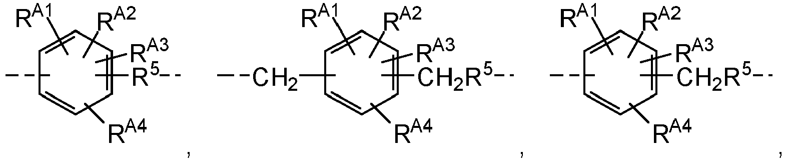 or 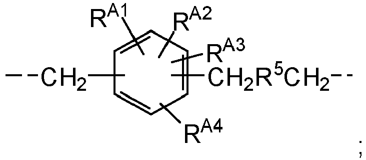
**R³** represents a bond, -CH₂-, 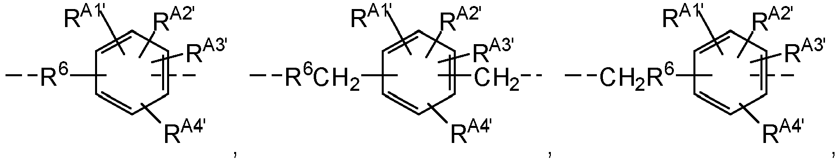 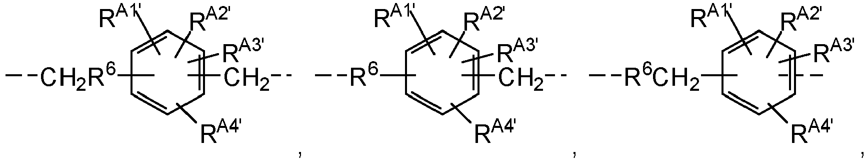 or 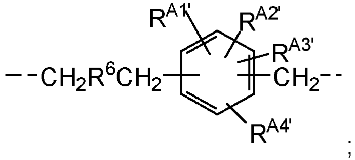
**R⁵** and **R⁶** independently of each other represent -NHCO-, -CONH-, -OCO-, -COO-, -NHCOO-, -OCONH-, -OCOO-, or -NHCONH-; and
   preferably
**R⁵** and **R⁶** independently of each other may represent -NHCO-, -CONH-, -OCO-, -COO-, -NHCOO-, or -OCONH,
   more preferably
**R⁵** and **R⁶** independently of each other may represent -NHCO-, -CONH-, -OCO-, or -COO-,
   most preferably
**R⁵** and **R⁶** independently of each other may represent -NHCO-, or -CONH-; and
**R^{A1}** - **R^{A4}**, and **R^{A1'}** - **R^{A4'}**, independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In preferred embodiments, the present invention relates to crosslinkers of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, or -R¹-OCONH-R²-;
**L²** represents -R³-NHCO-R⁴-, -R³-CONH-R⁴-, -R³-OCO-R⁴-,-R³-COO-R⁴-, -R³-NHCOO-R⁴-, or-R³-OCONH-R⁴-;
**R¹** and **R⁴** independently of each other represent a bond or -CH₂-;
**R²** represents a bond, -CH₂-, 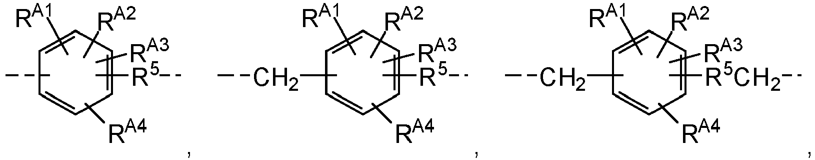 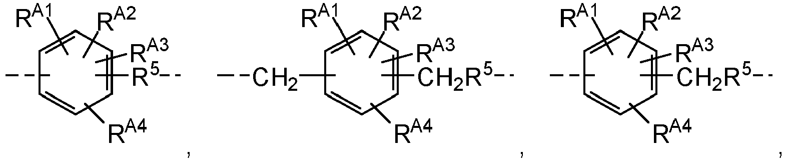 or 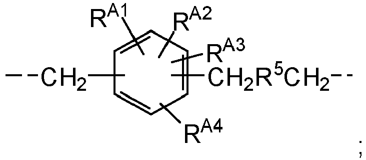
**R³** represents a bond, -CH₂-, 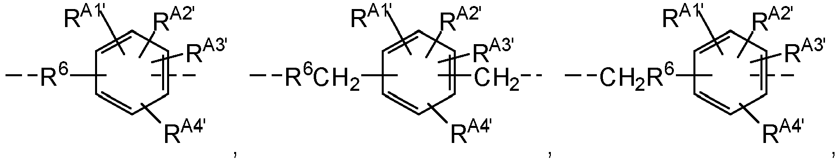 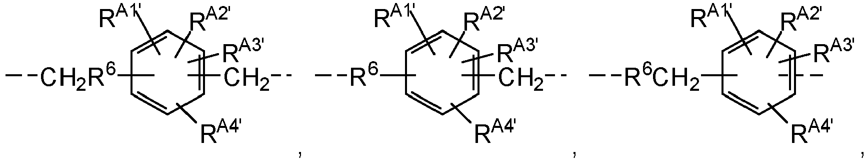 or 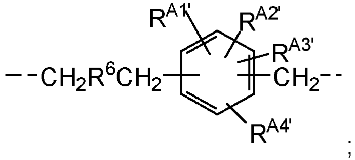
**R⁵** and **R⁶** independently of each other may represent -NHCO-, -CONH-, -OCO-, -COO-, -NHCOO-, or -OCONH,
**R^{A1}** - **R^{A4}**, and **R^{A1'}** - **R^{A4'}**, independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, the present invention relates to crosslinkers of general formula (**I**):

**BP¹-L¹-Z-L²-BP²** (**I**)

wherein
**L¹** represents -R¹-NHCO-R²-, -R¹-CONH-R²-, -R¹-OCO-R²-, or -R¹-COO-R²-;
**L²** represents -R³-NHCO-R⁴-, -R³-CONH-R⁴-, -R³-OCO-R⁴-, or -R³-COO-R⁴-;
**R¹** and **R⁴** independently of each other represent a bond or -CH₂-;
**R²** represents a bond, -CH₂-, 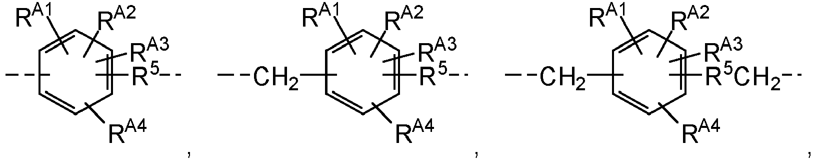 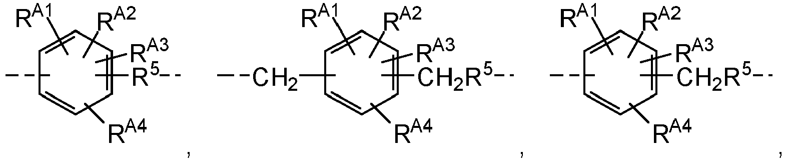 or 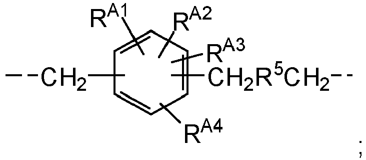
**R³** represents a bond, -CH₂-, 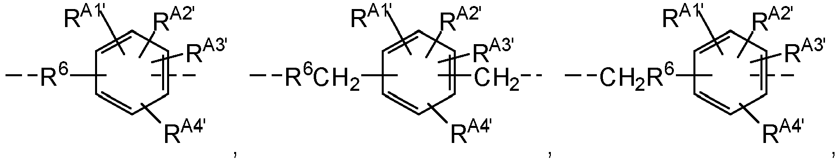 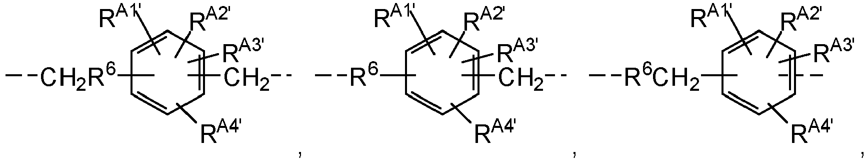 or 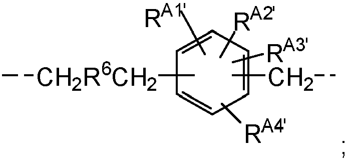
**R⁵** and **R⁶** independently of each other may represent -NHCO-, -CONH-, -OCO-, or -COO-;
**R^{A1}** - **R^{A4}**, and **R^{A1'}** - **R^{A4'}**, independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, the present invention relates to crosslinkers of general formula (**I**):

**BP¹-L1-Z-L²-BP²** (**I**)

wherein
**L¹** represents -R¹-OCO-R²-, or -R¹-COO-R²-;
**L²** represents -R³-OCO-R⁴-, or -R³-COO-R⁴-;
**R¹** and **R⁴** independently of each other represent a bond or -CH₂-;
**R²** represents a bond, -CH₂-, 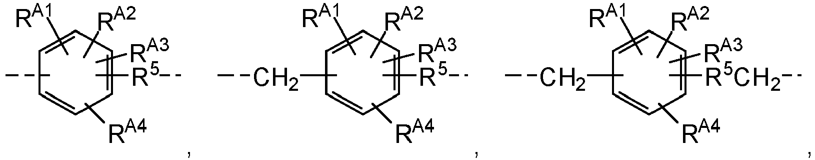 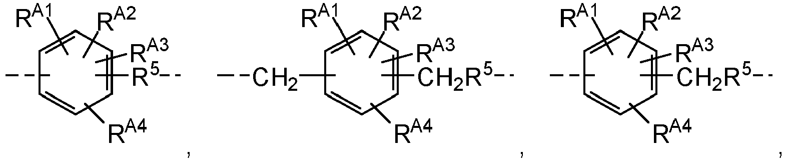 or 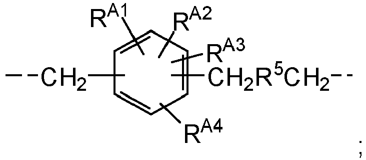
**R³** represents a bond, -CH₂-, 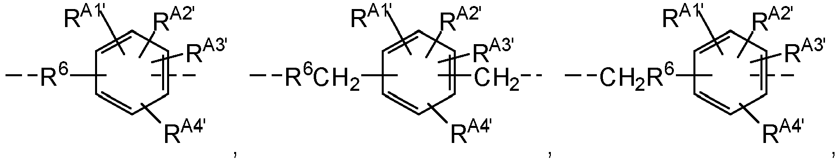 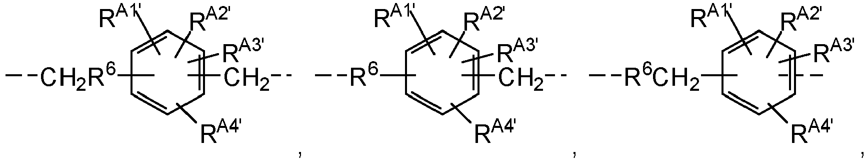 or 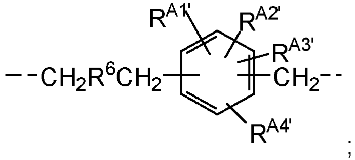
**R⁵** and **R⁶** independently of each other may represent -NHCO-, -CONH-, -OCO-, or -COO-;
**R^{A1}** - **R^{A4}**, and **R^{A1'}** - **R^{A4'}**, independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, the present invention relates to crosslinkers of general formula (**I**):

**BP¹-L¹-Z-L²-BP²** (**I**)

wherein
**L¹** represents -R¹-NHCO-R²-, or-R¹-CONH-R²-;
**L²** represents -R³-NHCO-R⁴-, or -R³-CONH-R⁴-;
**R¹** and **R⁴** independently of each other represent a bond or -CH₂-;
**R²** represents a bond, -CH₂-, 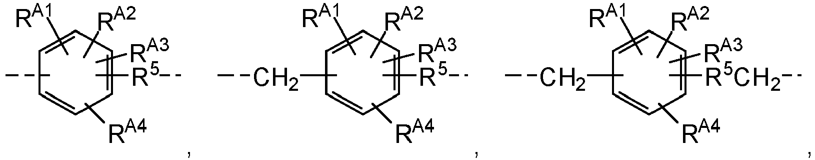 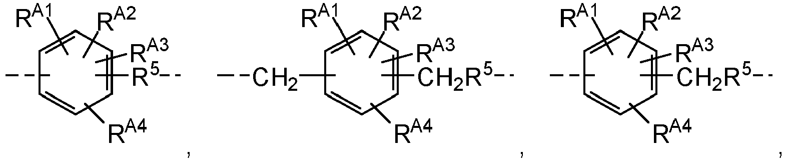 or 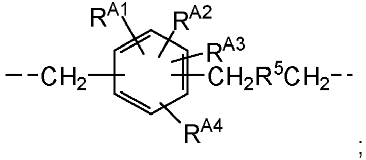
**R³** represents a bond, -CH₂-, 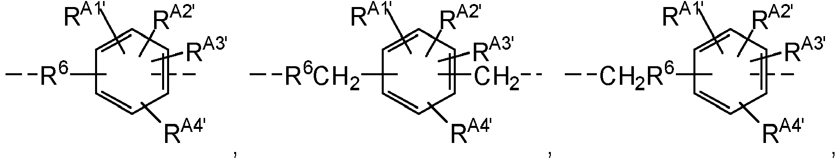 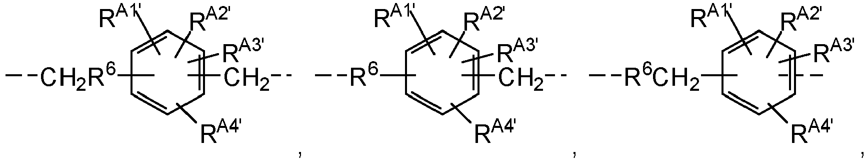 or 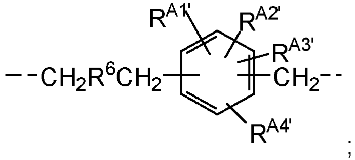
**R⁵** and **R⁶** independently of each other may represent -NHCO-, -CONH-, -OCO-, or -COO-;
**R^{A1}** - **R^{A4}**, and **R^{A1'}** - **R^{A4'}**, independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, the present invention relates to crosslinkers of general formula (**I**):

**BP¹-L1-Z-L²-BP²** (**I**)

wherein
**L¹** represents -R¹-NHCO-R²-, or-R¹-CONH-R²-;
**L²** represents -R³-NHCO-R⁴-, or -R³-CONH-R⁴-;
**R¹** and **R⁴** independently of each other represent a bond or -CH₂-;
**R²** represents a bond, -CH₂-, 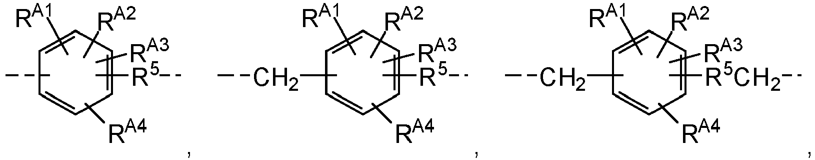 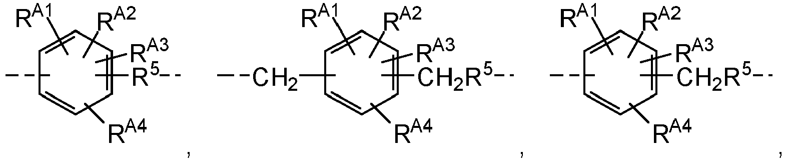 or 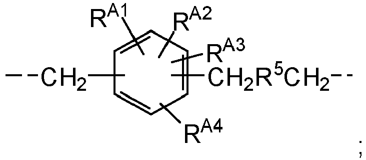
**R³** represents a bond, -CH₂-, 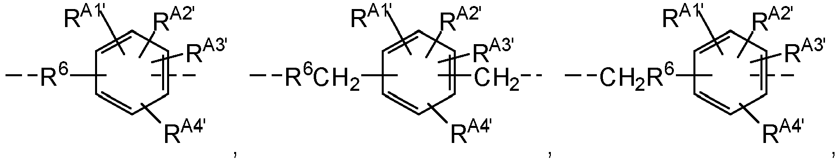 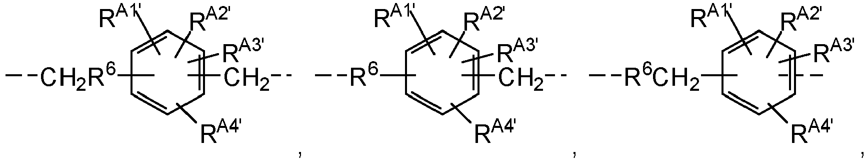 or 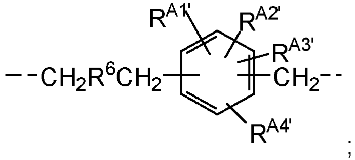
**R⁵** and **R⁶** independently of each other may represent -NHCO-, or -CONH-;
**R^{A1}** - **R^{A4}**, and **R^{A1'}** - **R^{A4'}**, independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

### Combination Alkyl-Linker and Aryl-Linker L¹ and L²

In some preferred embodiments, the present invention relates to crosslinkers of general formula (**I**):

**BP¹-L1-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, or -C₆H₁₂R⁵CH₂-;
**R³** represents a bond, -CH₂-, 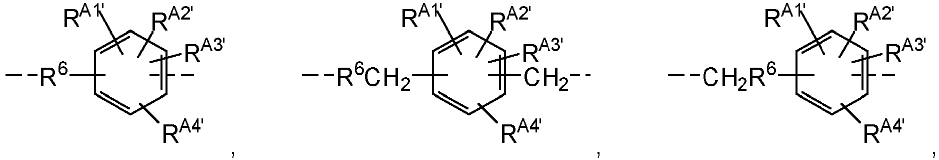 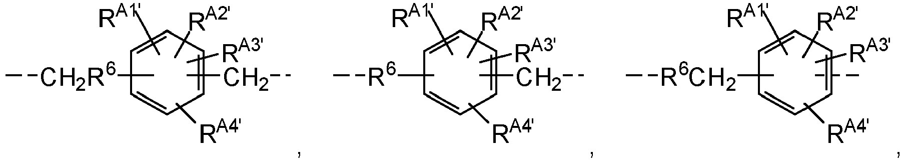 or 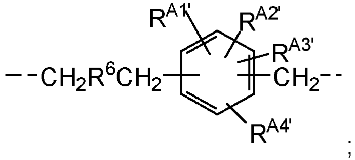
**R⁵** and **R⁶** independently of each other represent -NHCO-, -CONH-, -OCO-, -COO-, -NHCOO-, -OCONH-, -OCOO-, or -NHCONH-;
**R^{A1'}** - **R^{A4'}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃ ; and
**BP¹**, **BP²** and **Z** are as defined herein.

In some preferred embodiments, the present invention relates to crosslinkers of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, or -CH₂;
**R³** represents a bond, -CH₂-, 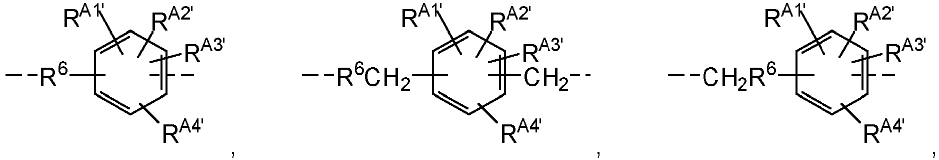 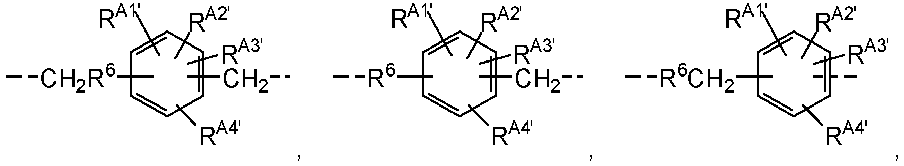 or 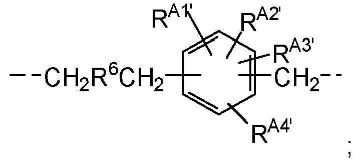
**R⁵** and **R⁶** independently of each other represent -NHCO-, -CONH-, -OCO-, -COO-, -NHCOO-, -OCONH-, -OCOO-, or -NHCONH-;
**R^{A1'}** - **R^{A4'}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃ ; and
**BP¹, BP²** and **Z** are as defined herein.

In some preferred embodiments, the present invention relates to crosslinkers of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, or -C₆H₁₂R⁵CH₂-;
**R³** represents a bond, or -CH₂-,
**R⁵** and **R⁶** independently of each other represent -NHCO-, -CONH-, -OCO-, -COO-, -NHCOO-, -OCONH-, -OCOO-, or -NHCONH-;
**R^{A1'}** - **R^{A4'}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃ ; and
**BP¹**, **BP²** and **Z** are as defined herein.

In some preferred embodiments, the present invention relates to crosslinkers of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²--R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, 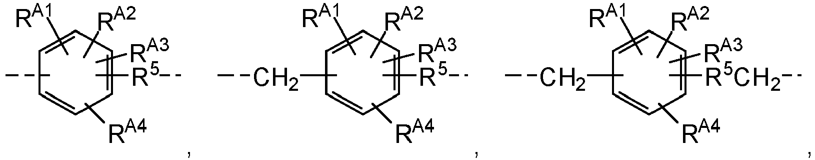 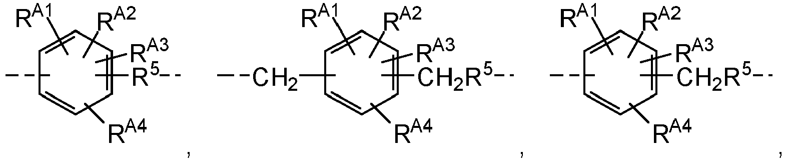 or 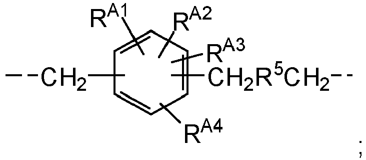
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, -CH₂R⁶C₆H₁₂-;
**R⁵** and **R⁶** independently of each other represent -NHCO-, -CONH-, -OCO-, -COO-, -NHCOO-, -OCONH-, -OCOO-, or -NHCONH-;
**R^{A1}** - **R^{A4}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃ ; and
**BP¹**, **BP²** and **Z** are as defined herein.

In some preferred embodiments, the present invention relates to crosslinkers of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, or -CH₂-,
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, -CH₂R⁶C₆H₁₂-;
**R⁵** and **R⁶** independently of each other represent -NHCO-, -CONH-, -OCO-, -COO-, -NHCOO-, -OCONH-, -OCOO-, or -NHCONH-;
**R^{A1}** - **R^{A4}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃ ; and
**BP¹**, **BP²** and **Z** are as defined herein.

In some preferred embodiments, the present invention relates to crosslinkers of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²--R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**R¹** and **R⁴** independently of each other represent a bond or -CH₂-;
**R²** represents a bond, -CH₂-, 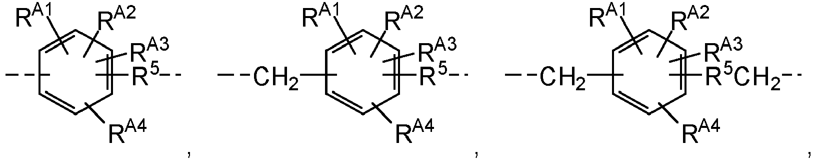 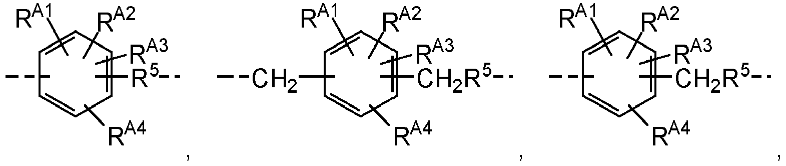 or 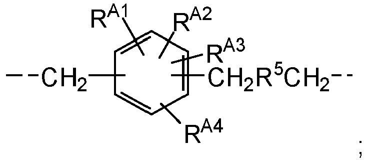
**R³** represents a bond, or -CH₂-;
**R⁵** and **R⁶** independently of each other represent -NHCO-, -CONH-, -OCO-, -COO-, -NHCOO-, -OCONH-, -OCOO-, or -NHCONH-;
**R^{A1}** - **R^{A4}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃ ; and
**BP¹**, **BP²** and **Z** are as defined herein.

### Preferred crosslinkers according to the invention

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²--R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
Z represents 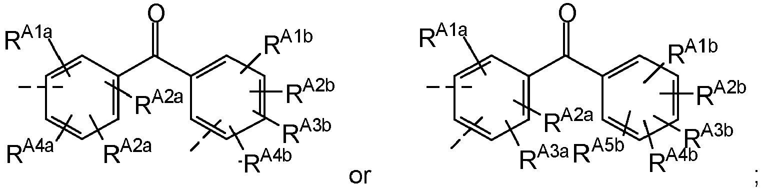
**BP¹** and **BP²** independently of each other represent 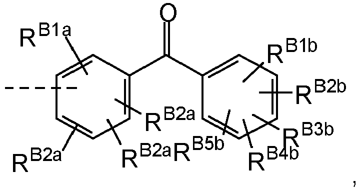
**R¹** and **R⁴** independently of each other represent a bond or -CH₂-;
**R²** represents a bond, or -CH₂-;
**R³** represents a bond, or -CH₂-;
**R^{A1a} - R^{A4a}, R^{A1b} - R^{A5b}, R^{B1a} - R^{B4a}** and **R^{B1b} - R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, or -R¹-OCONH-R²-;
**L²** represents -R³-NHCO-R⁴-, -R³-CONH-R⁴-, -R³-OCO-R⁴-, -R³-COO-R⁴-, -R³-NHCOO-R⁴-, or -R³-OCONH-R⁴-;
Z represents 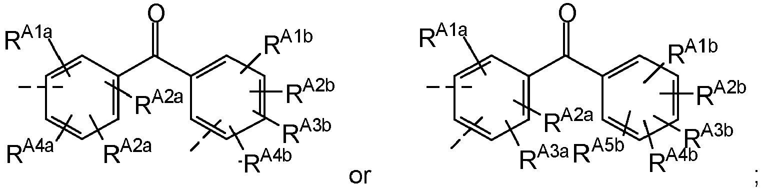
**BP¹** and **BP²** independently of each other represent 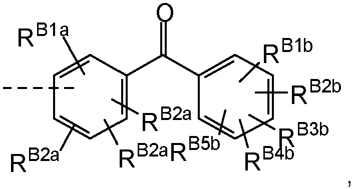
**R¹** and **R⁴** independently of each other represent a bond or -CH₂-;
**R²** represents a bond, or -CH₂-;
**R³** represents a bond, or -CH₂-;
**R⁵** and **R⁶** independently of each other represent -NHCO-, -CONH-, -OCO-, -COO-, -NHCOO-, -OCONH-, -OCOO-, or -NHCONH-; and
**R^{A1a}** - **R^{A4a}, R^{A1b}** - **R^{A5b}, R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, or -R¹-COO-R²-;
**L²** represents -R³-NHCO-R⁴-, -R³-CONH-R⁴-, -R³-OCO-R⁴-, or -R³-COO-R⁴-;
**Z** represents 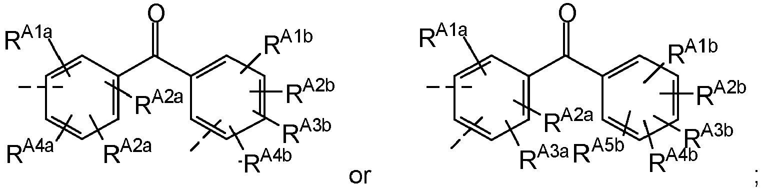
**BP¹** and **BP²** independently of each other represent 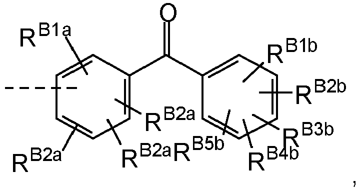
**R¹** and **R⁴** independently of each other represent a bond or -CH₂-;
**R²** represents a bond, or -CH₂-;
**R³** represents a bond, or -CH₂-;
**R^{A1a}** - **R^{A4a}, R^{A1b}** - **R^{A5b}, R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, or -R¹-OCONH-R²-;
**L²** represents -R³-NHCO-R⁴-, -R³-CONH-R⁴-, -R³-OCO-R⁴-, -R³-COO-R⁴-, -R³-NHCOO-R⁴-, or -R³-OCONH-R⁴-;
**Z** represents 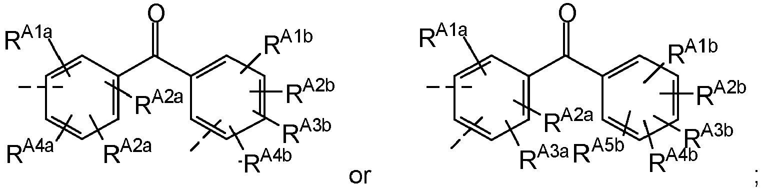
**BP¹** and **BP²** independently of each other represent 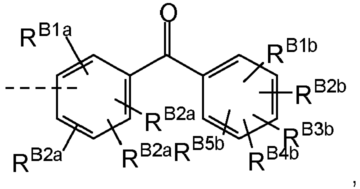
**R¹** and **R⁴** independently of each other represent a bond or -CH₂-;
**R²** represents a bond, or -CH₂-;
**R³** represents a bond, or -CH₂-;
**R⁵** and **R⁶** independently of each other represent -NHCO-, -CONH-, -OCO-, -COO-, -NHCOO-, -OCONH-, -OCOO-, or -NHCONH-; and
**R^{A1a}** - **R^{A4a}, R^{A1b}** - **R^{A5b}, R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, or -R¹-COO-R²-;
**L²** represents -R³-NHCO-R⁴-, -R³-CONH-R⁴-, -R³-OCO-R⁴-, or -R³-COO-R⁴-;
**Z** represents 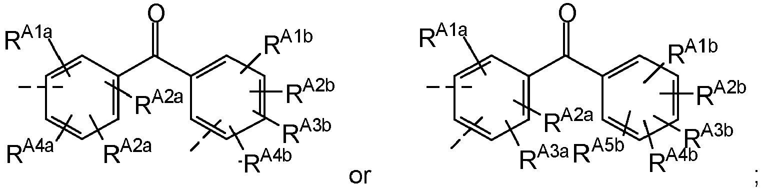
**BP¹** and **BP²** independently of each other represent 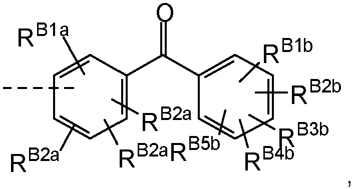
**R¹** and **R⁴** independently of each other represent a bond or -CH₂-;
**R²** represents a bond, or -CH₂-;
**R³** represents a bond, or -CH₂-;
**R^{A1a}** - **R^{A4a},** and **R^{A1b}** - **R^{A5b},** independently of each other represent -H, -COOH, or -CH₃.
**R^{B1a}** - **R^{B4a}** and **R^{B1b}** - **R^{B5b}** independently of each other represent -H, -COOH, - CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃.

The present invention relates to a crosslinker of general formula (**I**):

**BP¹-L¹-Z-L²-BP²** (**I**)

wherein
**L¹** and **L²** independently of each other represent -R¹-NHCO-R²-, or -R¹-CONH-R²-, and/or
**Z** represents 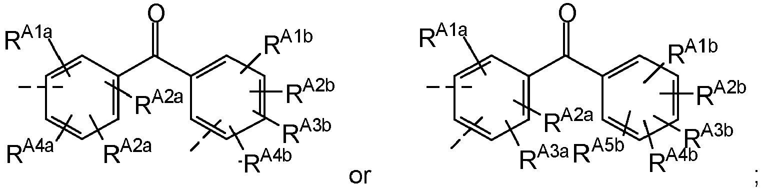
**BP¹** and **BP²** independently of each other represent 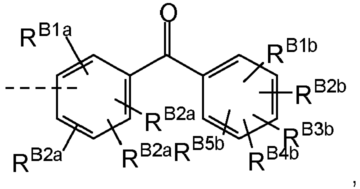
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, -C₆H₁₂R⁵CH₂-, 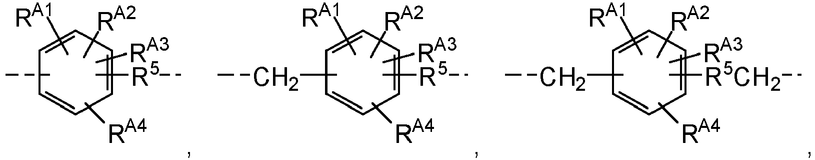 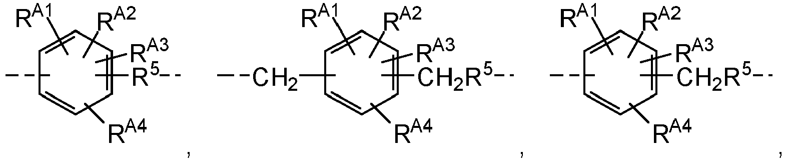 or 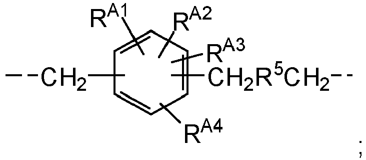
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, -CH₂R⁶C₆H₁₂-, 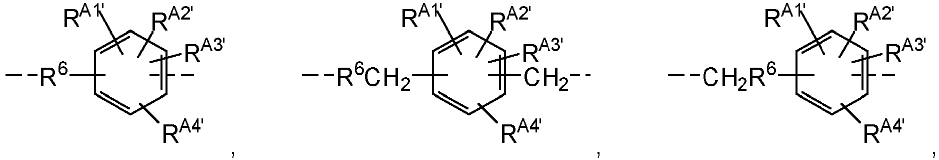 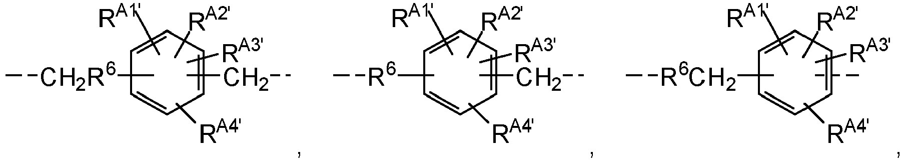 or 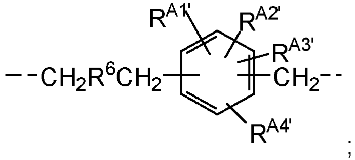
**R⁵** and **R⁶** independently of each other represent -NHCO-, or -CONH-; and
**R^{A1}** ₋ **R^{A4}, R^{A1'}** - **R^{A4'}, R^{A1a}** - **R^{A4a}, R^{A1b}** - **R^{A5b}**, **R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R1-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 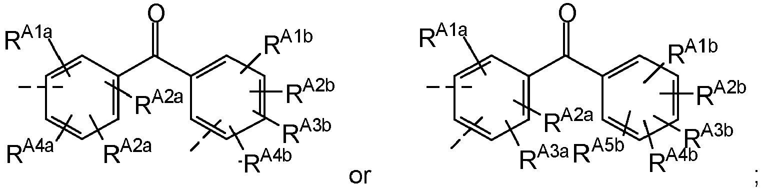
**BP¹** and **BP²** independently of each other represent 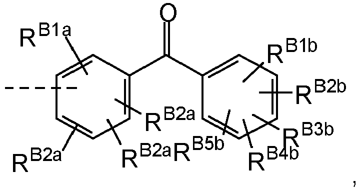
**R¹** and **R⁴** independently of each other represent a bond or -CH₂-;
**R²** represents a bond, -CH₂-, -CH₂R⁵-, -CH₂CH₂R⁵-, -CH₂CH₂CH₂R⁵-, -CH(CH₃)R⁵-, -C[(CH₃)₂]R⁵-, -CH₂CH(CH₃)R⁵-, -CH(CH₃)CH₂R⁵-, -CH(CH₂CH₃)R⁵-, -CH₂CH₂CH₂CH₂R⁵-, -CH₂C[(CH₃)₂]R⁵-, -C[(CH₃)₂]CH₂R⁵-, -CH(CH₃)CH₂CH₂R⁵-, -CH₂CH₂CH(CH₃)R⁵-, -CH₂CH(CH₂CH₃)R⁵-, -CH(CH₂CH₃)CH₂R⁵-, -CH₂CH(CH₃)CH₂R⁵-, -CH₂CH₂CH₂CH₂CH₂R⁵-, -CH₂CH(CH₃)CH(CH₃)R⁵-, -CH₂C[(CH₃)₂]CH₂R⁵-, -CH(CH₃)CH(CH₃)CH₂R⁵-, -C[(CH₃)₂]C(CH₃)R⁵-, - C(CH₃)C[(CH₃)₂]R⁵-, -CH(CH₃)CH(CH₃)R⁵-, -CH(CH₃)CH₂CH₂CH₂R⁵-, -C(CH₃)₂ CH₂CH₂R⁵-, -C(CH₂CH₃)₂R⁵-, -CH₂CH(CH₃)C₂H₄R⁵-, -CH(CH₃)C(CH₃)₂R⁵-, -CH₂CH₂C(CH₃)₂R⁵-, -CH(CH₃)₂C(CH₃)₂R⁵-, -CH₂CH₂CH₂CH₂CH₂CH₂R⁵-, -CH(CH₃)CH(CH₃)CH(CH₃)R⁵-, -CH₂CH₂CH₂CH(CH₃)₂R⁵-, -CH₂CH₂CH(CH₃)CH₂CH₂R⁵-, -CH₂CH(CH₃)CH₂CH₂CH₂R⁵-, -CH(CH₃)CH₂CH₂CH₂CH₂R⁵-, -CH(CH₃)CH₂C(CH₃)₂R⁵-, -C(CH₃)₂CH₂CH₂CH₂R⁵-, -CH(CH₃)CH(CH₃)CH₂CH₂R⁵-, -CH₂CH(CH₃)CH(CH₃)₂R⁵-, -CH₂C(CH₃)₂CH₂CH₂R⁵-, -C(CH₃)₂C(CH₃)₂R⁵-, -CH₂C(CH₂CH₃)₂R⁵, or -CH₂CH₂CH₂CH₂CH₂CH₂CH₃R⁵-; and/or
**R³** represents a bond, -CH₂-, -R⁶CH₂-, -R⁶CH₂CH₂-, -R⁶CH₂CH₂CH₂-, -R⁶CH(CH₃)-, -R⁶C[(CH₃)₂]-, -R⁶CH₂CH(CH₃)-, -R⁶CH(CH₃)CH₂-, -R⁶CH(CH₂CH₃)-, -R⁶CH₂CH₂CH₂CH₂-, -R⁶CH₂C[(CH₃)₂]-, -R⁶C[(CH₃)₂]CH₂-, -R⁶CH(CH₃)CH₂CH₂-, -R⁶CH₂CH₂CH(CH₃)-, -R⁶CH₂CH(CH₂CH₃) -, -R⁶CH(CH₂CH₃)CH₂-, -R⁶CH₂CH(CH₃)CH₂-, -R⁶CH₂CH₂CH₂CH₂CH₂-, -R⁶CH₂CH(CH₃)CH(CH₃)-, -R⁶CH₂C[(CH₃)₂]CH₂-, -R⁶CH(CH₃)CH(CH₃)CH₂-, -R⁶C[(CH₃)₂]C(CH₃)-, - R⁶C(CH₃)C[(CH₃)₂] -, -R⁶CH(CH₃)CH(CH₃) -, -R⁶CH(CH₃)CH₂CH₂CH₂-, -R⁶C(CH₃)₂ CH₂CH₂-, -R⁶C(CH₂CH₃)₂-, -R⁶CH₂CH(CH₃)C₂H₄-, -R⁶CH(CH₃)C(CH₃)₂-, -R⁶CH₂CH₂C(CH₃)₂-, -R⁶CH(CH₃)₂C(CH₃)₂-, -R⁶CH₂CH₂CH₂CH₂CH₂CH₂-, -R⁶CH(CH₃)CH(CH₃)CH(CH₃)--R⁶CH₂CH₂CH₂CH(CH₃)₂-, -R⁶CH₂CH₂CH(CH₃)CH₂CH₂-, -R⁶CH₂CH(CH₃)CH₂CH₂CH₂-, -R⁶CH(CH₃)CH₂CH₂CH₂CH₂-, -R⁶CH(CH₃)CH₂C(CH₃)₂-, -R⁶C(CH₃)₂CH₂CH₂CH₂-, -R⁶CH(CH₃)CH(CH₃)CH₂CH₂-, -R⁶CH₂CH(CH₃)CH(CH₃)₂-, -R⁶CH₂C(CH₃)₂CH₂CH₂-, -R⁶C(CH₃)₂C(CH₃)₂-, -R⁶CH₂C(CH₂CH₃)₂, or -R⁶CH₂CH₂CH₂CH₂CH₂CH₂CH₃-; and
**R⁵** and **R⁶** independently of each other represent -NHCO-, -CONH-, -OCO-, -COO-, -NHCOO-, -OCONH-, -OCOO-, or -NHCONH-; and
**R^{A1}** - **R^{A4}, R^{A1'}** - **R^{A4'}, R^{A1a}** - **R^{A4a}, R^{A1b}** - **R^{A5b}**, **R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H_{7,} -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃.

The present invention relates to a crosslinker of general formula (**I**):

**BP¹-L¹-Z-L²-BP²** (**I**)

wherein
**L¹** and **L²** independently of each other represent -R¹-NHCO-R²-, or -R¹-CONH-R²-, and/or
Z represents 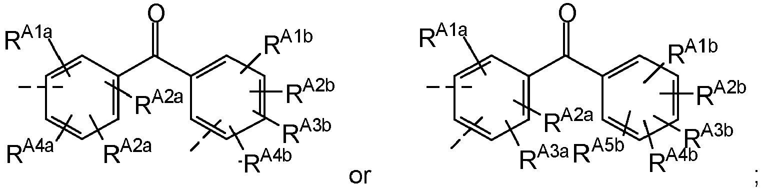
**BP¹** and **BP²** independently of each other represent 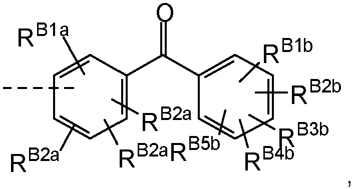
**R¹** and **R⁴** independently of each other represent a bond or -CH₂-;
**R²** represents a bond, -CH₂-, -CH₂R⁵-, -CH₂CH₂R⁵-, -CH₂CH₂CH₂R⁵-, -CH(CH₃)R⁵-, -C[(CH₃)₂]R⁵-, -CH₂CH(CH₃)R⁵-, -CH(CH₃)CH₂R⁵-, -CH(CH₂CH₃)R⁵-, -CH₂CH₂CH₂CH₂R⁵-, -CH₂C[(CH₃)₂]R⁵-, -C[(CH₃)_{2]}CH₂R⁵-, -CH(CH₃)CH₂CH₂R⁵-, -CH₂CH₂CH(CH₃)R⁵-, -CH₂CH(CH₂CH₃)R⁵-, -CH(CH₂CH₃)CH₂R⁵-, -CH₂CH(CH₃)CH₂R⁵-, -CH₂CH₂CH₂CH₂CH₂R⁵-, -CH₂CH(CH₃)CH(CH₃)R⁵-, -CH₂C[(CH₃)_{2]}CH₂R⁵-, -CH(CH₃)CH(CH₃)CH₂R⁵-, -C[(CH₃)₂]C(CH₃)R⁵-, -C(CH₃)C[(CH₃)₂]R⁵-, -CH(CH₃)CH(CH₃)R⁵-, -CH(CH₃)CH₂CH₂CH₂R⁵-, -C(CH₃)₂ CH₂CH₂R⁵-, -C(CH₂CH₃)₂R⁵-, -CH₂CH(CH₃)C₂H₄R⁵-, -CH(CH₃)C(CH₃)₂R⁵-, -CH₂CH₂C(CH₃)₂R⁵-, -CH(CH₃)₂C(CH₃)₂R⁵-, -CH₂CH₂CH₂CH₂CH₂CH₂R⁵-, -CH(CH₃)CH(CH₃)CH(CH₃)R⁵-, -CH₂CH₂CH₂CH(CH₃)₂R⁵-, -CH₂CH₂CH(CH₃)CH₂CH₂R⁵-, -CH₂CH(CH₃)CH₂CH₂CH₂R⁵-, -CH(CH₃)CH₂CH₂CH₂CH₂R⁵-, -CH(CH₃)CH₂C(CH₃)₂R⁵-, -C(CH₃)₂CH₂CH₂CH₂R⁵-, -CH(CH₃)CH(CH₃)CH₂CH₂R⁵-, -CH₂CH(CH₃)CH(CH₃)₂R⁵-, -CH₂C(CH₃)₂CH₂CH₂R⁵-, -C(CH₃)₂C(CH₃)₂R⁵-, -CH₂C(CH₂CH₃)₂R⁵, or -CH₂CH₂CH₂CH₂CH₂CH₂CH₃R⁵-; and/or
**R³** represents a bond, -CH₂-, -R⁶CH₂-, -R⁶CH₂CH₂-, -R⁶CH₂CH₂CH₂-, -R⁶CH(CH₃)-, -R⁶C[(CH₃)₂]-, -R⁶CH₂CH(CH₃)-, -R⁶CH(CH₃)CH₂-, -R⁶CH(CH₂CH₃)-, -R⁶CH₂CH₂CH₂CH₂-, -R⁶CH₂C[(CH₃)₂]-, -R⁶C[(CH₃)₂]CH₂-, -R⁶CH(CH₃)CH₂CH₂-, -R⁶CH₂CH₂CH(CH₃)-, -R⁶CH₂CH(CH₂CH₃)-, -R⁶CH(CH₂CH₃)CH₂-, -R⁶CH₂CH(CH₃)CH₂-, -R⁶CH₂CH₂CH₂CH₂CH₂-, -R⁶CH₂CH(CH₃)CH(CH₃)-, -R⁶CH₂C[(CH₃)₂]CH₂-, -R⁶CH(CH₃)CH(CH₃)CH₂-, -R⁶C[(CH₃)₂]C(CH₃)-, - R⁶C(CH₃)C[(CH₃)₂]-, -R⁶CH(CH₃)CH(CH₃)-, -R⁶CH(CH₃)CH₂CH₂CH₂-, -R⁶C(CH₃)₂ CH₂CH₂-, -R⁶C(CH₂CH₃)₂-, -R⁶CH₂CH(CH₃)C₂H₄-, -R⁶CH(CH₃)C(CH₃)₂-, -R⁶CH₂CH₂C(CH₃)₂-, -R⁶CH(CH₃)₂C(CH₃)₂-, -R⁶CH₂CH₂CH₂CH₂CH₂CH₂-, -R⁶CH(CH₃)CH(CH₃)CH(CH₃)- -R⁶CH₂CH₂CH₂CH(CH₃)₂-, -R⁶CH₂CH₂CH(CH₃)CH₂CH₂-, -R⁶CH₂CH(CH₃)CH₂CH₂CH₂-, -R⁶CH(CH₃)CH₂CH₂CH₂CH₂-, -R⁶CH(CH₃)CH₂C(CH₃)₂-, -R⁶C(CH₃)₂CH₂CH₂CH₂-, -R⁶CH(CH₃)CH(CH₃)CH₂CH₂-, -R⁶CH₂CH(CH₃)CH(CH₃)₂-, -R⁶CH₂C(CH₃)₂CH₂CH₂-, -R⁶C(CH₃)₂C(CH₃)₂-, -R⁶CH₂C(CH₂CH₃)₂, or -R⁶CH₂CH₂CH₂CH₂CH₂CH₂CH₃-; and
**R⁵** and **R⁶** independently of each other represent -NHCO-, or -CONH-; and
**R^{A1}** - **R^{A4}**, **R^{A1'}** - **R^{A4'}**, **R^{A1a}** - **R^{A4a}**, **R^{A1b}** - **R^{A5b}**, **R^{B1a}** - **R^{B4a}** and **R^{B1b}** - **R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 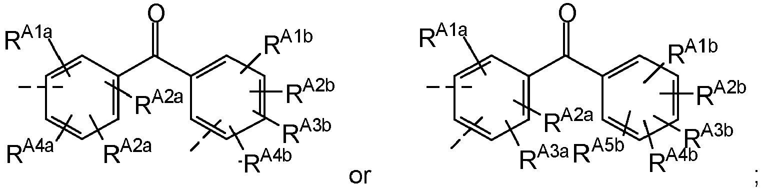
**BP¹** and **BP²** independently of each other represent 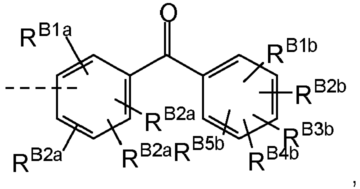
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂- -C₅H₁₀R⁵CH₂-, -C₆H₁₂R⁵CH₂-, 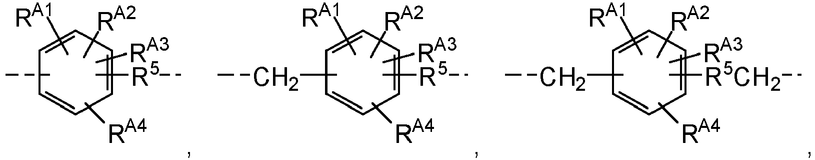 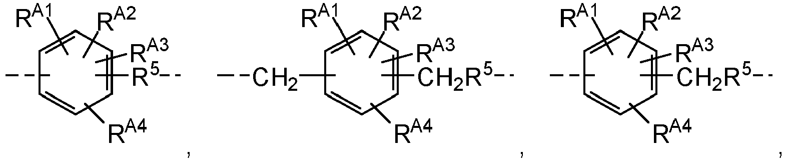 or 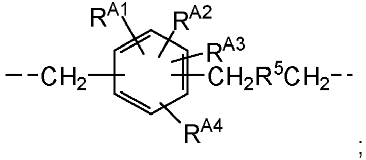
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, -CH₂R⁶C₆H₁₂-, 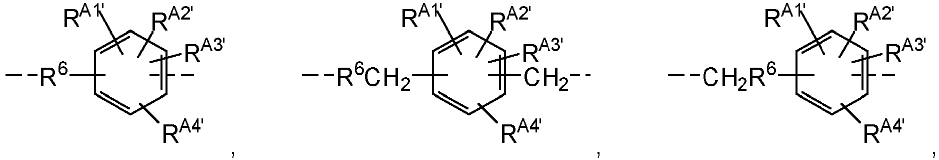 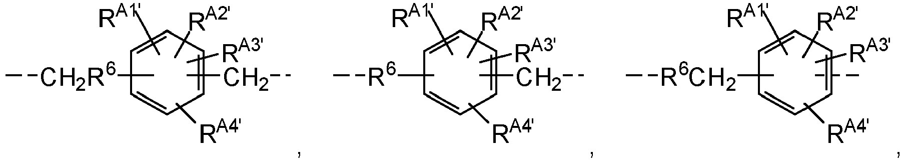 or 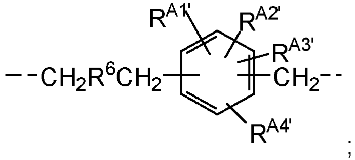
**R⁵** and **R⁶** independently of each other represent -NHCO-, -CONH-, -OCO-, -COO-, -NHCOO-, -OCONH-, -OCOO-, or -NHCONH-; and
**R^{A1a}** - **R^{A4a}**, and **R^{A1b}** - **R^{A5b}**, independently of each other represent -H, -COOH, or -CH₃.
**R^{B1a}** - **R^{B4a}** and **R^{B1b}** - **R^{B5b}** independently of each other represent -H, ...-OH, -NO₂, -COCH₃, -COOH, -CONH₂, -SO₃H, -SO₂NH₂, -OCH₃, -OCF₃, - NH₂, -NH(CH)₃, -N(CH₃)₂, and -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 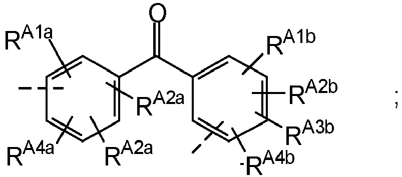
**BP¹** and **BP²** independently of each other represent 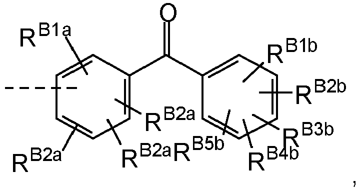
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, -C₆H₁₂R⁵CH₂-, 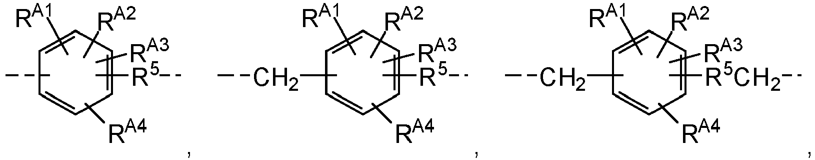 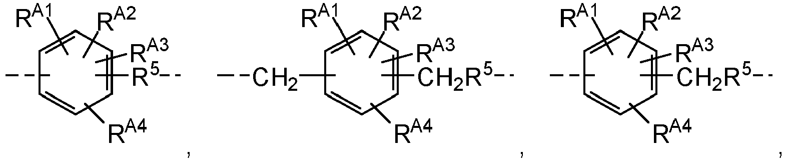 or 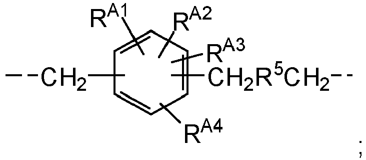
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, -CH₂R⁶C₆H₁₂-, 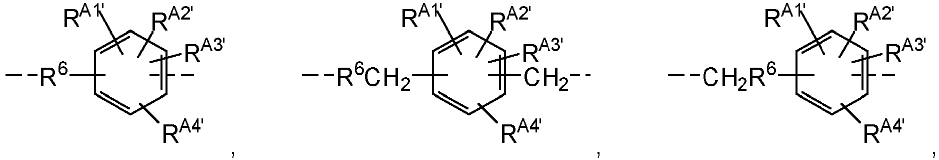 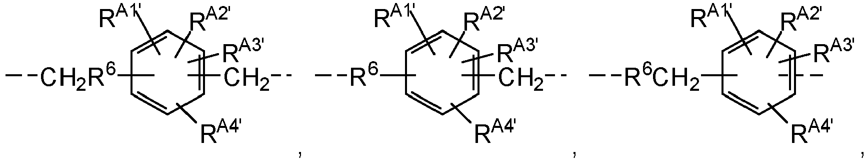 or 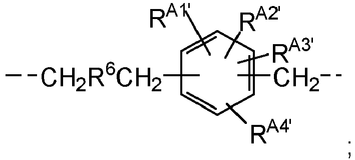
**R⁵** and **R⁶** independently of each other represent -NHCO-, -CONH-, -OCO-, -COO-, -NHCOO-, -OCONH-, -OCOO-, or -NHCONH-; and
**R^{A1}** - **R^{A4}**, **R**^{**A1**'} - **R^{A4'}**, **R^{A1a}** - **R^{A4a}**, **R^{A1b}** - **R^{A5b}** , **R^{B1a}** - **R^{B4a}** and **R^{B1b}** - **R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²- , or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 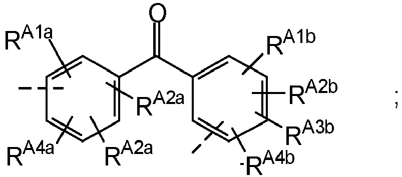
**BP¹** and **BP²** independently of each other represent 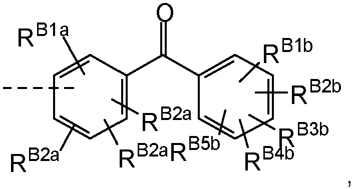
**R¹** and **R⁴** independently of each other represent a bond or -CH₂-;
**R²** represents a bond, or -CH₂-;
**R³** represents a bond, or -CH₂-;
**R^{A1a}** - **R^{A4a}**, **R^{A1b}** - **R^{A5b}**, **R^{B1a}** - **R^{B4a}** and **R^{B1b}** - **R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 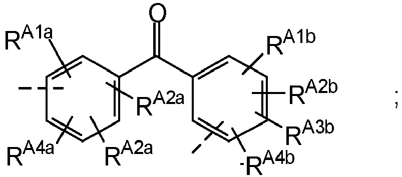
**BP¹** and **BP²** independently of each other represent 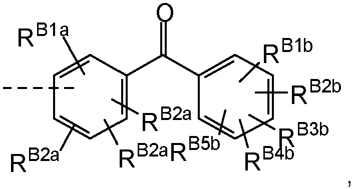
**R¹** and **R⁴** independently of each other represent a bond or -CH₂-;
**R²** represents a bond, or -CH₂-;
**R³** represents a bond, or -CH₂-;
**R^{A1a}** - **R^{A4a}**, and **R^{A1b}** - **R^{A5b}**, independently of each other represent -H, -COOH, or -CH₃.
**R^{B1a}** - **R^{B4a}** and **R^{B1b}** - **R^{B5b}** independently of each other represent -H, ...-OH, -NO₂, -COCH₃, -COOH, -CONH₂, -SO₃H, -SO₂NH₂, -OCH₃, -OCF₃, - NH₂, -NH(CH)₃, -N(CH₃)₂, and -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 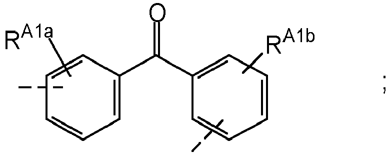
**BP¹** and **BP²** independently of each other represent 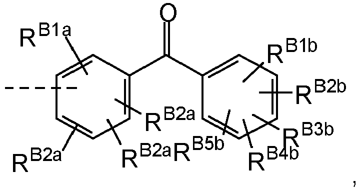
**R¹** and **R⁴** independently of each other represent a bond or -CH₂-;
**R²** represents a bond, or -CH₂-;
**R³** represents a bond, or -CH₂-;
**R^{A1a}**, and **R^{A1b}**, independently of each other represent -H, -COOH, or -CH₃.
**R^{B1a}** - **R^{B4a}** and **R^{B1b}** - **R^{B5b}** independently of each other represent -H, ...-OH, -NO₂, -COCH₃, -COOH, -CONH₂, -SO₃H, -SO₂NH₂, -OCH₃, -OCF₃, - NH₂, -NH(CH)₃, -N(CH₃)₂, and -SOCH₃.

The present invention relates to a crosslinker of general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 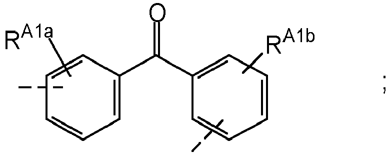
**BP¹** and **BP²** represent 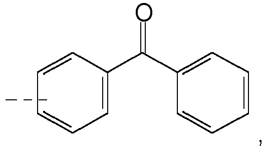 preferably 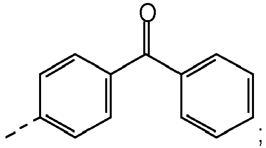
**R¹** and **R⁴** independently of each other represent a bond or -CH₂-;
**R²** represents a bond, or -CH₂-;
**R³** represents a bond, or -CH₂-;
**R^{A1a}**, and **R^{A1b}**, independently of each other represent -H, -COOH, or -CH₃.
**R^{B1a}** - **R^{B4a}** and **R^{B1b}** - **R^{B5b}** independently of each other represent -H, ...-OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃.

Particularly preferred are crosslinkers selected from the group comprising or consisting of: 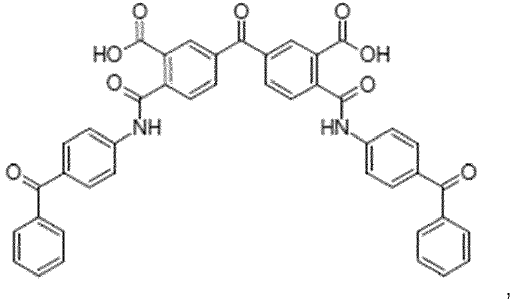 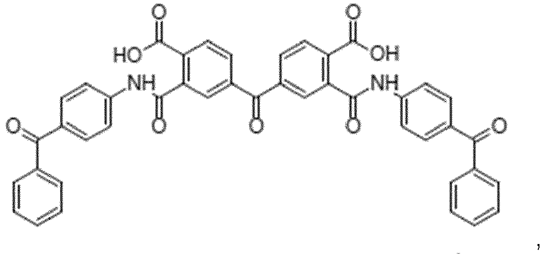 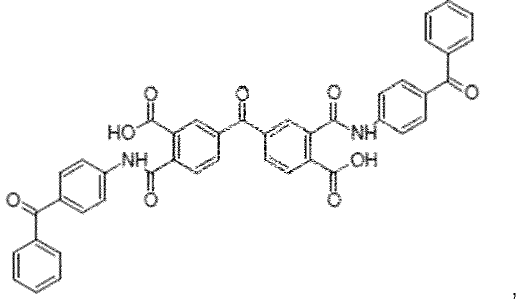 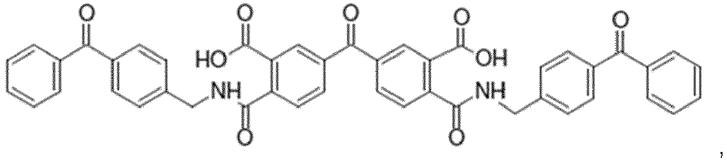 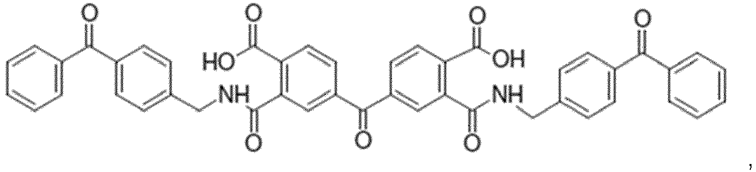 and 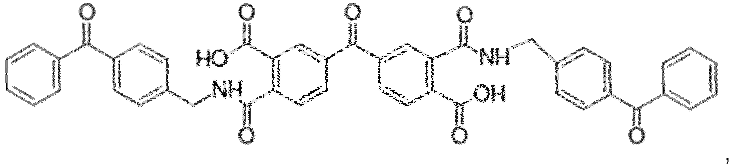 or mixtures thereof.

### Molecule colloids

Surprisingly, it has been shown that crosslinkers of the present invention can be reacted with the at least one polymer after activation via light, forming a molecular colloid network. Thus, the crosslinkers of the present invention are particularly advantageous for preparing molecular colloid networks from at least one polymer or polymerizate and a crosslinker as described herein. In general, crosslinking reactions involve linking one or more individual macromolecules to form a three-dimensional network. Macromolecules can be long-chain, high-molecular-weight molecules, but also branched, high-molecular-weight molecules. The macromolecules used to produce the molecular colloids are polymers, preferably hydrophilic polymers. The polymers suitable for the preparation of the molecular colloids include both homopolymers and mixed polymers or copolymers. Crosslinking can be achieved either directly during the assembly of the polymers or by reactions on preexisting polymers. Linking of already existing polymers is also referred to as crosslinking. The crosslinker according to the invention and the at least one polymer are preferably crosslinked and, after crosslinking has taken place, together form the molecular colloid network.

In general, the properties of a polymer are changed by the process of crosslinking. An increase in hardness, toughness, melting point and a decrease in solubility are usually observed. The change in properties increases with the degree of crosslinking, i.e. with the proportion of crosslinked sites relative to the total polymer quantity.

Surprisingly, it has been found that the crosslinkers according to the invention advantageously influence the properties of the resulting molecular colloid through the process of crosslinking and that the properties of the molecular colloid can be specifically controlled with these crosslinkers.

The molecular colloid networks of the present invention exhibit excellent hydrophilicity, stability, biocompatibility and hemocompatibility, and are particularly suitable for applications related to medical devices and medical aids. The molecular colloids of the present invention can thus be used to provide low-friction, inflammation-free medical devices and medical aids that can be used over the long term. However, these properties can also be used in a variety of ways in other areas such as hygiene articles, ointments, creams, pastes, lotions, cosmetic articles, and any other suitable personal care or hygienic care product etc..

The molecular colloid network formed by the at least one polymer and the crosslinker after activation via light or heat is suitable for use as or in any medical device or medical aid that can be used in or on the body on a short-term or long-term basis. The molecular colloids of the present invention can be used, among other things, as coatings for tubes, catheters, or other materials that require insertion into the body. Examples of these include, but are not limited to, balloon catheters, bladder catheters, stomach tubes, endoscopes, colonoscopes, needles, cannulas, endotracheal tubes, tracheal cannulas, implants such as stents, ocular lenses, prosthetic joints, screws, fixations, artificial exits, nasal implants, vocal cords, larynx, artificial and native valves such as cardiac or venous valves, esophageal sphincter, sphincter ani, bone graft substitutes, and ventilator tubes. However, the molecule colloids of the present invention can also be used as cartilage substitutes, nerve sheaths, occlusion of venous valves, seals ex corpora and in corpora, subcutaneous or intramuscular implants, as moisturizing and/or active ingredient gels/ointments, micro- and macrocapsules, wound protectors, breast implants, generally as filling materials, plasters, pads, etc., or as components of the products mentioned herein. Thus, an enormously diverse product range of medical devices and medical aids is made possible. Likewise, these molecular colloids can be used helpfully in the field of cosmetic articles and care products. Examples include skin lotions, moisturizers, eye creams, soaps, syndets, shower and bath preparations, sunscreen preparations, insect repellents, acne preparations, shaving products, etc.

At least one polymer and at least one crosslinker according to the invention are used to prepare the molecular colloids of the present invention. All biocompatible or hemocompatible, biodegradable, bioresorbable and biostable polymers are suitable as the at least one polymer. The selection of the polymer or polymers is based on the function that the molecular colloid is to fulfill.

A polymeric additive may be admixed to the at least one polymer or added to a solution of the at least one polymer and the crosslinker of the invention before or after molecular colloid network formation. Similarly, suitable monomers may be added during the polymerization reaction to produce the polymer. Similarly, a polymer may be covalently bonded to a monomer and polymerized as such functionalized monomer to form a polymer.

The combination of at least one polymer with other polymers also offers a wide range of possibilities. All biocompatible, biodegradable, bioresorbable and biostable polymers can be used as additional polymers. The selection of the added polymer(s) is based on the function that the molecular colloid must fulfill. In this way, for example, the hydrophilicity and hydrophobicity of a coating can be adjusted as desired. This can be done, for example, by mixing the coating with hydrophobic polymers or by copolymerization. Further monomers or oligomers of biodegradable, bioabsorbable or and biostable or very slowly biodegrading polymers can be added and participate in the polymerization reaction and be incorporated into the polymer chain, forming a binary, ternary, quaternary, etc. copolymer is formed.

By selectively adding further monomers, oligomers or polymers, the properties of the at least one polymer or the molecular colloid can thus be changed as desired. The possible addition of further components, e.g. a higher molecular weight polymer as a third component and a low molecular weight polymer as a fourth component, can additionally increase the hydrophilicity. The hydrophilicity or hydrophobicity, the frictional resistance, the solubility, the strength, the flexibility - and in the presence of a thermo- or photoactive functionalization - also the photo- or thermoreactivity can be specifically influenced in this way. In this way, the chemical and physical properties of the at least one polymer can influence the properties of the resulting molecular colloid network, such as the degree of crosslinking, elasticity, adhesion, strength, "shelf life", durability, and/or sterilizability.

Other monomers suitable for the polymerization reaction of the at least one polymer include, but are not limited to, fumaric acids, di-t-butyl fumarates, di-trimethylsilyl fumarates, maleic acids, maleic anhydrides, dimethyl and diethyl aminoethyl vinyl ethers, N-vinyl imidazole, N-vinyl imidazoline, vinyl pyridines, N-vinyl formamide, N-vinyl caprolactam, etc. as well as mixtures of the monomers and their derivatives.

It is also possible to incorporate unsaturated compounds, which cannot form macropolymers on their own, as a constituent in a copolymer and thus form new copolymers. Examples of such compounds include α-methylstyrene, allyl chloride, stilbene, allyl alcohols, dichloroethylene, maleic acid, maleic anhydride and trichlorostyrene and their derivatives.

Preferably, the at least one polymer is itself a molecular colloid already endowed with hydrophilic properties. This includes, in particular, the monomers of hydrophilic polymers which, after polymerization, already exhibit hydrophilic properties and, as such, can be used as the at least one polymer. Nevertheless, suitable biodegradable, biodegradable, bioresorbable or/and biostable or very slowly biodegradable polymers can additionally be admixed to the at least one polymer.

### Preferred Polymers and Copolymers

The present invention further relates to copolymers of general formula (v): 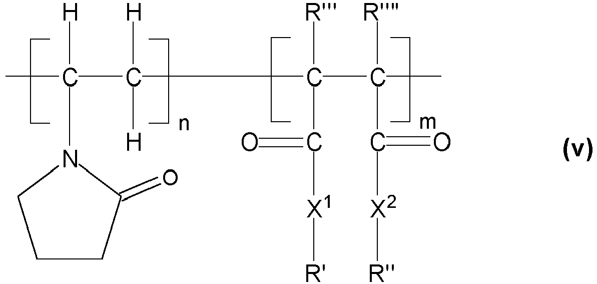 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, a substituted or unsubstituted alkyl, a substituted or unsubstituted aryl, a substituted or unsubstituted arylalky, a substituted or unsubstituted alkoxy, a substituted or unsubstituted aryloxy, a substituted or unsubstituted alkylhydroxy, a substituted or unsubstituted arylhydroxy, a substituted or unsubstituted alkylaryloxy, a substituted or unsubstituted alkylamino, a substituted or unsubstituted arylamino, a substituted or unsubstituted alkylarylamino, silyl groups, organometallic groups, and -H; wherein wherein **R^{'}** and **R^{"}** are not both -H;
**R‴** and **R""** represent independently of each other -H, -CH₃, -CH₂CH₃, or -F;
**L** represents -CH₂-;
**BP** represents 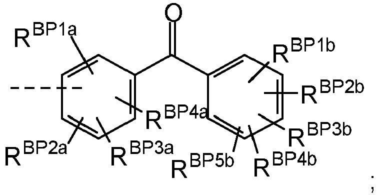
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.

The present invention further relates to copolymers of general formula (v): 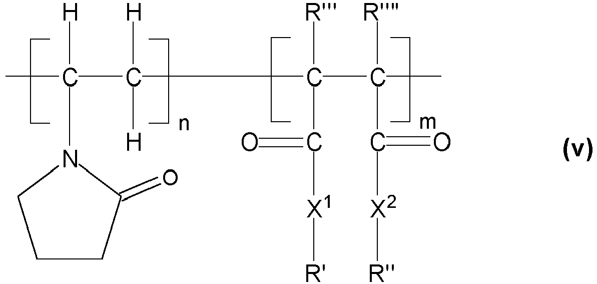 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, -CH₃, -C₂H₅, -C₃H₇, -CH(CH₃)₂, -C₄H₉, -CH₂CH(CH₃)₂, -CH(CH₃)C₂H₅, -C(CH₃)₃, -C₅H₁₁, -CH(CH₃) C₃H₇, -CH₂-CH(CH₃)C₂H₅, -CH(CH₃)-CH(CH₃)₂, -C(CH₃)₂C₂H₅, -CH₂C(CH₃)₃, -CH(C₂H₅)₂, -C₂H₄CH(CH₃)₂, -C₆H₁₃, -C₃H₆CH(CH₃)₂, -C₂H₄CH(CH₃)C₂H₅, -CH(CH₃)C₄H₉, -CH₂CH(CH₃)C₃H₇, -CH(CH₃)CH₂CH(CH₃)₂, -CH(CH₃)-CH(CH₃)C₂H₅, -CH₂CH(CH₃)CH(CH₃)₂, -CH₂C(CH₃)₂C₂H₅, -C(CH₃)₂C₃H₇, -C(CH₃)₂CH(CH₃)₂, -C₂H₄C(CH₃)₃, -CH₂CH(C₂H₅)₂, -CH(CH₃)C(CH₃)₃, -CH₃, -CH₂CH₃, -CH₂CH₂CH₃, -CH(CH₃)₂, -CH₂CH₂CH₂CH₃, -CH₂CH₂CH₂CH₂CH₃, -CH₂CH₂CH₂CH₂CH₂CH₃, -CH₂CH₂CH₂CH₂CH₂CH₂CH₃, -CH₂CH(CH₃)₂, -CH(CH₃)CH₂CH₃, -CH(C₂H₅)₂, -CH₂CH(C₂H₅)₂, -C(CH₃)₃, -CH₂-C(CH₃)₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂-Ph, -CH₂OCH₃, -CH₂OCH₂CH₃, -CH₂CH₂OCH₃, -CH₂CH₂OCH₂CH₃, -CH₂CO₂CH₃, -CH₂CO₂CH₂CH₃, -CH₂CH₂NHCH₃, -CH₂CH₂N(CH₃)₂, -CH₂S(O)₂-(4-methyl-phenyl), and -H; wherein R^{'} and R^{"} are not both -H;
**R‴** and **R""** represent independently of each other -H, -CH₃, -CH₂CH₃, or -F;
L represents -CH₂-;
**BP** represents 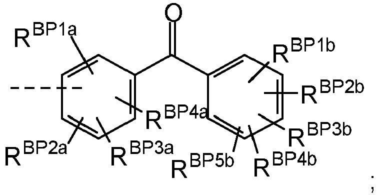
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.

The present invention further relates to copolymers of general formula (v): 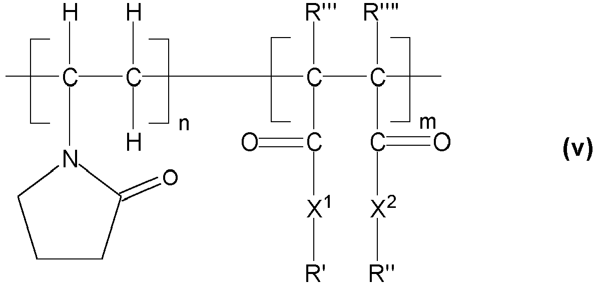 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, and -H; wherein **R^{'}** and **R^{"}** are not both -H;
**R‴** and **R""** represent independently of each other -H, -CH₃, -CH₂CH₃, or -F;
L represents -CH₂-;
**BP** represents 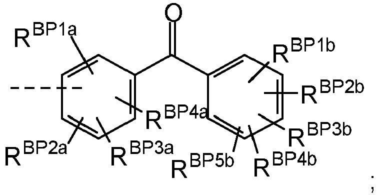
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.

The present invention further relates to copolymers of general formula (v): 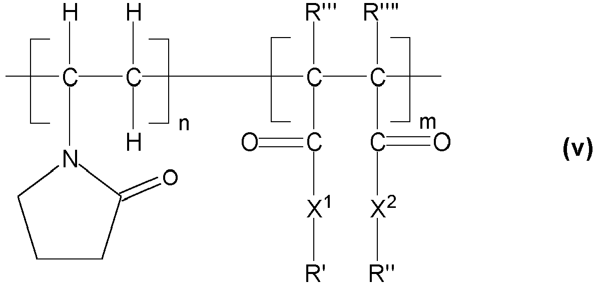 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, and -H; wherein **R^{'}** and **R^{"}** are not both -H;
**R‴** and **R""** represent independently of each other -H, -CH₃, -CH₂CH₃, or -F;
L represents -CH₂-;
**BP** represents 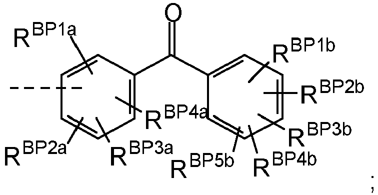
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 50.

The present invention further relates to copolymers of general formula (v): 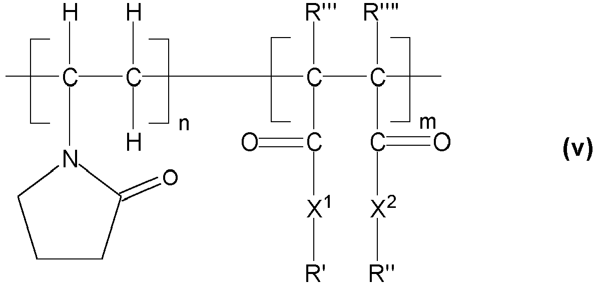 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, and -H; wherein **R^{'}** and **R^{"}** are not both -H;
**R‴** and **R""** represent independently of each other -H, -CH₃, -CH₂CH₃, or -F;
**L** represents -CH₂-;
**BP** represents 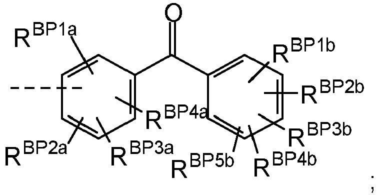
**R^{BP1a}** - **R^{BP4a}** and **R^{BP1b}** - **R^{BP5b}** represent -H;
**q** is an integer between 5 and 50.

The present invention further relates to copolymers of general formula (v): 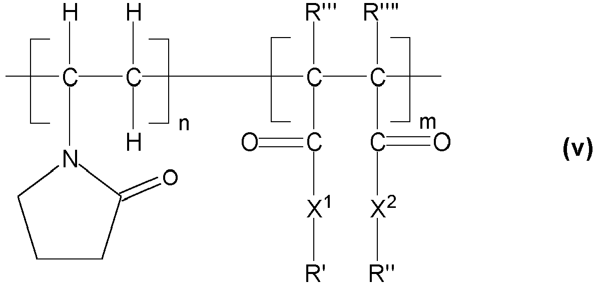 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, and -H; wherein **R^{'}** and **R"** are not both -H;
**R‴** and **R""** represent independently of each other -H, -CH₃, -CH₂CH₃, or -F;
**L** represents -CH₂-;
**BP** represents 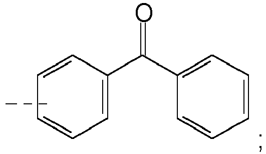
**q** is an integer between 5 and 50.

The present invention further relates to copolymers of general formula (**v**): 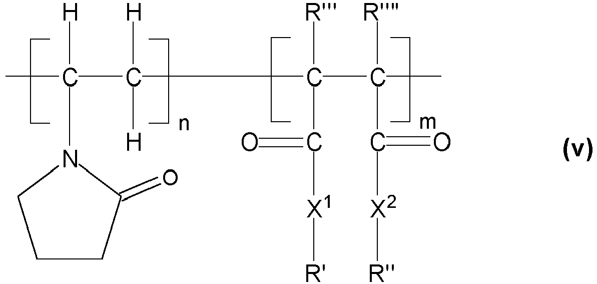 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** represents -BP or -L-BP, and R^{"} represents -(CH₂CH₂O)_{q}-OH, or -H, or
**R^{"}** represents -BP or -L-BP, and **R^{'}** represents -(CH₂CH₂O)_{q}-OH, or -H;
**R‴** and **R""** represent independently of each other -H, -CH₃, -CH₂CH₃, or -F;
**L** represents -CH₂-;
**BP** represents 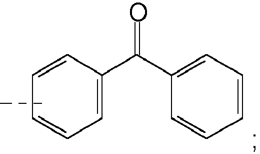
**q** is an integer between 5 and 50.

The present invention further relates to copolymers of general formula (v): 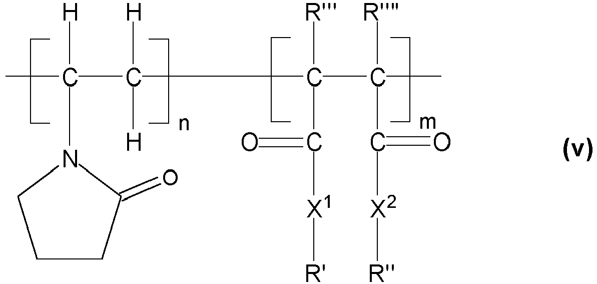 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** represents -BP or -L-BP, and **R^{"}** represents -H, or **R^{"}** represents -BP or -L-BP, and **R^{'}** represents -H;
**R‴** and **R""** represent independently of each other -H, -CH₃, -CH₂CH₃, or -F;
L represents -CH₂-;
**BP** represents 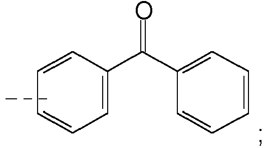
**q** is an integer between 5 and 50.

The present invention further relates to copolymers of general formula (v): 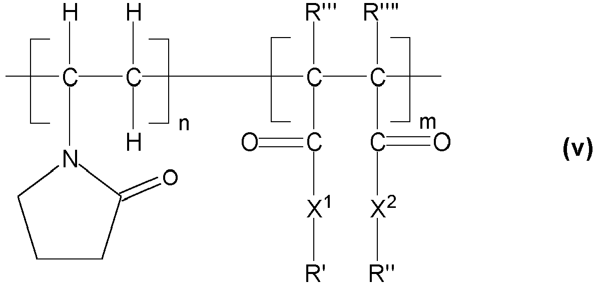 wherein
**m** = 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** represents -BP or -L-BP, and **R^{"}** represents -H, or **R^{"}** represents -BP or -L-BP, and **R^{'}** represents -H;
**R‴** and **R""** represent independently of each other -H, -CH₃, -CH₂CH₃, or -F;
**L** represents -CH₂-;
**BP** represents 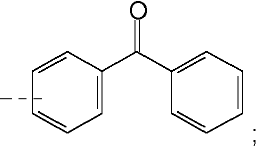
**q** is an integer between 5 and 50.

The present invention further relates to copolymers of general formula (**v**): 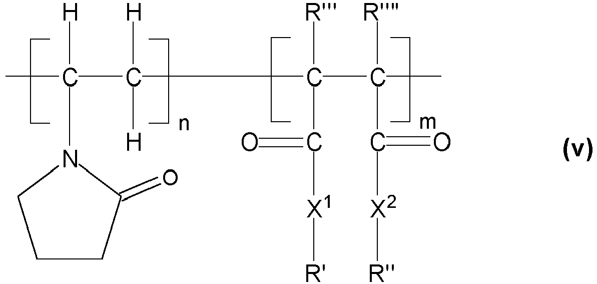 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** represents -BP or -L-BP,, and **R^{"}** represents -(CH₂CH₂O)_{q}-OH, or -H, or
**R^{"}** represents -BP or -L-BP,, and **R^{'}** represents -(CH₂CH₂O)_{q}-OH, or -H;
**R‴** and **R""** represent independently of each other -H, -CH₃, -CH₂CH₃, or -F;
**L** represents -CH₂-;
**BP** represents 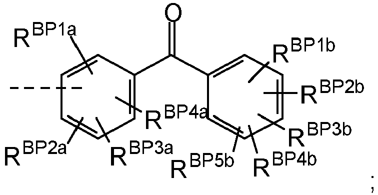
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 50.

The present invention further relates to copolymers of general formula (v): 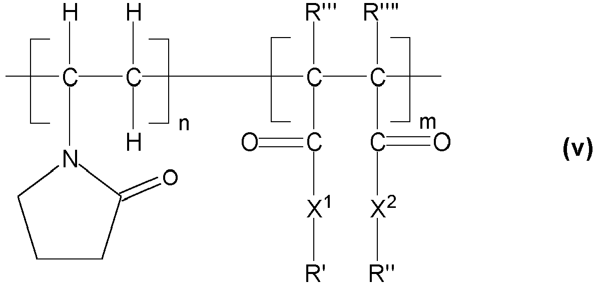 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** represents -BP or -L-BP, and **R^{"}** represents -H, or **R^{"}** represents -BP or -L-BP, and **R^{'}** represents -H;
**R‴** and **R""** represent independently of each other -H, -CH₃, -CH₂CH₃, or -F;
**L** represents -CH₂-;
**BP** represents 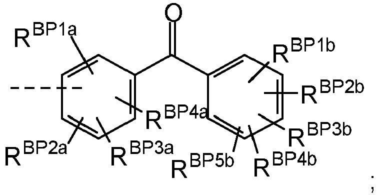
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 50.

The present invention further relates to copolymers of general formula (v): 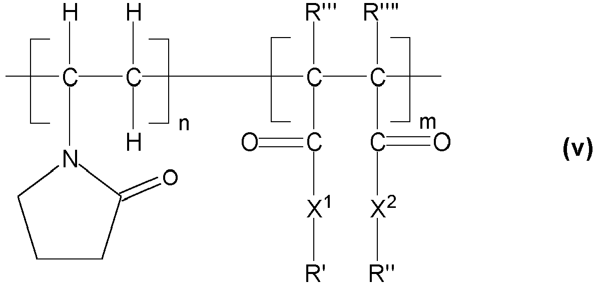 wherein
**m** = 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** represents -BP or -L-BP, and **R^{"}** represents -H, or **R^{"}** represents -BP or -L-BP, and **R^{'}** represents -H;
**R‴** and **R""** represent independently of each other -H, -CH₃, -CH₂CH₃, or -F;
**L** represents -CH₂-;
**BP** represents 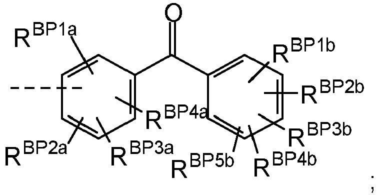
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
q is an integer between 5 and 50.

The present invention preferably relates to copolymers of the following structure: 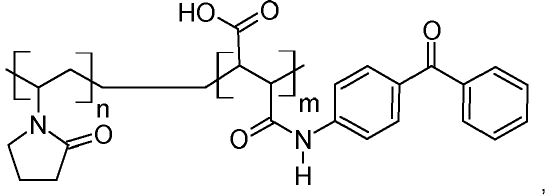 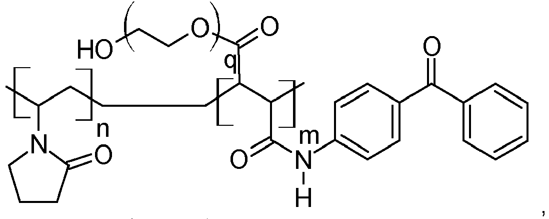 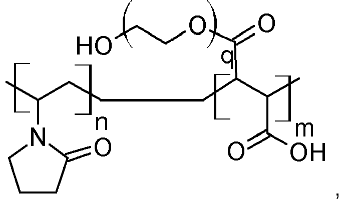 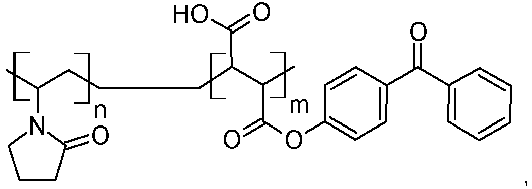 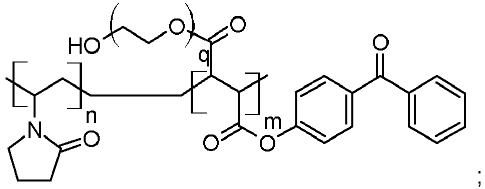 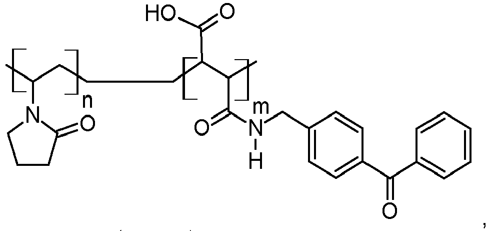 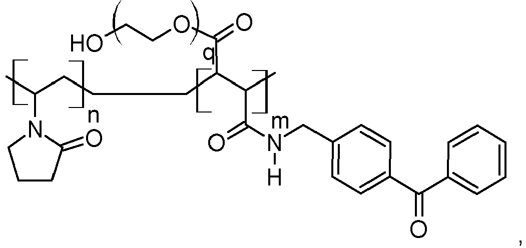 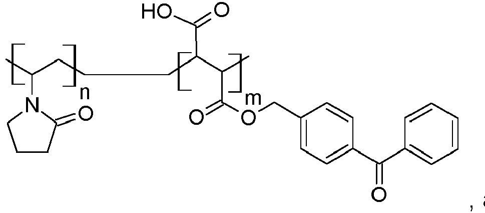 and 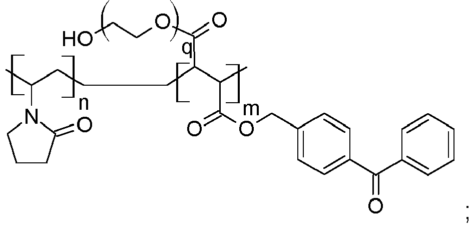 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**q** is an integer between 5 and 50.

Further preferred are copolymers of the following structure: 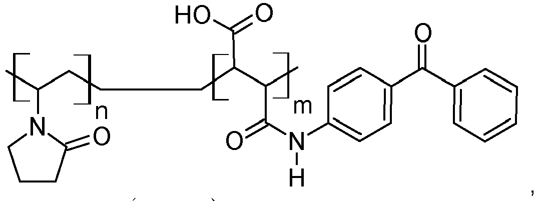 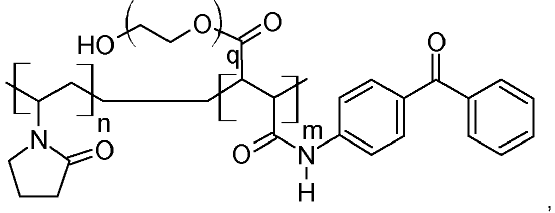 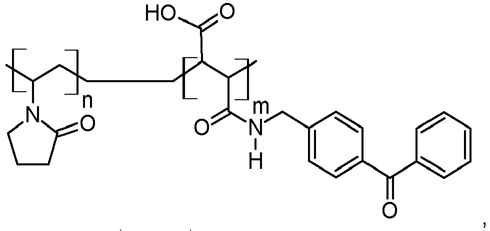 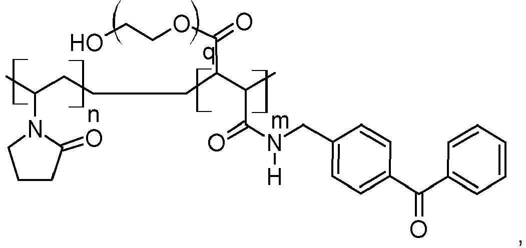 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**q** is an integer between 5 and 50.

Preferred copolymers according to the invention have an average molecular weight of 1,000 to 1,000,000, preferably 10,000 to 700,000, and particularly preferably between 5,000 to 500,000. Examples of copolymers according to the invention and their determined molecular weight and polydispersity index are listed in Table 1 as non-limiting examples.

**Table 1: Copolymers according to the invention and molecular weight and polydispersity index (MBM: N-[(4-benzoylphenyl)]-maleic acid monoamide)**

| Proportion MBM in copolymer with N-vinylpyrrolidon [mol%] | Number average molecular weight Copolymer (Mₙ) [kDa] | Weight average Molecular weight Copolymer (M_{w}) [kDa] | polydispersity index (PDI) M_{w}/Mₙ |
|---|---|---|---|
| 5.2 | 26 | 90 | 3.5 |
| 2.5 | 32 | 101 | 3.1 |
| 1.1 | 37 | 123 | 3.3 |
| 0.9 | 27 | 103 | 3.8 |
| 0.8 | 32 | 102 | 3.2 |
| 0.5 | 37 | 119 | 3.3 |

It has been shown that the crosslinkers according to the invention in combination with polymers are particularly well suited for the production of molecular colloid networks, which can be used in many areas, preferably in the area of medical products in the short term and moreover especially in the long term. Particularly in combination with the polymers described herein, it is shown, among other things, that there is an excellent improvement in hydrophilic properties with simultaneous improvement in adhesion to surfaces, thus preventing detachment.

The synthesis of the crosslinkers according to the invention is preferably carried out in a simple one-pot process with selection of suitable solvents. In the simplest case, the choice of solvent is based on the solubility of the reactants and the insolubility of the resulting product in a solvent for isolation and purification.

Thus, the choice of solvent is critical to the process and the quality of the product. Therefore, depending on the synthesis and solubility of the reactants and products of the components B according to the invention, ethanol, acetone, n-heptane, n-hexane, cyclohexane, tetrahydrofuran, diethyl ether, toluene, chloroform, dichloromethane, methanol, n-octanol, dimethylformamide, isopropanol, water, ethyl acetate, etc., and mixtures thereof are used as solvents.

### Synthesis

The polymerization reaction of the (photo- and/or thermosensitive) α,β-unsaturated carboxylic acid monomer with a further monomer is preferably carried out by free radicals and preferably as a solution polymerization, but is not limited to this polymerization process. The monomers or oligomers capable of polymerization are preferably prepared in a "one-pot" process involving a free radical initiator (organic peroxides such as peroxodisulfates, dibenzoyl peroxide, benzpinacol, cyclohexanone peroxide, di-t-butyl peroxide, methyl ethyl ketone peroxide, t-butyl peroctoate, di-(2-ethylhexyl) peroxydicarbonate, diisopropyl percarbonate, cumene hydroperoxide, etc., inorganic peroxides such as potassium peroxodisulfate, etc., or azo compounds such as AIBN, 2,2'-azo-bis(2-methylpropionitrile, etc.) or polymerized with boroalkylene (in the presence of oxygen or peroxide) while monitoring and controlling the pot temperature. This yields a linear or nearly linear molecular colloidal product with already good hydrophilic properties and with readily activatable functional groups available for further reaction.

The reaction ideally takes place under a protective atmosphere (e.g. argon, nitrogen) and at an elevated temperature depending on the reaction initiator used. Organic or aqueous solvents or fully demineralized (DI) water (also known as demineralized water or deionized water) can be used as the reaction medium. If the (photo/thermosensitive) α,β-unsaturated carboxylic acid monomer is soluble in the further monomer, a solvent is not essential for the polymerization reaction.

For example, APMA-BBC is readily soluble in N-vinylpyrrolidone as a monomer and the reaction can take place without a solvent. In contrast, HEMA-BBA is only slowly soluble in N-vinylpyrrolidone, so a suitable solvent is helpful.

Common solvents suitable for use are: tetrahydrofurane, acetone, ethanol, isopropanol, ether, methanol, n-hexane, n-heptane, n-octanol, isooctanol, dimethylformamide, dimethyl sulfoxide, chloroform, cyclohexane, 1,4-butanediol, ethyl acetate, methyl acetate, petroleum ether, toluene, diethylene glycol, ethylene glycol, dichloromethane, t-butyl methyl ether, acetonitrile, deionized water (optionally pyrogen-free), etc. as mixtures thereof.

The selection is usually based on the solubility of the reactants and on the solubility of the product or of the at least one polymer, taking into account that the solvent has an influence on the polymerization reaction and thus also on the molecular weight average (Mw) of the at least one polymer.

For example, NVP polymerizes with MABP under argon introduction with water as solvent (under these conditions, crosslinked structures of NVP-co-MABP are already formed, which obviously also influences the degree of crosslinking). The preparation also succeeds with dimethylformamide under nitrogen atmosphere and is stopped with water. Isopropanol is also suitable, although argon atmosphere is preferred here and the reaction is precipitated by acetone. Synthesis in 40% degassed dimethylformamide is particularly preferred, since the synthesis is fully controllable and leads to high yields of about 95%, but also succeeds in situ, in dist. Water, isopropanol.

The molecular colloid obtained from the polymerization reaction has a molecular weight average of 10000Da - 1000000Da, preferably 30000Da - 700000Da, more preferably 50000Da - 5000000Da. In some preferred embodiments 80000Da-400000Da.

Preferably, the polymer is prepared from a monomer and a (photo/thermosensitive) α,β-unsaturated carboxylic acid monomer, the molar ratio of monomer to carboxylic acid monomer is between 10:1 and 200:1, preferably between 50:1 and 150: 1 and more preferably between 80:1 and 130:1.

These polymers are next reacted with a crosslinker of the invention to produce the molecular colloid networks of the present invention. Preferred photosensitive and/or thermosensitive polymers are listed in table 2.

**Table 2 Examples of preferred photosensitive and/or thermosensitive polymers.**

| No. | Polymer |
|---|---|
| A1 | VP-co-APMA |
| A2 | VP-co-MABP |
| A3 | VP-co-HEMA-BBA |
| A4 | VP-co-glycidylmethacrylate |
| A5 | VP-co-benzophenone maleate |
| A6 | VP-co-maleate-4-aminobenzophenone-PEG-derivative |

### Combination of polymer and crosslinker

To prepare the molecular colloid network, the at least one polymer (i.e. molecular colloid) is dissolved with the crosslinker in a suitable solvent. After activation of the component mixture of polymer and crosslinker via heat and/or light of suitable wavelength ranges in the UV-vis region, the reaction between the components occurs after the formation of radicals, whereby a crosslinked molecular colloid is formed, the degree of crosslinking depends on the proportion of compounds capable of forming radicals and the necessary activation energy in the mixture of polymer and crosslinker.

The crosslinker serves to stabilize the radicals of the polymer formed by photo/thermoactivation, which, however, simultaneously leads to crosslinking of the linear polymers with each other and crosslinking of the polymer with the crosslinker. As a result, there is a noticeable increase in mechanical, physical and chemical properties. The resulting molecular colloid network has improved flexibility, lower frictional resistance, higher stability and improved moisture retention, which in turn means an increase in hydrophilicity as well as other properties.

The light and/or thermo-activated reaction of the crosslinker with the at least one polymer can, depending on the procedure and wavelength range, also lead to reaction with materials present that are not part of the mixture, such as a surface to which the polymer and the crosslinker are applied, thus increasing the adhesion to this surface by forming covalent bonds, whereby stronger adhesion also enables a longer period of use, repeatable application included. This reaction with the substrate is facilitated by conditioning the material that comes into contact with the component mixture. This is done, for example, by pre-wetting with a suitable solvent, but can also be achieved by irradiation with light, heat, plasma processes, etc.

In general, it can be stated that the properties of the product obtained from the at least one polymer and crosslinker can be varied as desired with the specific selection of the building blocks of polymer and crosslinker.

The coefficients of friction determined on various substrates (nylon, Pebax, polypropylene, silicone) show particularly low values for these preferred combinations. It has been found that especially the combination of the components A2/B3 and photo-PVP-co-maleate (A5)/A2/B3 and photo-PVP-co-maleate(A5)/B3 lead to particularly low coefficients of friction of far below 0.1 on various materials. A2/B3, for example, has an amazingly low coefficient of friction of 0.008 (on nylon).

The coefficients of friction determined on various substrates (nylon, Pebax, polypropylene, silicone) show particularly low values for these preferred combinations. It has been found that especially the combination of components A2/B3 and PVP-co-maleate(+benzophenone)/A2/B3 and PVP-co-maleate(+benzophenone)/B3 lead to particularly low coefficients of friction of well below 0.1 on various materials. A2/B3, for example, has an amazingly low coefficient of friction of 0.008 (on nylon).

Thus, the present invention further relates to compositions comprising at least one polymer and at least one crosslinker according to the present invention. Therefore, the present invention further relates to compositions comprising at least one polymer and at least one crosslinker, wherein the polymer is a copolymer of general formula (v):
The present invention further relates to copolymers of general formula (v): 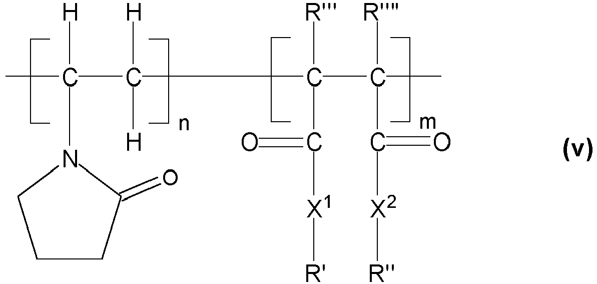 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, a substituted or unsubstituted alkyl, a substituted or unsubstituted aryl, a substituted or unsubstituted arylalky, a substituted or unsubstituted alkoxy, a substituted or unsubstituted aryloxy, a substituted or unsubstituted alkylhydroxy, a substituted or unsubstituted arylhydroxy, a substituted or unsubstituted alkylaryloxy, a substituted or unsubstituted alkylamino, a substituted or unsubstituted arylamino, a substituted or unsubstituted alkylarylamino, silyl groups organometallic groups and -H; wherein wherein **R^{'}** and **R^{"}** are not both -H;
**R‴** and **R""** represent independently of each other -H, -CH₃, -CH₂CH₃, or -F;
**L** represents -CH₂-;
**BP** represents 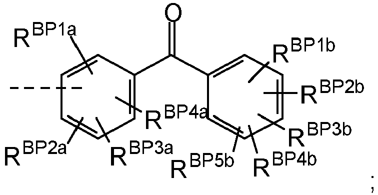
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 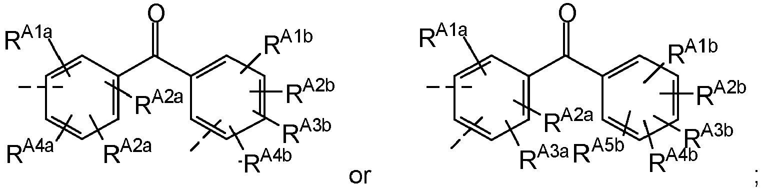
**BP¹** and **BP²** independently of each other represent 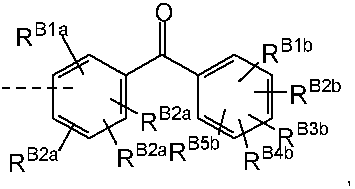
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, -C₆H₁₂R⁵CH₂-, 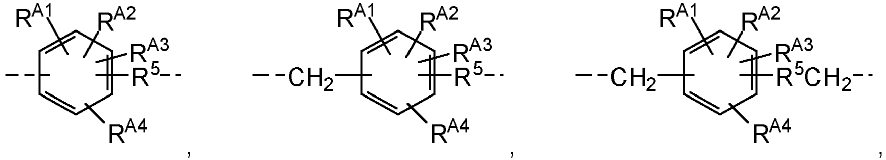 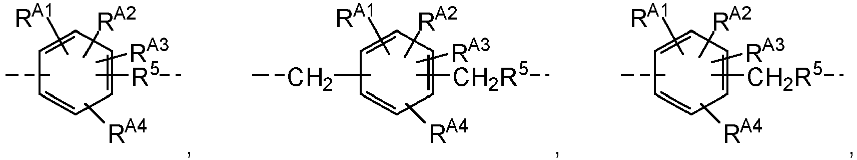 or 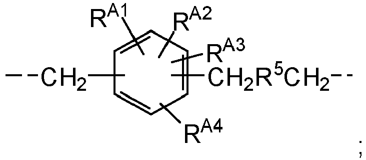
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, -CH₂R⁶C₆H₁₂-, 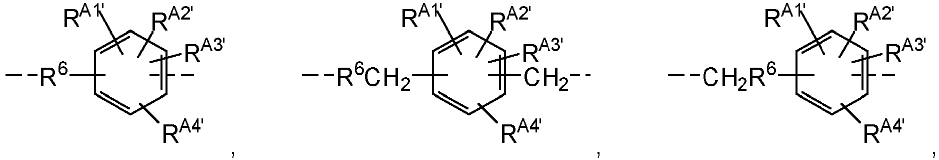 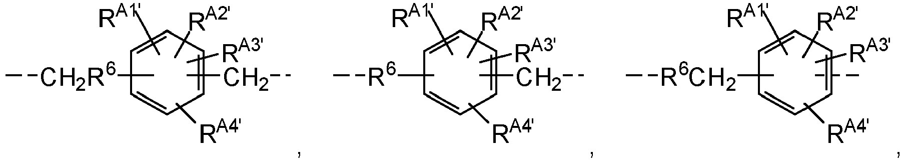 or 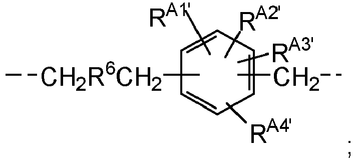
**R⁵** and **R⁶** independently of each other represent -NHCO-, -CONH-, -OCO-, -COO-, -NHCOO-, -OCONH-, -OCOO-, or -NHCONH-; and
**R^{A1}** - **R^{A4}, R^{A1'}** - **R^{A4'}, R^{A1a}** - **R^{A4a}, R^{A1b}** - **R^{A5b}**, **R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Thus, the present invention further relates to compositions comprising at least one polymer and at least one crosslinker according to the present invention. Therefore, the present invention further relates to compositions comprising at least one polymer and at least one crosslinker, wherein the polymer is a copolymer of general formula (v): 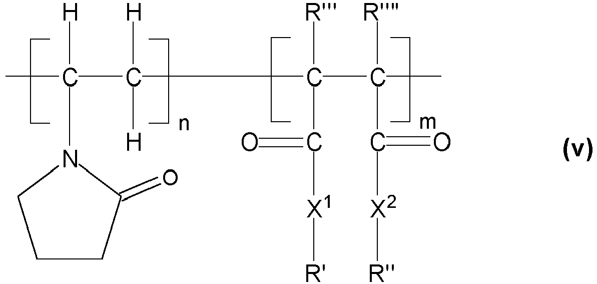 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R'** and **R"** independently of each other are selected from -BP, -L-BP, -(CH₂CH₂O)_{q}-OH, and -H; wherein **R'** and **R"** are not both -H;
**R‴** and **Rʺʺ** represent independently of each other -H, -CH₃, -CH₂CH₃, or -F;
**L** represents -CH₂-;
**BP** represents 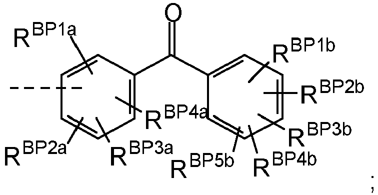
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 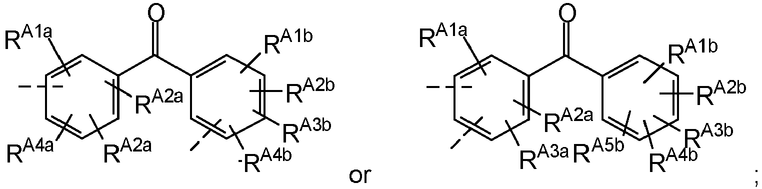
**BP¹** and **BP²** independently of each other represent 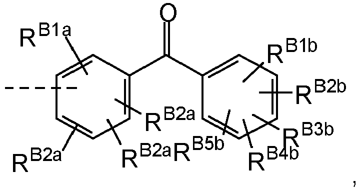
**R¹** and **R⁴** independently of each other represent a bond or -CH₂-;
**R²** represents a bond, or -CH₂-;
**R³** represents a bond, or -CH₂-;
**R^{A1a}** - **R^{A4a}**, **R^{A1b}** - **R^{A5b}**, **R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Thus, the present invention further relates to compositions comprising at least one polymer and at least one crosslinker according to the present invention. Therefore, the present invention further relates to compositions comprising at least one polymer and at least one crosslinker, wherein the polymer is a copolymer of general formula (**v**): 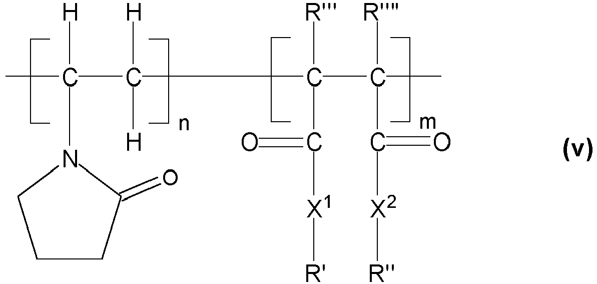 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R'** and **R"** independently of each other are selected from -BP, -L-BP, -(CH₂CH₂O)_{q}-OH, and -H; wherein **R'** and R" are not both -H;
**R‴** and **Rʺʺ** represent independently of each other -H, -CH₃, -CH₂CH₃, or -F;
**L** represents -CH₂-;
**BP** represents 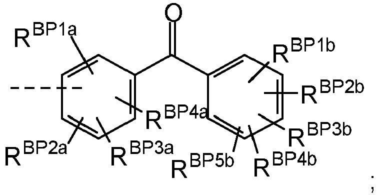
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 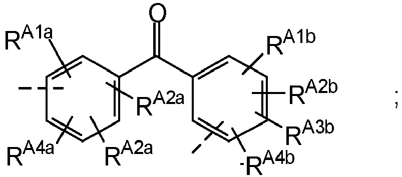
**BP¹** and **BP²** independently of each other represent 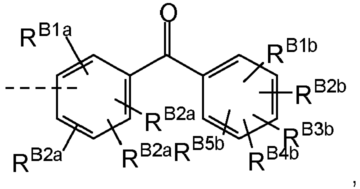
**R¹** and **R⁴** independently of each other represent a bond or -CH₂-;
**R²** represents a bond, or -CH₂-;
**R³** represents a bond, or -CH₂-;
**R^{A1a}** - **R^{A4a}**, **R^{A1b}** - **R^{A5b}**, **R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Thus, the present invention further relates to compositions comprising at least one polymer and at least one crosslinker according to the present invention. Therefore, the present invention further relates to compositions comprising at least one polymer and at least one crosslinker, wherein the polymer is a copolymer of general formula (v): 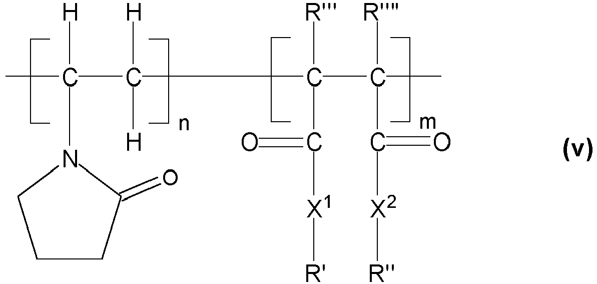 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R'** and **R"** independently of each other are selected from -BP, -L-BP, -(CH₂CH₂O)_{q}-OH, and -H; wherein **R'** and **R"** are not both -H;
**R‴** and **Rʺʺ** represent independently of each other -H, -CH₃, -CH₂CH₃, or -F;
**L** represents -CH₂-;
**BP** represents 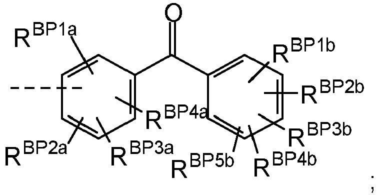
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, or -R¹-OCONH-R²;
**L²** represents -R³-NHCO-R⁴-, -R³-CONH-R⁴-, -R³-OCO-R⁴-, -R³-COO-R⁴-, -R³-NHCOO-R⁴-, or -R³-OCONH-R⁴-;
**Z** represents 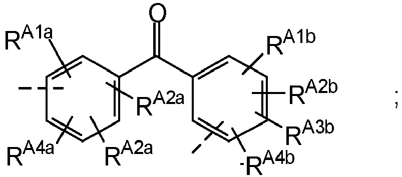
**BP¹** and **BP²** independently of each other represent 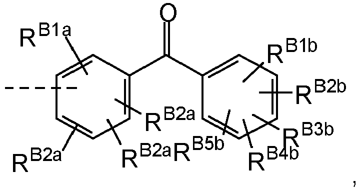
**R¹** and **R⁴** independently of each other represent a bond or -CH₂-;
**R²** represents a bond, or -CH₂-;
**R³** represents a bond, or -CH₂-;
**R^{A1a}** - **R^{A4a}**, **R^{A1b}** - **R^{A5b}**, **R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Thus, the present invention further relates to compositions comprising at least one polymer and at least one crosslinker according to the present invention. Therefore, the present invention further relates to compositions comprising at least one polymer and at least one crosslinker, wherein the polymer is a copolymer of general formula (**v**): 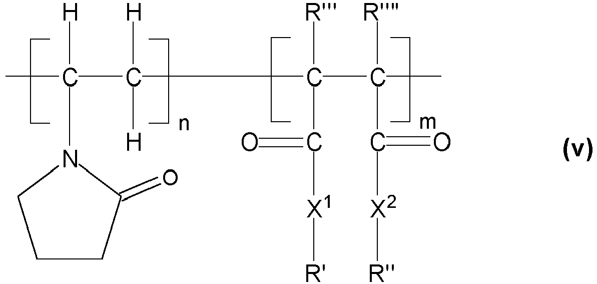 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R'** and **R"** independently of each other are selected from -BP, -L-BP, -(CH₂CH₂O)_{q}-OH, and -H; wherein **R'** and **R"** are not both -H;
**R‴** and **Rʺʺ** represent independently of each other -H, -CH₃, -CH₂CH₃, or -F;
**L** represents -CH₂-;
**BP** represents 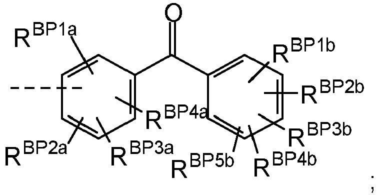
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, or -R¹-COO-R²-:
**L²** represents -R³-NHCO-R⁴-, -R³-CONH-R⁴-, -R³-OCO-R⁴-, or -R³-COO-R⁴-;
**Z** represents 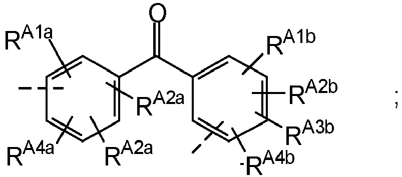
**BP¹** and **BP²** independently of each other represent 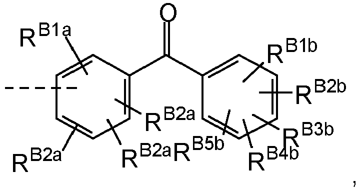
**R¹** and **R⁴** independently of each other represent a bond or -CH₂-;
**R²** represents a bond, or -CH₂-;
**R³** represents a bond, or -CH₂-;
**R^{A1a}** - **R^{A4a}**, **R^{A1b}** - **R^{A5b}**, **R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Thus, the present invention further relates to compositions comprising at least one polymer and at least one crosslinker according to the present invention. Therefore, the present invention further relates to compositions comprising at least one polymer and at least one crosslinker, wherein the polymer is a copolymer of general formula (v): 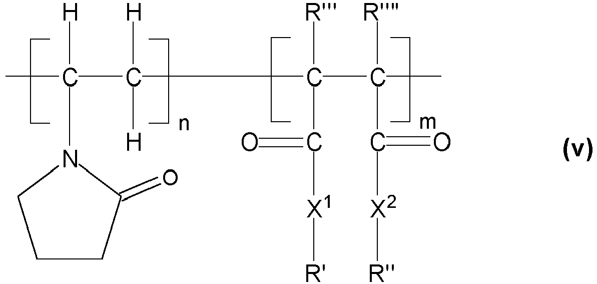 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, and -H; wherein **R^{'}** and **R^{"}** are not both -H;
**R‴** and **Rʺʺ** represent independently of each other -H, -CH₃, -CH₂CH₃, or -F;
**L** represents -CH₂-;
**BP** represents 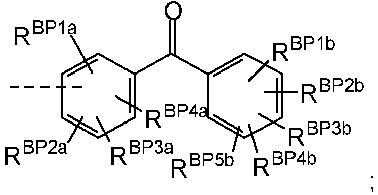
**R^{BP1a}** - **R^{BP4a}** and **R^{BP1b}** - **R^{BP5b}** represent -H;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, or -R¹-COO-R²-:
**L²** represents -R³-NHCO-R⁴-, -R³-CONH-R⁴-, -R³-OCO-R⁴-, or -R³-COO-R⁴-;
**Z** represents 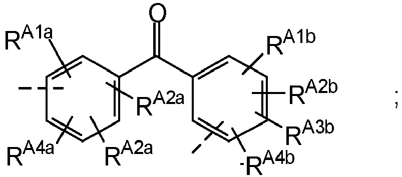
**BP¹** and **BP²** independently of each other represent 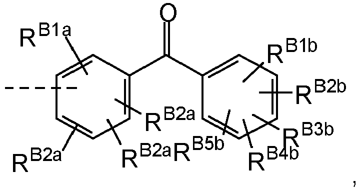
**R¹** and **R⁴** independently of each other represent a bond or -CH₂-;
**R²** represents a bond, or -CH₂-;
**R³** represents a bond, or -CH₂-;
**R^{A1a}**, and **R^{A1b}**, independently of each other represent -H, -COOH, or -CH₃.
**R^{B1a}** - **R^{B4a}** and **R^{B1b}** - **R^{B5b}** independently of each other represent -H,...-OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH_{3.}

Thus, the present invention further relates to compositions comprising at least one polymer and at least one crosslinker according to the present invention. Therefore, the present invention further relates to compositions comprising at least one polymer and at least one crosslinker, wherein the polymer is a copolymer of general formula (**v**): 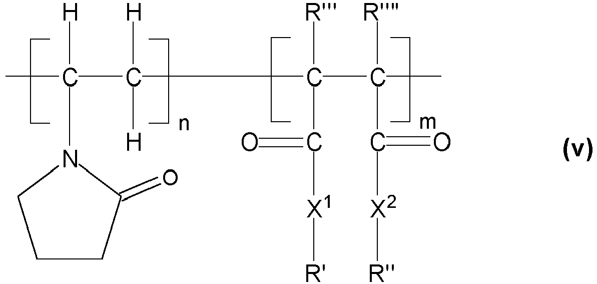 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, and -H; wherein **R^{'}** and **R^{"}** are not both -H;
**R‴** and **Rʺʺ** represent independently of each other -H, -CH₃, -CH₂CH₃, or -F;
**L** represents -CH₂-;
**BP** represents 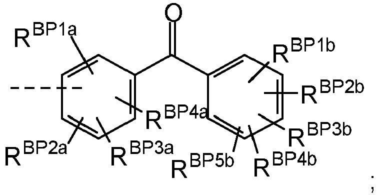
**R^{BP1a} - R^{BP4a}** and **R^{BP1b} - R^{BP5b}** represent -H;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, or -R¹-COO-R²-:
**L²** represents -R³-NHCO-R⁴-, -R³-CONH-R⁴-, -R³-OCO-R⁴-, or -R³-COO-R⁴-;
**Z** represents 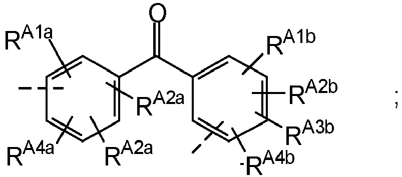
**BP¹** and **BP²** independently of each other represent 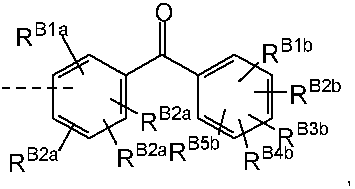
**R¹** and **R⁴** independently of each other represent a bond or -CH₂-;
**R²** represents a bond, or -CH₂-;
**R³** represents a bond, or -CH₂-;
**R^{A1a},** and **R^{A1b},** independently of each other represent -H, -COOH, or -CH₃.
**R^{B1a} - R^{B4a}** and **R^{B1b}** - **R^{B5b}** represent -H

Particularly preferably, the present invention further relates to compositions comprising at least one polymer and at least one crosslinker, wherein the polymer is a copolymer selected from 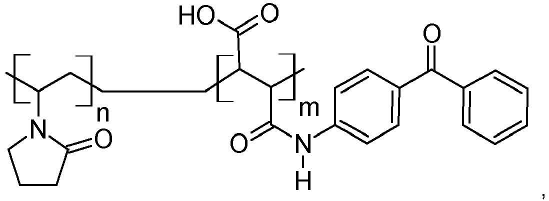 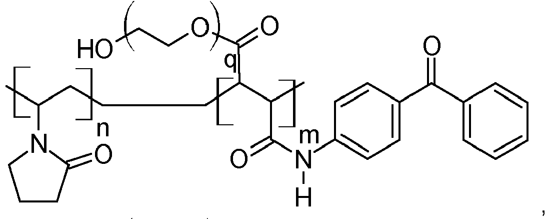 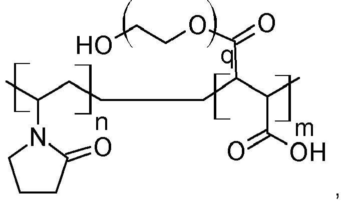 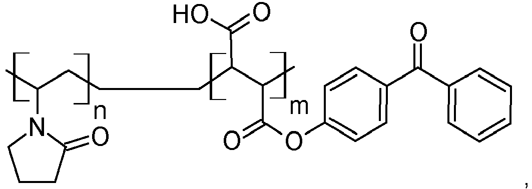 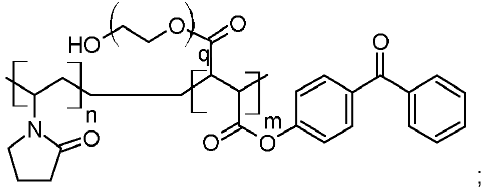 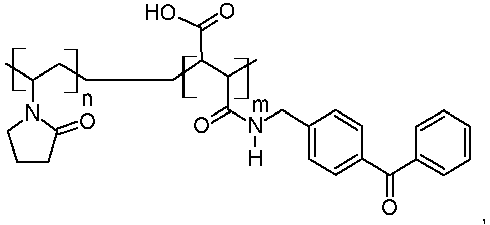 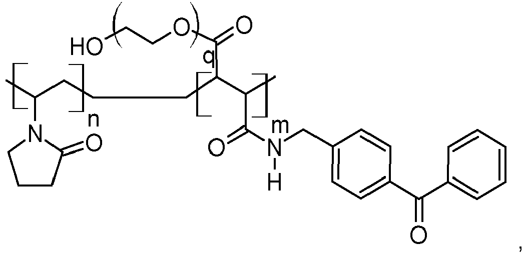 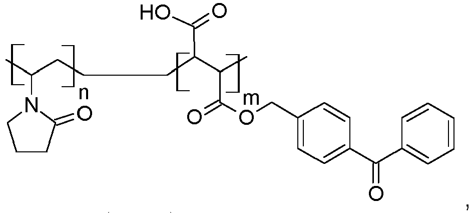 and 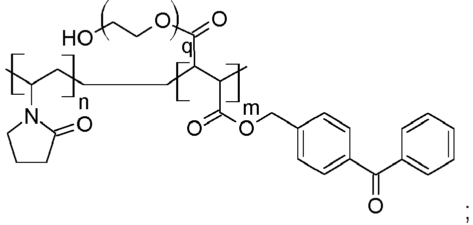 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**q** is an integer between 5 and 50,
and the crosslinker is selected from 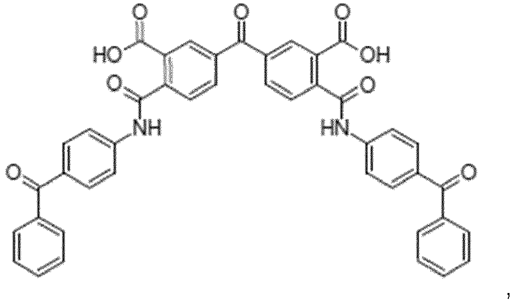 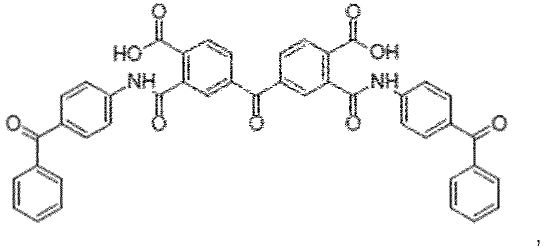 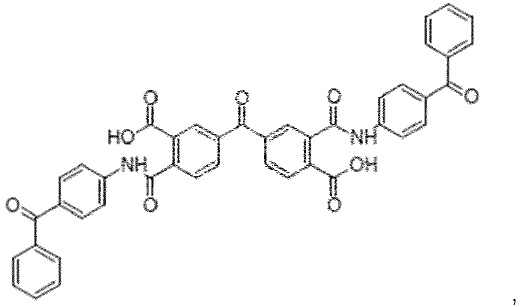 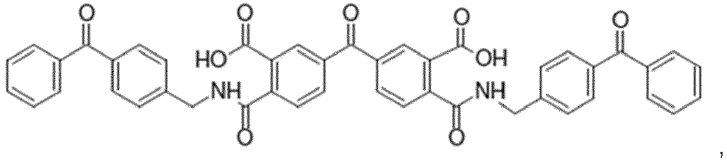 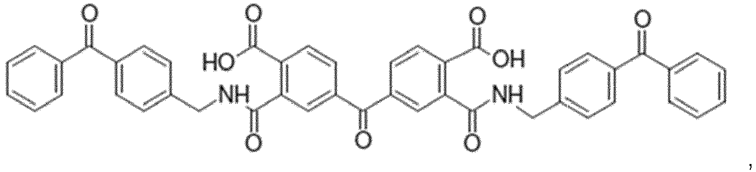 and 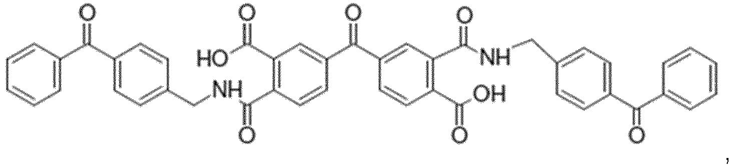 or mixtures thereof.

### Coated Medical Devices

The present invention further relates to a medical device having a coating of a cross-linked composition comprising at least one polymer and at least one crosslinker according to the present invention

The present invention further relates to the use of a molecular colloid according to the invention as a medical device, component of a medical device, coating substance, coating of a medical device such as catheters, balloon catheters, stents, guide wires, needles, cannulas, additive in cosmetics, care products, hygiene products, ointments, gels, pads, medical aid, lubricant, for moisture regulation or absorption and for friction reduction of surfaces.

The present invention further relates to the use of a molecular colloid according to the invention as a medical device, component of a medical device, coating substance, coating of a medical device such as catheters, balloon catheters, stents, guide wires, needles, cannulas, additive in cosmetics, care products, hygiene products, ointments, gels, pads, medical aids, lubricants, for moisture regulation or absorption, for friction reduction of surfaces, containing at least one pharmaceutically active substance which can be released in a controlled manner.

### Preferred Coated Medical Devices

The present invention relates to a medical device having a coating of a **cross-linked** vinylpyrrolidone copolymer and at least one crosslinker according to the present invention, wherein the polymer is a copolymer of general formula (v): 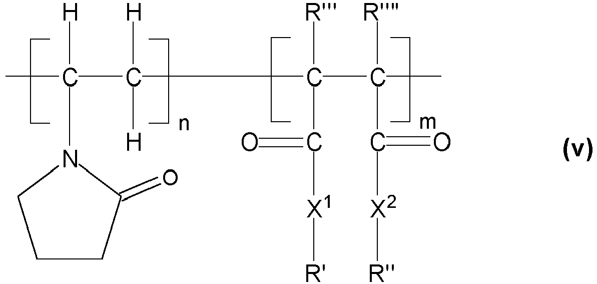 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, a substituted or unsubstituted alkyl, a substituted or unsubstituted aryl, a substituted or unsubstituted arylalky, a substituted or unsubstituted alkoxy, a substituted or unsubstituted aryloxy, a substituted or unsubstituted alkylhydroxy, a substituted or unsubstituted arylhydroxy, a substituted or unsubstituted alkylaryloxy, a substituted or unsubstituted alkylamino, a substituted or unsubstituted arylamino, a substituted or unsubstituted alkylarylamino, silyl groups organometallic groups and -H; wherein wherein **R^{'}** and **R^{"}** are not both -H;
**R‴** and **Rʺʺ** represent independently of each other -H, -CH₃, -CH₂CH₃, or -F;
**L** represents -CH₂-;
**BP** represents 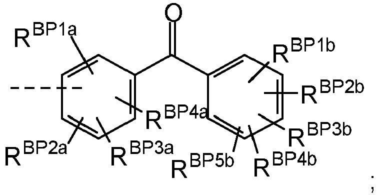
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 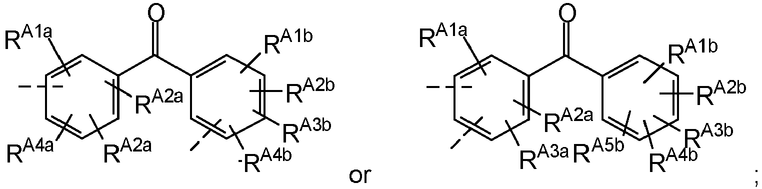
**BP¹** and **BP²** independently of each other represent 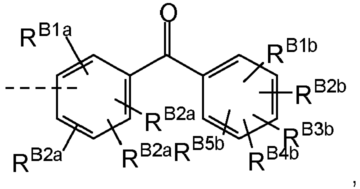
**R¹** and **R⁴** independently of each other represent a bond or -CH₂-;
**R²** represents a bond, -CH₂-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂- -C₅H₁₀R⁵CH₂-, -C₆H₁₂R⁵CH₂-, 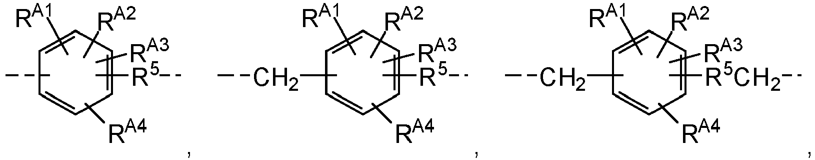 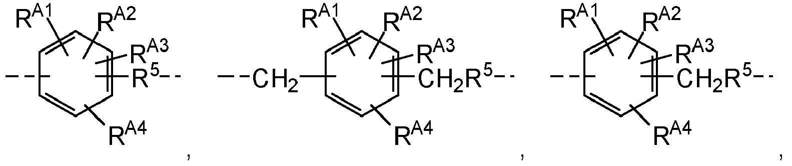 or 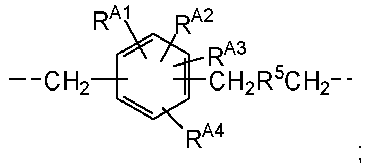
**R³** represents a bond, -CH₂-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, -CH₂R⁶C₆H₁₂-, 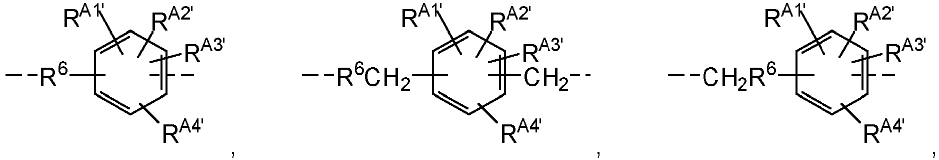 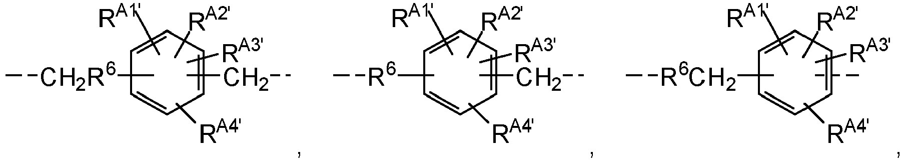 or 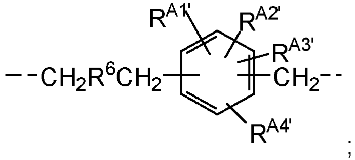
**R⁵** and **R⁶** independently of each other represent -NHCO-, -CONH-, -OCO-, -COO-, -NHCOO-, -OCONH-, -OCOO-, or -NHCONH-; and
**R^{A1}** - **R^{A4}, R^{A1'}**, - **R^{A4'}**, **R^{A1a}** - **R^{A4a}**, **R^{A1b}** - **R^{A5b}**, **R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

The present invention relates to a medical device having a coating of a **cross-linked** vinylpyrrolidone copolymer and at least one crosslinker according to the present invention, wherein the polymer is a copolymer of general formula (v): 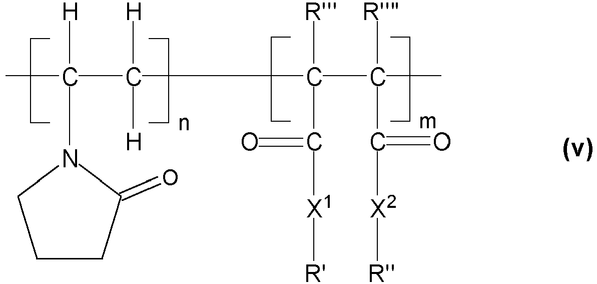 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, and -H; wherein R' and R" are not both -H;
**R‴** and **Rʺʺ** represent independently of each other -H, -CH₃, -CH₂CH₃, or -F;
**L** represents -CH₂-;
**BP** represents 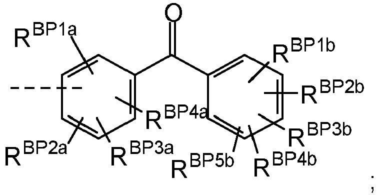
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 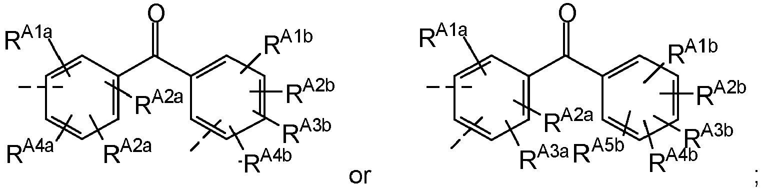
**BP¹** and **BP²** independently of each other represent 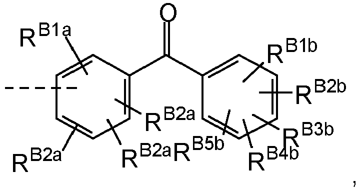
**R¹** and **R⁴** independently of each other represent a bond or -CH₂-;
**R²** represents a bond, or -CH₂-;
**R³** represents a bond, or -CH₂-;
**R^{A1a}** - **R^{A4a}, R^{A1b}** - **R^{A5b}**, **R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

The present invention relates to a medical device having a coating of a **cross-linked** vinylpyrrolidone copolymer and at least one crosslinker according to the present invention, wherein the polymer is a copolymer of general formula (v): 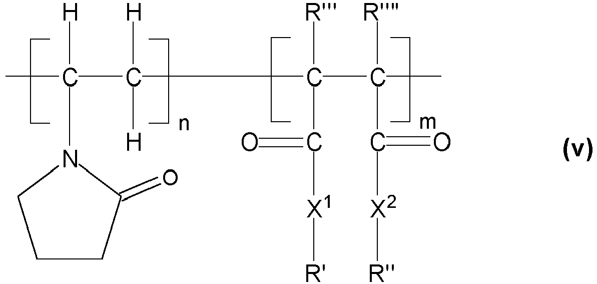 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R^{'}** and **R^{"}** independently of each other are selected from -BP, -L-BP, -(CH₂CH₂O)_{q}-OH, and -H; wherein **R^{'}** and **R^{"}** are not both -H;
**R‴** and **Rʺʺ** represent independently of each other -H, -CH₃, -CH₂CH₃, or -F;
**L** represents -CH₂-;
**BP** represents 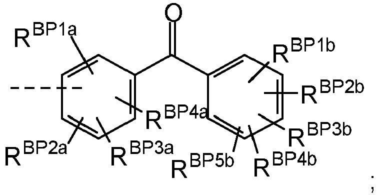
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, -R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** represents 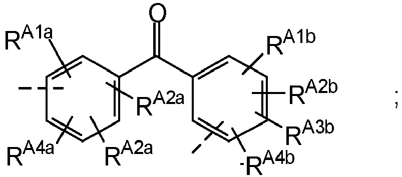
**BP¹** and **BP²** independently of each other represent 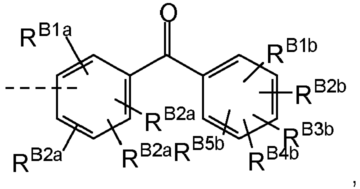
**R¹** and **R⁴** independently of each other represent a bond or -CH₂-;
**R²** represents a bond, or -CH₂-;
**R³** represents a bond, or -CH₂-;
**R^{A1a} - R^{A4a}, R^{A1b} - R^{A5b}, R^{B1a} - R^{B4a}** and **R^{B1b} - R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

The present invention relates to a medical device having a coating of a **cross-linked** vinylpyrrolidone copolymer and at least one crosslinker according to the present invention, wherein the polymer is a copolymer of general formula (v): 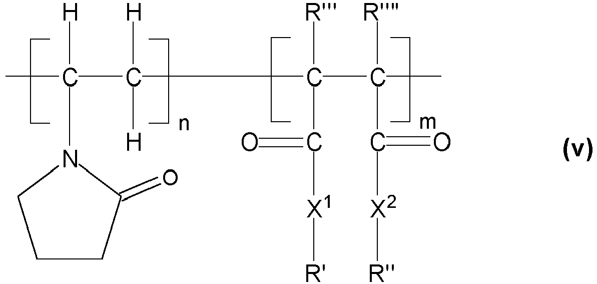 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R'** and **R"** independently of each other are selected from -BP, -L-BP, -(CH₂CH₂O)_{q}-OH, and -H; wherein **R'** and **R"** are not both -H;
**R‴** and **Rʺʺ** represent independently of each other -H, -CH₃, -CH₂CH₃, or -F;
**L** represents -CH₂-;
**BP** represents 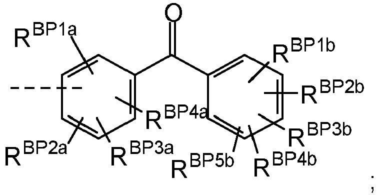
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, -R¹-COO-R²-, -R¹-NHCOO-R²-, or -R¹-OCONH-R²;
**L²** represents -R³-NHCO-R⁴-, -R³-CONH-R⁴-, -R³-OCO-R⁴-, -R³-COO-R⁴-, -R³-NHCOO-R⁴-, or -R³-OCONH-R⁴-;
**Z** represents 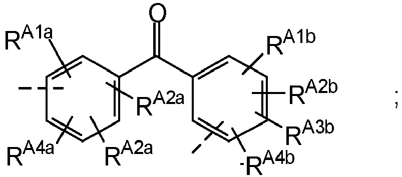
**BP¹** and **BP²** independently of each other represent 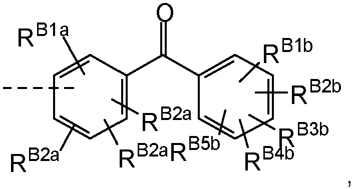
**R¹** and **R⁴** independently of each other represent a bond or -CH₂-;
**R²** represents a bond, or -CH₂-;
**R³** represents a bond, or -CH₂-;
**R^{A1a}** - **R^{A4a}, R^{A1b}** - **R^{A5b}, R^{B1a}** - **R^{B4a}** and **R^{B1b}** - **R^{B5b}** independently of each other represent -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

The present invention relates to a medical device having a coating of a **cross-linked** vinylpyrrolidone copolymer and at least one crosslinker according to the present invention, wherein the polymer is a copolymer of general formula (v): 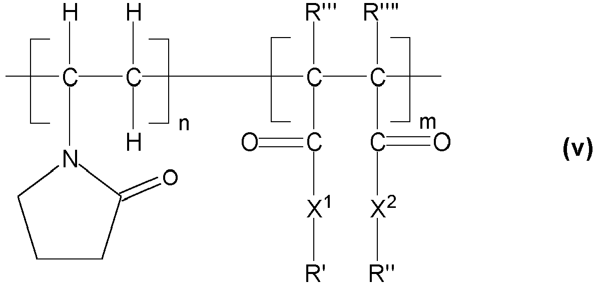 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R'** and **R"** independently of each other are selected from -BP, -L-BP, -(CH₂CH₂O)_{q}-OH, and -H; wherein **R'** and **R"** are not both -H;
**R‴** and **Rʺʺ** represent independently of each other -H, -CH₃, -CH₂CH₃, or -F;
**L** represents -CH₂-;
**BP** represents 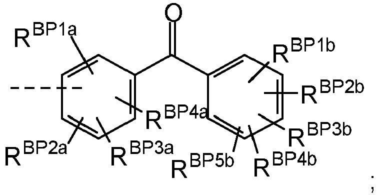
**R^{BP1a}** - **R^{BP4a}** and **R^{BP1b}** - **R^{BP5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, or -R¹-COO-R²-:
**L²** represents -R³-NHCO-R⁴-, -R³-CONH-R⁴-, -R³-OCO-R⁴-, or -R³-COO-R⁴-;
**Z** represents 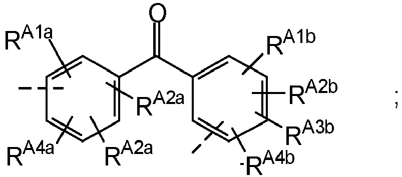
**BP¹** and **BP²** independently of each other represent 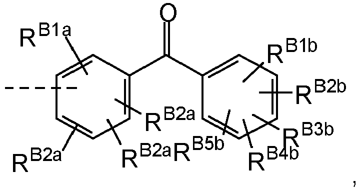
**R¹** and **R⁴** independently of each other represent a bond or -CH₂-;
**R²** represents a bond, or -CH₂-;
**R³** represents a bond, or -CH₂-;
**R^{A1a}** - **R^{A4a}, R^{A1b}** - **R^{A5b}, R^{B1a}** - **R^{B4a}** and **R^{B1b}** - **R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

The present invention relates to a medical device having a coating of a **cross-linked** vinylpyrrolidone copolymer and at least one crosslinker according to the present invention, wherein the polymer is a copolymer of general formula (v): 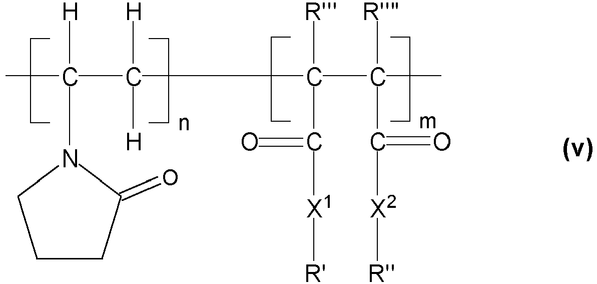 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R'** and **R"** independently of each other are selected from -BP, -L-BP, -(CH₂CH₂O)_{q}-OH, and -H; wherein **R'** and **R"** are not both -H;
**R‴** and **Rʺʺ** represent independently of each other -H, -CH₃, -CH₂CH₃, or -F;
**L** represents -CH₂-;
**BP** represents 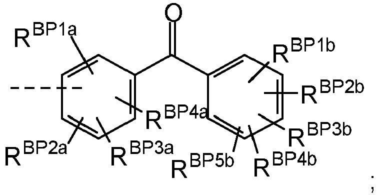
**R^{BP1a}** - **R^{BP4a}** and **R^{BP1b}** - **R^{BP5b}** represent -H;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, or -R¹-COO-R²-:
**L²** represents -R³-NHCO-R⁴-, -R³-CONH-R⁴-, -R³-OCO-R⁴-, or -R³-COO-R⁴-;
**Z** represents 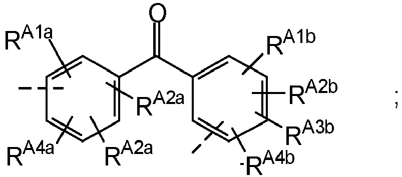
**BP¹** and **BP²** independently of each other represent 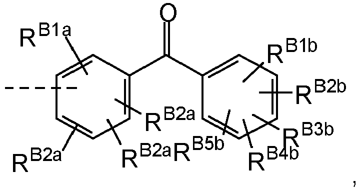
**R¹** and **R⁴** independently of each other represent a bond or -CH₂-;
**R²** represents a bond, or -CH₂-;
**R³** represents a bond, or -CH₂-;
**R^{A1a},** and **R^{A1b},** independently of each other represent -H, -COOH, or -CH₃.
**R^{B1a}** - **R^{B4a}** and **R^{B1b}** - **R^{B5b}** independently of each other represent -H,...-OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃.

The present invention relates to a medical device having a coating of a **cross-linked** vinylpyrrolidone copolymer and at least one crosslinker according to the present invention, wherein the polymer is a copolymer of general formula (v): 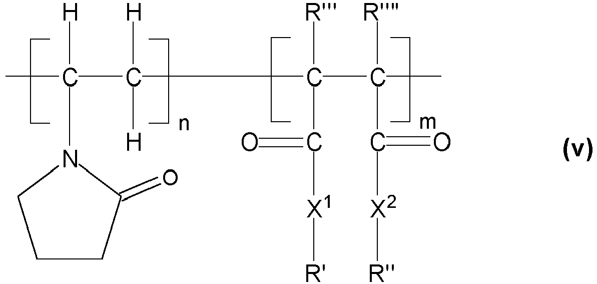 wherein
**m** = 0.1 - 50 mol%, preferably 0.1 - 10 mol%, preferably 0.1 - 0.9 mol%, more preferably 0.1 - 0.85 mol%, more preferably 0.5 - 0.85 mol%;
**n** = 100 mol% - m;
**X¹** and **X²** represent independently of each other -O- or -NH-;
**R'** and **R"** independently of each other are selected from -BP, -L-BP, -(CH₂CH₂O)_{q}-OH, and -H; wherein **R'** and **R"** are not both -H;
**R‴** and **Rʺʺ** represent independently of each other -H, -CH₃, -CH₂CH₃, or -F;
**L** represents -CH₂-;
**BP** represents 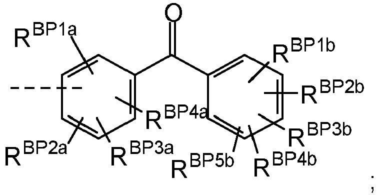
**R^{BP1a}** - **R^{BP4a}** and **R^{BP1b}** - **R^{BP5b}** represent -H;
**q** is an integer between 5 and 900.
and wherein the crosslinker has general formula (**I**):

**BP¹-L¹-Z-L²-BP²** (**I**)

wherein
**L¹** represents -R¹-NHCO-R²-, -R¹-CONH-R²-, -R¹-OCO-R²-, or -R¹-COO-R²-:
**L²** represents -R³-NHCO-R⁴-, -R³-CONH-R⁴-, -R³-OCO-R⁴-, or -R³-COO-R⁴-;
**Z** represents 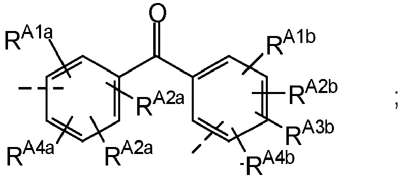
**BP¹** and **BP²** independently of each other represent 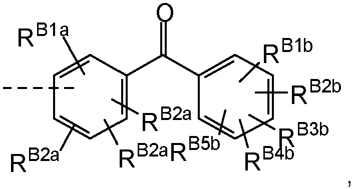
**R¹** and **R⁴** independently of each other represent a bond or -CH₂-;
**R²** represents a bond, or -CH₂-;
**R³** represents a bond, or -CH₂-;
**R^{A1a},** and **R^{A1b},** independently of each other represent -H, -COOH, or -CH₃.
**R^{B1a}** - **R^{B4a}** and **R^{B1b} - R^{B5b}** represent -H

Particularly preferably, the present invention further relates to compositions comprising at least one polymer and at least one crosslinker, wherein the polymer is a copolymer selected from 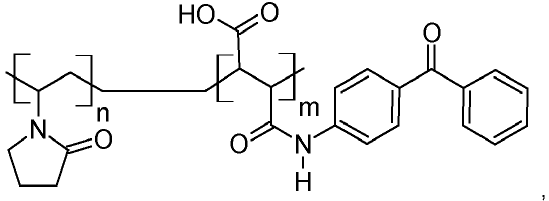 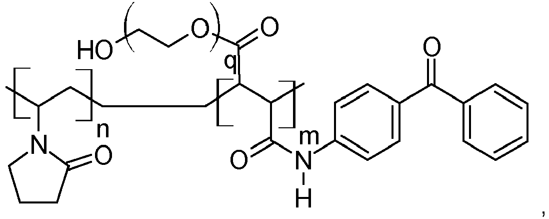 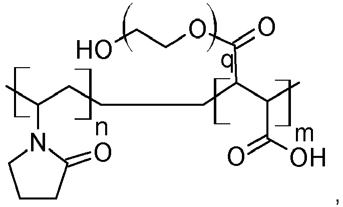 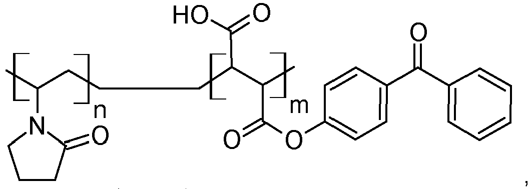 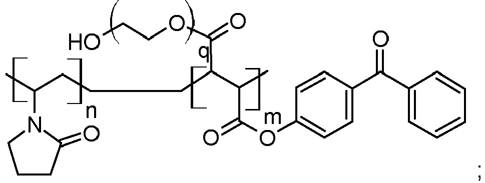 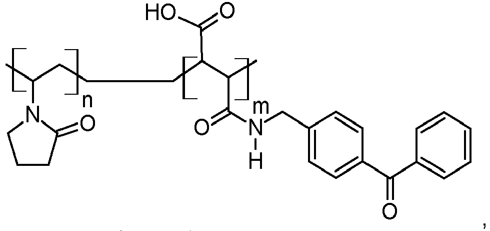 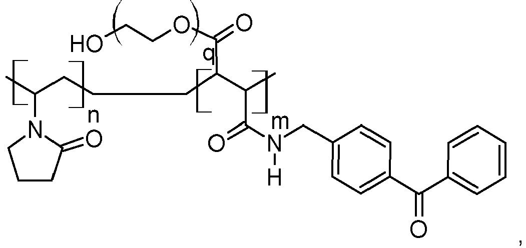 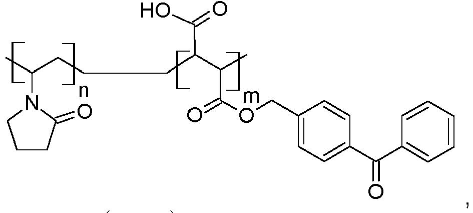 and 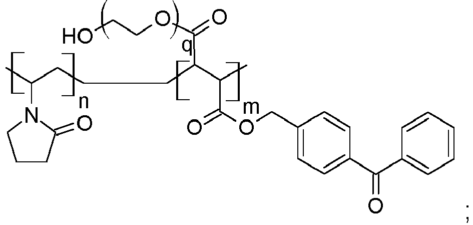 wherein
m = 0.1 - 50 mol%, preferably 0.1 - 10 mol%, preferably 0.1 - 0.9 mol%, more preferably 0.1 - 0.85 mol%, more preferably 0.5 - 0.85 mol%;
q is an integer between 5 and 50,
and the crosslinker is selected from 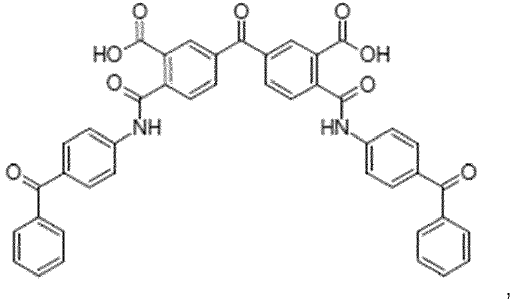 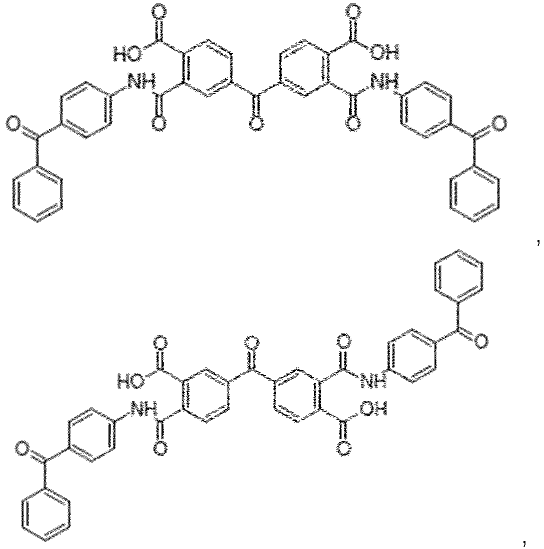 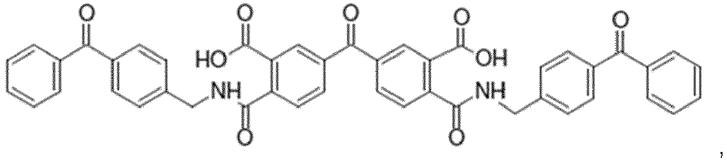 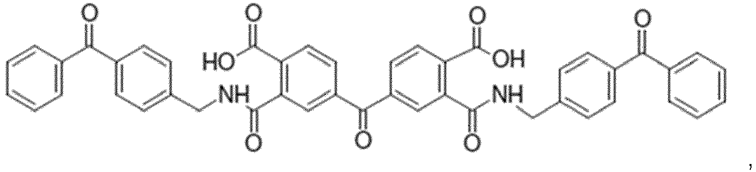 and 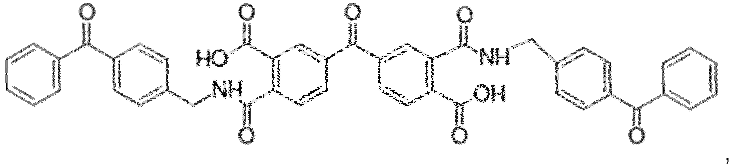 or mixtures thereof.

### Further components / optional additives

The addition of further additives to the mixture of the at least one polymer and the crosslinker offer many options to shape the properties of the intended end product, be it in the form of coatings, pads, plasters, fillings, gaskets, components of cremes and lotions, etc.

Biostable, biodegradable, biocompatible, biodegradable, bioresistant polymers and oligomers, low molecular weight compounds, active ingredients, metals, metal compounds, complexes, stabilizers, dyes, salts, glycosaminoglycans, carbohydrates, polysaccharides, proteins and peptides, etc., their mixtures and derivatives, can be added. It is important that the additives should further optimize the final product. The number and type of additives depends on what one wants to achieve. For example, it has been shown that the addition of polyvinylpyrrolidone LMW and HMW to the component mixture of the at least one polymer and the crosslinker can be advantageous.
In general, all additives are suitable that are biocompatible and don't decompose due to short-term irradiation with light or exposure to temperatures below 35°C , unless the decomposition products serve to optimize the final product.

Further therapeutic possibilities arise from additives such as active ingredients that can be added to the component mixture prior to the crosslinking step, although the choice is limited to UV-stable active ingredients and to active ingredients that are activated by irradiation. They can also be added subsequently, e.g. by a swelling process with the solution containing the active ingredient or applied superficially.

All biocompatible or hemocompatible biodegradable, bioresorbable and biostable polymers are suitable polymers. The selection of the polymer(s) to be added depends on the function to be fulfilled by the medical device, auxiliary, cosmetic and hygienic product.

In summary, the procedure for preparing the molecular colloid network is as follows:
1. providing at least one monomer (A1)
2. synthesis of the unsaturated photo- and/or thermosensitive α,β-unsaturated carboxylic acid component (A2).
3. preparation of component A from at least one monomer (A1)
   i) in the presence of the unsaturated photo- and/or α,β-unsaturated carboxylic acid components (A2) synthesized under 2.) to give a photoactive and/or thermoactive molecular colloidal copolymer,
      or
   ii) in the absence of the photoactive or thermoactive compounds synthesized under 2.) to give a molecular colloidal polymer/mixed polymer.
4. synthesizing the at least bifunctional thermosensitive or photosensitive compound (component B) according to the invention by reacting at least one diaryl ketone (B1) with at least one bifunctional compound (B2) to form ether, amide urethane, ester, thiourethane or urea bonds.
5. mixing of component A and component B
6. optional: addition of further components (polymers, active ingredients) to the mixture of components A and B
7. optional: in case of contact of the mixture with surfaces, prior conditioning of the surfaces with solvents, plasma, heat and/or light.
8. application of the mixture onto a surface or into a shaping vessel (template) by means of dipping, spraying, pipetting, brushing, dropping, pouring, etc.) or add to a fluid, paste
9. exposure/heating to produce the molecular colloid network
10. drying or resting of the final product to remove solvents and, if necessary, removal from shaping production material
11. sterilization and packaging

### Active Agents

The possible addition of active ingredients, e.g. applied over, in or under the coating of a medical device or medical tool according to the invention or covalently bonded to the coating, enables a therapeutic diversity which, in symbiosis with the properties of the coating, ensure optimal healing conditions. It is important that the active ingredients can develop the desired and intended effect. The choice of active ingredients is based on the physical and chemical interactions with the coating or the matrix containing the coating. These can come from any group of active ingredients and there is a wide choice depending on the therapeutic need, although many active ingredients can be assigned to several groups based on their mode of action.

Thus, the coating of the present invention preferably contains at least one anti-inflammatory, cytostatic, cytotoxic, antiproliferative, antimicrotubule, antiangiogenic, antiviral, antibacterial, antibiotic, antimitotic, antirestenotic, antifungal antineoplastic, antimigrative, anticancerogenic, vasodilator, athrombogenic, and antithrombogenic agent, or a mixture thereof. Also preferred is a coating containing at least one antiproliferative, immunosuppressive, antiangiogenic, antiinflammatory, fungicidal, antibiotic, antimigratory, athrombogenic or antithrombotic active agent or a mixture thereof.

The following anti-inflammatory, cytostatic, cytotoxic, antiproliferative, antimicrotubule, antiangiogenic, antiviral, antibacterial, antibiotic, antimitotic, antirestenotic, antifungal antineoplastic, antimigrative, anticancerogenic, vasodilator, athrombogenic, and antithrombogenic agents may be preferred: Sirolimus (rapamycin) all limus derivatives such as. everolimus, biolimus, zotarolimus, temsirolimus, somatostatin, tacrolimus, roxithromycin, dunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoboside, teniposide, nimustine, carmustine, lomustine, cyclophosphamide, 4-hydroxyoxycyclophosphamide, estramustine, melphalan, ifosfamide, tropfosfamide, chlorambucil, bendamustine, dacarbazine, busulfan, procarbazine, treosulfan, tremozolomide, thiotepa, daunorubicin, doxorubicin, aclarubicin, epirubicin, Mitoxantrone, idarubicin, bleomycin, mitomycin, dactinomycin, methotrexate, fludarabine, fludarabine 5'-dihydrogen phosphate, cladribine, mercaptopurine, thioguanine, cytarabine, fluorouracil, gemcitabine, capecitabine, docetaxel, Carboplatin, cisplatin, oxaliplatin, amsacrine, irinotecan, topotecan, hydroxycarbamide, miltefosine, pentostatin, aldesleukin, tretinoin, asparaginase, pegasparase, anastrozole, exemestane, letrozole, formestane, aminoglutethemide, adriamycin, Azithromycin, spiramycin, cepharantine, 8-ergolines, dimethylergolines, agroclavin, 1-allylisuride, 1-allylterguride, bromerguride, bromocriptine (ergotaman-3',6',18-trione, 2-bromo-12'-hydroxy-2'-(1-methylethyl)-5'-(2-methylpropyl)-,(5'alpha)-), elymoclavine, ergocristine (ergotaman-3', 6', 18-trione, 12'-hydroxy-2'-(1-methylethyl)-5'-(phenylmethyl)-, (5'-alpha)-), ergocristinine, ergocornine (ergotaman-3',6',18-trione, 12'-hydroxy-2',5'-bis(1-methylethyl)-, (5'-alpha)-), ergocorninine, ergocryptin (ergotaman-3',6',18-trione, 12'-hydroxy-2'-(1-methylethyl)-5'-(2-methylpropyl)-, (5'alpha)- (9CI)), ergocryptinine, ergometrine, ergonovine (ergobasin, INN: Ergometrine, (8beta(S))-9,10-didehydro-N-(2-hydroxy-1-methylethyl)-6-methyl-ergoline-8-carboxamide), ergosine, ergosinine, ergotmetrinine, ergotamine (ergotaman-3',6',18-trione, 12'-hydroxy-2'-methyl-5'-(phenylmethyl)-, (5'-alpha)-(9CI)), ergotaminin, ergovalin (ergotaman-3',6', 18-trione, 12'-hydroxy-2'-methyl-5'-(1-methylethyl)-, (5'alpha)-), lergotril, lisuride (CAS no.: 18016-80-3, 3-(9,10-didehydro-6-methylergolin-8alpha-yl)-1,1-diethylurea), lysergol, lysergic acid (D-lysergic acid), lysergic acid amide (LSA, D-lysergic acid amide), lysergic acid diethylamide (LSD, D-lysergic acid diethylamide, INN: lysergamide, (8β)-9,10-didehydro-N,N-diethyl-6-methyl-ergoline-8-carboxamide), isolysergic acid (D-isolysergic acid), isolysergic acid amide (D-isolysergic acid amide), isolysergic acid diethylamide (D-isolysergic acid diethylamide), mesulergin, metergoline, methergine (INN: methylergometrin, (8beta(S))-9,10-didehydro-N-(1-(hydroxymethyl)propyl)-6-methyl-ergoline-8-carboxamide), methylergometrin, methysergide (INN: methysergide, (8beta)-9,10-didehydro-N-(1-(hydroxymethyl)propyl)-1,6-dimethyl-ergoline-8-carboxamide), pergolide ((8β)-8-((methylthio)methyl)-6-propyl-ergoline), proterguride and terguride, celecoxip, thalidomide, Fasudil^{®,} cyclosporine, SMC proliferation inhibitor-2w, epothilone A and B, mitoxanthrone, azathioprine, mycophenolate mofetil, c-myc antisense, b-myc antisense, betulinic acid, camptothecin, PI-88 (sulfated oligosaccharide), melanocyte-stimulating hormone (α -MSH), activated protein C, IL1-β inhibitor, thymosinα -1, fumaric acid and its esters, calcipotriol, tacalcitol, lapachol, β-lapachone,podophyllotoxin, betulin, podophyllic acid-2-ethylhydrazide, molgramostim (rhuGM-CSF), peginterferon α-2b, lanograstim (r-HuG-CSF), filgrastim, macrogol, dacarbazine, basiliximab, daclizumab, Selectin (cytokine antagonist), CETP inhibitor, cadherins, cytokine inhibitors, COX-2 inhibitor, NFkB, angiopeptin, ciprofloxacin, camptothecin, fluroblastine, monoclonal antibodies that inhibit muscle cell proliferation, bFGF antagonists, probucol, prostaglandins, 1,11-dimethoxycanthin-6-one, 1-hydroxy-11-methoxycanthin-6-one, scopolectin, colchicine, NO donors such as pentaerythrityl tetranitrate, and syndnoeimines, S-nitroso derivatives, tamoxifen, staurosporine, β-estradiol,α -estradiol, estriol, estrone, ethinylestradiol, fosfestrol, medroxyprogesterone, estradiolcypionate, estradiolbenzoate, tranilast, camebacaurine and other terpenoids used in cancer therapy, verapamil, tyrosine kinase inhibitors (tyrphostins), cyclosporine A and B, paclitaxel and its derivatives such as 6-α -hydroxy-paclitaxel, baccatin, taxotere, synthetically produced as well as macrocyclic oligomers of carbon suboxide (MCS) obtained from native sources and its derivatives, mofebutazone, acemetacin, diclofenac, lonazolac, dapsone, o-carbamoylphenoxyacetic acid, lidocaine, ketoprofen, mefenamic acid, piroxicam, meloxicam, chloroquine phosphate, penicillamine, tumstatin, avastin, D-24851, SC-58125, hydroxychloroquine, auranofin, sodium aurothiomalate, oxaceprol, celecoxib, β-sitosterol, ademetionin, myrtecaine, polidocanol, nonivamide, levomenthol, benzocaine, aescin, ellipticin, D-24851 (Calbiochem), colcemid, cytochalasin A-E, indanocins, nocadazoles, S 100 protein, bacitracin, vitronectin receptor antagonists, azelastine, guanidyl cyclase stimulator tissue inhibitor of metalloproteinase-1 and 2, free nucleic acids, Nucleic acids incorporated into viral carriers, DNA and RNA fragments, plaminogen activator inhibitor-1, plasminogen activator inhibitor-2, antisense oligonucleotides, VEGF inhibitors, IGF-1, Agents from the group of antibiotics such as cefadroxil, cefazolin, cefaclor, cefotixin, tobramycin, gentamycin, penicillins such as dicloxacillin, oxacillin, sulfonamides, metronidazole, antithrombotics such as argatroban, aspirin, abciximab, synthetic antithrombin, bivalirudin, coumadin, enoxoparin, desulfated and N-reacetylated heparin, tissue plasminogen activator, GpIIb/IIIa platelet membrane receptor, factor Xₐ inhibitor antibody, interleukin inhibitors, heparin, hirudin, r-hirudin, PPACK, protamine, sodium salt of 2-methylthiazolidine-2,4-dicarboxylic acid prourokinase, streptokinase, warfarin, urokinase, Vasodilators such as dipyramidol, trapidil, nitroprusside, PDGF antagonists such as triazolopyrimidine and seramine, ACE inhibitors such as captopril, cilazapril, lisinopril, enalapril, losartan, thioprotease inhibitors, Prostacyclin, vasiprost, interferonα , β and γ, histamine antagonists, serotonin blockers, apoptosis inhibitors, apoptosis regulators such as p65, NF-kB, or Bcl-xL antisense oligonucleotides, halofuginone, nifedipine, tocopherol, vitamins B1, B2, B6, and B12, folic acid, tranilast, molsidomine, tea polyphenols, epicatechin gallate, epigallocatechin gallate, boswellic acids and their derivatives, leflunomide, Anakinra, etanercept, sulfasalazine, etoposide, dicloxacylline, tetracycline, triamcinolone, mutamycin, procainimide, D24851, SC-58125, retinoic acid, quinidine, disopyrimide, flecainide, propafenone, sotolol, amidoron, natural and synthetically produced steroids such as bryophyllin A, inotodiol, maquiroside A, ghalakinoside, mansonin, strebloside, hydrocortisone, betamethasone, dexamethasone, non-steroidal anti-inflammatory drugs (NSAIDS) such as fenoporfen, ibuprofen, indomethacin, naproxen, phenylbutazone and other antiviral agents such as acyclovir, ganciclovir and zidovudine, antifungal agents such as clotrimazole, flucytosine, griseofulvin, ketoconazole, miconazole, nystatin, terbinafine, antiprozoal agents like chloroquine, mefloquine, quinine, furthermore natural terpenoids like hippocaesculin, barringtogenol-C21-angelat, 14-dehydroagrostistachin, agroskerin, agroskerin, agrostistachin, 17-hydroxyagrostistachin, ovatodiolides, 4,7-oxycycloanisomelic acid, baccharinoids B1, B2, B3 and B7, tubeimoside, bruceanols A, B and C, bruceantinosides C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, furthermore cymarin, apocymarin, aristolochic acid, anopterin, hydroxyanopterin, anemonin, protoanemonin, berberin, cheliburin chloride, cictoxin, sinococulin, bombrestatin A and B, cudraisoflavone A, curcumin, dihydronitidine, nitidine chloride, 12-beta-hydroxypregnadiene-3,20-dione, bilobol, ginkgol, ginkgolic acid, helenalin, indicin, indicin-N-oxide, lasiocarpin, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetin, pancratistatin, liriodenin, oxoushinsunin, aristolactam-AII, bisparthenolidin, periplocoside A, ghalakinoside, ursolic acid, deoxypsorospermine, psycorubin, ricin A, sanguinarin, manwuweizic acid, methylsorbifolin, sphatheliachromene, stizophyllin, mansonin, strebloside, akagerin, dihydrousambaraensin, hydroxyusambarin, strychnopentamine, strychnophyllin, usambarin, usambarensin, berberin, liriodenin, oxoushinsunin, daphnoretin, lariciresinol, methoxylariciresinol, syringaresinol, umbelliferon, afromoson, acetylvismion B, desacetylvismion A, vismion A and B, and sulfur-containing amino acids such as cystine, as well as salts, hydrates, solvates, enantiomers, racemates, enantiomeric mixtures, diastereomeric mixtures; metabolites, prodrugs and mixtures of the active ingredients. Other possible additives with a therapeutic effect include halogens, minerals, metals, metal salts, hydrophilic and hydrophobic polymers or oligomers, polysaccharides such as glycosaminoglycans, carragenans, chitosans, oligosaccharides, alginates, peptides, proteins, etc., wherein derivatives and combinations with each other and with the active ingredients lead to possible variations, thus enabling an individually adapted therapy.

### Further Polymers

The combination of the coating according to the invention with other polymers also offers a wide range of possibilities. Suitable polymers include all biocompatible, biodegradable, bioresorbable and biostable polymers. The selection of the added polymer(s) is based on the function that the medical device must fulfill. In this way, for example, the hydrophilicity and hydrophobicity of the coating can be adjusted as desired. This can be done, for example, by mixing the coating with hydrophobic polymers or also by copolymerization.

Other monomers or oligomers of biodegradable, bioabsorbable or biostable or very slowly biodegrading polymers can be added and participate in the polymerization reaction and be incorporated into the polymer chain forming a binary, ternary, quaternary, etc. mixed polymer.

Biodegradable, biodegradable or bioabsorbable polymers include polyvalerolactones, poly-ε-decalactones, polylactonic acid, polyglycolic acid polylactides, polyglycolides, copolymers of polylactides and polyglycolides, poly-ε-caprolactone, polyhydroxybutyric acid, polyhydroxybutyrates, polyhydroxybutyrate-co-valerates, poly(1,4-dioxane-2,3-diones), poly(1,3-dioxane-2-ones), poly-p-dioxanones, polyanhydrides such as polymaleic anhydrides, polyhydroxymethacrylates, fibrin, polycyanoacrylates, polycaprolactone dimethylacrylates, poly-b-maleic acid polycaprolactone butyl acrylates, multiblock polymers such as.e.g., of oligocaprolactone diols and oligodioxanone diols, polyether ester multiblock polymers such as PEG and polybutylene terephthalate. Polypivotolactones, polyglycolic acid trimethyl carbonates, polycaprolactone glycolides, poly-g-ethylglutamate, poly(DTH-iminocarbonate), poly(DTE-co-DT-carbonate), poly(bisphenol A-iminocarbonate), polyorthoester, polyglycolic acid trimethyl carbonate, polytrimethyl carbonate polyiminocarbonate, poly(N-vinyl) pyrolidone, polyvinyl alcohols, polyesteramides, glycolated polyesters, polyphosphoesters, polyphosphazenes, poly[p-carboxyphenoxy)propane], polyhydroxypentanoic acid, polyanhydrides, polyethylene oxide-propylene oxide, soft polyurethanes, polyurethanes with amino acid residues in the backbone, polyether esters such as polyethylene oxide, polyalkenoxalates, polyorthoesters and their copolymers, lipids, carrageenans, fibrinogen, starch, collagen, protein-based polymers, polyamino acids, synthetic polyamino acids, zein, modified zein, polyhydroxyalkanoates, pectic acid, actinic acid, modified and unmodified fibrin and casein, carboxymethyl sulfate, albumin, furthermore hyaluronic acid, heparan sulfate, heparin, chondroitin sulfate, dextran, b-cyclodextrins, and copolymers with PEG and polypropylene glycol, gum arabic, guar, gelatin, collagen, collagen-N-hydroxysuccinimide, lipids, phospholipids, modifications and copolymers and/or mixtures of the aforementioned substances are used herein. Preferred biodegradable, polymers are povidones, such as Povidone 90 and Povidone 90. Also preferred herein as biodegradable polymers are the commercially available povidones Kollidon^{®} 90F with a molecular weight of 1000kDa - 1500kDa and Kollidon^{®} 30 with a molecular weight of 44kDa - 54 kDa.

Biostable or extremely slowly degradable polymers may also be present or admixed. The following can be used as biostable substances for the biostable layer(s): polyacrylic acid, polyacrylates such as polymethyl methacrylate, polybutyl methacrylate, polyacrylamide, polyacrylonitrile, polyamides, polyetheramides, polyethyleneamine, polyimides, polycarbonates, polycarbourethanes, polyvinyl ketones, polyvinyl halides, polyvinylidene halides, polyvinyl ethers, polyisobutylenes, polyvinyl aromatics, polyvinyl esters, polyvinyl pyrollidones, polyoxymethylenes, polytetramethylene oxide, polyethylene, polypropylene, polytetrafluoroethylene, polyurethanes, polyetherurethanes silicone-polyetherurethanes, silicone-polyurethanes, silicone-polycarbonate urethanes, polyolefin elastomers, polyisobutylenes, EPDM rubbers, fluorosilicones, carboxymethyl chitosans, polyarylether ether ketones, polyether ether ketones, polyethylene terephthalate, polyvalerates, carboxymethyl cellulose, cellulose, Rayon, Rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, EPDM rubbers, silicones such as polysiloxanes, polydimethylsiloxanes, polyvinyl halogens and copolymers, cellulose ethers, cellulose triacetates, chitosans and copolymers and/or mixtures of these substances.

The possible addition of further components, e.g. a higher molecular weight molecular colloid as component C and a lower molecular weight molecular colloid as component D, can further increase the hydrophilicity.

### Manufacturing process of coated medical devices and coated medical tools

The coating of surfaces is preferably carried out from an immersion solution, but can also be applied by spraying, pipetting, brushing, tattooing, spherical, drop-drag processes, filling of molds, etc., as well as in combinations of the above-mentioned processes. The choice of the process depends on the optimal functionality of the medical device or the auxiliary device to be achieved, whether special layer thicknesses, partial coatings, uniformity or non-uniformity of the layer thicknesses, inner surface or outer surface coatings, filling of cavities, etc. are required.

The coating is carried out as follows in four successive simple steps, whereby steps 1-3) can also be carried out on site and immediately before use.
In an application in which the components of the coating are only mixed shortly before use ("to go"), the sterilization (step 4) of the individual solutions is necessarily carried out in advance for each separately.
1) single dipping of the optionally pre-treated surface to be coated into the coating solution,
   or
   brushing/pipetting/spotting of a surface, e.g. when partially coating (e.g. only inside or outside, folds, corners, ends, center, recesses, i.e. only certain specified areas);
2) irradiation with UV/VIS light max. 300 sec. max 5 min or temperature increase or both or even longer (conveniently also by exposure to daylight until the solvent has also evaporated in the ideal case);
3) drying to remove solvents and residues, if necessary.

In the simplest case, the coating solution consisting of at least the mixture of component A and component B is applied to the surface of the medical device by a single immersion. Subsequently, the radical formation is activated briefly with UV light or or and temperature increase, so that the subsequent radical polymerization of the components takes place directly on the surface and leads to crosslinking.

The irradiation time depends on the components and varies due to their adsorption maxima. As a rule, UV irradiation of max. 300 sec. max 5 min is completely sufficient. In the case of VIS activation, the irradiation may well last considerably longer, which can conveniently be combined with evaporation of the solvent.

The surface to be coated can consist of a wide variety of common materials. Examples include polyamides (such as PA 12, PA 6, PA 66), stainless steel, nitinol, pebax, polyethylenes, polypropylenes, polyvinyls, polystyrenes, polyimides, PTFE, silicones, polyurethanes, polyesters such as polymethacrylates, polyacrylates, polyacrylamides, polycarbonates, polylactides, polyglycolides, polylactic acids, polyacetates, polycarbonates, etc., and their copolymers and derivatives.

Depending on the component mixture, a different preference for the material surface can be seen, which means that the optimum composition must be found experimentally for different substrates.

Depending on the material, prior conditioning of the surface may be advantageous to achieve the desired adhesion with the molecular colloidal coating. Conditioning can be achieved in different ways depending on the template, e.g. by wetting the surface with a suitable solvent, in the best case the solvent used in the component mixture, or by plasma treatment, heat or cold, irradiation or other suitable conditioning.

It is just as easy to fill components A and B - optionally with further additives (see above) - into molds (e.g. for breast implants, gel pads, depot patches, subcutaneous implants as depots, seals, etc.), where they either remain in the mold or are removed from it after irradiation or heat after the crosslinking reaction has taken place, or are injected, for example, into interstices, intracorporally such as joint spaces, and then irradiated externally or body temperature is sufficient for crosslinking.

### Description of the figures

**Fig. 1****:** shows a diagram of a first sample per material measured at three positions: Ra < 3 µm and Rz < 18µm.
**Fig. 2****:** shows a diagram of a second sample per material measured at three positions: Ra < 3 µm and Rz < 18µm.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Example 1

### Synthesis of α,β-unsaturated carboxylic acid monomers and photo/thermoreactive α,β-unsaturated carboxylic acid monomers.

### Example 1.1:

### Synthesis of N-[3-(4-benzoylbenzamido)propyl]methacrylamide (APMA-BBC)

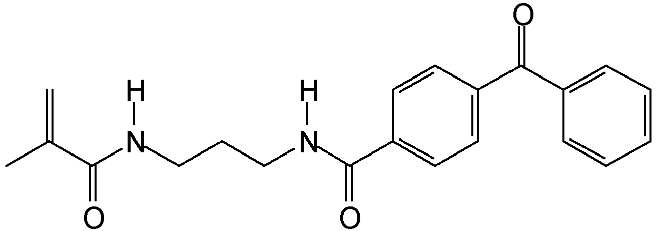

5 g of N-(3-aminopropyl)methacrylamide and 5 mg of phenothiazine were transferred to a 3-neck flask and suspended in 35 mL of chloroform with stirring. The suspension was then cooled to approximately 0° C in a cooling bath. To the cooled suspension, 7.265 g of 4-benzoylbenzoic acid chloride was added. Then, 2.5 mL of chloroform and 8.624 mL of triethylamine were slowly dropped into the suspension. After 1.5 hours, the cooling bath was removed. The solution was then stirred further for 2.5 hours. It was then washed 3x with conc. HCl in and then dried over sodium sulfate. The solution was then filtered and the solvent was removed to dryness at low temperature *in vacuo.* Next, 20 mL of chloroform and a spatula tip of phenothiazine were added to the dry residue. 80 mL of toluene was then added and the suspension was briefly heated to 75° C without completely returning the solid to solution. The suspension was cooled to room temperature. An additional 100 mL of toluenechloroform solution (4:1) was then added to the suspension. The solid was then filtered off by vacuum filtration and dried overnight with rotary vane pump vacuum.
Solubility in THF: Freely soluble
Solubility in N-vinylpyrrolidone: Freely soluble

### Example 1.2:

### Optimized synthesis of N-[3-(4-benzoylbenzamido)propyl]methacrylamide (APMA-BBC)

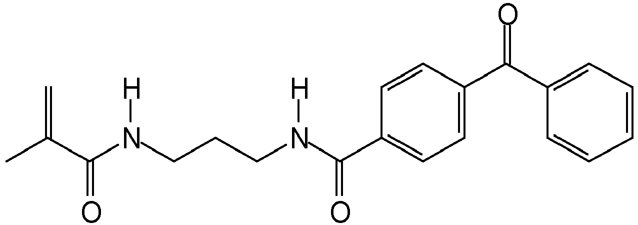

1g of N-(3-aminopropyl)methacrylamide hydrochloride (APMA*HCl) was suspended in 15 mL of tetrahydrofuran (THF). A spatula tip of phenothiazine and 0.836 mL of triethylamine were then added. In another beaker, 1.267 g of benzoylbenzoic acid (BBA) was weighed and dissolved in 15 mL of THF. Then, 0.960 mL of diisopropylcarbodiimide (DIC) was added. Then, the BBA-DIC solution and the APMA suspension were combined and the suspension was stirred overnight at room temperature. After filtration, the clear solution was added to 120 mL n hexane, whereupon the APMA-BBA crystallized out. The solid was separated by vacuum filtration and washed with n-hexane. Finally, the solid was dried at room temperature and checked by IR spectroscopy.

The alternative and newly developed process for the preparation of APMA-BBA represents a considerable simplification to the already known process in Example 1.

### Example 1.3:

### Synthesis of N-(methacryloyl)-4-aminobenzophenone (MABP)

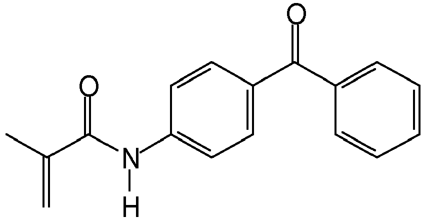

10g of 4-aminobenzophenone was suspended in 20 mL of chloroform with stirring and cooled to approximately 0° C. In a beaker, 8.3 mL methacrylic anhydride and a spatula tip of phenothiazine were dissolved in 10 mL chloroform and slowly added to the cooled 4-aminobenzophenone suspension. After removing the ice bath, a golden yellow solution was formed within the next 90 minutes, which turned green after 3.5 hours and was then stirred further at room temperature overnight. The solution was then transferred to a separatory funnel, 30 mL of deionized water added, and shaken out. The chloroform phase was then transferred back to the separatory funnel and 30 mL of 2 N sodium hydroxide solution was added and shaken out, resulting in a color change from green to pink. The chloroform phase was dried with sodium sulfate and somewhat concentrated using a rotary evaporator. The constricted chloroform solution was then slowly added to the precipitate in n-hexane and stirred. The crystallized solid was separated and dried overnight.

### Example 1.4:

### Synthesis of 1-(4-benzoylbenzoic acid)-2-methaacrylic acid-glycolester (HEMA-BBA)

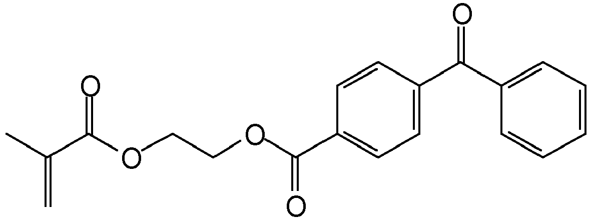

5g of benzoylbenzoic acid was dissolved in 50 mL of THF. Then 2 drops of triethylamine and 3.765 mL of diisopropylamide were added. Then 2.690 mL of hydroxyethyl methacrylate was pipetted into the solution and a white solid precipitated. The solid was separated and washed with tetrahydrofuran or ethanol or acetone and dried.

The product is highly soluble in chloroform, moderately soluble in THF and slowly soluble in vinylpyrrolidone, product is slightly to sparingly soluble in n-heptane and insoluble in ethanol, acetone, ethyl acetate and deionized water.

HEMA-BBA has high UV absorbance at 260 nm, with no further maxima at higher wavelengths.

### Example 1.5: PVP-co-Maleat+benzophenones (A4, MBM)

### 1.5.1: Mono-Benzophenone-Maleate (MBM)

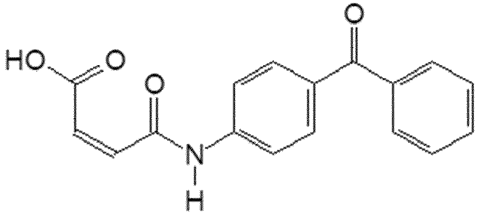

4-aminobenzophenone (160 g) was transferred in a three-neck-flask (3 L), equipped with a mechanical stirrer, and dissolved in ethyl acetate (2.25 L) under ambient temperature. Maleic anhydride (96 g, 1.2 eq.) was added in one portion to the solution and dissolved within seconds. After stirring overnight at ambient temperature reaction mixture was filtered over a filter and washed with ethyl acetate (3 x 500 ml). Solids were collected and dried, under reduced pressure, at 50°C overnight to yield 230 g (96%) of a yellow solid.

### 1.5.2: Synthesis of N-[(4-Benzoylphenyl)]maleic acid monoamide (alternative method)

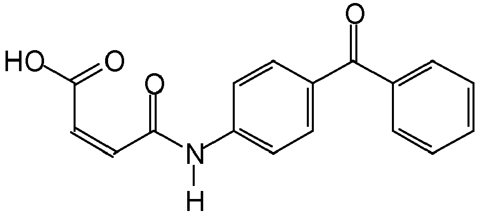

In a 250 mL single-neck flask, 1.525 g (0.0155 mol) of maleic anhydride and 3.062 g (0.0155 mol, or 1.0 moleq) of 4-aminobenzophenone were weighed and suspended or partially dissolved in 75 mL of ethyl acetate at rt. Then, the solution was gassed with inert gas (argon) for 2 min and stirred further at rt for 2 hrs. Subsequently, the resulting solid was separated from the solvent by vacuum filtration. The resulting white to pale yellow dry residue (or solid) was then washed 3 times in 100 mL n-heptane. Vacuum filtration was used to separate the solid from the solvent or precipitant. The resulting white solid was then dried overnight (approximately 18 hours).

### Example 1.6: PEG-Maleate (MPM2000)

PEG2000 methyl ether (55 g) and maleic anhydride (4 g) were added in a three necked flask (500 ml). The mixture was stirred, using a magnetic stirring bar, at 80°C overnight.

Mixture was dissolved in dichloromethane (125 ml), followed by dropwise addition in cold diethylether in 40 minutes. The formed precipitate was collected via filtration and washed with cold diethylether (250 ml) after which it was collected and dried under reduced pressure.

### Example 2

### Synthesis of polymers

### Example 2.1: A1

### Polymerization of NVP and APMA-BBC toPoly(VP-co-APMA-BBC)

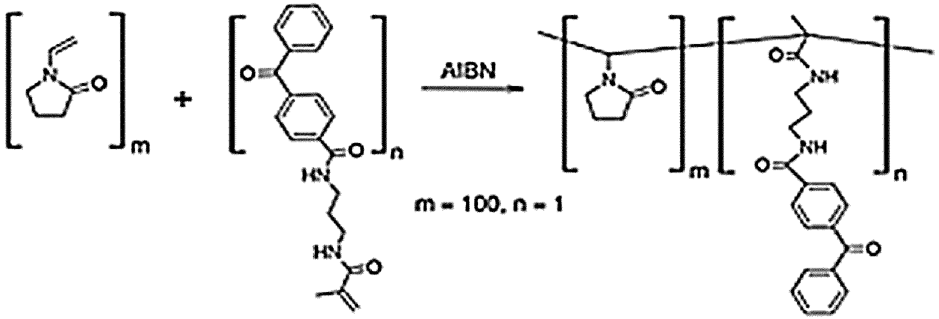

0.25 g of N-[3-(4-benzoylbenzamido)propyl]methacrylamide (APMA-BBA, see Example 1.2) was weighed and 5 mL of N-vinyl-2-pyrrolidone was added under nitrogen introduction. 76 mg of azobisisobutyronitrile (AIBN) was added to the monomer solution. Then the temperature of the solution was slowly increased to about 60° C. After a reaction time of about half an hour, the solution had first become viscous and shortly thereafter the polymer cured spontaneously. The polymer was then dissolved in ethanol/demineralized water (1:1) while heating to about 90° C. The solution was then evaporated. Using a rotary evaporator (bath temperature 40° C), the solution was then evaporated to about half its volume, transferred to four dialysis tubes (MWCO: 6 to 8 kD) long and dialyzed overnight against deionized water. After further concentrating the dialyzed solution, the concentrated highly viscous milky-turbid solution was then frozen overnight and finally freeze-dried. N-[3-(4-benzoylbenzamido)propyl]methacrylamide]-vinylpyrrolidone was polymerized with NVP at a ratio of 1:100.
Mn: 19500, Mw: 282000, Pdi (Mw/Mn): 14.44
Molar mass: > 6000 g/mol (molecular chain length greater than 53 repeat unitsof monomer).

### Example 2.2:A2

### Polymerization of NVP and MABP to Poly(VP-co-MABP)

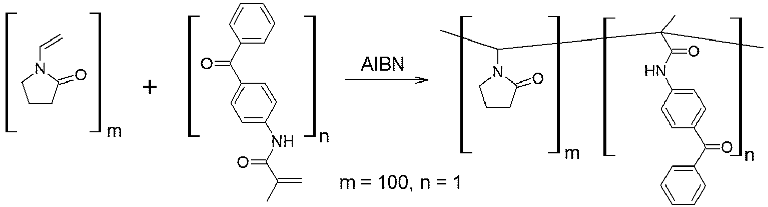

### Example 2.2.1: A2

### Preparation of copolymer poly(VP-co-MABP) with water as solvent

In a two-neck flask, 0.446 g of N-(methacryloyl)-4-aminobenzophenone (MABP) was weighed. Under argon introduction, 16.5 mL of N-vinyl-2-pyrrolidone and 11 mL of water were then added. With stirring, 110 mg of azobis(isobutyronitrile) (AIBN) was added to the monomer solution. Then the temperature of the solution was slowly increased to about 60°C. After a total reaction time of 45 min, the solution was viscous. After the reaction mixture had cooled, an oil bath was used to slowly heat it to 80°C. After 75 min total reaction time, 40 ml water was added at 60°C, followed by another 40 ml water and 50 ml isopropanol. Finally, 80 ml of water- isopropanol 1:1 and 40 ml of isopropanol were added. At 70°C, the polymer dissolved to a milky turbid solution. After cooling, the copolymer was precipitated in acetone. The precipitated polymer no longer contained N-vinyl-2-pyrrolidone. The precipitated polymer was dried in a desiccator under vacuum.

### Example 2.2.2: A2

### Preparation of copolymer poly(VP-co-MABP) with dimethylformamide (DMF) as solvent

To 4.0 g of N-(methacryloyl)-4-aminobenzophenone (MABP), 150.0 mL of N-vinyl-2-pyrrolidone and 100 mL of dimethylformamide (DMF) were added under nitrogen introduction. With stirring, 1.000 g of azobis(isobutyronitrile) (AIBN) was added to the monomer solution, and the temperature of the solution was slowly raised to about 60° C. After a total of 55 min reaction time, the first small bubbles formed in the reaction solution, but the temperature of the reaction solution remained constant at 60° C during this experiment. After a total of 65 min. reaction time, the water bath was replaced by cold water (approx. 10° C). After a total of 80 min reaction time, the bath temperature was gradually increased again to 60° C. After a total of 140 min reaction time, the reaction was terminated by adding 400 mL deionized water. After increasing the temperature to 80° C, a milky cloudy polymer solution was obtained, which was cooled. The cooled "polymer-solvent dough" was then slurried several times with acetone, each time decanting off the milky cloudy dimethylformamide-water-acetone solution formed over the polymer and then the polymer was dried.

Molar mass: > 6000 g/mol (molecular chain length greater than 53 repeat units monomer).

N-(methacryloyl)-4-aminobenzophenone-vinylpyrrolidone was polymerized with NVP at a ratio of 1:100. Mn: 49000, Mw: 324000, Pdi (Mw/Mn): 6.61

### Example 2.2.3: A2

### Preparation of copolymer poly(VP-co-MABP) with isopropanol as solvent

In a two-neck flask, 0.4 g of N-(methacryloyl)-4-aminobenzophenone (MABP) was weighed and 15 mL of N-vinyl-2-pyrrolidone, 10 mL of isopropanol and 100 mg of azobisisobutyronitrile (AIBN) were added to the monomer solution under argon atmosphere. Then, the temperature of the solution was slowly increased to about 60° C and after 80 min of reaction time the solution was viscous. The reaction mixture was cooled to about 40°C and 100 ml of isopropanol/water 1:1 was added. The solid polymer dissolved gradually when heated to about 60°C and another 25 ml of isopropanol was added. A milky turbid solution was formed from which the polymer was precipitated by addition of acetone.

### Example 2.3:

### Preparation of VP-co-glycidylmethacrylate

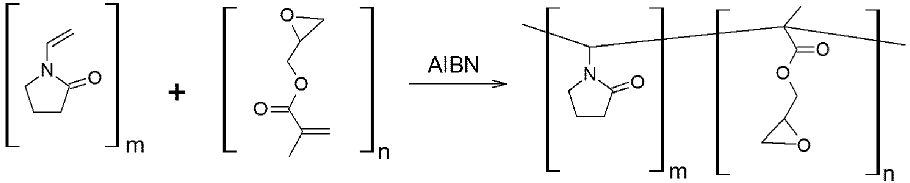

m = 75, n = 25

The synthesis was analogous to MABP using purchasable glycidyl methacrylate THF as solvent.

### Example 2.4:

### 2.4.1 Preparation of polv(VP-co-MBM) with NVP + N-[4-(Benzoylphenyl)]maleic acid monoamide

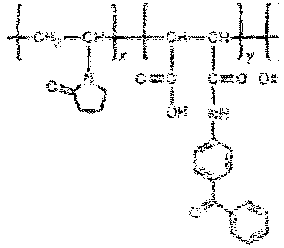

### PVP-co-4-aminobenzophenone-Maleate (0.75 mol%)

In a 250 mL three-neck flask, 0.315 g of MBM (set value: 0.314 g, or 0.001065 mol) was weighed. Under argon injection, 15.0 mL of N-vinyl-2-pyrrolidone (15.6 g, or 0.1420 mol) and 10 mL of degassed dimethylformamide were then added. The flask was then sonicated for 15 min. 150 mg (0.926 mmol) of azobis(isobutyronitrile) (AIBN) were weighed and added to the monomer solution and the temperature of the solution was slowly increased (within 25 min) to about 60° C (oil bath temperature 60° C). After 75 min the viscous polymer dimethylformamide solution was then mixed with 40 mL deionized water and heated to about 90° C. A milky turbid solution was obtained, which after cooling to room temperature was transferred to dialysis tubes (MWCO: 6 to 8 kD) and dialyzed against deionized water overnight. After dialysis, 150 mL of polymer solution was in the dialysis tubes, which was transferred to 2 preweighed 250 mL single-neck flasks. The polymer solution was then frozen and finally freeze-dried for 4 days (extended weekend).

### 2.4.2: Preparation of PVP-Maleate-copolvmer with Benzophenone

N-Vinylpyrrolidone (2930.97 g) and MBM (68.82 g) were mixed together to give a clear solution (monomer mix). Of this mixture (1002.04 g) was added to a double walled reaction vessel, fitted with a mechanical stirrer, two dropping funnels, a condenser and ethanol (2100 ml).

To the remaining monomer mixture was added ethanol (1200 ml) and transferred in one of the dropping funnels. In the other dropping funnel was added a solution containing 4,4'-Azobis(4-cyanopentanoic acid) (17.26 g) dissolved in ethanol (1400 ml). The entire reaction set-up and reactants were inerted with nitrogen gas and the temperature of the reactor was set at 75°C. Upon reaching 75°C, the monomer mix solution feed was added over a period of 2 hours and the initiator solution added over a period of 3 hours. The reaction was allowed to proceed for a total of 18 hours at 75°C. The polymer solution was discharged and cooled to ambient temperature.

### Example 2.5: Preparation of Poly(VP-co-MBM-co-MBMPEG) (PVP-Maleate-copolymer with Benzophenone and PEG2000

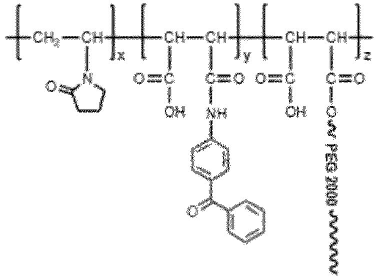

N-Vinylpyrrolidone (41 g), PEG2000 (7.9 g), MBM (1.1 g) and ethanol (25 ml) were mixed together to give a clear solution (monomer mix). Of this mixture 1/3 wt% was added to a three necked round bottom flask, fitted with a mechanical stirrer, two dropping funnels, condenser and heated to 75°C.

The remaining monomer mixture was transferred in one of the dropping funnels. In the other dropping funnel was added a solution containing 2,2'-Azobis(2-methylpropionitrile) (0.29 g) dissolved in ethanol (16 ml). The entire reaction set-up and reactants were inerted with nitrogen gas and the temperature of the reactor was set at 75°C. Upon reaching 75°C, the monomer mix solution feed was added over a period of 2 hours and the initiator solution added over a period of 3 hours. The reaction was allowed to proceed for a total of 18 hours at 75°C. The polymer solution was discharged and cooled to ambient temperature.

### Example 2.6:

### Example 2.6: Preparation of Poly-(VP-co-HEMA) (90:10)

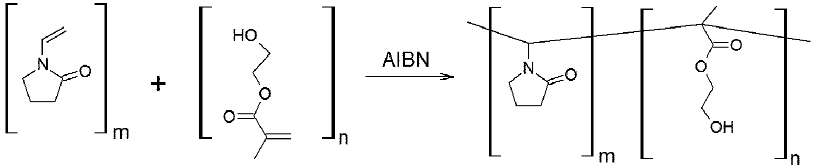

m: 75 - 95 n: 25 - 5

10.0 mL (equivalent to 10.4 g, or 0.0936 mol) of vinylpyrrolidone (VP), 1.265 mL of hydroxyethyl methacrylate (HEMA), 0.085g of azobis(isobutyronitrile) (AIBN), and 6 mL of isopropanol were combined. With stirring, the resulting solution was tempered to 65°C. Further addition of small portions of isopropanol maintained the temperature of the solution at approximately 70° C. After 45 min, the solution had become so viscous that approximately 30 mL isopropanol was added, whereupon the resulting polymer slowly dissolved again. The solution was then cooled to RT with stirring. The cooled solution was transferred to 6 kD dialysis tubes and dialyzed against deionized water. The dialyzed polymer solution was concentrated to approximately half and frozen with liquid nitrogen. Finally, the frozen solution was freeze-dried.

### Example 2.7:

### Acrylation of Poly-(VP-co-HEMA) (90:10)

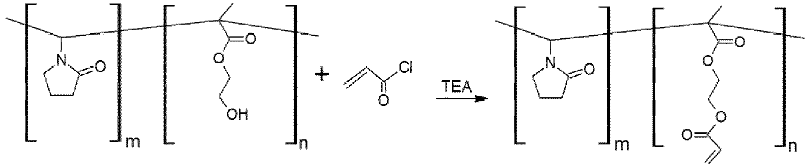

In a 250 mL three-neck flask, 5.058 g of poly-(VP-co-HEMA) (90:10) was weighed. The mass fraction of HEMA in this polymer is calculated to be 10.84 %. Accordingly, 5.0 g of poly-(VP-co-HEMA) (90:10) should contain 0.548 g (equivalent to 4.2147 mmol) of HEMA, or free hydroxyl groups. The polymer was dissolved in 75 mL of chloroform, then 1.930 mL (equivalent to 1.407 g, or 13.9084 mmol, or 3.1 moleq) of TEA was added. The solution was then cooled to -8° C using an ice-acetone bath, after which 1.035 mL (equivalent to 1.144 g, or 12.644 mmol, or 3.0 moleq) of acryloyl chloride (dissolved in 25 mL of chloroform) was slowly added to the solution over a period of 5 min using a dropping funnel with a pressure equalization tube. The solution was then stirred further for one hour at about -10° C and then for another 16 hours at RT with light excluded. The solution was then precipitated in acetone and dried by rotary vane pump vacuum. The dry residue (approximately 1.5 g) was then suspended in 25 mL of deionized water, transferred to a dialysis tube (MWCO 6kD) and dialyzed against 60 L of deionized water. The dialyzed solution was then transferred to a tared 100 mL single-neck flask, frozen and freeze-dried under light exclusion.

### Example 3

### Synthesis of crosslinkers according to the invention and prior art crosslinkers

### Example 3.1: Crosslinker B1 (reference example)

### Synthesis of dibenzotirone (BBBD; 4,5-bis(4-benzoylphenylmethyleneoxy) benzene-1,3-disulfonate.

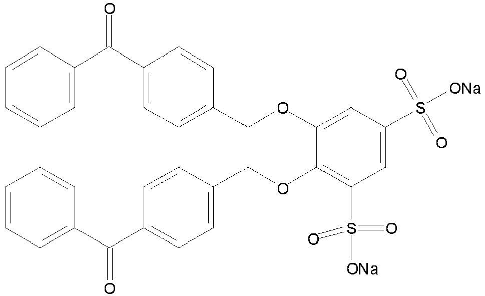

9.0 g of tiron (pyrocatechol-3,5-disulfonic acid disodium salt) and 2.167 g of sodium hydroxide were dissolved in 45 mL of deionized water and tempered to about 75° C under reflux. A clear yellow solution was obtained. In a 100 mL beaker, 15.670 g of 4-(bromomethyl)benzophenone (96% strength) was weighed and dissolved in 45 mL of 1,4 dioxane under slight heating (<50° C) and added to the basic tiron solution heated to 75° C. The solution was then refluxed. The solution turbid in short time and turned deep dark green and within 1 hour returned to yellow and completely clear. After 3 hours of reaction, post-dosing was then carried out with 4.015 g of 4-(bromomethyl)benzophenone (96%) in 10 mL of 1,4 dioxane, whereupon the yellow coloration of the solution intensified. Then 0.880 g sodium hydroxide dissolved in deionized water was added, which then turned a rust red color. Within 30 min, the solution then turned yellow again. After 20 h at boiling temperature and under reflux, the solution was i.v. concentrated to dryness. The yellowish solid was suspended in approximately 50 mL of chloroform. After filtration, the solid was transferred to a 200 mL single-neck flask and 67 mL of deionized water was added. Upon heating to approximately 80° C, the solid dissolved completely to form a yellow solution to which 67 mL of methanol was added. The solution was then transferred to a 200 mL iodine number flask and slowly cooled with stirring until a white solid crystallized at about 50° C. The solid was then removed from the flask. The solid (dibenzotirone) was separated by vacuum filtration, washed 2x with 100 mL methanol/VE water (1:1) and dried at RT followed by further post-drying with rotary vane pump vacuum.

### Example 3.2: Crosslinker B3

### Synthesis of carbonyl-bis-((4-benzoylphenyl)carbamoyl)-benzophenone [BCBAC].

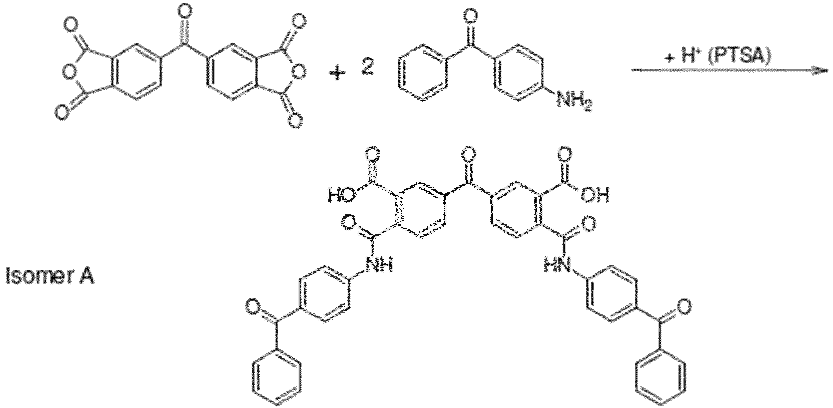

*IUPAC: 5,5'-Carbonylbis(2-(4-benzoylphenyl)carbamoyl)-Benzoesäure* 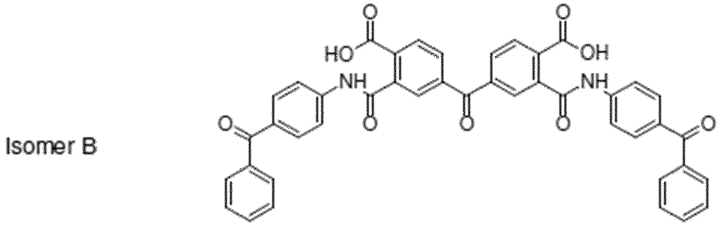 *IUPAC: 4.4'-Carbonylbis(2-(4-benzoylbenzyl)carbamoyl)-Benzoesäure* 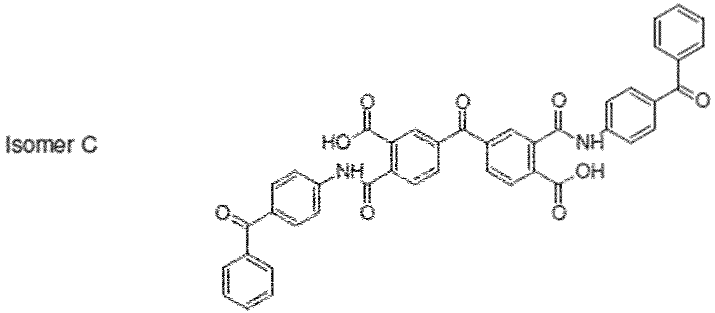 *IUPAC: 4,5'-Carbonylbis(2-(4-benzoylbenzyl)carbamoyl)-Benzoesäure*

In a 500 mL single-neck flask, 5.009 g of benzophenone-3,3',4,4' tetracarboxyl dianhydride and 6.128 g of 4-aminobenzophenone were weighed and dissolved in 100 mL of THF + 30 mL of acetone. 0.213 g of para-toluenesulfonic acid monohydrate was added. It was then briefly gassed with nitrogen and refluxed to 60° C overnight. The resulting golden yellow solution was then concentrated *in vacuo* to dryness. The resulting brown-yellow dry residue was taken up in 50 mL chloroform + 10 mL acetone, and then precipitated in 300 mL n-heptane. Vacuum filtration was used to separate the solid from the solvent, and the resulting white, very fine powdery solid was then dried under vacuum overnight.

### Example 3.3: Crosslinker B4

### Benzophenone di-carboxyl di-N-(4-(benzoylphenyl)-methylaminocarboxylate) [BCBMC].

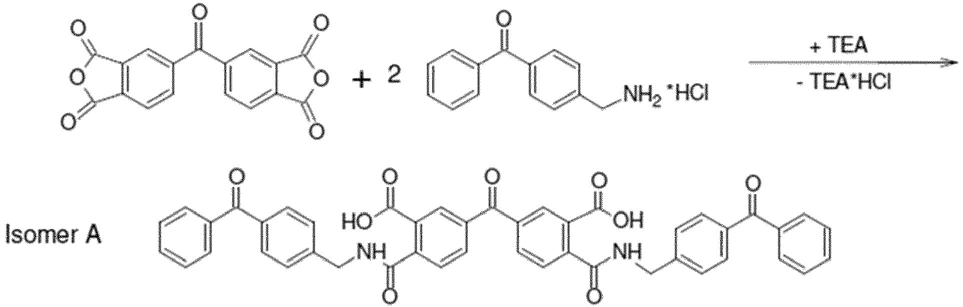

*Benzophenon 2.2'-di-carboxyl-3.3'-di-N-((4-benzoylphenyl)-methylaminocarboxylat) IUPAC: 5,5'-Carbonylbis(2-(4-benzoylbenzyl)carbamoyl)-Benzoesäure* 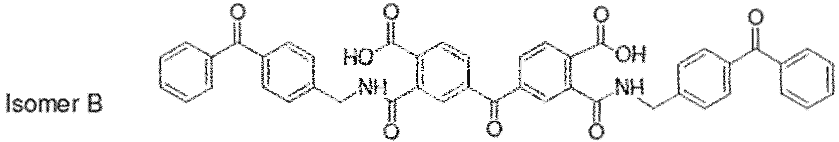 *Benzophenon 3.3'-di-carboxyl-2,2'-di-N-((4-benzoylphenyl)-methylaminocarboxylat) IUPAC: 4,4'-Carbonylbis(2-(4-benzoylbenzyl)carbamoyl)-Benzoesäure* 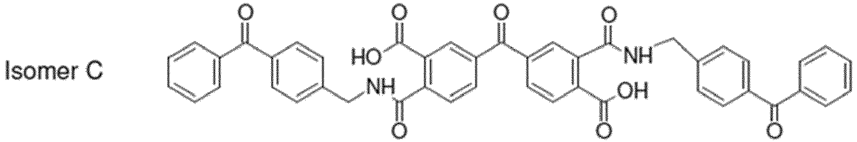 *Benzophenon 2,3'-di-carboxyl-3,2'-di-N-((4-benzoylphenyl)-methylaminocarboxylat) IUPAC: 4,5'-Carbonylbis(2-(4-benzoylbenzyl)carbamoyl)-Benzoesäure*

In a 250 mL single-neck flask, 5.000 g of 4-benzoylbenzylamine hydrochloride and 3.260 g of benzophenone-3,3',4,4' tetracarboxyldianhydride were weighed and dissolved in 150 mL of tetrahydrofuran (THF). Then, 2.825 mL of triethylamine was also added to the reaction solution and tempered at reflux to 60 °C for 3 days. Then, the yellow and slightly turbid solution was cooled to room temperature. Triethylammonium chloride precipitated from the cooled solution and was separated. To precipitate the solid, 600 mL of n-heptane was added to the yellow solution and after decantation, the precipitated yellow amorphous solid was separated from the solvent. The milky white solvent mixture was concentrated to dryness. Both solids were then dried with rotary vane pump vacuum. The solubility of both solids was first investigated before further purification. It was found that the dry residue of the solvent mixture was insoluble or only partially soluble in chloroform, whereas the precipitated yellow solid dissolved readily in chloroform. The yellow solid was dissolved in chloroform for further purification and precipitated again by adding n-heptane. The solvent mixture became milky turbid and after centrifugation, the milky turbid solution was separated again. The milky turbid solution was concentrated to dryness, and the resulting white dry residue was washed 2x with about 100 mL of acetone-ethanol (1:1), adding first the acetone portion and then the ethanol portion. The washed solid was separated from the solvent mixture by vacuum filtration using a glass suction filter and transferred to a tared 100 mL single-neck flask. The solid remaining in the falcon tubes was dissolved in chloroform, transferred to a tared single-neck flask, and concentrated to dryness. Then, rotary vane pump vacuum was used to dry for 24 hours.

### Example 4

### Preparation of solutions of at least one polymer and crosslinkers according to the invention without and with additives, and coating of surfaces with these solutions.

**Example 4.1:** Preparation of a coating solution with 4 components and coating of catheter outer tube pieces.

### Solution:

2.50 g VP-co-MABP (component A = A2)
0.438 g BBBD (component B = crosslinker B1)
5.0 g Kollidon 90F (component C)
10.0 g Kollidon 30K (component D
are dissolved in isopropanol/water (70:30 v/v) in a suitably sized Schott flask (overnight).

### Coating:

Catheter outer tubing is cut into 12 cm pieces (5 samples) and then drawn on matching stylet wire. After wiping with isopropanol for conditioning, the pieces are immersed in the coating solution.
Immersion speed: 20 mm/sec
Incubation time: 10 sec
Dipping speed: 5 mm/sec
UV curing: 3 min
Drying: 5 min at rt

**Example 4.2:** Preparation of a coating solution of 4 components and coating of nylon tubing by immersion method with subsequent measurement of the coefficient of friction. (Comparison with state of the art crosslinker).

### Solution:

22 g Kollidon 30K (Component D)
11 g Kollidon 90F (Component C)
5.5 g VP-co-MABP (Component A = A2)
0.963 g BBBD (Component B = Crosslinker B1)
are dissolved in deionized water/isopropanol (70:30 v/v).

### Coating:

Immersion speed: 20 mm/sec
Incubation time: 30 sec
Dipping speed: 10 mm/sec
Curing in UV oven: 2 min
Drying: 15 min at 50°C

**CoF : 0.132 +/- 0.016 (>0.1)**

**Example 4.3:** Preparation of a coating solution from 5 components and coating of nylon tubing by immersion method with subsequent determination of the coefficient of friction on nylon tubing and polypropylene tubing.

### Solution:

5.4 g VP-co-MABP (Component A)
0.105 g BCBAC (Component B = Crosslinker B3)
0.6 g VP-co-Maleat (Component E)
12.0 g PVP 90 F (Component C)
24.0 g PVP 30 K (Component D)

The components are dissolved in 600 ml isopropanol/acetone (85:15 v/v).

The coating is applied by immersion (30sec) followed by UV irradiation for 3 min. After drying for 5 min at room temperature, the lubricity was tested with Harland FTS6000.
**Log CoF: MW = 0.046 +/- 0.009 nylon**
**Log CoF: MW = 0.061 +/- 0.021 pp tubes**

**Example 4.4:** Preparation of a coating solution from 4 components and coating of polypropylene hose by immersion method with subsequent measurement of the coefficient of friction.

### Solution:

5,4 g VP-co-MABP (Component A) 1%
0,105 g BCBAC (Component B = Crosslinker B3)
0,5 g Kollidon 90F (Component C)
1,0 g Kollidon 30K (Component D)
are dissolved in isopropanol/acetone (85:15 v/v)

### Coating

Immersion speed: 10 mm/sec
Incubation time: 10 sec
Dipping speed: 5 mm/sec
Curing in UV oven: 3 min
Drying: 5 min at room temperature

### Log CoF: MW = 0.044 +/- 0.010

**Example 4.5:** Preparation of a coating solution of 4 components and coating of nylon tubing by immersion method with subsequent measurement of the coefficient of friction.

### Solution:

0.25 g poly(VP-co-N-[(4-benzoylphenyl)]maleic acid monoamide VP-co-maleate-benzophenonderivative (Component A)
0.963 g BCBAC (Component B = Crosslinker B3)
0.5 g Kollidon 90F (Component C)
1.0 g Kollidon 30K (Component D)
are dissolved in isopropanol/0.067mol/INaOH_{aq} (85:15 v/v).

### Coating:

Immersion speed: 20 mm/sec
Incubation time: 30 sec
Dipping speed: 10 mm/sec
Curing in UV oven: 3 min
Drying: 5 min at 50°C

**CoF : 0.068 +/- 0.012**

**Example 4.6:** Preparation of a coating solution of 4 components and coating of nylon tubing by immersion method with subsequent measurement of the coefficient of friction.

### Solution:

4.0 g Kollidon 30K (Component D)
2.0 g Kollidon 90F (Component C)
0.25 g VP-co-MABP (Component A) 1%
0.175 g BCBAC (Component B = Crosslinker B3)
are dissolved in isopropanol/VE water (85:15 v/v) and filtered.

### Coating:

Immersion speed: 20 mm/sec
Incubation time: 30 sec
Dipping speed: 10 mm/sec
Curing in UV oven: 3 min
Irradiation dose: 4055.80 mJ/cm²
Drying: 15 min at room temperature

**CoF : 0.008 +/- 0.002**

**Example 4.7:** Preparation of a coating solution of 3 components and coating of nylon tubing by immersion method with subsequent measurement of the coefficient of friction.

### Solution:

0.5 g VP-co-MABP (Component A) 1%
0.04 g BCBAC (Component B = Crosslinker B3)
2.8 g Kollidon 90F (Component C)
are dissolved in isopropanol/demineralized water (85:15 v/v) and filtered.

### Coating:

Immersion speed: 20 mm/sec
Incubation time: 30 sec
Dipping speed: /sec
Curing in UV oven: 3 min
Irradiation dose: 4055.80 mJ/cm²
Drying: 15 min at room temperature

**CoF : 0.016 +/- 0.002**

**Example 4.8:** Preparation of a coating solution of 4 components and coating of silicone hose by immersion method with subsequent measurement of the coefficient of friction.

### Primer solution:

The silicone tubes are pretreated with plasma. They are then placed in the primer solution for 60 min:
Ethanol (70%), HEMA (13.6%), PVP 90F (7%), aminosilane (10%) and methacrylic anhydride (6.5%).

Then dried at room temperature for 20 min and subsequently at 135° C for 2h.

### Dip solution:

0.25 g VP-co-MABP (Component A) 1%.
0.044 g BCBAC (Component B = Crosslinker B3)
0.5 g Collidon 90F (Component C)
1.0 g Collidon 30K (Component D)
are dissolved in isopropanol/VE water (85:15 v/v) and filtered.

### Coating:

Immersion speed: 20 mm/sec
Incubation time: 30 sec
Dipping speed: 10 mm/sec
Curing in UV oven: 3 min
Drying: 5 min at 50°C

**Example 4.9:** Preparation of a coating solution from 4 components and coating of silicone hose by immersion process.

### Primer solution:

The silicone tubes are added to a primer solution of n-heptane (60%), 6670 Part A (20%) and 6670 Part B (20%).

### Dip solution:

0.25 g VP-co-Maleate (Component A) 1%.
0.044 g BCBAC (Component B = Crosslinker B3)
0.5 g Collidon 90F (Component C)
1.0 g Collidon 90F (Component D)
are dissolved in isopropanol/NaOH_{aq}. (85:15 v/v) and filtered.

### Coating:

Immersion speed: 20 mm/sec
Incubation time: 30 sec
Dipping speed: 10 mm/sec
Curing in UV oven: 3 min
Drying: 5 min at 50°C

**Example 4.10:** Preparation of a coating solution from 4 components and coating of Pebax hose by immersion process. (Comparison with state of the art crosslinker).

### Dip solution:

0.25 g VP-co-MABP (Component A) 1%
0.044 g BBBD (Component B = Crosslinker B1)
1.0 g Kollidon 90F (Component C)
2.0 g Kollidon 30k (Component D)
are dissolved in deionized water/isopropanol (70:30 v/v).

### Coating procedure:

Immersion speed: 20 mm/sec
Incubation time: 10 sec
Dipping speed: 5 mm/sec
Curing in UV oven: 2.0 min and 2.5 min per 3 samples
Drying: 15 min at 70°C

**Example 4.11:** Preparation of a coating solution of 3 components and coating of nylon, pellethane and pebax hose by immersion process.

### Dip solution:

0.25 g VP-co-Maleat + Benzophenon (Component A) 0,5%
0.01 g BCBAC (Component B = Crosslinker B3)
0.7 g Kollidon 90F (Component C)
are dissolved in isopropanol/VE water (85:15 v/v).

### Coating:

Immersion speed: 20 mm/sec
Incubation time: 30 sec
Dipping speed: 10 mm/sec
Curing in UV oven: 3.0 min
Drying: 5 min at rt and 15 min at 50°C

Coefficient of friction:
**CoF (nylon): 0.023 +/-0.004**
**CoF (pellethan) : 0.020 +/-0.004**
**CoF (pebax): 0.021 +/-0.005**

**Example 4.12:** Lubricity of nylon pieces coated with VP-co-MABP alone as a comparison to the above examples.

**CoF: 0.416 +/-0.066**

**Example 5:** Mean roughness (Ra) and difference from lowest point to highest point (Rc-d) of the surfaces of Vestamed, Nylon, Pebax and PET.

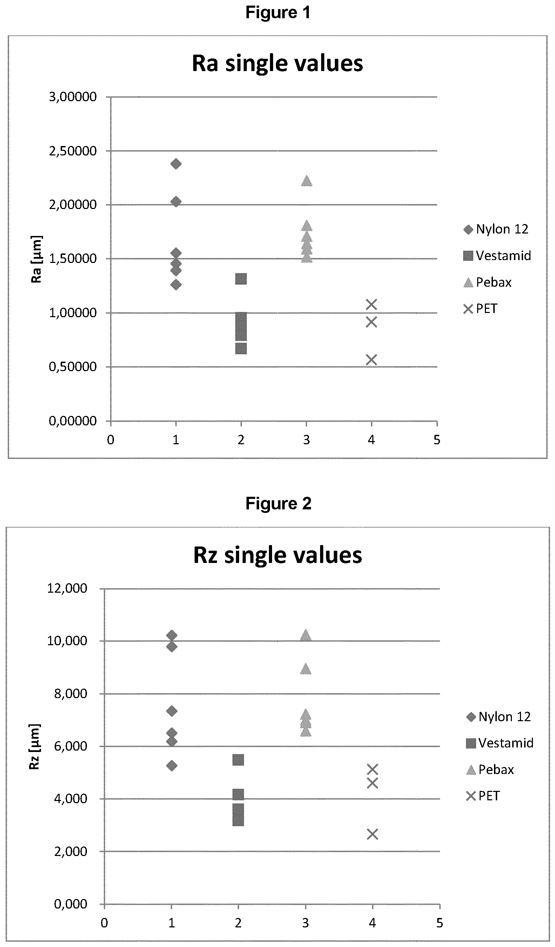

Two samples per material were measured at three positions: Ra < 3 µm and Rz < 18µm (table 3, Fig. 1; table 4, Fig. 2)

**Table 3 First sample per material were measured at three positions: Ra < 3 µm and Rz < 18µm.**

| Ra | | |
|---|---|---|
| | Mean value [µm] | Standard dev. [µm] |
| Nylon | 1,68 | 0,43 |
| Vestamid | 0,91 | 0,22 |
| Pebax | 1,75 | 0,25 |
| PET | 0,85 | 0,26 |

**Table 4 Second sample per material were measured at three positions: Ra < 3 µm and Rz < 18µm.**

| Rz | | |
|---|---|---|
| | Mean value [µm] | Standard dev. [µm] |
| Nylon | 7,56 | 2,02 |
| Vestamid | 3,86 | 0,87 |
| Pebax | 7,82 | 1,45 |
| PET | 4,13 | 1,29 |

### Example 6: Particle test of A4/B3/C

### a) coating solution:

isopropanol (85%) 1020 ml
demineralized water (15%) 180 ml
Kollidon 90F (2,8%) 33.604 mg
PVPMBM (0,5%) 6.004 mg
BCBAC (0,04%) 0.482 mg

One catheter as dummy (remains uncoated for blank) and 5 catheters each (material nylon) are wiped with isopropanol and after threading onto a stylet, 5 catheters are immersed (for some selected catheters, distal ends are masked with silicone tubing):
Immersion speed : 20 mm/s
Incubation time in the immersion solution : 30 s
Immersion speed : 20 mm/s
UV oven : 30 min

### c) Result of measurement of particle size and number

| Particle size | Nylon catheter uncoated | A4/B3/C (7.175%) | A4/B3/C (3.34%) | Max. Limit according to USP 788 (*Ph. Eur. 2.9.19)* |
|---|---|---|---|---|
| ≥ 10 µm | 246 | 1388 | 778 | 6000 |
| ≥ 25 µm | 14 | 139 | 29 | 600 |

### Adhesion of the coating on a surface.

The adhesion is shown by the measurement of the coefficient of friction and is obtained by measuring the coefficient of friction as a function of the cycles performed. The higher the value of the coefficient of friction becomes over the cycles, the more the coating detaches and adhesion decreases.

If the coefficient of friction does not change or hardly changes over the successive cycles (measured up to 25 cycles), this indicates a stable coating with good adhesion to the surface of the coated object.

### Shelf Life (storage stability)

To determine the shelf life and durability of the hydrophilic coating material, a number of samples are coated, dried and packaged as specified for the planned time period. During a specified time frame, at least two samples are taken, visually inspected and the coefficient of friction and adhesion are measured.

### Shelf life von A2/B1/C/D

### Stock solution:

2-propanol 60 ml
demineralized water 340 ml
Kollidon 90F (2% w/v) 8 g
Kollidon 30K (4% w/v) 16 g
Immersion solution: stock solution 140 ml
poly(VP-co-MABP) (1% w/v) 1400 mg
dibenzotirone (0.175% w/v) 245 mg

The nylon catheters used here are threaded onto a stylet and then immersed in the solution at a speed of 20 mm/s. The nylon catheter remains fully immersed in the solution for 30 s and is then withdrawn again at a speed of 10 mm/s. The fully immersed nylon catheter remains in the solution for 30 s and is then pulled out again at a speed of 10 mm/s. This is followed by UV irradiation for 2 min. The final drying is carried out in the drying oven for 15 min. The finished parts are cured for 4h at 177°C, cleaned and packaged.

## Claims

1. A crosslinker of general formula (**I**):
**BP¹-L¹-Z-L²-BP²** (**I**)
wherein
**L¹** represents -R¹-NHCO-R²-, -R¹-CONH-R²-, -R¹-OCO-R²- -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴-, -R³-NHCOO-R⁴-, -R³-OCONH-R⁴-, -R³-OCOO-R⁴-, or -R³-NHCONH-R⁴-;
**Z** is 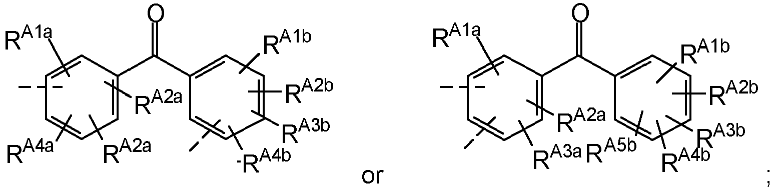
**BP¹** and **BP²** independently of each other represent 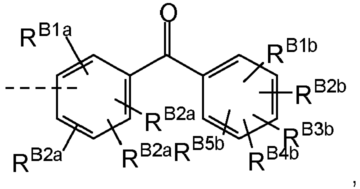
**R¹** and **R⁴** independently of each other represent a bond or -CH₂-;
**R²** represents a bond, -C_{H2}-, -CH₂R⁵-, -C₂H₄R⁵-, -C₃H₆R⁵-, -C₄H₈R⁵-, -C₅H₁₀R⁵-, -C₆H₁₂R⁵-, -CH₂R⁵CH₂-, -C₂H₄R⁵CH₂-, -C₃H₆R⁵CH₂-, -C₄H₈R⁵CH₂-, -C₅H₁₀R⁵CH₂-, -C₆H₁₂R⁵CH₂-, 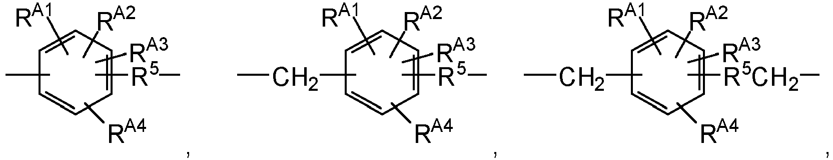 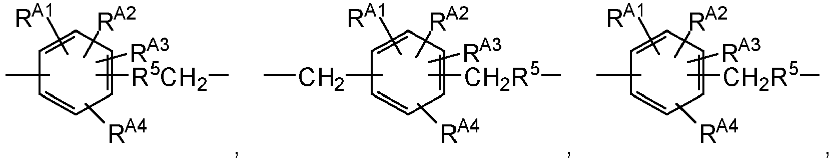 or 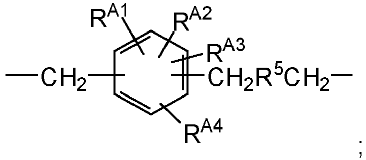
**R³** represents a bond, -C_{H2}-, -R⁶CH₂-, -R⁶C₂H₄-, -R⁶C₃H₆-, -R⁶C₄H₈-, -R⁶C₅H₁₀-, -R⁶C₆H₁₂-, -CH₂R⁶CH₂-, -CH₂R⁶C₂H₄-, -CH₂R⁶C₃H₆-, -CH₂R⁶C₄H₈-, -CH₂R⁶C₅H₁₀-, -CH₂R⁶C₆H₁₂-, 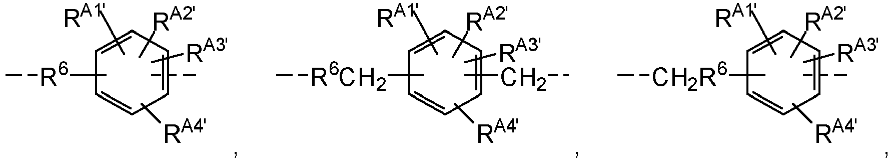 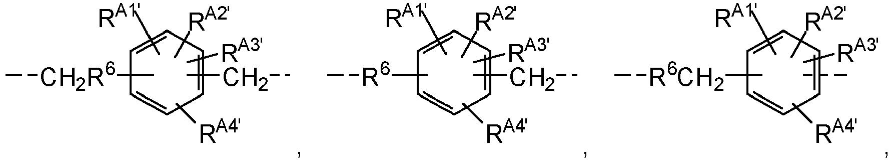 or 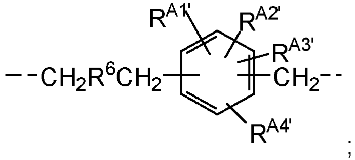
**R⁵** and **R⁶** independently of each other represent -NHCO-, -CONH-, -OCO-, -COO-, -NHCOO-, -OCONH-, -OCOO-, or -NHCONH-; and
**R^{A1} - R^{A4}, R^{A1'} - R^{A4'}**, **R^{A1a} - R^{A4a}, R^{A1b} - R^{A5b}**, **R^{B1a}** - **R^{B4a}** and **R^{B1b} - R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

2. The crosslinker according to claim 1, wherein
**L¹** and **L²** independently of each other represent -R¹-NHCO-R²-, or -R¹-CONH-R²-, and/or
**R⁵** and **R⁶** independently of each other represent -NHCO-, or -CONH-; and
**R¹** and **R²** have the same meaning as defined in claim 1.

3. The crosslinker according to claim 1 or 2, wherein
**R²** represents a bond, -C_{H2}-, -CH₂R⁵-, -CH₂CH₂R⁵-, -CH₂CH₂CH₂R⁵-,-CH(CH₃)R⁵-, -C[(CH₃)₂]R⁵-, -CH₂CH(CH₃)R⁵-, -CH(CH₃)CH₂R⁵-, -CH(CH₂CH₃)R⁵-, -CH₂CH₂CH₂CH₂R⁵-, -CH₂C[(CH₃)₂]R⁵-, -C[(CH₃)₂]CH₂R⁵-, -CH(CH₃)CH₂CH₂R⁵-, -CH₂CH₂CH(CH₃)R⁵-, -CH₂CH(CH₂CH₃)R⁵-, -CH(CH₂CH₃)CH₂R⁵-, -CH₂CH(CH₃)CH₂R⁵-, -CH₂CH₂CH₂CH₂CH₂R⁵-, -CH₂CH(CH₃)CH(CH₃)R⁵-, -CH₂C[(CH₃)₂]CH₂R⁵-, -CH(CH₃)CH(CH₃)CH₂R⁵-, -C[(CH₃)₂]C(CH₃)R⁵-,-C(CH₃)C[(CH₃)₂]R⁵-, -CH(CH₃)CH(CH₃)R⁵-, -CH(CH₃)CH₂CH₂CH₂R⁵-,-C(CH₃)₂ CH₂CH₂R⁵-, -C(CH₂CH₃)₂R⁵-, -CH₂CH(CH₃)C₂H₄R⁵-, -CH(CH₃)C(CH₃)₂R⁵-, -CH₂CH₂C(CH₃)₂R⁵-, -CH(CH₃)₂C(CH₃)₂R⁵-, -CH₂CH₂CH₂CH₂CH₂CH₂R⁵-, -CH(CH₃)CH(CH₃)CH(CH₃)R⁵--CH₂CH₂CH₂CH(CH₃)₂R⁵-, -CH₂CH₂CH(CH₃)CH₂CH₂R⁵-, -CH₂CH(CH₃)CH₂CH₂CH₂R⁵-, -CH(CH₃)CH₂CH₂CH₂CH₂R⁵-, -CH(CH₃)CH₂C(CH₃)₂R⁵-, -C(CH₃)₂CH₂CH₂CH₂R⁵-, -CH(CH₃)CH(CH₃)CH₂CH₂R⁵-, -CH₂CH(CH₃)CH(CH₃)₂R⁵-, -CH₂C(CH₃)₂CH₂CH₂R⁵-, -C(CH₃)₂C(CH₃)₂R⁵-, -CH₂C(CH₂CH₃)₂R⁵, or -CH₂CH₂CH₂CH₂CH₂CH₂CH₃R⁵-; and/or
**R³** represents a bond, -C_{H2}-, -R⁶CH₂-, -R⁶CH₂CH₂-, -R⁶CH₂CH₂CH₂-, -R⁶CH(CH₃)-, -R⁶C[(CH₃)₂]-, -R⁶CH₂CH(CH₃)-, -R⁶CH(CH₃)CH₂-, -R⁶CH(CH₂CH₃)-, -R⁶CH₂CH₂CH₂CH₂-, -R⁶CH₂C[(CH₃)₂]-, -R⁶C[(CH₃)₂]CH₂-, -R⁶CH(CH₃)CH₂CH₂-, -R⁶CH₂CH₂CH(CH₃)-, -R⁶CH₂CH(CH₂CH₃) -, -R⁶CH(CH₂CH₃)CH₂-, -R⁶CH₂CH(CH₃)CH₂-, -R⁶CH₂CH₂CH₂CH₂CH₂-, -R⁶CH₂CH(CH₃)CH(CH₃)-, -R⁶CH₂C[(CH₃)₂]CH₂-, -R⁶CH(CH₃)CH(CH₃)CH₂-, -R⁶C[(CH₃)₂]C(CH₃)-, - R⁶C(CH₃)C[(CH₃)₂]-, -R⁶CH(CH₃)CH(CH₃)-, -R⁶CH(CH₃)CH₂CH₂CH₂-, -R⁶C(CH₃)₂ CH₂CH₂-, -R⁶C(CH₂CH₃)₂-, -R⁶CH₂CH(CH₃)C₂H₄-, -R⁶CH(CH₃)C(CH₃)₂-, -R⁶CH₂CH₂C(CH₃)₂-, -R⁶CH(CH₃)₂C(CH₃)₂-, -R⁶CH₂CH₂CH₂CH₂CH₂CH₂-, -R⁶CH(CH₃)CH(CH₃)CH(CH₃)-, -R⁶CH₂CH₂CH₂CH(CH₃)₂-, -R⁶CH₂CH₂CH(CH₃)CH₂CH₂-, -R⁶CH₂CH(CH₃)CH₂CH₂CH₂-, -R⁶CH(CH₃)CH₂CH₂CH₂CH₂-, -R⁶CH(CH₃)CH₂C(CH₃)₂-, -R⁶C(CH₃)₂CH₂CH₂CH₂-, -R⁶CH(CH₃)CH(CH₃)CH₂CH₂-, -R⁶CH₂CH(CH₃)CH(CH₃)₂-, -R⁶CH₂C(CH₃)₂CH₂CH₂-, -R⁶C(CH₃)₂C(CH₃)₂-, -R⁶CH₂C(CH₂CH₃)₂, or -R⁶CH₂CH₂CH₂CH₂CH₂CH₂CH₃-; and
**R⁵** and **R⁶** have the same meaning as defined in claim 1 or 2.

4. The crosslinker according to any one of the claims 1 - 3, wherein **R¹, R², R³,** and **R⁴** independently of each other represent a bond or -CH₂-.

5. The crosslinker according to any one of the claims 1 - 4, wherein each of **R^{B1a}-R^{B4a}** and **R^{B1b}** - **R^{B5b}** represent -H, ...-OH, -NO₂, -COCH₃, -COOH,-CONH₂, -SO₃H, -SO₂NH₂, -OCH₃, -OCF₃, -NH₂, -NH(CH)₃,-N(CH₃)₂, and -SOCH₃

6. The crosslinker according to any one of the claims 1 - 5, wherein **BP¹** and **BP²** represent 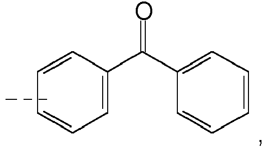 preferably

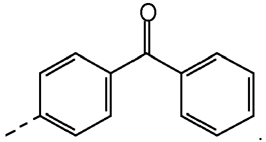

7. The crosslinker according to any one of the claims 1 - 6, wherein each of R^{A1} - R^{A4} represent -H, ... -OH, -NO₂, -COCH₃, -COOH, -CONH₂, -SO₃H, - SO₂NH₂, -OCH₃, -OCF₃, -NH₂, -NH(CH)₃, -N(CH₃)₂, and -SOCH₃

8. The crosslinker according to any one of the claims 1 - 7, wherein **R^{A1a} - R^{A4a}** and **R^{A1b} - R^{A5b}** independently of each other represent -H, -COOH, or -CH₃.

9. The crosslinker according to any one of the claims 1 - 8, wherein
Z represents 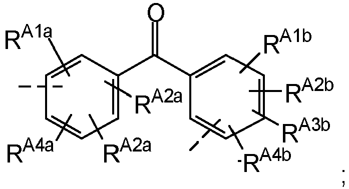 and **R^{A1a}** - **R^{A4a}** and **R^{A1b}** - **R**^{**A5b** 6} have the same meaning as defined in any of the previous claims.

10. The crosslinker according to any one of the claims 1 - 9, wherein
**Z** represents 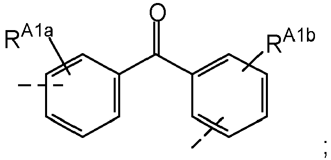 and **R^{A1a}** and **R^{A1b}** have the same meaning as defined in any of the previous claims.

11. A crosslinker selected from the group comprising or consisting of: 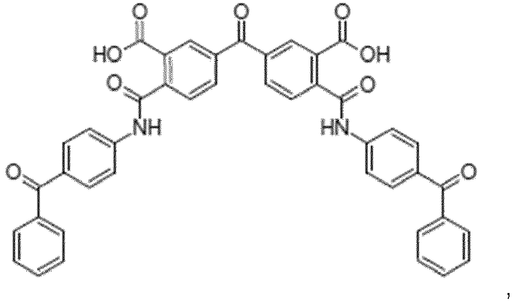 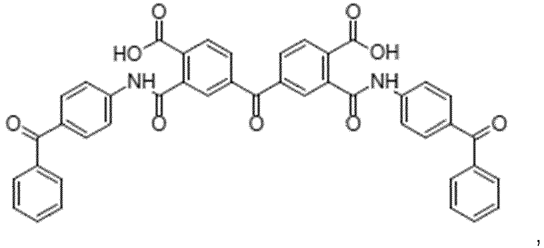 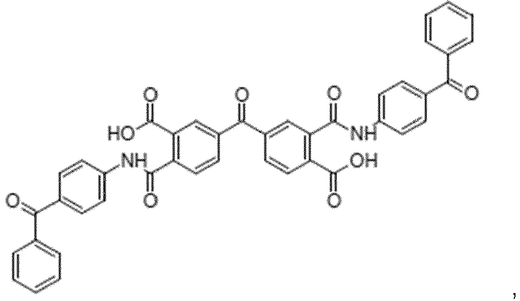 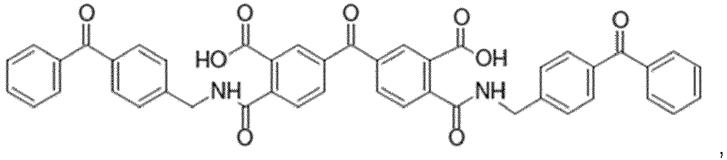 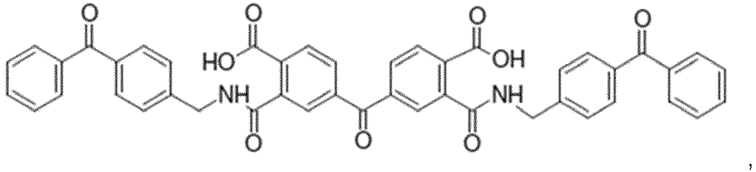 and 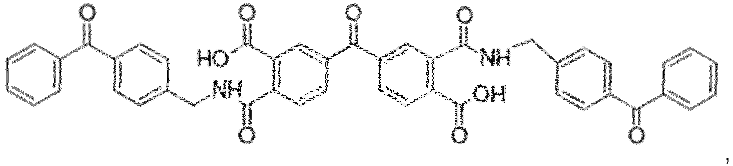 or mixtures thereof.

12. A composition comprising a polymer and a crosslinker according to any one of the claims 1-11.

13. A molecule colloid consisting of a crosslinked composition comprising a polymer and a crosslinker according to any one of the claims 1-11.

14. A medical device having a coating of a crosslinked composition according to claim 12.

15. Use of the molecular colloid according to claim 13 as a medical device, component of a medical device, coating substance, coating of a medical device such as catheters, balloon catheters, stents, guide wires, needles, cannulas, additive in cosmetics, care products, hygiene products, ointments, gels, pads, medical aids, lubricants, for moisture regulation or absorption, for friction reduction of surfaces, containing at least one pharmaceutically active substance which can be released in a controlled manner.
